# EUROPEAN PATENT SPECIFICATION

(11) **EP 3 092 231 B1**
(45) Date of publication and mention of the grant of the patent: **27.06.2018**
(21) Application number: 14824644.0
(22) Date of filing: 10.12.2014
(51) Int. Cl.: C07D 405/14, C07D 405/04, C07D 487/04, A61K 31/53, A61K 31/506, A61K 31/519, A61P 3/00, A61P 11/00, A61P 25/00

(54) **SGC STIMULATORS**
SGC-STIMULATOREN
STIMULATEURS DE LA SGC

(30) Priority: 11.12.2013 US 201361914908 P; 17.09.2014 US 201462051539 P
(43) Date of publication of application: 16.11.2016
(73) Proprietor: Ironwood Pharmaceuticals, Inc., Cambridge, Massachusetts A 02142 (US)
(72) Inventor: RENHOWE, Paul Allen, Sudbury, Massachusetts 01776 (US); SHEPPECK, James Edward, Newtown, Pennsylvania 18940 (US); NAKAI, Takashi, Newton, Massachusetts 02458 (US); IYER, Karthik, Cambridge, Massachusetts 02140 (US); PERL, Nicholas Robert, Brookline, Massachusetts 02446 (US); RENNIE, Glen Robert, Somerville, Massachusetts 02144 (US); CURRIE, Mark G., Boston, MA 02129 (US); LONG, Kimberly Kafadar, Boston, MA 02130 (US); MILNE, George Todd, Brookline, MA 02446 (US); IYENGAR, Rajesh R., West Newton, MA 02465 (US)
(74) Representative: Mullen, Lee Bryan
(86) International application number: PCT/US2014/069537
(87) International publication number: WO 2015/089182

(56) References cited:
- WO-A1-2012/003405
- WO-A1-2012/064559
- WO-A1-2013/092512
- WO-A1-2013/101830
- WO-A1-2014/202588
- DAVID L SELWOOD ET AL: "Synthesis and biological evaluation of novel pyrazoles and indazoles as activators of the nitric oxide receptor, soluble guanylate cyclase", JOURNAL OF MEDICINAL CHEMISTRY, AMERICAN CHEMICAL SOCIETY, US, vol. 44, no. 1, 1 January 2001 (2001-01-01), pages 78-93, XP002658947, ISSN: 0022-2623, DOI: 10.1021/JM001034K [retrieved on 2000-11-22]

## Description

### CROSS-REFERENCE TO RELATED APPLICATIONS

This application claims priority of United Stated provisional applications 61/914,908, filed December 11, 2013, and 62/051,539, filed September 17, 2014.

### FIELD OF THE INVENTION

The present disclosure relates to stimulators of soluble guanylate cyclase (sGC), pharmaceutical formulations comprising them and their uses thereof, alone or in combination with one or more additional agents, for treating and/or preventing various diseases, wherein an increase in the concentration of nitric oxide (NO) or an increase in the concentration of cyclic Guanosine Monophosphate (cGMP) might be desirable.

### BACKGROUND OF THE INVENTION

Soluble guanylate cyclase (sGC) is the primary receptor for nitric oxide (NO) *in vivo.* sGC can be activated *via* both NO-dependent and NO-independent mechanisms. In response to this activation, sGC converts GTP into the secondary messenger cyclic GMP (cGMP). The increased level of cGMP, in turn, modulates the activity of downstream effectors including protein kinases, phosphodiesterases (PDEs) and ion channels.

In the body, NO is synthesized from arginine and oxygen by various nitric oxide synthase (NOS) enzymes and by sequential reduction of inorganic nitrate. Three distinct isoforms of NOS have been identified: inducible NOS (iNOS or NOS II) found in activated macrophage cells; constitutive neuronal NOS (nNOS or NOS I), involved in neurotransmission and long term potentiation; and constitutive endothelial NOS (eNOS or NOS III) which regulates smooth muscle relaxation and blood pressure.

Experimental and clinical evidence indicates that reduced bioavailability and/or responsiveness to endogenously produced NO contributes to the development of cardiovascular, endothelial, renal and hepatic disease, as well as erectile dysfunction and other sexual disorders (e.g.,female sexual disorder or vaginal atrophy). In particular, the NO signaling pathway is altered in cardiovascular diseases, including, for instance, systemic and pulmonary hypertension, heart failure, angina, stroke, thrombosis and other thromboembolic diseases, peripheral arterial disease, fibrosis of the liver, lung or kidney and atherosclerosis.

sGC stimulators are also useful in the treatment of lipid related disorders such as e.g., dyslipidemia, hypercholesterolemia, hypertriglyceridemia, sitosterolemia, fatty liver disease, and hepatitis.

Pulmonary hypertension (PH) is a disease characterized by sustained elevation of blood pressure in the pulmonary vasculature (pulmonary artery, pulmonary vein and pulmonary capillaries), which results in right heart hypertrophy, eventually leading to right heart failure and death. In PH, the bioactivity of NO and other vasodilators such as prostacyclin is reduced, whereas the production of endogenous vasoconstrictors such as endothelin is increased, resulting in excessive pulmonary vasoconstriction. sGC stimulators have been used to treat PH because they promote smooth muscle relaxation, which leads to vasodilation.

Treatment with NO-independent sGC stimulators also promoted smooth muscle relaxation in the corpus cavernosum of healthy rabbits, rats and humans, causing penile erection, indicating that sGC stimulators are useful for treating erectile dysfunction.

NO-independent, heme-dependent, sGC stimulators, such as those disclosed herein, have several important differentiating characteristics, including crucial dependency on the presence of the reduced prosthetic heme moiety for their activity, strong synergistic enzyme activation when combined with NO and stimulation of the synthesis of cGMP by direct stimulation of sGC, independent of NO. The benzylindazole compound YC-1 was the first sGC stimulator to be identified. Additional sGC stimulators with improved potency and specificity for sGC have since been developed. These compounds have been shown to produce anti-aggregatory, anti-proliferative and vasodilatory effects.

Since compounds that stimulate sGC in an NO-independent manner offer considerable advantages over other current alternative therapies, there is a need to develop novel stimulators of sGC. They are potentially useful in the prevention, management and treatment of disorders such as pulmonary hypertension, arterial hypertension, heart failure, atherosclerosis, inflammation, thrombosis, renal fibrosis and failure, liver cirrhosis, lung fibrosis, erectile dysfunction, female sexual arousal disorder and vaginal atrophy and other cardiovascular disorders; they are also potentially useful for the prevention, management and treatment of lipid related disorders.

In D.L. Selwood et al., "Synthesis and Biological Evaluation of Novel Pyrazoles and Indazoles as Activators of the Nitric Oxide Receptor, Soluble Guanylate Cyclase", J. Med. Chem., 2001, 44 (1), pp 78-93, 1-benzyl-3-(3-dimethylaminopropyloxy)indazole was investigated as an activator of the nitric oxide receptor, soluble guanylate cyclase.

WO 2012/064559 discloses various compounds as stimulators of sGC, particularly NO-independent, heme-dependent stimulators. These compounds may be useful for treating, preventing or managing various disorders.

WO 2014/202588 discloses heteroaryl substituted pyrazoles and their use as pharmaceuticals.

WO 2013/092512 discloses substituted benzylpyrazoles and their use as pharmaceuticals.

### SUMMARY OF THE INVENTION

This invention is directed to a compound according to Formula I, or a pharmaceutically acceptable salt thereof; it is also directed to pharmaceutical compositions comprising said compounds. 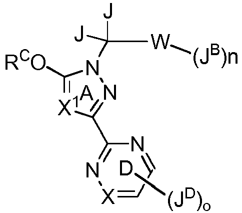 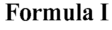
wherein X is selected from N or C;
X¹ is selected from N, CH, C(C₁₋₄ alkyl), C(C₁₋₄ fluoroalkyl), C(Cl) and CF;
W is either
   i) absent, with J^{B} connected directly to the carbon atom bearing two J groups, each J is independently selected from hydrogen, methyl or fluorine, n is 1 and J^{B} is a C₁₋₆ alkyl chain optionally substituted by up to 6 instances of fluorine; or
   ii) a ring B selected from phenyl or a 5 or 6-membered heteroaryl ring, containing 1 or 2 ring heteroatoms selected from N, O or S; wherein when W is ring B:
      each J is hydrogen;
      n is 0 or an integer selected from 1 to 3;
      and each J^{B} is independently selected from halogen, -CN, a C₁₋₆ aliphatic, -OR^{B} or a C₃₋₈ cycloaliphatic group; wherein each said C₁₋₆ aliphatic and each said C₃₋₈ cycloaliphatic group is optionally and independently substituted with up to 3 instances of R³;
      each R^{B} is independently selected from hydrogen, a C₁₋₆ aliphatic or a C₃₋₈ cycloaliphatic; wherein each said R^{B} that is a C₁₋₆ aliphatic and each said R^{B} that is a C₃₋₈ cycloaliphatic ring is optionally and independently substituted with up to 3 instances of R^{3a};
      each R³ is independently selected from halogen, -CN, C₁₋₄ alkyl, C₁₋₄ haloalkyl, -O(C₁₋₄ alkyl) or -O(C₁₋₄ haloalkyl);
      each R^{3a} is independently selected from halogen, -CN, C₁₋₄ alkyl, C₁₋₄ haloalkyl, -O(C₁₋₄ alkyl) or -O(C₁₋₄ haloalkyl);
o is 0 or an integer selected from 1 to 3;
each J^{D} is either absent or independently selected from hydrogen, halogen, -CN, -NO₂, -OR^{D}, -SR^{D}, -C(O)R^{D}, -C(O)OR^{D}, -OC(O)R^{D}, -C(O)N(R^{D})₂, -N(R^{D})₂, -N(R^{D})C(O)R^{D}, -N(R^{d})C(O)OR^{D}, -N(R^{D})C(O)N(R^{D})₂, -OC(O)N(R^{D})₂, -SO₂R^{D}, -SO₂N(R^{D})₂, -N(R^{d})SO₂R^{D}, a C₁₋₆ aliphatic, -(C₁₋₆ aliphatic)-R^{D}, a C₃₋₈ cycloaliphatic ring, a 6 to 10-membered aryl ring, a 4 to 8-membered heterocyclic ring or a 5 to 10-membered heteroaryl ring; wherein each said 4 to 8-membered heterocyclic ring and each said 5 to 10-membered heteroaryl ring contains between 1 and 3 heteroatoms independently selected from O, N or S; and wherein each of said C₁₋₆ aliphatic chains, each said C₃₋₈ cycloaliphatic ring, each said 6 to 10-membered aryl ring, each said 4 to 8-membered heterocyclic ring and each said 5 to 10-membered heteroaryl ring is optionally and independently substituted with up to 5 instances of R⁵;
each R^{D} is independently selected from hydrogen, a C₁₋₆ aliphatic, -(C₁₋₆ aliphatic)-R^{f}, a C₃₋₈ cycloaliphatic ring, a 4 to 8-membered heterocyclic ring and phenyl; wherein each said 4 to 8-membered heterocyclic ring contains between 1 and 3 heteroatoms independently selected from O, N and S; and wherein each of said C₁₋₆ aliphatic chains, each said C₃₋₈ cycloaliphatic ring, each said 4 to 8-membered heterocyclic ring and each said phenyl is optionally and independently substituted with up to 5 instances of R^{5a}; wherein when any R^{D} is one of a C₁₋₆ aliphatic or a -(C₁₋₆ aliphatic)-R^{f} group, one or two -CH₂- units that form said C₁₋₆ aliphatic chains may, optionally, be replaced by a group independently selected from -C(O)-, -N(R^{d}) - or -O-;
each R^{d} is independently selected from hydrogen, a C₁₋₆ aliphatic, -(C₁₋₆ aliphatic)-R^{f}, a C₃₋₈ cycloaliphatic ring, a 4 to 8-membered heterocyclic ring, phenyl or a 5 to 6-membered heteroaryl ring; wherein each said 4 to 8-membered heterocyclic ring and each said 5 or 6-membered heteroaryl ring contains between 1 and 3 heteroatoms independently selected from O, N or S; and wherein each of said C₁₋₆ aliphatic chains, each said C₃₋₈ cycloaliphatic ring, each said 4 to 8-membered heterocyclic ring, each said phenyl and each said 5 to 6-membered heteroaryl ring is optionally and independently substituted by up to 5 instances of R^{5b};
each R^{f} is independently selected from a C₁₋₃ alkyl, a C₃₋₈ cycloaliphatic ring, a 4 to 8-membered heterocyclic ring, phenyl or a 5 to 6-membered heteroaryl ring; wherein each said 4 to 8-membered heterocyclic ring and each said 5 to 6-membered heteroaryl ring contains between 1 and 4 heteroatoms independently selected from O, N or S; and wherein each said C₃₋₈ cycloaliphatic ring, each said 4 to 8-membered heterocyclic ring, each said phenyl and each said 5 to 6-membered heteroaryl ring is optionally and independently substituted by up to 5 instances of R^{5c};
when J^{D} is -C(O)N(R^{D})₂, -N(R^{D})₂, -N(R^{d})C(O)N(R^{D})₂, -OC(O)N(R^{D})₂ or -SO₂N(R^{D})₂, the two R^{D} groups together with the nitrogen atom attached to the two R^{D} groups may form a 4 to 8-membered heterocyclic ring or a 5-membered heteroaryl ring; wherein each said 4 to 8-membered heterocyclic ring and each said 5-membered heteroaryl ring optionally contains up to 3 additional heteroatoms independently selected from N, O or S, in addition to the nitrogen atom to which the two R^{D} groups are attached; and wherein each said 4 to 8-membered heterocyclic ring and each said 5-membered heteroaryl ring is optionally and independently substituted by up to 5 instances of R^{5d};
when J^{D} is -N(R^{d})C(O)R^{D}, the R^{D} group together with the carbon atom attached to the R^{D} group, with the nitrogen atom attached to the R^{d} group, and with the R^{d} group may form a 4 to 8-membered heterocyclic ring or a 5-memere heteroaryl ring; wherein each said 4 to 8-membered heterocyclic ring and each said 5-membered heteroaryl ring optionally contains up to 2 additional heteroatoms independently selected from N, O or S, in addition to the nitrogen atom to which the R^{d} group is attached; and wherein each said 4 to 8-membered heterocyclic ring and each said 5-membered heteroaryl ring is optionally and independently substituted by up to 5 instances of R^{5a};
when J^{D} is -N(R^{d})C(O)OR^{D}, the R^{D} group together with the oxygen atom attached to the R^{D} group, with the carbon atom of the -C(O)- portion of the -N(R^{d})C(O)OR^{D} group, with the nitrogen atom attached to the R^{d} group, and with said R^{d} group, may form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
when J^{D} is -N(R^{d})C(O)N(R^{D})₂, one of the R^{D} groups attached to the nitrogen atom, together with said nitrogen atom, and with the N atom attached to the R^{d} group and said R^{d} group may form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
when J^{D} is -N(R^{d})SO₂R^{D}, the R^{D} group together with the sulfur atom attached to the R^{D} group, with the nitrogen atom attached to the R^{d} group, and with said R^{d} group may combine to form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
each R⁵ is independently selected from halogen, -CN, C₁₋₆ alkyl, -(C₁₋₆alkyl)-R⁶, -OR⁶, -SR⁶, -COR⁶, -OC(O)R⁶, -C(O)OR⁶, -C(O)N(R⁶)₂, -N(R⁶)C(O)R⁶-N(R⁶)C(O)OR⁶, -N(R⁶)C(O)N(R⁶)₂, -N(R⁶)₂, -SO₂R⁶, -SO₂OH, -SO₂NHOH, -SO₂N(R⁶)₂, -SO₂N(R⁶)(CO)-R⁶, -N(R⁶)SO₂R⁶, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, said C₇₋₁₂ aralkyl, said C₃₋₈ cycloalkyl ring, said 4 to 7-membered heterocyclic ring, said 5 or 6-membered heteroaryl ring or said phenyl group is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -CONH₂, -COO(C₁₋₄ alkyl), -O(C₁₋₄ alkyl), -O(C₁₋₄ haloalkyl) or oxo;
alternatively, two instances of R⁵ attached to the same or different atoms of J^{D}, together with said atom or atoms of J^{D} to which they are attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -NR(CO)O(C₁₋₄ alkyl), -CONH₂, -OH or halogen; wherein R is hydrogen or a C₁₋₂ alkyl;
each R^{5a} is independently selected from halogen, -CN, C₁₋₆ alkyl, -(C₁₋₆alkyl)-R^{6a}, -OR^{6a}, -SR^{6a}, -COR^{6a}, -OC(O)R^{6a}, -C(O)OR^{6a}, -C(O)N(R^{6a})₂, -N(R^{6a})C(O)R^{6a}-N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, each said C₇₋₁₂ aralkyl, said C₃₋₈ cycloalkyl ring, said 4 to 7-membered heterocyclic ring, said 5 or 6-membered heteroaryl ring or phenyl group is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -COO(C₁₋₄ alkyl), -CONH₂, -O(C₁₋₄ alkyl), -O(C₁₋₄ haloalkyl) or oxo;
each R^{5b} is independently selected from halogen, -CN, C₁₋₆ alkyl, -(C₁₋₆alkyl)-R^{6a}, -OR^{6a}, -SR^{6a}, -COR^{6a}, -OC(O)R^{6a}, -C(O)OR^{6a}, -C(O)N(R^{6a})₂, -N(R^{6a})C(O)R^{6a}-N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, each said C₇₋₁₂ aralkyl, said C₃₋₈ cycloalkyl ring, said 4 to 7-membered heterocyclic ring, said 5 or 6-membered heteroaryl ring or phenyl group is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -COO(C₁₋₄ alkyl), -CONH₂, -O(C₁₋₄ alkyl), -O(C₁₋₄ haloalkyl) or oxo;
alternatively, two instances of R^{5a} or two instances of R^{5b} attached to the same or different atoms of R^{D} or R^{d}, respectively, together with said atom or atoms to which they are attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -NR(CO)O(C₁₋₄ alkyl), -CONH₂, -OH or halogen; wherein R is hydrogen or a C₁₋₂ alkyl;
each R^{5c} is independently selected from halogen, -CN, C₁₋₆ alkyl, -(C₁₋₆alkyl)-R^{6b}, -OR^{6b}, -SR^{6b}, -COR^{6b}, -OC(O)R^{6B}, -C(O)OR^{6b}, -C(O)N(R^{6b})₂, -N(R⁶)C(O)R^{6b}, -N(R^{6b})C(O)OR^{6b}, -N(R^{6b})C(O)N(R^{6b})₂, -N(R^{6b})₂, -SO₂R^{6b}, -SO₂OH, -SO₂NHOH, -SO₂N(R^{6b})(CO)-R^{6b}, -SO₂N(R⁶¹)₂, -N(R^{6b})SO₂R^{6b}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, said C₇₋₁₂ aralkyl, said C₃₋₈ cycloalkyl ring, said 4 to 7-membered heterocyclic ring, said 5 or 6-membered heteroaryl ring or said phenyl groups is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -CONH₂, -COO(C₁₋₄ alkyl), -O(C₁₋₄ alkyl), -O(C₁₋₄ haloalkyl) or oxo;
alternatively, two instances of R^{5c} attached to the same or different atoms of R^{f}, together with said atom or atoms to which it is attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -CONH₂, -NR(CO)O(C₁₋₄ alkyl), -OH or halogen; wherein R is hydrogen or a C₁₋₂ alkyl;
each R^{5d} is independently selected from halogen, -CN, C₁₋₆ alkyl, -(C₁₋₆alkyl)-R⁶, -OR⁶, -SR⁶, -COR⁶, -OC(O)R⁶, -C(O)OR⁶, -C(O)N(R⁶)₂, -N(R⁶)C(O)R⁶-N(R⁶)C(O)OR⁶, -N(R⁶)C(O)N(R⁶)₂, -N(R⁶)₂, -SO₂OH, -SO₂NHOH, -SO₂N(R⁶)(CO)-R⁶, -SO₂R⁶, -SO₂N(R⁶)₂, -N(R⁶)SO₂R⁶, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, said C₇₋₁₂ aralkyl, said C₃₋₈ cycloalkyl ring, said 4 to 7-membered heterocyclic ring, said 5 or 6-membered heteroaryl ring or said phenyl groups is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -CONH₂, -COO(C₁₋₄ alkyl), -O(C₁₋₄ alkyl), -O(C₁₋₄ haloalkyl) or oxo;
two instances of R⁵ or two instances of R^{5d}, attached to the same or different atoms of J^{D}, together with said atom or atoms to which they are attached, may optionally form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship, wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -CONH₂, -NR(CO)O(C₁₋₄ alkyl), -OH or halogen; wherein R is hydrogen or a C₁₋₂ alkyl;
each R⁶ is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S;
each R^{6a} is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S;
each R^{6b} is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S; wherein
two instances of R⁶ linked to the same nitrogen atom of R⁵ or R^{5d}, together with said nitrogen atom of R⁵ or R^{5d}, respectively, may form a 5 to 8-membered heterocyclic ring or a 5-membered heteroaryl ring; wherein each said 5 to 8-membered heterocyclic ring and each said 5-membered heteroaryl ring optionally contains up to 2 additional heteroatoms independently selected from N, O or S;
two instances of R^{6a} linked to a nitrogen atom of R^{5a} or R^{5b}, together with said nitrogen, may form a 5 to 8-membered heterocyclic ring or a 5-membered heteroaryl ring; wherein each said 5 to 8-membered heterocyclic ring and each said 5-membered heteroaryl ring optionally contains up to 2 additional heteroatoms independently selected from N, O or S;
two instances of R^{6b} linked to a nitrogen atom of R^{5c}, together with said nitrogen, may form a 5 to 8-membered heterocyclic ring or a 5-membered heteroaryl ring; wherein each said 5 to 8-membered heterocyclic ring and each said 5-membered heteroaryl ring optionally contains up to 2 additional heteroatoms independently selected from N, O or S;
alternatively, two J^{D} groups attached to two vicinal ring D atoms, taken together with said two vicinal ring D atoms, may form a 5 to 7-membered heterocycle or a 5-membered heteroaryl ring that is fused to ring D; wherein said 5 to 7-membered heterocycle or said 5-membered ring heteroaryl contains from 1 to 3 heteroatoms independently selected from N, O or S; and wherein said 5 to 7-membered heterocycle or said 5-membered heteroaryl ring is optionally and independently substituted by up to 3 instances of oxo or -(Y)-R⁹;
wherein Y is either absent or is a C₁₋₆ alkyl chain, optionally substituted by up to 6 instances of fluoro; and wherein when Y is said C₁₋₆ alkyl chain, up to 3 methylene units of this alkyl chain, can be replaced by a group selected from -O-, -C(O) - or -N((Y¹)-R⁹⁹)-;
Y¹ is either absent or a C₁₋₆ alkyl chain, optionally substituted by up to 6 instances of fluoro;
when Y¹ is absent, each R⁹⁹ is independently selected from hydrogen, C₁₋₆ alkyl optionally substituted with up to 9 fluorine atoms, -COR¹⁰, -C(O)OR¹⁰,-C(O)N(R¹⁰)₂, -C(O)N(R¹⁰)SO₂R¹⁰, -SO₂R¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, a C₃₋₆ cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
when Y¹ is present, each R⁹⁹ is independently selected from hydrogen, halogen, -CN, C₁₋₆ alkyl optionally substituted with up to 9 fluorine atoms, -COR¹⁰, -OR¹⁰, -OC(O)R¹⁰, -C(O)OR¹⁰,-C(O)N(R¹⁰)₂, -C(O)N(R¹⁰)SO₂R¹⁰, -SO₂R¹⁰, -SOR¹⁰, -SR¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, a C₃₋₆ cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
each R⁹ is independently selected from hydrogen, -CN, -OR¹⁰, -COR¹⁰, -OC(O)R¹⁰, -C(O)OR¹⁰, -C(O)N(R¹⁰)₂, -C(O)N(R¹⁰)SO₂R¹⁰, -N(R¹⁰)C(O)R¹⁰, -N(R¹⁰)C(O)OR¹⁰, -N(R¹⁰)C(O)N(R¹⁰)₂, -N(R¹⁰)₂, -SO₂R¹⁰, -SO₂N(R¹⁰)₂, -SO₂N(R¹⁰)COOR¹⁰, -SO₂N(R¹⁰)C(O)R¹⁰, -N(R¹⁰)SO₂R¹⁰, -SO₂OH, -SO₂NHOH, -SO₂N(R¹⁰)(CO)-R¹⁰, a C₃₋₆ cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
each R¹⁰ is independently selected from hydrogen, a C₁₋₆ alkyl, -(C₁₋₆ alkyl)-R¹³, phenyl, benzyl, a C₃₋₆ cycloalkyl ring, a 4 to 7-membered heterocyclic ring or a 5 or 6-membered heteroaryl ring, wherein each 5 or 6-membered heteroaryl ring or 4 to 7-membered heterocyclic ring contains up to 4 ring heteroatoms independently selected from N, O and S; and wherein each of said C₁₋₆ alkyl, each said phenyl, each said benzyl, each said C₃₋₈ cycloalkyl group, each said 4 to 7-membered heterocyclic ring and each 5 or 6-membered heteroaryl ring is optionally and independently substituted with up to 3 instances of R^{11b};
each R¹³ is independently selected from a phenyl, a benzyl, a C₃₋₆ cycloalkyl ring, a 4 to 7-membered heterocyclic ring or a 5 or 6-membered heteroaryl ring, wherein each 5 or 6-membered heteroaryl ring or 4 to 7-membered heterocyclic ring contains up to 4 ring heteroatoms independently selected from N, O and S; and wherein each said phenyl, each of said benzyl, each said C₃₋₈ cycloalkyl group, each said 4 to 7-membered heterocyclic ring and each 5 or 6-membered heteroaryl ring is optionally and independently substituted with up to 3 instances of R^{11c};
each R^{11a} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹;
each R^{11b} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹; and
each R^{11c} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹;
each R¹² is selected from hydrogen, C₁₋₄ alkyl, C₁₋₄ (fluoroalkyl), -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ fluoroalkyl) or oxo;
each R¹²¹ is selected from C₁₋₄ alkyl, C₁₋₄ (fluoroalkyl), -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ fluoroalkyl) or oxo;
R^{C} is selected from hydrogen, C₁₋₆ aliphatic, -(C₁₋₆ alkyl)-R^{N}, a 5 or 6-membered heteroaryl, phenyl, a 4 to 7 membered heterocyclic, a C₃₋₈ cycloaliphatic, -C(O)R⁷, -C(O)OR⁷, -C(O)N(R⁷)₂, and -C(O)N(R⁷)SO₂R⁷; wherein each of said 5 or 6-membered heteroaryl ring and 4 to 7-membered heterocyclic ring contains up to 4 ring heteroatoms independently selected from N, O and S; wherein each said C₁₋₆ aliphatic and each C₁₋₆ alkyl portion of said -(C₁₋₆ alkyl)-R^{N}, is optionally and independently substituted with up to 6 instances of halogen and up to 2 instances of-CN, -COOR⁸, -OR⁸, oxo, -N(R⁸)₂, -C(O)N(R⁸)₂, -N(R⁸)C(O)R⁸, -N(R⁸)C(O)OR⁸, -N(R⁸)C(O)N(R⁸)₂, -SO₂R⁸, -SO₂N(R⁸)₂, -NHOR⁸, -SO₂N(R⁸)COOR⁸, -SO₂N(R⁸)C(O)R⁸ and -N(R⁸)SO₂R⁸;
wherein each R⁷ is independently selected from hydrogen, C₁₋₆ alkyl, C₁₋₆ fluoroalkyl, a C₃₋₈ cycloalkyl ring, phenyl, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo;
each R⁸ is independently selected from hydrogen, C₁₋₆ alkyl, C₁₋₆ fluoroalkyl, a C₃₋₈ cycloalkyl ring, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo;
each R^{N} is independently selected from a phenyl ring, a monocyclic 5 or 6-membered heteroaryl ring, a monocyclic C₃₋₆ cycloaliphatic ring, or a monocyclic 4 to 6-membered heterocycle; wherein said monocyclic 5 or 6-membered heteroaryl ring or said monocyclic 4 to 6-membered heterocycle contain between 1 and 4 heteroatoms selected from N, O or S; wherein said monocyclic 5 or 6-membered heteroaryl ring is not a 1,3,5-triazinyl ring; and wherein said phenyl, said monocyclic 5 to 6-membered heteroaryl ring, said monocyclic C₃₋₆ cycloaliphatic ring, or said monocyclic 4 to 6-membered heterocycle is optionally and independently substituted with up to 6 instances of fluoro and/or up to 3 instances of J^{M};
each J^{M} is independently selected from -CN, a C₁₋₆aliphatic, -OR^{M}, -SR^{M}, -N(R^{M})₂, a C₃₋₈ cycloaliphatic ring or a 4 to 8-membered heterocyclic ring; wherein said 4 to 8-membered heterocyclic ring contains 1 or 2 heteroatoms independently selected from N, O or S; wherein each said C₁₋₆ aliphatic, each said C₃₋₈ cycloaliphatic ring and each said 4 to 8-membered heterocyclic ring, is optionally and independently substituted with up to 3 instances of R^{7c}; and
each R^{M} is independently selected from hydrogen, a C₁₋₆ aliphatic, a C₃₋₈ cycloaliphatic ring or a 4 to 8-membered heterocyclic ring; wherein each said 4 to 8-membered heterocylic ring contains between 1 and 3 heteroatoms independently selected from O, N or S; and
each R^{7c} is independently selected from halogen, -CN, -NO₂, C₁₋₄ alkyl, C₁₋₄ haloalkyl, C₃₋₈ cycloalkyl ring, -OR^{8b}, -SR^{8b}, -N(R^{8b})₂, -C(O)O(C₁₋₄ alkyl), -C(O)OH, -NR(CO)CO(C₁₋₄ alkyl) or an oxo group; wherein each said cycloalkyl group is optionally and independently substituted with up to 3 instances of halogen;
each R^{8b} is independently selected from hydrogen, C₁₋₆ alkyl, C₁₋₆ fluoroalkyl, a C₃₋₈ cycloalkyl ring, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo;
wherein the heterocyclic ring described above is completely saturated or contains one or more units of unsaturation but is not aromatic;
provided that the compound is not one represented by the general structure: 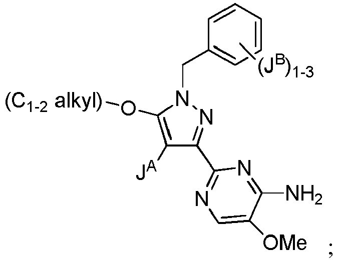
wherein J^{A} is either hydrogen or C₁₋₄ alkyl; and J^{B} is either halogen or C₁₋₄ (alkoxy).

The invention is also directed to a pharmaceutical composition comprising a compound disclosed herein, or a pharmaceutically acceptable salt thereof, and at least one pharmaceutically acceptable excipient.

The invention is also directed to a pharmaceutical formulation or dosage form comprising the pharmaceutical composition and at least one excipient or carrier.

Also disclosed is a method of treating or preventing a disease, health condition or disorder in a subject in need thereof, comprising administering, alone or in combination therapy, a therapeutically effective amount of a compound of Formula I or a pharmaceutically acceptable salt thereof to the subject; wherein the disease, health condition or disorder is a peripheral, pulmonary, hepatic, kidney, digestive, cardiac or cerebral vascular/endothelial disorder or condition, a urogenital-gynecological or sexual disorder or condition, a thromboembolic disease, an ischemic disorder, a fibrotic disorder, a pulmonary or respiratory disorder, renal or hepatic disorder, an ocular disorder, a hearing disorder, a CNS disorder, a circulation disorder, a topical or skin disorder, a metabolic disorder, an autoimmune disorder, an inflammation mediated disorder, atherosclerosis, a wound or bone healing disorder, alopecia, certain cancers, or a lipid related disorder that benefits from sGC stimulation or from an increase in the concentration of NO or cGMP.

### DETAILED DESCRIPTION OF THE INVENTION

Reference will now be made in detail to certain embodiments of the invention, examples of which are illustrated in the accompanying structures and formulae.

### Definitions and general terminology

For purposes of this disclosure, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, and "March's Advanced Organic Chemistry", 5th Ed., Smith, M. B. and March, J., eds. John Wiley & Sons, New York: 2001.

As described herein, compounds of Formula I may be optionally substituted with one or more substituents, such as illustrated generally below, or as exemplified by particular classes, subclasses and species of the invention. The phrase "optionally substituted" is used interchangeably with the phrase "substituted or unsubstituted." In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position unless otherwise specified. As will be apparent to one of ordinary skill in the art, groups such as -H, halogen, -NO₂, -CN, -OH, -NH₂ or -OCF₃ would not be substitutable groups.

The phrase "up to", as used herein, refers to zero or any integer number that is equal or less than the number following the phrase. For example, "up to 3" means any one of 0, 1, 2, or 3. As described herein, a specified number range of atoms includes any integer therein. For example, a group having from 1-4 atoms could have 1, 2, 3 or 4 atoms. When any variable occurs more than one time at any position, its definition on each occurrence is independent from every other occurrence.

Selection of substituents and combinations envisioned by this disclosure are only those that result in the formation of stable or chemically feasible compounds. Such choices and combinations will be apparent to those of ordinary skill in the art and may be determined without undue experimentation. The term "stable", as used herein, refers to compounds that are not substantially altered when subjected to conditions to allow for their production, detection, and, in some embodiments, their recovery, purification, and use for one or more of the purposes disclosed herein. In some embodiments, a stable compound is one that is not substantially altered when kept at a temperature of 25°C or less, in the absence of moisture or other chemically reactive conditions, for at least a week. A chemically feasible compound is a compound that can be prepared by a person skilled in the art based on the disclosures herein supplemented, if necessary, relevant knowledge of the art.

A compound, such as the compounds of Formula I or other compounds herein disclosed, may be present in its free form (e.g.,an amorphous form, or a crystalline form or a polymorph). Under certain conditions, compounds may also form co-forms. As used herein, the term co-form is synonymous with the term multi-component crystalline form. When one of the components in the co-form has clearly transferred a proton to the other component, the resulting co-form is referred to as a "salt". The formation of a salt is determined by how large the difference is in the pKas between the partners that form the mixture. For purposes of this disclosure, compounds include pharmaceutically acceptable salts, even if the term "pharmaceutically acceptable salts" is not explicitly noted.

Unless only one of the isomers is drawn or named specifically, structures depicted herein are also meant to include all stereoisomeric (e.g., enantiomeric, diastereomeric, atropoisomeric and cis-trans isomeric) forms of the structure; for example, the *R* and *S* configurations for each asymmetric center, *Ra* and *Sa* configurations for each asymmetric axis, (*Z*) and (*E*) double bond configurations, and *cis* and *trans* conformational isomers. Therefore, single stereochemical isomers as well as racemates, and mixtures of enantiomers, diastereomers, and *cis-trans* isomers (double bond or conformational) of the present compounds are within the scope of the present disclosure. Unless otherwise stated, all tautomeric forms of the compounds of the present disclosure are also within the scope of the invention. As an example, a substituent drawn as below: 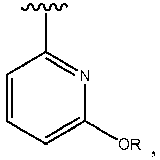 wherein R may be hydrogen, would include both compounds shown below:

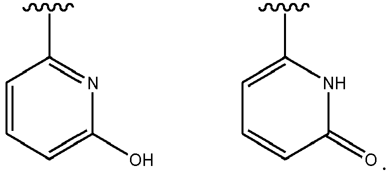

The present disclosure also embraces isotopically-labeled compounds which are identical to those recited herein, but for the fact that one or more atoms are replaced by an atom having an atomic mass or mass number different from the atomic mass or mass number usually found in nature. All isotopes of any particular atom or element as specified are contemplated within the scope of the compounds of the invention, and their uses. Exemplary isotopes that can be incorporated into compounds of the invention include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I, and ¹²⁵I, respectively. Certain isotopically-labeled compounds of the present invention (e.g., those labeled with ³H and ¹⁴C) are useful in compound and/or substrate tissue distribution assays. Tritiated (i.e., ³H) and carbon-14 (i.e., ¹⁴C) isotopes are useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements) and hence may be preferred in some circumstances. Positron emitting isotopes such as ¹⁵O, ¹³N, ¹¹C, and ¹⁸F are useful for positron emission tomography (PET) studies to examine substrate receptor occupancy. Isotopically labeled compounds of the present invention can generally be prepared by following procedures analogous to those disclosed in the Schemes and/or in the Examples herein below, by substituting an isotopically labeled reagent for a non-isotopically labeled reagent.

The term "aliphatic" or "aliphatic group", as used herein, means a straight-chain (i.e., unbranched) or branched, substituted or unsubstituted hydrocarbon chain that is completely saturated or that contains one or more units of unsaturation. Unless otherwise specified, aliphatic groups contain 1-20 aliphatic carbon atoms. In some embodiments, aliphatic groups contain 1-10 aliphatic carbon atoms. In other embodiments, aliphatic groups contain 1-8 aliphatic carbon atoms. In still other embodiments, aliphatic groups contain 1-6 aliphatic carbon atoms. In other embodiments, aliphatic groups contain 1-4 aliphatic carbon atoms and in yet other embodiments, aliphatic groups contain 1-3 aliphatic carbon atoms. Suitable aliphatic groups include, but are not limited to, linear or branched, substituted or unsubstituted alkyl, alkenyl, or alkynyl groups. Specific examples of aliphatic groups include, but are not limited to: methyl, ethyl, propyl, butyl, isopropyl, isobutyl, vinyl, sec-butyl, tert-butyl, butenyl, propargyl, acetylene and the like. To be perfectly clear, the term "aliphatic chain" may be used interchangeably with the term "aliphatic" or "aliphatic group".

The term "alkyl" (as in "alkyl chain" or "alkyl group"), as used herein, refers to a saturated linear or branched-chain monovalent hydrocarbon radical. Unless otherwise specified, an alkyl group contains 1-20 carbon atoms (e.g., 1-20 carbon atoms, 1-10 carbon atoms, 1-8 carbon atoms, 1-6 carbon atoms, 1-4 carbon atoms or 1-3 carbon atoms). Examples of alkyl groups include, but are not limited to, methyl, ethyl, n-propyl, isopropyl, n-butyl, isobutyl, s-butyl, t-butyl, pentyl, hexyl, heptyl, octyl and the like.

The term "alkenyl" (as in "alkenyl chain" or "alkenyl group"), refers to a linear or branched-chain monovalent hydrocarbon radical with at least one site of unsaturation, i.e., a carbon-carbon, sp² double bond, wherein the alkenyl radical includes radicals having "cis" and "trans" orientations, or alternatively, "E" and "Z" orientations. Unless otherwise specified, an alkenyl group contains 2-20 carbon atoms (e.g., 2-20 carbon atoms, 2-10 carbon atoms, 2-8 carbon atoms, 2-6 carbon atoms, 2-4 carbon atoms or 2-3 carbon atoms). Examples include, but are not limited to, vinyl, allyl and the like.

The term "alkynyl" (as in "alkynyl chain" or "alkynyl group"), refers to a linear or branched monovalent hydrocarbon radical with at least one site of unsaturation, i.e., a carbon-carbon sp triple bond. Unless otherwise specified, an alkynyl group contains 2-20 carbon atoms (e.g., 2-20 carbon atoms, 2-10 carbon atoms, 2-8 carbon atoms, 2-6 carbon atoms, 2-4 carbon atoms or 2-3 carbon atoms). Examples include, but are not limited to, ethynyl, propynyl, and the like.

The term "carbocyclic" refers to a ring system formed only by carbon and hydrogen atoms. Unless otherwise specified, throughout this disclosure, carbocycle is used as a synonym of "non-aromatic carbocycle" or "cycloaliphatic". In some instances the term can be used in the phrase "aromatic carbocycle", and in this case it refers to an "aryl group" as defined below.

The term "cycloaliphatic" (or "non-aromatic carbocycle", "non-aromatic carbocyclyl", "non-aromatic carbocyclic") refers to a cyclic hydrocarbon that is completely saturated or that contains one or more units of unsaturation but which is not aromatic, and which has a single point of attachment to the rest of the molecule. Unless otherwise specified, a cycloaliphatic group may be monocyclic, bicyclic, tricyclic, fused, spiro or bridged. In one embodiment, the term "cycloaliphatic" refers to a monocyclic C₃-C₁₂ hydrocarbon or a bicyclic C₇-C₁₂ hydrocarbon. In some embodiments, any individual ring in a bicyclic or tricyclic ring system has 3-7 members. Suitable cycloaliphatic groups include, but are not limited to, cycloalkyl, cycloalkenyl, and cycloalkynyl. Examples of aliphatic groups include cyclopropyl, cyclobutyl, cyclopentyl, cyclopentenyl, cyclohexyl, cyclohexenyl, cycloheptyl, cycloheptenyl, norbornyl, cyclooctyl, cyclononyl, cyclodecyl, cycloundecyl, cyclododecyl, and the like.

The term "cycloaliphatic" also includes polycyclic ring systems in which the non-aromatic carbocyclic ring can be "fused" to one or more aromatic or non-aromatic carbocyclic or heterocyclic rings or combinations thereof, as long as the radical or point of attachment is on the non-aromatic carbocyclic ring.

"Cycloalkyl", as used herein, refers to a ring system in which is completely saturated and which has a single point of attachment to the rest of the molecule. Unless otherwise specified, a cycloalkyl group may be monocyclic, bicyclic, tricyclic, fused, spiro or bridged. In one embodiment, the term "cycloalkyl" refers to a monocyclic C₃-C₁₂ saturated hydrocarbon or a bicyclic C₇-C₁₂ saturated hydrocarbon. In some embodiments, any individual ring in a bicyclic or tricyclic ring system has 3-7 members. Suitable cycloalkyl groups include, but are not limited to, cyclopropyl, cyclobutyl, cyclopentyl, cyclohexyl, cycloheptyl, cycloheptenyl, norbornyl, cyclooctyl, cyclononyl, cyclodecyl, cycloundecyl, cyclododecyl, and the like.

"Heterocycle" (or "heterocyclyl" or "heterocyclic), as used herein, refers to a ring system in which one or more ring members are an independently selected heteroatom, which is completely saturated or that contains one or more units of unsaturation but which is not aromatic, and which has a single point of attachment to the rest of the molecule. Unless otherwise specified, through this disclosure, heterocycle is used as a synonym of "non-aromatic heterocycle". In some instances the term can be used in the phrase "aromatic heterocycle", and in this case it refers to a "heteroaryl group" as defined below. The term heterocycle also includes fused, spiro or bridged heterocyclic ring systems. Unless otherwise specified, a heterocycle may be monocyclic, bicyclic or tricyclic. In some embodiments, the heterocycle has 3-18 ring members in which one or more ring members is a heteroatom independently selected from oxygen, sulfur or nitrogen, and each ring in the system contains 3 to 7 ring members. In other embodiments, a heterocycle may be a monocycle having 3-7 ring members (2-6 carbon atoms and 1-4 heteroatoms) or a bicycle having 7-10 ring members (4-9 carbon atoms and 1-6 heteroatoms). Examples of bicyclic heterocyclic ring systems include, but are not limited to: adamantanyl, 2-oxa-bicyclo[2.2.2]octyl, 1-aza-bicyclo[2.2.2]octyl.

As used herein, the term "heterocycle" also includes polycyclic ring systems wherein the heterocyclic ring is fused with one or more aromatic or non-aromatic carbocyclic or heterocyclic rings, or with combinations thereof, as long as the radical or point of attachment is on the heterocyclic ring.

Examples of heterocyclic rings include, but are not limited to, the following monocycles: 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-tetrahydropiperazinyl, 2-tetrahydropiperazinyl, 3-tetrahydropiperazinyl, 1-piperidinyl, 2-piperidinyl, 3-piperidinyl, 1-pyrazolinyl, 3-pyrazolinyl, 4-pyrazolinyl, 5-pyrazolinyl, 1-piperidinyl, 2-piperidinyl, 3-piperidinyl, 4-piperidinyl, 2-thiazolidinyl, 3-thiazolidinyl, 4-thiazolidinyl, 1-imidazolidinyl, 2-imidazolidinyl, 4-imidazolidinyl, 5-imidazolidinyl; and the following bicycles: 3-1H-benzimidazol-2-one, 3-(1-alkyl)-benzimidazol-2-one, indolinyl, tetrahydroquinolinyl, tetrahydroisoquinolinyl, benzothiolane, benzodithiane, and 1,3-dihydro-imidazol-2-one.

As used herein, the term "aryl" (as in "aryl ring" or "aryl group"), used alone or as part of a larger moiety, as in "aralkyl", "aralkoxy", "aryloxyalkyl", refers to a carbocyclic ring system wherein at least one ring in the system is aromatic and has a single point of attachment to the rest of the molecule. Unless otherwise specified, an aryl group may be monocyclic, bicyclic or tricyclic and contain 6-18 ring members. The term also includes polycyclic ring systems where the aryl ring is fused with one or more aromatic or non-aromatic carbocyclic or heterocyclic rings, or with combinations thereof, as long as the radical or point of attachment is in the aryl ring. Examples of aryl rings include, but are not limited to, phenyl, naphthyl, indanyl, indenyl, tetralin, fluorenyl, and anthracenyl.

The term "aralkyl" refers to a radical having an aryl ring substituted with an alkylene group, wherein the open end of the alkylene group allows the aralkyl radical to bond to another part of the compound of Formula I. The alkylene group is a bivalent, straight-chain or branched, saturated hydrocarbon group. As used herein, the term "C₇₋₁₂ aralkyl" means an aralkyl radical wherein the total number of carbon atoms in the aryl ring and the alkylene group combined is 7 to 12. Examples of "aralkyl" include, but not limited to, a phenyl ring substituted by a C₁₋₆ alkylene group, e.g., benzyl and phenylethyl, and a naphthyl group substituted by a C₁₋₂ alkylene group.

The term "heteroaryl" (or "heteroaromatic" or "heteroaryl group" or "aromatic heterocycle") used alone or as part of a larger moiety as in "heteroaralkyl" or "heteroarylalkoxy" refers to a ring system wherein at least one ring in the system is aromatic and contains one or more heteroatoms, wherein each ring in the system contains 3 to 7 ring members and which has a single point of attachment to the rest of the molecule. Unless otherwise specified, a heteroaryl ring system may be monocyclic, bicyclic or tricyclic and have a total of five to fourteen ring members. In one embodiment, all rings in a heteroaryl system are aromatic. Also included in this definition are heteroaryl radicals where the heteroaryl ring is fused with one or more aromatic or non-aromatic carbocyclic or heterocyclic rings, or combinations thereof, as long as the radical or point of attachment is in the heteroaryl ring. Bicyclic 6, 5 heteroaromatic system, as used herein, for example, is a six membered heteroaromatic ring fused to a second five membered ring wherein the radical or point of attachment is on the six-membered ring.

Heteroaryl rings include, but are not limited to the following monocycles: 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, N-pyrrolyl, 2-pyrrolyl, 3-pyrrolyl, 2-pyridyl, 3-pyridyl, 4-pyridyl, 2-pyrimidinyl, 4-pyrimidinyl, 5-pyrimidinyl, pyridazinyl (e.g., 3-pyridazinyl), 2-thiazolyl, 4-thiazolyl, 5-thiazolyl, tetrazolyl (e.g., 5-tetrazolyl), triazolyl (e.g., 2-triazolyl and 5-triazolyl), 2-thienyl, 3-thienyl, pyrazolyl (e.g., 2-pyrazolyl), isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, pyrazinyl, 1,3,5-triazinyl, and the following bicycles: benzimidazolyl, benzofuryl, benzothiophenyl, benzopyrazinyl, benzopyranonyl, indolyl (e.g., 2-indolyl), purinyl, quinolinyl (e.g., 2-quinolinyl, 3-quinolinyl, 4-quinolinyl), and isoquinolinyl (e.g., 1-isoquinolinyl, 3-isoquinolinyl, or 4-isoquinolinyl).

As used herein, "cyclo" (or "cyclic", or "cyclic moiety") encompasses mono-, bi- and tri-cyclic ring systems including cycloaliphatic, heterocyclic, aryl or heteroaryl, each of which has been previously defined.

"Fused" bicyclic ring systems comprise two rings which share two adjoining ring atoms.

"Bridged" bicyclic ring systems comprise two rings which share three or four adjacent ring atoms. As used herein, the term "bridge" refers to an atom or a chain of atoms connecting two different parts of a molecule. The two atoms that are connected through the bridge (usually but not always, two tertiary carbon atoms) are referred to as "bridgeheads". In addition to the bridge, the two bridgeheads are connected by at least two individual atoms or chains of atoms. Examples of bridged bicyclic ring systems include, but are not limited to, adamantanyl, norbornanyl, bicyclo[3.2.1]octyl, bicyclo[2.2.2]octyl, bicyclo[3.3.1]nonyl, bicyclo[3.2.3]nonyl, 2-oxa-bicyclo[2.2.2]octyl, 1-aza-bicyclo[2.2.2]octyl, 3-aza-bicyclo[3.2.1]octyl, and 2,6-dioxa-tricyclo[3.3.1.03,7]nonyl. "Spiro" bicyclic ring systems share only one ring atom (usually a quaternary carbon atom) between the two rings.

The term "ring atom" refers to an atom such as C, N, O or S that is part of the ring of an aromatic ring, a cycloaliphatic ring, a heterocyclic or a heteroaryl ring. A "substitutable ring atom" is a ring carbon or nitrogen atom bonded to at least one hydrogen atom. The hydrogen can be optionally replaced with a suitable substituent group. Thus, the term "substitutable ring atom" does not include ring nitrogen or carbon atoms which are shared when two rings are fused. In addition, "substitutable ring atom" does not include ring carbon or nitrogen atoms when the structure depicts that they are already attached to one or more moiety other than hydrogen and no hydrogens are available for substitution.

"Heteroatom" refers to one or more of oxygen, sulfur, nitrogen, phosphorus, or silicon, including any oxidized form of nitrogen, sulfur, phosphorus, or silicon, the quaternized form of any basic nitrogen, or a substitutable nitrogen of a heterocyclic or heteroaryl ring, for example N (as in 3,4-dihydro-2H-pyrrolyl), NH (as in pyrrolidinyl) or NR⁺ (as in N-substituted pyrrolidinyl).

In some embodiments, two independent occurrences of a variable may be taken together with the atom(s) to which each variable is bound to form a 5-8-membered, heterocyclyl, aryl, or heteroaryl ring or a 3-8-membered cycloaliphatic ring. Exemplary rings that are formed when two independent occurrences of a substituent are taken together with the atom(s) to which each variable is bound include, but are not limited to the following: a) two independent occurrences of a substituent that are bound to the same atom and are taken together with that atom to form a ring, where both occurrences of the substituent are taken together with the atom to which they are bound to form a heterocyclyl, heteroaryl, cycloaliphatic or aryl ring, wherein the group is attached to the rest of the molecule by a single point of attachment; and b) two independent occurrences of a substituent that are bound to different atoms and are taken together with both of those atoms to form a heterocyclyl, heteroaryl, cycloaliphatic or aryl ring, wherein the ring that is formed has two points of attachment with the rest of the molecule. For example, where a phenyl group is substituted with two occurrences of -OR° as in Formula D1:

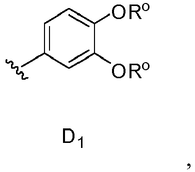

these two occurrences of -OR° are taken together with the carbon atoms to which they are bound to form a fused 6-membered oxygen containing ring as in Formula D2:

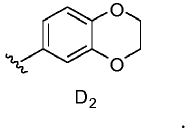

It will be appreciated that a variety of other rings can be formed when two independent occurrences of a substituent are taken together with the atom(s) to which each substituent is bound and that the examples detailed above are not intended to be limiting.

In general, the term "vicinal" refers to the placement of substituents on a group that includes two or more carbon atoms, wherein the substituents are attached to adjacent carbon atoms.

In general, the term "geminal" refers to the placement of substituents on a group that includes two or more carbon atoms, wherein the substituents are attached to the same carbon atom.

The terms "terminally" and "internally" refer to the location of a group within a substituent. A group is terminal when the group is present at the end of the substituent not further bonded to the rest of the chemical structure. Carboxyalkyl, i.e., R^{x}O(O)C-alkyl is an example of a carboxy group used terminally. A group is internal when the group is present in the middle of a substituent at the end of the substituent bound to the rest of the chemical structure. Alkylcarboxy (e.g., alkyl-C(O)O- or alkyl-O(CO)-) and alkylcarboxyaryl (e.g., alkyl-C(O)O-aryl- or alkyl-O(CO)-aryl-) are examples of carboxy groups used internally.

As described herein, a bond drawn from a substituent to the center of one ring within a multiple-ring system (as shown below), represents substitution of the substituent at any substitutable position in any of the rings within the multiple ring system. For example, formula D3 represents possible substitution in any of the positions shown in formula D4:

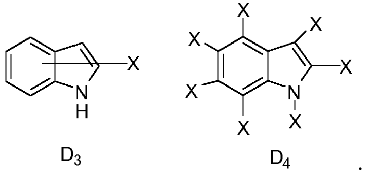

This also applies to multiple ring systems fused to optional ring systems (which would be represented by dotted lines). For example, in Formula D5, X is an optional substituent both for ring A and ring B.

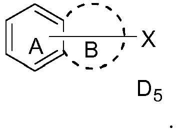

If, however, two rings in a multiple ring system each have different substituents drawn from the center of each ring, then, unless otherwise specified, each substituent only represents substitution on the ring to which it is attached. For example, in Formula D6, Y is an optional substituent for ring A only, and X is an optional substituent for ring B only.

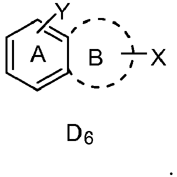

As used herein, the terms "alkoxy" or "alkylthio" refer to an alkyl group, as previously defined, attached to the molecule, or to another chain or ring, through an oxygen ("alkoxy" i.e., -O-alkyl) or a sulfur ("alkylthio" i.e., -S-alkyl) atom.

The terms Cₙ₋ₘ "alkoxyalkyl", Cₙ₋ₘ "alkoxyalkenyl", Cₙ₋ₘ "alkoxyaliphatic", and Cₙ₋ₘ "alkoxyalkoxy" mean alkyl, alkenyl, aliphatic or alkoxy, as the case may be, substituted with one or more alkoxy groups, wherein the combined total number of carbons of the alkyl and alkoxy groups, alkenyl and alkoxy groups, aliphatic and alkoxy groups or alkoxy and alkoxy groups, combined, as the case may be, is between the values of n and m. For example, a C₄₋₆ alkoxyalkyl has a total of 4-6 carbons divided between the alkyl and alkoxy portion; e.g., it can be -CH₂OCH₂CH₂CH₃, -CH₂CH₂OCH₂CH₃ or -CH₂CH₂CH₂OCH₃.

When the moieties described in the preceding paragraph are optionally substituted, they can be substituted in either or both of the portions on either side of the oxygen or sulfur. For example, an optionally substituted C₄ alkoxyalkyl could be, for instance, -CH₂CH₂OCH₂(Me)CH₃ or -CH₂(OH)O CH₂CH₂CH₃; a C₅ alkoxyalkenyl could be, for instance, -CH=CHO CH₂CH₂CH₃ or -CH=CHCH₂OCH₂CH₃.

The terms aryloxy, arylthio, benzyloxy or benzylthio, refer to an aryl or benzyl group attached to the molecule, or to another chain or ring, through an oxygen ("aryloxy", benzyloxy e.g., -O-Ph, -OCH₂Ph) or sulfur ("arylthio" e.g., -S-Ph, -S-CH₂Ph) atom. Further, the terms "aryloxyalkyl", "benzyloxyalkyl" "aryloxyalkenyl" and "aryloxyaliphatic" mean alkyl, alkenyl or aliphatic, as the case may be, substituted with one or more aryloxy or benzyloxy groups, as the case may be. In this case, the number of atoms for each aryl, aryloxy, alkyl, alkenyl or aliphatic will be indicated separately. Thus, a 5-6-membered aryloxy(C₁₋₄alkyl) is a 5-6 membered aryl ring, attached via an oxygen atom to a C₁₋₄ alkyl chain which, in turn, is attached to the rest of the molecule *via* the terminal carbon of the C₁₋₄ alkyl chain.

As used herein, the terms "halogen" or "halo" mean F, Cl, Br, or I.

The terms "haloalkyl", "haloalkenyl", "haloaliphatic", and "haloalkoxy" mean alkyl, alkenyl, aliphatic or alkoxy, as the case may be, substituted with one or more halogen atoms. For example a C₁₋₃ haloalkyl could be -CFHCH₂CHF₂ and a C₁₋₂ haloalkoxy could be -OC(Br)HCHF₂. This term includes perfluorinated alkyl groups, such as -CF₃ and -CF₂CF₃.

As used herein, the term "cyano" refers to -CN or -C≡N.

The terms "cyanoalkyl", "cyanoalkenyl", "cyanoaliphatic", and "cyanoalkoxy" mean alkyl, alkenyl, aliphatic or alkoxy, as the case may be, substituted with one or more cyano groups. For example a C₁₋₃ cyanoalkyl could be -C(CN)₂CH₂CH₃ and a C₁₋₂ cyanoalkenyl could be =CHC(CN)H₂.

As used herein, an "amino" group refers to -NH₂.

The terms "aminoalkyl", "aminoalkenyl", "aminoaliphatic", and "aminoalkoxy" mean alkyl, alkenyl, aliphatic or alkoxy, as the case may be, substituted with one or more amino groups. For example a C₁₋₃ aminoalkyl could be -CH(NH₂)CH₂CH₂NH₂ and a C₁₋₂ aminoalkoxy could be -OCH₂CH₂NH₂.

The term "hydroxyl" or "hydroxy" refers to -OH.

The terms "hydroxyalkyl", "hydroxyalkenyl", "hydroxyaliphatic", and "hydroxyalkoxy" mean alkyl, alkenyl, aliphatic or alkoxy, as the case may be, substituted with one or more -OH groups. For example a C₁₋₃ hydroxyalkyl could be -CH₂(CH₂OH)CH₃ and a C₄ hydroxyalkoxy could be -OCH₂C(CH₃)(OH)CH₃.

As used herein, a "carbonyl", used alone or in connection with another group refers to -C(O) - or -C(O)H. For example, as used herein, an "alkoxycarbonyl," refers to a group such as -C(O)O(alkyl).

As used herein, an "oxo" refers to =O, wherein oxo is usually, but not always, attached to a carbon atom (e.g., it can also be attached to a sulfur atom). An aliphatic chain can be optionally interrupted by a carbonyl group or can optionally be substituted by an oxo group, and both expressions refer to the same: e.g., -CH₂-C(O)-CH₃.

As used herein, in the context of resin chemistry (e.g., using solid resins or soluble resins or beads), the term "linker" refers to a bifunctional chemical moiety attaching a compound to a solid support or soluble support.

In all other situations, a "linker", as used herein, refers to a divalent group in which the two free valences are on different atoms (e.g., carbon or heteroatom) or are on the same atom but can be substituted by two different substituents. For example, a methylene group can be C₁ alkyl linker (-CH₂-) which can be substituted by two different groups, one for each of the free valences (e.g., as in Ph-CH₂-Ph, wherein methylene acts as a linker between two phenyl rings). Ethylene can be C₂ alkyl linker (-CH₂CH₂-) wherein the two free valences are on different atoms. The amide group, for example, can act as a linker when placed in an internal position of a chain (e.g., -CONH-). A linker can be the result of interrupting an aliphatic chain by certain functional groups or of replacing methylene units on said chain by said functional groups, e.g., a linker can be a C₁₋₆ aliphatic chain in which up to two methylene units are substituted by -C(O)- or -NH- (as in -CH₂-NH-CH₂-C(O)-CH₂- or - CH₂-NH-C(O)-CH₂-). An alternative way to define the same -CH₂-NH-CH₂-C(O)-CH₂- and - CH₂-NH-C(O)-CH₂- groups is as a C₃ alkyl chain optionally interrupted by up to two -C(O) - or -NH-moieties. Cyclic groups can also form linkers: e.g., a 1,6-cyclohexanediyl can be a linker between two R groups, as in 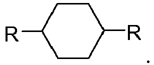 A linker can additionally be optionally substituted in any portion or position.

Divalent groups of the type R-CH= or R₂C=, wherein both free valences are in the same atom and are attached to the same substituent, are also possible. In this case, they will be referred to by their IUPAC accepted names. For instance an alkylidene (such as, for example, a methylidene (=CH₂) or an ethylidene (=CH-CH₃)) would not be encompassed by the definition of a linker in this disclosure.

The term "protecting group", as used herein, refers to an agent used to temporarily block one or more desired reactive sites in a multifunctional compound. In certain embodiments, a protecting group has one or more, or preferably all, of the following characteristics: a) reacts selectively in good yield to give a protected substrate that is stable to the reactions occurring at one or more of the other reactive sites; and b) is selectively removable in good yield by reagents that do not attack the regenerated functional group. Exemplary protecting groups are detailed in Greene, T. W. et al., "Protective Groups in Organic Synthesis", Third Edition, John Wiley & Sons, New York: 1999. The term "nitrogen protecting group", as used herein, refers to an agents used to temporarily block one or more desired nitrogen reactive sites in a multifunctional compound. Preferred nitrogen protecting groups also possess the characteristics exemplified above, and certain exemplary nitrogen protecting groups are detailed in Chapter 7 in Greene, T. W., Wuts, P. G in "Protective Groups in Organic Synthesis", Third Edition, John Wiley & Sons, New York: 1999.

As used herein, the term "displaceable moiety" or "leaving group" refers to a group that is associated with an aliphatic or aromatic group as defined herein and is subject to being displaced by nucleophilic attack by a nucleophile.

As used herein, "amide coupling agent" or "amide coupling reagent" means a compound that reacts with the hydroxyl moiety of a carboxy moiety thereby rendering it susceptible to nucleophilic attack. Exemplary amide coupling agents include DIC (diisopropylcarbodiimide), EDCI (1-ethyl-3-(3-dimethylaminopropyl)carbodiimide), DCC (dicyclohexylcarbodiimide), BOP (benzotriazol-1-yloxy-tris(dimethylamino)-phosphonium hexafluorophosphate), pyBOP ((benzotriazol-1-yloxy)tripyrrolidinophosphonium hexafluorophosphate), etc.

The compounds of the invention are defined herein by their chemical structures and/or chemical names. Where a compound is referred to by both a chemical structure and a chemical name, and the chemical structure and chemical name conflict, the chemical structure is determinative of the compound's identity.

In a first aspect, the invention is directed to compounds according to **Formula I,** or a pharmaceutically acceptable salt thereof, 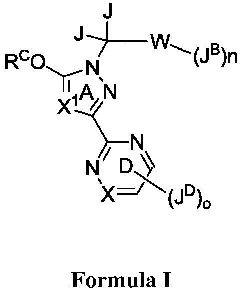
wherein X is selected from N or C;
X¹ is selected from N, CH, C(C₁₋₄ alkyl), C(C₁₋₄ fluoroalkyl), C(Cl), and CF;
W is either
   i) absent, with J^{B} connected directly to the carbon atom bearing two J groups, each J is independently selected from hydrogen, methyl or fluorine, n is 1 and J^{B} is a C₁₋₆ alkyl chain optionally substituted by up to 6 instances of fluorine; or
   ii) a ring B selected from phenyl or a 5 or 6-membered heteroaryl ring, containing 1 or 2 ring heteroatoms selected from N, O or S; wherein when W is ring B:
      each J is hydrogen;
      n is 0 or an integer selected from 1 to 3;
      and each J^{B} is independently selected from halogen, -CN, a C₁₋₆ aliphatic, -OR^{B} or a C₃₋₈ cycloaliphatic group; wherein each said C₁₋₆ aliphatic and each said C₃₋₈ cycloaliphatic group is optionally and independently substituted with up to 3 instances of R³;
      each R^{B} is independently selected from hydrogen, a C₁₋₆ aliphatic or a C₃₋₈ cycloaliphatic; wherein each said R^{B} that is a C₁₋₆ aliphatic and each said R^{B} that is a C₃₋₈ cycloaliphatic ring is optionally and independently substituted with up to 3 instances of R^{3a};
      each R³ is independently selected from halogen, -CN, C₁₋₄ alkyl, C₁₋₄ haloalkyl, -O(C₁₋₄ alkyl) or -O(C₁₋₄ haloalkyl);
      each R^{3a} is independently selected from halogen, -CN, C₁₋₄ alkyl, C₁₋₄ haloalkyl, -O(C₁₋₄ alkyl) or -O(C₁₋₄ haloalkyl);
o is 0 or an integer selected from 1 to 3;
each J^{D} is either absent or independently selected from hydrogen, halogen, -CN, -NO₂, -OR^{D}, -SR^{D}, -C(O)R^{D}, -C(O)OR^{D}, -OC(O)R^{D}, -C(O)N(R^{D})₂, -N(R^{D})₂, -N(R^{D})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂, -N(R^{d})SO₂R^{D}, a C₁₋₆aliphatic, -(C₁₋₆ aliphatic)-R^{D}, a C₃₋₈ cycloaliphatic ring, a 6 to 10-membered aryl ring, a 4 to 8-membered heterocyclic ring or a 5 to 10-membered heteroaryl ring; wherein each said 4 to 8-membered heterocyclic ring and each said 5 to 10-membered heteroaryl ring contains between 1 and 3 heteroatoms independently selected from O, N or S; and wherein each of said C₁₋₆ aliphatic chains, each said C₃₋₈ cycloaliphatic ring, each said 6 to 10-membered aryl ring, each said 4 to 8-membered heterocyclic ring and each said 5 to 10-membered heteroaryl ring is optionally and independently substituted with up to 5 instances of R⁵;
each R^{D} is independently selected from hydrogen, a C₁₋₆ aliphatic, -(C₁₋₆ aliphatic)-R^{f}, a C₃₋₈ cycloaliphatic ring, a 4 to 8-membered heterocyclic ring, phenyl; wherein each said 4 to 8-membered heterocyclic ring contains between 1 and 3 heteroatoms independently selected from O, N or S; and wherein each of said C₁₋₆ aliphatic chains, each said C₃₋₈ cycloaliphatic ring, each said 4 to 8-membered heterocyclic ring, each said phenyl is optionally and independently substituted with up to 5 instances of R^{5a}; wherein when any R^{D} is one of a C₁₋₆ aliphatic or a -(C₁₋₆ aliphatic)-R^{f} group, one or two -CH₂- units that form said C₁₋₆ aliphatic chains may, optionally, be replaced by a group independently selected from -C(O)-, -N(R^{d})- or -O- ;
each R^{d} is independently selected from hydrogen, a C₁₋₆ aliphatic, -(C₁₋₆ aliphatic)-R^{f}, a C₃₋₈ cycloaliphatic ring, a 4 to 8-membered heterocyclic ring, phenyl or a 5 to 6-membered heteroaryl ring; wherein each said 4 to 8-membered heterocyclic ring and each said 5 or 6-membered heteroaryl ring contains between 1 and 3 heteroatoms independently selected from O, N or S; and wherein each of said C₁₋₆ aliphatic chains, each said C₃₋₈ cycloaliphatic ring, each said 4 to 8-membered heterocyclic ring, each said phenyl and each said 5 to 6-membered heteroaryl ring is optionally and independently substituted by up to 5 instances of R^{5b};
each R^{f} is independently selected from a C₁₋₃ alkyl, a C₃₋₈ cycloaliphatic ring, a 4 to 8-membered heterocyclic ring, phenyl or a 5 to 6-membered heteroaryl ring; wherein each said 4 to 8-membered heterocyclic ring and each said 5 to 6-membered heteroaryl ring contains between 1 and 4 heteroatoms independently selected from O, N or S; and wherein each said C₃₋₈ cycloaliphatic ring, each said 4 to 8-membered heterocyclic ring, each said phenyl and each said 5 to 6-membered heteroaryl ring is optionally and independently substituted by up to 5 instances of R^{5c};
when J^{D} is -C(O)N(R^{D})₂, -N(R^{D})₂, -N(R^{d})C(O)N(R^{D})₂, -OC(O)N(R^{D})₂ or -SO₂N(R^{D})₂, the two R^{D} groups together with the nitrogen atom attached to the two R^{D} groups may form a 4 to 8-membered heterocyclic ring or a 5-membered heteroaryl ring; wherein each said 4 to 8-membered heterocyclic ring and each said 5-membered heteroaryl ring optionally contains up to 3 additional heteroatoms independently selected from N, O or S, in addition to the nitrogen atom to which the two R^{D} groups are attached; and wherein each said 4 to 8-membered heterocyclic ring and each said 5-membered heteroaryl ring is optionally and independently substituted by up to 5 instances of R^{5d};
when J^{D} is -N(R^{d})C(O)R^{D}, the R^{D} group together with the carbon atom attached to the R^{D} group, with the nitrogen atom attached to the R^{d} group, and with the R^{d} group may form a 4 to 8-membered heterocyclic ring or a 5-membered heteroaryl ring; wherein each said 4 to 8-membered heterocyclic ring and each said 5-membered heteroaryl ring optionally contains up to 2 additional heteroatoms independently selected from N, O or S, in addition to the nitrogen atom to which the R^{d} group is attached; and wherein each said 4 to 8-membered heterocyclic ring and each said 5-membered heteroaryl ring is optionally and independently substituted by up to 5 instances of R^{5d};
when J^{D} is -N(R^{d})C(O)OR^{D}, the R^{D} group together with the oxygen atom attached to the R^{D} group, with the carbon atom of the -C(O)- portion of the -N(R^{d})C(O)OR^{D} group, with the nitrogen atom attached to the R^{d} group, and with said R^{d} group, may form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
when J^{D} is -N(R^{d})C(O)N(R^{D})₂, one of the R^{D} groups attached to the nitrogen atom, together with said nitrogen atom, and with the N atom attached to the R^{d} group and said R^{d} group may form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
when J^{D} is -N(R^{d})SO₂R^{D}, the R^{D} group together with the sulfur atom attached to the R^{D} group, with the nitrogen atom attached to the R^{d} group, and with said R^{d} group may combine to form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
each R⁵ is independently selected from halogen, -CN, C₁₋₆ alkyl, -(C₁₋₆alkyl)-R⁶, -OR⁶, -SR⁶, -COR⁶, -OC(O)R⁶, -C(O)OR⁶, -C(O)N(R⁶)₂, -N(R⁶)C(O)R⁶, -N(R⁶)C(O)OR⁶, -N(R⁶)C(O)N(R⁶)₂, -N(R⁶)₂, -SO₂R⁶, -SO₂OH, -SO₂NHOH, -SO₂N(R⁶)₂, -SO₂N(R⁶)(CO)-R⁶, -N(R⁶)SO₂R⁶, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, saidC₇₋₁₂ aralkyl, said C₃₋₈ cycloalkyl ring, said 4 to 7-membered heterocyclic ring, said 5 or 6-membered heteroaryl ring or said phenyl group is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -CONH₂, -COO(C₁₋₄ alkyl), -O(C₁₋₄ alkyl), -O(C₁₋₄ haloalkyl) or oxo;
alternatively, two instances of R⁵ attached to the same or different atoms of J^{D}, together with said atom or atoms of J^{D} to which they are attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -NR(CO)O(C₁₋₄ alkyl), -CONH₂, -OH or halogen; wherein R is hydrogen or a C₁₋₂ alkyl;
each R^{5a} is independently selected from halogen, -CN, C₁₋₆ alkyl, -(C₁₋₆alkyl)-R^{6a}, -OR^{6a}, -SR^{6a}, -COR^{6a}, -OC(O)R^{6a}, -C(O)OR^{6a}, -C(O)N(R^{6a})₂, -N(R^{6a})C(O)R^{6a}, -N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, each said C₇₋₁₂ aralkyl, said C₃₋₈ cycloalkyl ring, said 4 to 7-membered heterocyclic ring, said 5 or 6-membered heteroaryl ring or phenyl group is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -COO(C₁₋₄ alkyl), -CONH₂, -O(C₁₋₄ alkyl), -O(C₁₋₄ haloalkyl) or oxo;
each R^{5b} is independently selected from halogen, -CN, C₁₋₆ alkyl, -(C₁₋₆alkyl)-R^{6a}, -OR^{6a}, -SR^{6a}, -COR^{6a}, -OC(O)R^{6a}, -C(O)OR^{6a}, -C(O)N(R^{6a})₂, -N(R^{6a})C(O)R^{6a}, -N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, each said C₇₋₁₂ aralkyl, said C₃₋₈ cycloalkyl ring, said 4 to 7-membered heterocyclic ring, said 5 or 6-membered heteroaryl ring or phenyl group is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -COO(C₁₋₄ alkyl), -CONH₂, -O(C₁₋₄ alkyl), -O(C₁₋₄ haloalkyl) or oxo;
alternatively, two instances of R^{5a} or two instances of R^{5b} attached to the same or different atoms of R^{D} or R^{d}, respectively, together with said atom or atoms to which they are attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -NR(CO)O(C₁₋₄ alkyl), -CONH₂, -OH or halogen; wherein R is hydrogen or a C₁₋₂ alkyl;
each R^{5c} is independently selected from halogen, -CN, C₁₋₆ alkyl, -(C₁₋₆alkyl)-R^{6b}, -OR^{6b}, -SR^{6b}, -COR^{6b}, -OC(O)R^{6b}, -C(O)OR^{6b}, -C(O)N(R^{6b})₂, -N(R^{6b})C(O)R^{6b}, -N(R^{6b})C(O)OR^{6b}, -N(R^{6b})C(O)N(R^{6b})₂, -N(R^{6b})₂, -SO₂R^{6b}, -SO₂OH, -SO₂NHOH, -SO₂N(R^{6b})(CO)-R^{6b}, -SO₂N(R^{6b})₂, -N(R^{6b})SO₂R^{6b}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, said C₇₋₁₂ aralkyl, said C₃₋₈ cycloalkyl ring, said 4 to 7-membered heterocyclic ring, said 5 or 6-membered heteroaryl ring or said phenyl groups is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -CONH₂, -COO(C₁₋₄ alkyl), -O(C₁₋₄ alkyl), -O(C₁₋₄ haloalkyl) or oxo;
alternatively, two instances of R^{5c} attached to the same or different atoms of R^{f}, together with said atom or atoms to which it is attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄haloalkoxy, oxo, -C(O)O(C₁₋₄ alkyl), -C(O)OH, -CONH₂, -NR(CO)O(C₁₋₄ alkyl), -OH or halogen; wherein R is hydrogen or a C₁₋₂ alkyl;
each R^{5d} is independently selected from halogen, -CN, C₁₋₆ alkyl, -(C₁₋₆alkyl)-R⁶, -OR⁶, -SR⁶, -COR⁶, -OC(O)R⁶, -C(O)OR⁶, -C(O)N(R⁶)₂, -N(R⁶)C(O)R⁶, -N(R⁶)C(O)OR⁶, -N(R⁶)C(O)N(R⁶)₂, -N(R⁶)₂, -SO₂OH, -SO₂NHOH, -SO₂N(R⁶)(CO)-R⁶, -SO₂R⁶, -SO₂N(R⁶)₂, -N(R⁶)SO₂R⁶, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, said C₇₋₁₂ aralkyl, said C₃₋₈ cycloalkyl ring, said 4 to 7-membered heterocyclic ring, said 5 or 6-membered heteroaryl ring or said phenyl groups is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -CONH₂, -COO(C₁₋₄ alkyl), -O(C₁₋₄ alkyl), -O(C₁₋₄ haloalkyl) or oxo;
two instances of R⁵ or two instances of R^{5d}, attached to the same or different atoms of J^{D}, together with said atom or atoms to which they are attached, may optionally form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship, wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -CONH₂, -NR(CO)O(C₁₋₄ alkyl), -OH or halogen; wherein R is hydrogen or a C₁₋₂ alkyl;
each R⁶ is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S;
each R^{6a} is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S;
each R^{6b} is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S; wherein
two instances of R⁶ linked to the same nitrogen atom of R⁵ or R^{5d}, together with said nitrogen atom of R⁵ or R^{5d}, respectively, may form a 5 to 8-membered heterocyclic ring or a 5-membered heteroaryl ring;
wherein each said 5 to 8-membered heterocyclic ring and each said 5-membered heteroaryl ring optionally contains up to 2 additional heteroatoms independently selected from N, O or S;
two instances of R^{6a} linked to a nitrogen atom of R^{5a} or R^{5b}, together with said nitrogen, may form a 5 to 8-membered heterocyclic ring or a 5-membered heteroaryl ring; wherein each said 5 to 8-membered heterocyclic ring and each said 5-membered heteroaryl ring optionally contains up to 2 additional heteroatoms independently selected from N, O or S;
two instances of R^{6b} linked to a nitrogen atom of R^{5c}, together with said nitrogen, may form a 5 to 8-membered heterocyclic ring or a 5-membered heteroaryl ring; wherein each said 5 to 8-membered heterocyclic ring and each said 5-membered heteroaryl ring optionally contains up to 2 additional heteroatoms independently selected from N, O or S;
alternatively, two J^{D} groups attached to two vicinal ring D atoms, taken together with said two vicinal ring D atoms, may form a 5 to 7-membered heterocycle or a 5-membered heteroaryl ring that is fused to ring D; wherein said 5 to 7-membered heterocycle or said 5-membered ring heteroaryl contains from 1 to 3 heteroatoms independently selected from N, O or S; and wherein said 5 to 7-membered heterocycle or said 5-membered heteroaryl ring is optionally and independently substituted by up to 3 instances of oxo or -(Y)-R⁹;
wherein Y is either absent or is a C₁₋₆ alkyl chain, optionally substituted by up to 6 instances of fluoro; and wherein when Y is said C₁₋₆ alkyl chain, up to 3 methylene units of this alkyl chain, can be replaced by a group selected from -O-, -C(O)- or -N((Y¹)-R⁹⁹)-;
Y¹ is either absent or a C₁₋₆ alkyl chain, optionally substituted by up to 6 instances of fluoro;
when Y¹ is absent, each R⁹⁹ is independently selected from hydrogen, C₁₋₆ alkyl optionally substituted with up to 9 fluorine atoms, -COR¹⁰, -C(O)OR¹⁰, -C(O)N(R¹⁰)₂, -C(O)N(R¹⁰)SO₂R¹⁰, -SO₂R¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, a C₃₋₆ cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
when Y¹ is present, each R⁹⁹ is independently selected from hydrogen, halogen, -CN, C₁₋₆ alkyl optionally substituted with up to 9 fluorine atoms, -COR¹⁰, -OR¹⁰, -OC(O)R¹⁰, -C(O)OR¹⁰, -C(O)N(R¹⁰)₂, -C(O)N(R¹⁰)SO₂R¹⁰, -SO₂R¹⁰, -SOR¹⁰, -SR¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, a C₃₋₆ cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
each R⁹ is independently selected from hydrogen, -CN, -OR¹⁰, -COR¹⁰, -OC(O)R¹⁰, -C(O)OR¹⁰, -C(O)N(R¹⁰)₂, -C(O)N(R¹⁰)SO₂R¹⁰ -N(R¹⁰)C(O)R¹⁰, -N(R¹⁰)C(O)OR¹⁰, -N(R¹⁰)C(O)N(R¹⁰)₂, -N(R¹⁰)₂, -SO₂R¹⁰, -SO₂N(R¹⁰)₂, -SO₂N(R¹⁰)COOR¹⁰, -SO₂N(R¹⁰)C(O)R¹⁰, -N(R¹⁰)SO₂R¹⁰, -SO₂OH, -SO₂NHOH, -SO₂N(R¹⁰)(CO)-R¹⁰, a C₃₋₆ cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
each R¹⁰ is independently selected from hydrogen, a C₁₋₆ alkyl, -(C₁₋₆ alkyl)-R¹³, phenyl, benzyl, a C₃₋₆ cycloalkyl ring, a 4 to 7-membered heterocyclic ring or a 5 or 6-membered heteroaryl ring, wherein each 5 or 6-membered heteroaryl ring or 4 to 7-membered heterocyclic ring contains up to 4 ring heteroatoms independently selected from N, O and S; and wherein each of said C₁₋₆ alkyl, each said phenyl, each said benzyl, each said C₃₋₈ cycloalkyl group, each said 4 to 7-membered heterocyclic ring and each 5 or 6-membered heteroaryl ring is optionally and independently substituted with up to 3 instances of R^{11b};
each R¹³ is independently selected from a phenyl, a benzyl, a C₃₋₆ cycloalkyl ring, a 4 to 7-membered heterocyclic ring or a 5 or 6-membered heteroaryl ring, wherein each 5 or 6-membered heteroaryl ring or 4 to 7-membered heterocyclic ring contains up to 4 ring heteroatoms independently selected from N, O and S; and wherein each said phenyl, each of said benzyl, each said C₃₋₈ cycloalkyl group, each said 4 to 7-membered heterocyclic ring and each 5 or 6-membered heteroaryl ring is optionally and independently substituted with up to 3 instances of R^{11c};
each R^{11a} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹² )₂, -N(R¹² )₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹;
each R^{11b} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹² )₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹; and
each R^{11c} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹² )₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹;
each R¹² is selected from hydrogen, C₁₋₄ alkyl, C₁₋₄ (fluoroalkyl), -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ fluoroalkyl) or oxo;
each R¹²¹ is selected from C₁₋₄ alkyl, C₁₋₄ (fluoroalkyl), -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ fluoroalkyl) or oxo;
R^{C} is selected from hydrogen, C₁₋₆ aliphatic, -(C₁₋₆ alkyl)-R^{N}, a 5 or 6-membered heteroaryl, phenyl, a 4 to 7 membered heterocyclic, a C₃₋₈ cycloaliphatic, -C(O)R⁷, -C(O)OR⁷, -C(O)N(R⁷)₂, and -C(O)N(R⁷)SO₂R⁷ ; wherein each of said 5 or 6-membered heteroaryl ring and 4 to 7-membered heterocyclic ring contains up to 4 ring heteroatoms independently selected from N, O and S; wherein each said C₁₋₆ aliphatic and each C₁₋₆ alkyl portion of said -(C₁₋₆ alkyl)-R^{N}, is optionally and independently substituted with up to 6 instances of halogen and up to 2 instances of-CN, -COOR⁸, -OR⁸, oxo, -N(R⁸)₂, -C(O)N(R⁸)₂, -N(R⁸)C(O)R⁸, -N(R⁸)C(O)OR⁸, -N(R⁸)C(O)N(R⁸)₂, -SO₂R⁸, -SO₂N(R⁸)₂, -NHOR⁸, -SO₂N(R⁸)COOR⁸, -SO₂N(R⁸)C(O)R⁸ and -N(R⁸)SO₂R⁸;
wherein each R⁷ is independently selected from hydrogen, C₁₋₆ alkyl, C₁₋₆ fluoroalkyl, a C₃₋₈ cycloalkyl ring, phenyl, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo;
each R⁸ is independently selected from hydrogen, C₁₋₆ alkyl, C₁₋₆ fluoroalkyl, a C₃₋₈ cycloalkyl ring, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo;
each R^{N} is independently selected from a phenyl ring, a monocyclic 5 or 6-membered heteroaryl ring, a monocyclic C₃₋₆ cycloaliphatic ring, or a monocyclic 4 to 6-membered heterocycle; wherein said monocyclic 5 or 6-membered heteroaryl ring or said monocyclic 4 to 6-membered heterocycle contain between 1 and 4 heteroatoms selected from N, O or S; wherein said monocyclic 5 or 6-membered heteroaryl ring is not a 1,3,5-triazinyl ring; and wherein said phenyl, said monocyclic 5 to 6-membered heteroaryl ring, said monocyclic C₃₋₆ cycloaliphatic ring, or said monocyclic 4 to 6-membered heterocycle is optionally and independently substituted with up to 6 instances of fluoro and/or up to 3 instances of J^{M};
each J^{M} is independently selected from -CN, a C₁₋₆ aliphatic, -OR^{M}, -SR^{M}, -N(R^{M})₂, a C₃₋₈ cycloaliphatic ring or a 4 to 8-membered heterocyclic ring; wherein said 4 to 8-membered heterocyclic ring contains 1 or 2 heteroatoms independently selected from N, O or S; wherein each said C₁₋₆ aliphatic, each said C₃₋₈ cycloaliphatic ring and each said 4 to 8-membered heterocyclic ring, is optionally and independently substituted with up to 3 instances of R^{7c}; and
each R^{M} is independently selected from hydrogen, a C₁₋₆ aliphatic, a C₃₋₈ cycloaliphatic ring or a 4 to 8-membered heterocyclic ring; wherein each said 4 to 8-membered heterocylic ring contains between 1 and 3 heteroatoms independently selected from O, N or S;
each R^{7c} is independently selected from halogen, -CN, -NO₂, C₁₋₄ alkyl, C₁₋₄ haloalkyl, C₃₋₈ cycloalkyl ring, -OR^{8b}, -SR^{8b}, -N(R^{8b})₂, -C(O)O(C₁₋₄ alkyl), -C(O)OH, -NR(CO)CO(C₁₋₄ alkyl) or an oxo group; wherein each said cycloalkyl group is optionally and independently substituted with up to 3 instances of halogen;
each R^{8b} is independently selected from hydrogen, C₁₋₆ alkyl, C₁₋₆ fluoroalkyl, a C₃₋₈ cycloalkyl ring, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo;
provided that the compound is not one represented by the general structure: 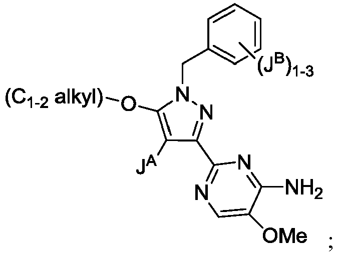
wherein J^{A} is either hydrogen or C₁₋₄ alkyl; and J^{B} is either halogen or C₁₋₄ (alkoxy).

In some embodiments of the compounds of **Formula I,** W is absent. In some of these embodiments, the compound is a compound of **Formula IIa:** 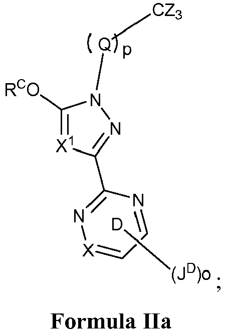 wherein Q represents a -CZ₂- group; each Z is independently selected from hydrogen or fluorine; and p is an integer selected from 1, 2, 3, 4 and 5. In some embodiments of **Formula IIa** up to 5 instances of Z are fluorine and the remaining instances of Z are hydrogen.

In other embodiments, the compound is a compound of **Formula IIIa:**

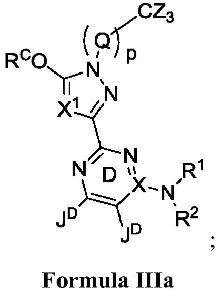

In some embodiments of the compounds of **Formula IIIa,** X is N, and the moiety -N(R¹)(R²) is absent. In some embodiments of the compounds of **Formula IIIa,** X is C, and the moiety -N(R¹)(R²) is present. In some of these embodiments:
R¹ and R², together with the nitrogen atom to which they are attached, form a 4 to 8-membered heterocyclic ring or 5-membered heteroaryl ring; wherein said 4 to 8-membered heterocyclic ring or 5-membered heteroaryl ring optionally contains, in addition to the nitrogen atom to which both R¹ and R² are attached, up to 3 ring heteroatoms independently selected from N, O or S, and is optionally substituted by up to 5 instances of R^{5e};
each R^{5e} is independently selected from halogen, -CN, C₁₋₆ alkyl, -(C₁₋₄ alkyl)-R⁶, a C₃₋₈ cycloalkyl ring, C₁₋₄ (cyanoalkyl), -OR⁶, -SR⁶, -OCOR⁶, -COR⁶, -C(O)OR⁶, -C(O)N(R⁶)₂, -N(R⁶)C(O)R⁶, -N(R⁶)₂, -SO₂R⁶, -SO₂N(R⁶)₂, -N(R⁶)SO₂R⁶, -SO₂OH, -SO₂NHOH, -SO₂N(R⁶)(CO)-R⁶, benzyl, phenyl or an oxo group; wherein each said phenyl ring and each said benzyl group, is optionally and independently substituted with up to 3 instances of halogen, -OH, -NH₂, -NH(C₁₋₄ alkyl), -N(C₁₋₄ alkyl)₂, -CN, C₁₋₄ alkyl, C₁₋₄ haloalkyl, -CONH₂, -O(C₁₋₄ alkyl) or -O(C₁₋₄ haloalkyl); and wherein each said C₁₋₆ alkyl or C₁₋₄ alkyl chains and each said C₃₋₈ cycloalkyl ring is optionally and independently substituted with up to 3 instances of halogen; wherein
each R⁶ is independently selected from hydrogen, a C₁₋₆ alkyl, a C₂₋₄ alkenyl, phenyl, benzyl, or a C₃₋₈ cycloalkyl ring; wherein each said C₁₋₆ alkyl, each said C₂₋₄ alkenyl, each said phenyl, each said benzyl and each said C₃₋₈ cycloalkyl group is optionally and independently substituted with up to 3 instances of halogen;
alternatively, two of the instances of R^{5e} attached to the same or different atoms of said ring formed by R¹, R² and the nitrogen to which R¹ and R² are attached, together with said atom or atoms, optionally form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship, wherein said 4 to 6-membered heterocycle or said 5 or 6-membered heteroaryl ring contains up to three ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -CONH₂, -C(O)OH, -NR(CO)O(C₁₋₄ alkyl), -OH or halogen; wherein R is hydrogen or a C₁₋₂ alkyl;
In some of these embodiments, alternatively, R¹ and R² are each independently selected from hydrogen, C₁₋₆ alkyl, a C₃₋₈ cycloalkyl ring, a 4 to 8-membered heterocyclic ring, a 5 or 6-membered heteroaryl, phenyl or a C₁₋₆ alkyl-R^{Y}; wherein each of said 4 to 8-membered heterocyclic ring and each of said 5 or 6-membered heteroaryl ring contains up to 3 ring heteroatoms independently selected from N, O and S; and wherein each of said C₁₋₆ alkyl, C₃₋₈ cycloalkyl ring, 4 to 8-membered heterocyclic ring group, 5 or 6-membered heteroaryl, phenyl and C₁₋₆ alkyl-R^{Y} is optionally and independently substituted with up to 5 instances of R^{5f};
R^{Y} is selected from a C₃₋₈ cycloalkyl ring, a 4 to 8-membered heterocyclic ring, phenyl, or a 5 to 6-membered heteroaryl ring; wherein each of said 4 to 8-membered heterocyclic ring or 5 to 6-membered heteroaromatic ring contains between 1 and 4 ring heteroatoms independently selected from N, O or S; and wherein each of said C₃₋₈ cycloalkyl ring, each of said 4 to 8-membered heterocyclic ring, each of said phenyl, and each of said 5 to 6-membered heteroaryl ring is optionally substituted with up to 5 instances of R^{5g};
each R^{5f} is independently selected from halogen, -CN, C₁₋₆ alkyl, -(C₁₋₄ alkyl)-R^{6a}, a C₇₋₁₂ aralkyl, C₃₋₈ cycloalkyl ring, C₁₋₄ (cyanoalkyl), -OR^{6a}, -SR^{6a}, -OCOR^{6a}, -COR^{6a}, -C(O)OR^{6a}, -C(O)N(R^{6a})₂, -N(R^{6a})C(O)R^{6a}, -N(R^{6a})₂, -SO₂R^{6a}, -SO₂N(R^{6a})₂, -N(R^{6a})SO₂R^{6a}, phenyl or an oxo group; wherein each said phenyl group is optionally and independently substituted with up to 3 instances of halogen, -OH, -NH₂, -NH(C₁₋₄ alkyl), -N(C₁₋₄ alkyl)₂, -NO₂, -CN, C₁₋₄ alkyl, C₁₋₄ haloalkyl, -O(C₁₋₄ alkyl) or -O(C₁₋₄ haloalkyl); and wherein each said C₇₋₁₂ aralkyl, each said C₁₋₄ alkyl chain and each said C₃₋₈ cycloalkyl group is optionally and independently substituted with up to three instances of halogen;
each R^{6a} is independently selected from hydrogen, a C₁₋₆ alkyl, a C₂₋₄ alkenyl, phenyl, benzyl, or a C₃₋₈ cycloalkyl ring; wherein each said C₁₋₆ alkyl, each said C₂₋₄ alkenyl, each said phenyl, each said benzyl and each said C₃₋₈ cycloalkyl group is optionally and independently substituted with up to 3 instances of halogen;
each R^{5g} is independently selected from halogen, -CN, C₁₋₆ alkyl, -(C₁₋₄ alkyl)-R^{6b}, a benzyl, C₃₋₈ cycloalkyl ring, C₁₋₄ (cyanoalkyl), -OR^{6b}, -SR^{6b}, -OCOR^{6b}, -COR^{6b}, -C(O)OR^{6b}, -C(O)N(R^{6b})₂, -N(R^{6b})C(O)R^{6b}, -N(R^{6b})₂, -SO₂R^{6b}, -SO₂OH, -SO₂NHOH, -SO₂N(R^{6b})(CO)-R^{6b}, -SO₂N(R^{6b})₂, -N(R^{6b})SO₂R^{6b}, phenyl or an oxo group; wherein each said phenyl and each said benzyl group is optionally and independently substituted with up to 3 instances of halogen, -OH, -NH₂, -NH(C₁₋₄ alkyl), -N(C₁₋₄ alkyl)₂, -NO₂, -CN, C₁₋₄ alkyl, C₁₋₄ haloalkyl, -O(C₁₋₄ alkyl) or -O(C₁₋₄ haloalkyl); and wherein each said C₁₋₄ alkyl chain and each said C₃₋₈ cycloalkyl group is optionally and independently substituted with up to 3 instances of halogen;
each R^{6b} is independently selected from hydrogen, a C₁₋₆ alkyl, a C₂₋₄ alkenyl, phenyl, benzyl, or a C₃₋₈ cycloalkyl ring; wherein each said C₁₋₆ alkyl, each said C₂₋₄ alkenyl, each said phenyl, each said benzyl and each said C₃₋₈ cycloalkyl group is optionally and independently substituted with up to 3 instances of halogen.

In some embodiments, alternatively, two instances of R^{5g} attached to the same or different ring atoms of R^{Y}, together with said ring atom or atoms, form a C₃₋₈ cycloalkyl ring, a 4 to 6-membered heterocyclic ring; a phenyl or a 5 or 6-membered heteroaryl ring, resulting in a bicyclic system wherein the two rings are in a spiro, fused or bridged relationship, wherein said 4 to 6-membered heterocycle or said 5 or 6-membered heteroaryl ring contains up to three heteroatoms independently selected from N, O or S; and wherein said C₃₋₈ cycloalkyl ring, 4 to 6-membered heterocyclic ring, phenyl or a 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -NR"(CO)CO(C₁₋₄ alkyl), -OH or halogen; and R" is hydrogen or a C₁₋₂ alkyl.

In those embodiments when one of R¹ or R² is the C₃₋₈ cycloalkyl ring, 4 to 8-membered heterocyclic ring or 5 or 6-membered heteroaryl substituted with up to 5 instances of R^{5f}, two of the instances of R^{5f} attached to the same or different ring atoms of said R¹ or R², together with said atom or atoms, form a C₃₋₈ cycloalkyl ring, a 4 to 6-membered heterocyclic ring, a phenyl or a 5 or 6-membered heterocyclic ring, resulting in a bicyclic system wherein the two rings are in a spiro, fused or bridged relationship, wherein said 4 to 6-membered heterocycle or said 5 or 6-membered heterocyclic ring contains up to two ring heteroatoms independently selected from N, O or S; and wherein said C₃₋₈ cycloalkyl ring, 4 to 6-membered heterocyclic ring, phenyl or 5 or 6-membered heterocyclic ring is optionally substituted by up to 2 instances of C₁₋₄ alkyl, C₁₋₄ haloalkyl, oxo, -(CO)CO(C₁₋₄ alkyl), -NR'(CO)CO(C₁₋₄ alkyl) or halogen; wherein R' is hydrogen or a C₁₋₂ alkyl.

In some embodiments of the compounds **of Formulae I, IIa and IIIa,** X is N. In other embodiments, X is C.

In other embodiments of the compounds of **Formula I,** the compounds have **Formula IVa:** 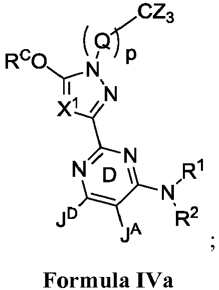 wherein J^{D} is absent or is selected from halogen, methyl, hydroxyl, methoxy, trifluoromethyl, trifluoromethoxy or -NR^{a}R^{b}; in some of these embodiments, R^{a} and R^{b} are each independently selected from hydrogen, C₁₋₆ alkyl or a 3-6 cycloalkyl ring; alternatively, R^{a} and R^{b}, together with the nitrogen atom to which they are both attached, may form a 4-8 membered heterocyclic ring, or a 5-membered heteroaryl ring optionally containing up to two additional heteroatoms selected from N, O and S; wherein each of said 4-8 membered heterocyclic ring and 5-membered heteroaryl ring is optionally and independently substituted by up to 5 instances of fluorine; J^{A} is selected from hydrogen or fluorine; and R¹ and R² are as defined *supra.*

In other embodiments of the compounds of **Formula I,** the compounds of the invention are compounds of **Formula IIb,** or pharmaceutically acceptable salts thereof: 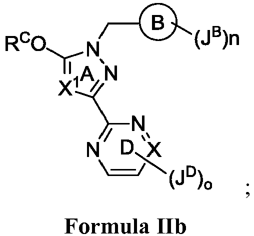 In some of these embodiments, ring B is a phenyl. In other embodiments, ring B is a 5 or 6-membered heteroaryl ring, containing 1 or 2 ring heteroatoms selected from N, O or S.

In some embodiments of the compounds of **Formula IIb,** X is C. In some of these embodiments, X is optionally substituted by J^{D}. In other embodiments of the compounds of **Formula IIb,** X is N.

In some embodiments of the compounds of **Formula IIb,** each J^{D} is independently selected from halogen, a C₁₋₆ aliphatic, C₁₋₆ haloaliphatic, -N(R^{D})₂, -N(R^{d})COR^{D}, -N(R^{d})COOR^{D}, -OR^{D}, -N(R^{d})SO₂R^{D} oxo or an optionally substituted C₃₋₈ cycloaliphatic ring. In other embodiments, o is 2 and each J^{D} is independently selected from a halogen atom or -N(R^{D})₂, -N(R^{d})COR^{D}, -OH, -N(R^{d})COOR^{D}, or -N(R^{d})SO₂R^{D}. In still other embodiments, o is 2 and one instance of J^{D} is fluoro or chloro and the other instance of J^{D} is -OH. In yet other embodiments, o is 2 and one instance of J^{D} is -NH₂ and the other one is independently selected from -N(R^{D})₂, wherein at least one instance of R^{D} is not hydrogen; or is -NHCOR^{D}, -N(R^{d})COOR^{D} or -N(R^{d})SO₂R^{D}. In further embodiments, o is 2 and one instance of J^{D} is independently selected from -N(R^{D})₂ or -NHCOR^{D} and the other instance of J^{D} is selected from fluoro or chloro. In further embodiments, o is 1 and J^{D} is amino.

In some embodiments of the compounds of **Formula I,** the compounds are compounds of **Formula IIIb:**

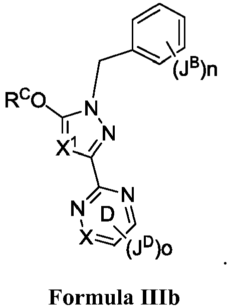

In other embodiments of the compounds of **Formula I,** the compounds have **Formula IVb:**

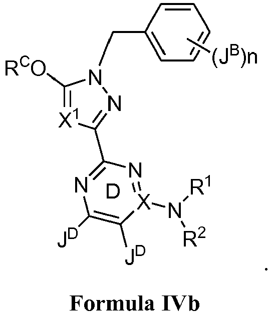

In some embodiments of the compounds of **Formula IVb,** X is N; in these embodiments, -NR¹R² is absent. In other embodiments, X is C.

In other embodiments of the compounds of **Formula I,** the compounds are represented by **Formula Vb:** 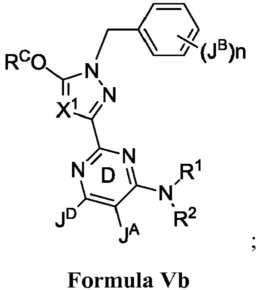 wherein, J^{D} is absent or is selected from halogen, methyl, hydroxyl, methoxy, trifluoromethyl, trifluoromethoxy or -NR^{a}R^{b}; in some of these embodiments, R^{a} and R^{b} are each independently selected from hydrogen, C₁₋₆ alkyl or a 3-6 cycloalkyl ring; alternatively, R^{a} and R^{b}, together with the nitrogen atom to which they are both attached, may form a 4-8 membered heterocyclic ring, or a 5-membered heteroaryl ring optionally containing up to two additional heteroatoms selected from N, O and S; wherein each of said 4-8 membered heterocyclic ring and 5-membered heteroaryl ring is optionally and independently substituted by up to 5 instances of fluorine; and J^{A} is selected from hydrogen or fluorine.

In other embodiments of the compounds of **Formula IIb,** ring B is phenyl or a 6-membered heteroaryl ring. In some of these embodiments, n is an integer selected from 1, 2, or3 and each J^{B} is independently selected from halogen, a C₁₋₆ aliphatic or -OR^{B}. In other embodiments, each J^{B} is independently selected from halogen. In still other embodiments, each J^{B} is independently selected from fluoro or chloro. In yet other embodiments, each J^{B} is fluoro. In further embodiments, each J^{B} is methyl or ethyl.

In other embodiments of the compounds of **Formulae IIb, IIIb, IVb or Vb,** n is 1. In some of these embodiments, J^{B} is selected from halogen. In other embodiments, J^{B} is fluoro or chloro. In still other embodiments, J^{B} is fluoro.

In further embodiments of **Formulae IIb, IIIb, IVb and VB,** at least one J^{B} is ortho to the attachment of the methylene linker between ring B and ring A. In some of these embodiments, each J^{B} is independently selected from halogen. In other embodiments, each J^{B} is independently selected from fluoro or chloro. In still other embodiments, each J^{B} is fluoro. In some of the embodiments wherein n is 1, the J^{B} ortho to the attachment of the methylene linker between ring B and ring A is fluoro.

In other embodiments of the compounds of **Formulae IIb, IIIb, IVb and Vb,** ring B is a 6-membered heteroaryl ring. In some of these embodiments, B is a pyridyl ring. In other of these embodiments, ring B is a pyrimidinyl ring.

In some embodiments of any one of the **Formulae** depicted above, o is an integer selected from 1, 2, or 3. In some of these embodiments, each J^{D} is independently selected from halogen, a C₁₋₆ aliphatic, C₁₋₆ haloaliphatic, -N(R^{D})₂, -N(R^{d})C(O)R^{D}, -N(R^{d})C(O)OR^{D}, -N(R^{d})C(O)N(R^{D})₂, -SO₂R^{D}, -SO₂N(R^{D})₂, -N(R^{d})SO₂R^{D}, -SR^{D}, -OR^{D} or an optionally substituted C₃₋₈ cycloaliphatic ring. In other embodiments, each J^{D} is independently selected from methyl, trifluoromethyl, chloro, fluoro, -N(R^{D})₂, N(R^{d})C(O)R^{D}, -N(R^{d})SO₂R^{D}, or -OR^{D}. In some of these embodiments, R^{d} is independently selected from hydrogen or C₁₋₄ alkyl. In yet other embodiments, o is 1 or 2 and at least one instance of J^{D} is independently selected from fluoro, chloro, hydroxyl and amino. In further embodiments, o is an integer selected from 1 or 2.

In some embodiments of the compounds of **Formula** I, compounds have **Formulae VIb, VIIb, Va or VIa:** 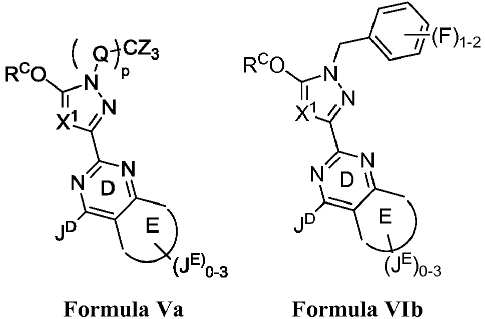 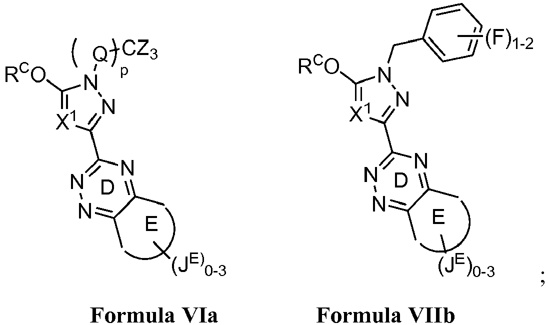 wherein ring E is a 5 or 6-membered heterocyclic ring, containing up to 3 heteroatoms selected from N, O and S; and wherein each J^{E} is independently selected from oxo or -(Y)-R⁹. In some of these embodiments, J^{D} is absent or is selected from halogen, -NH₂, or -OH. In other embodiments, ring E is a heterocyclic ring containing one nitrogen ring atom and at least one instance of J^{E} is oxo. In still other embodiments, one J^{E} is oxo and two other instances of J^{E} are independently selected from -(Y)-R⁹. In further embodiments, each -(Y)-R⁹ is independently selected from a C₁₋₆ alkyl; a 5 or 6-membered heteroaryl ring containing between 1 and 3 heteroatoms independently selected from N, O or S and optionally substituted by one or more instances of C₁₋₆ alkyl or halogen; and -(CO)NH-R¹⁰. In further embodiments, R¹⁰ is a C₃₋₆ cycloalkyl ring.

In other embodiments of the compounds of **Formula I,** the compounds of the invention are represented by one of **Formulae VIIa** or **VIIIb:**

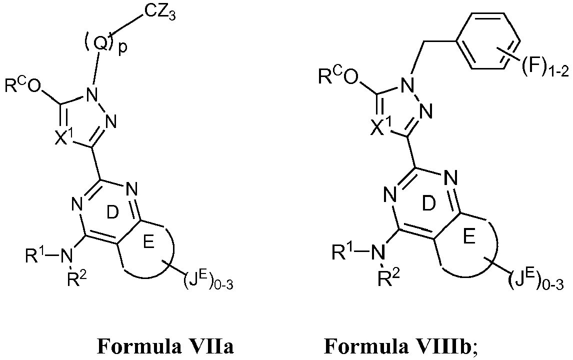

In some embodiments of the compounds of **Formulae VIIa or VIIIb,** one instance of J^{E} is oxo and two other instances of J^{E} are independently selected from C₁₋₆ alkyl; a 5 or 6-membered heteroaryl ring, containing between 1 and 3 heteroatoms independently selected from N, O, or S and optionally substituted by one or more instances of C₁₋₆ alkyl or halogen; and -(CO)NH-R¹⁰. In some of these embodiments, R¹⁰ is a C₃₋₆ cycloalkyl ring.

In some embodiments of the compounds of **Formula I,** the compounds of the invention are represented by one of **Formulae VIIIa** or **XIXb:** 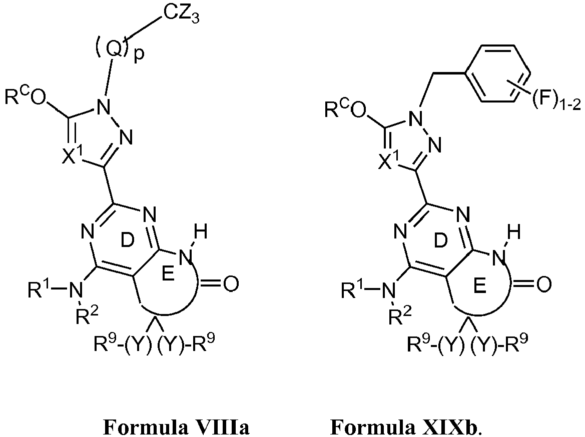 In these embodiments, both -(Y)-R⁹ substituents are attached to any ring carbon anywhere on the ring, provided that both -(Y)-R⁹ substituents are attached to the same ring carbon.

In other embodiments of the compounds of **Formula I,** the compounds of the invention are represented by one of **Formulae XIXa, Xa, Xb, XIb:** 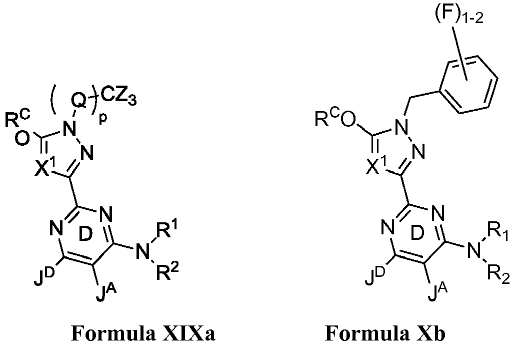 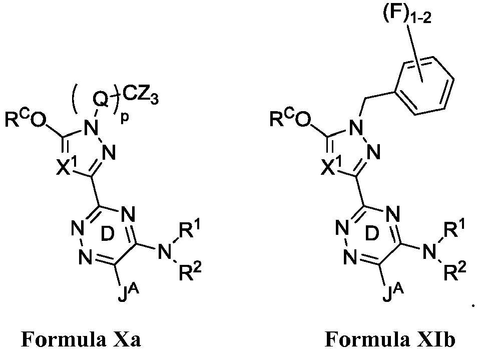 In these embodiments, each J^{D} is independently selected from -NH₂ or is absent; and wherein each J^{A} is alternatively: i) when R¹ and R² are not simultaneously hydrogen, each J^{A} is independently selected from hydrogen or halogen; or ii) when R¹ and R² are both simultaneously hydrogen, each J^{A} is independently selected from -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂ or -N(R^{d})SO₂R^{D}. In some of these embodiments, J^{A} is -NH₂, -OH, or hydrogen.

In some embodiments, R^{C} is C₁₋₆ aliphatic optionally substituted with up to 6 instances of fluoro. In some embodiments, R^{C} is C₁₋₆ alkyl optionally substituted with up to 6 instances of fluoro. In other embodiments, R^{C} is ethyl or methyl; the ethyl or methyl may be optionally substituted with up to 5 instances of fluoro. In still other embodiments, R^{C} is a C₃₋₆ cycloaliphatic, optionally substituted with up to 4 instances of fluoro.

In another embodiment, the compound is selected from those depicted in Table I below:

**Table I**

| | | |
|---|---|---|
| 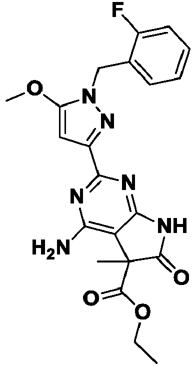 | 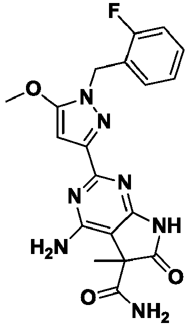 | 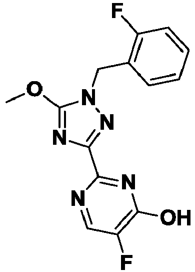 |
| I-1 | I-2 | I-3 |
| 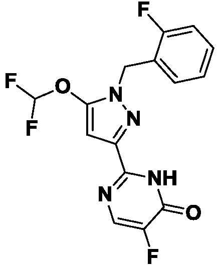 | 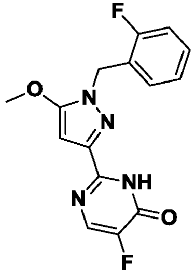 | 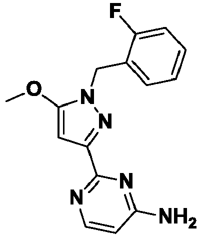 |
| I-4 | I-5 | I-6 |
| 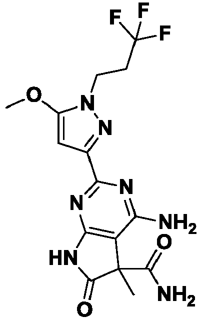 | 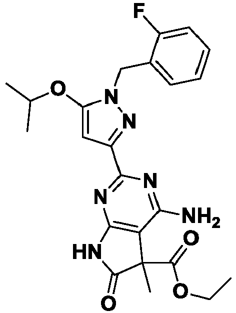 | 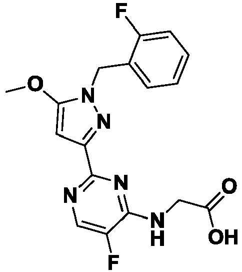 |
| I-7 | I-8 | I-9 |
| 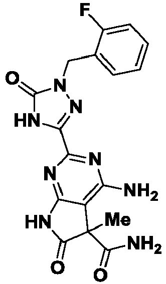 | 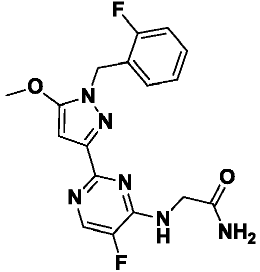 | 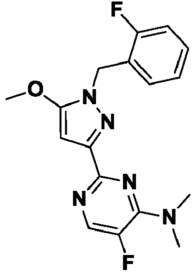 |
| I-10 | I-11 | I-12 |
| 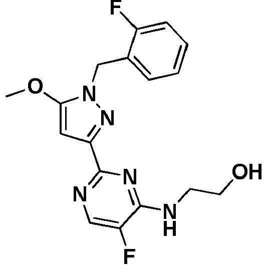 | 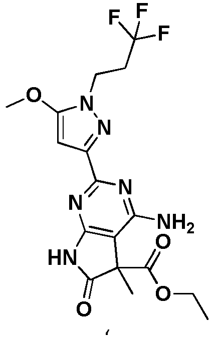 | 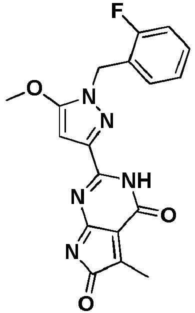 |
| I-13 | I-14 | I-15 |
| 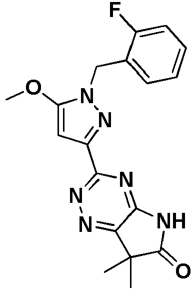 | 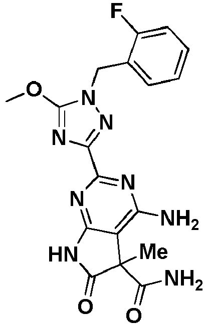 | |
| I-16 | I-17 | |
| 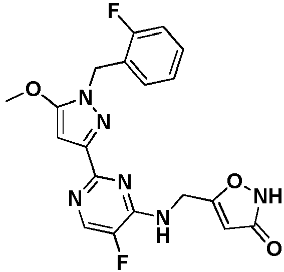 | 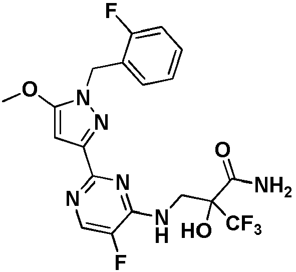 | 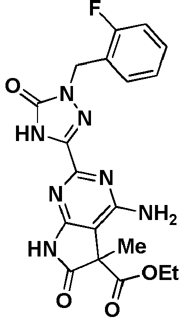 |
| I-19 | I-20 | I-21 |
| 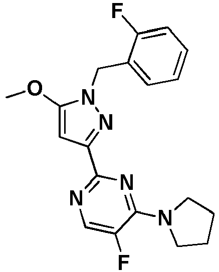 | 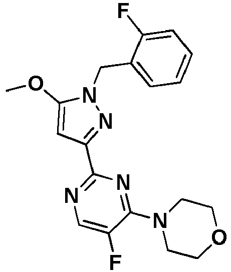 | 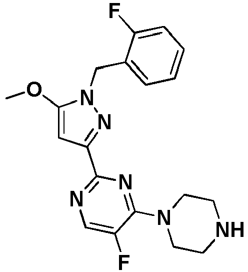 |
| I-22 | I-23 | I-24 |
| | 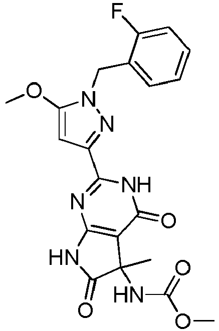 | 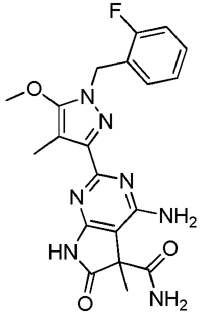 |
| | I-26 | I-27 |
| 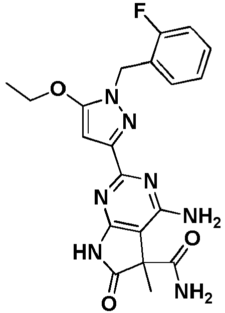 | | 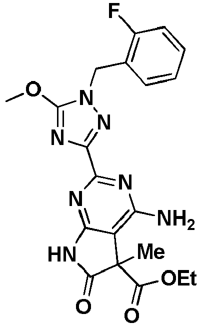 |
| I-28 | | I-30 |
| 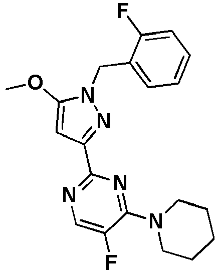 | 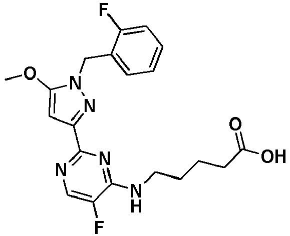 | 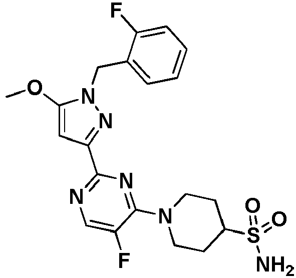 |
| I-31 | I-32 | I-33 |
| 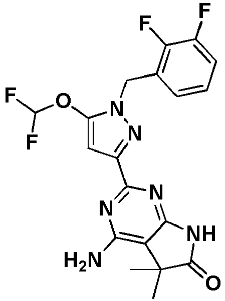 | 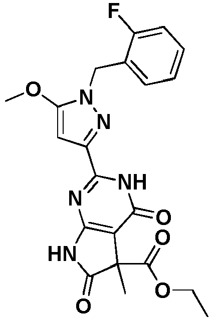 | 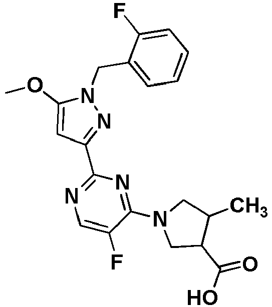 |
| I-34 | I-35 | I-36 |
| 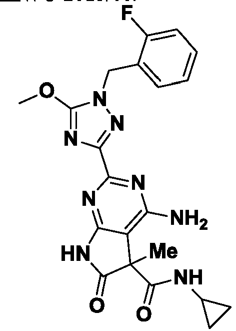 | 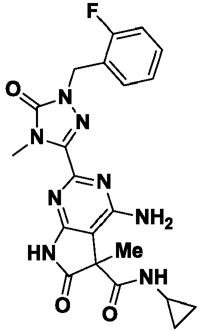 | 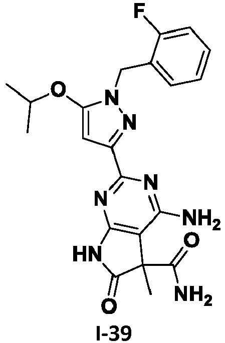 |
| I-37 | I-38 | |
| 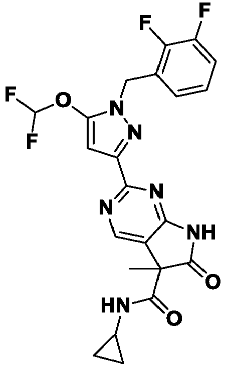 | 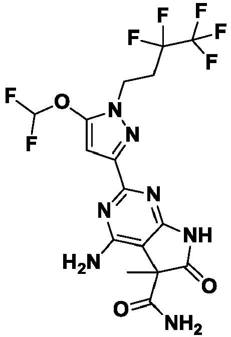 | 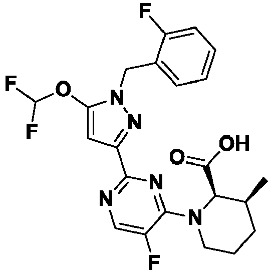 |
| I-40 | I-41 | I-42 |
| 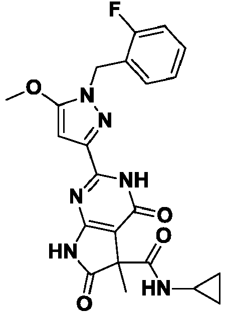 | 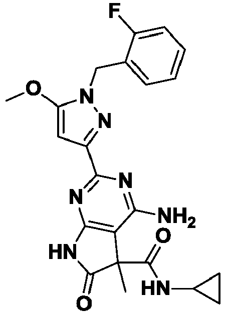 | 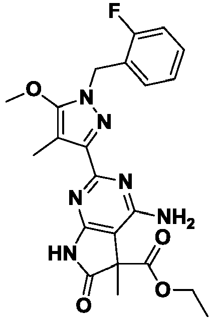 |
| I-43 | I-44 | I-45 |
| 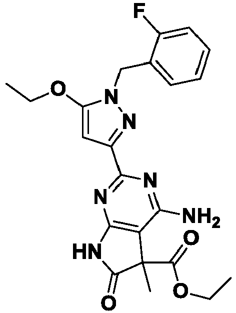 | 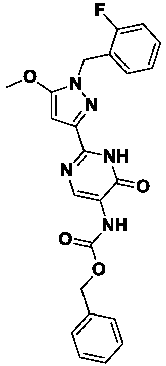 | 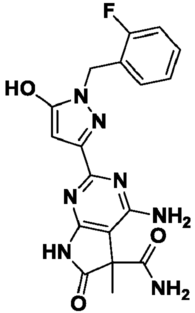 |
| I-46 | I-47 | I-48 |
| 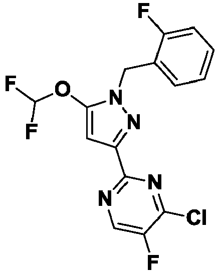 | 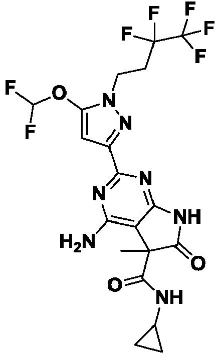 | 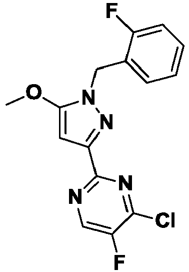 |
| I-49 | I-50 | I-51 |
| 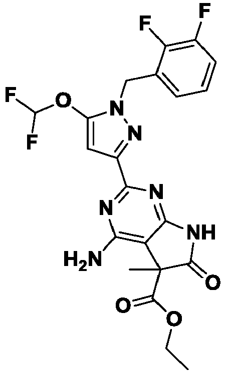 | 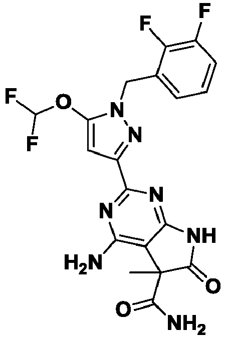 | 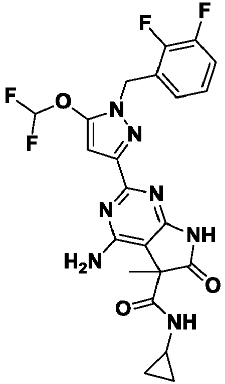 |
| I-52 | I-53 | I-54 |
| 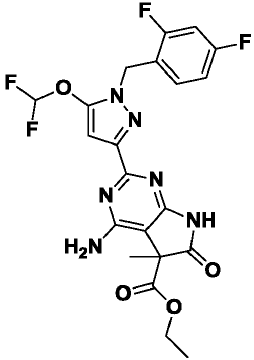 | 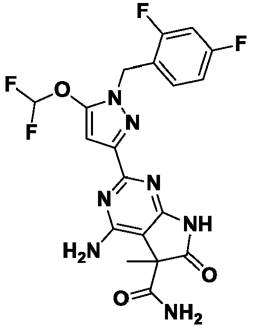 | 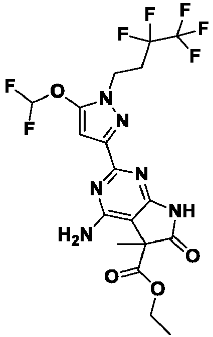 |
| I-55 | I-56 | I-57 |
| 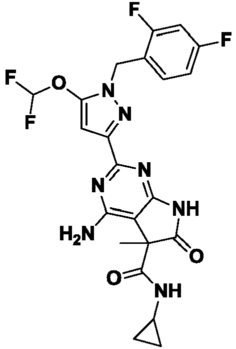 | 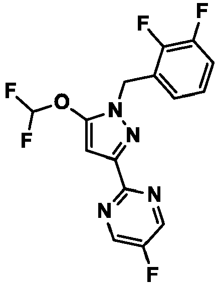 | 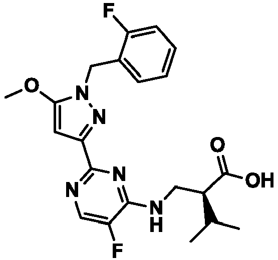 |
| I-58 | I-59 | I-60 |
| 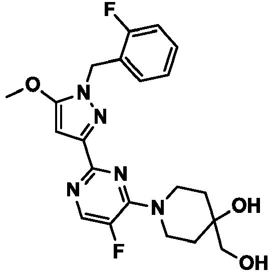 | 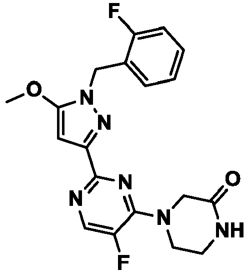 | 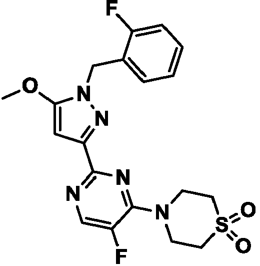 |
| I-61 | I-62 | I-63 |
| 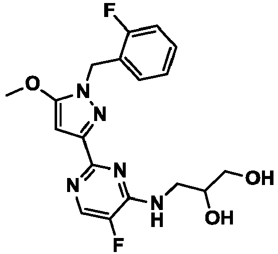 | 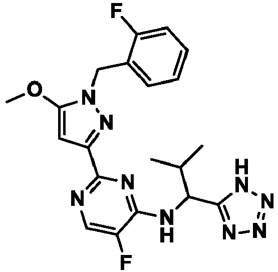 | 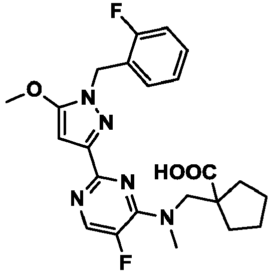 |
| I-64 | I-65 | I-66 |
| 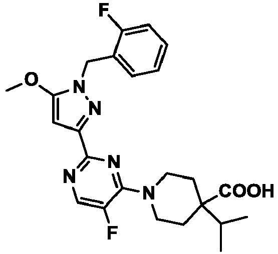 | 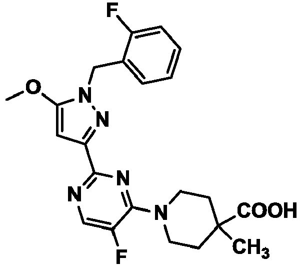 | 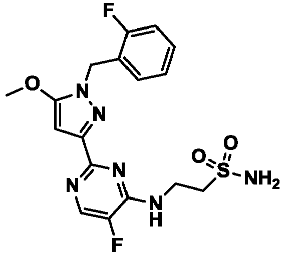 |
| I-67 | I-68 | I-69 |
| 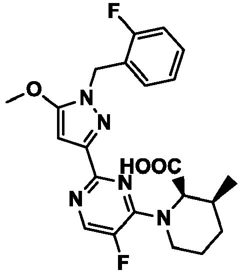 | 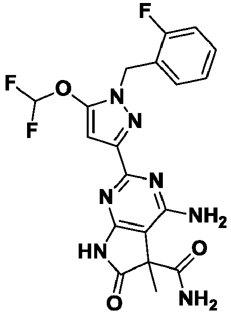 | 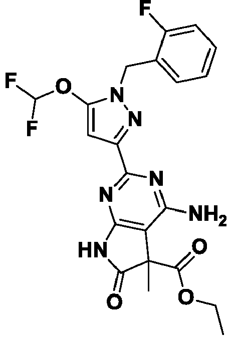 |
| I-70 | I-71 | I-72 |

### Methods of preparing the compounds

The compounds of **Formula I** may be prepared according to the schemes and examples depicted and described below. Unless otherwise specified, the starting materials and various intermediates may be obtained from commercial sources, prepared from commercially available compounds or prepared using well-known synthetic methods. Another aspect of the present invention is a process for preparing the compounds of Formula I as disclosed herein.

General synthetic procedures for the compounds of this invention are described below. The synthetic schemes are presented as examples and do not limit the scope of the invention in any way.

### General Procedures

Compounds of the present invention embodied in Formula I may be synthesized by those skilled in the art of synthetic organic chemistry using a variety of synthetic routes such as those depicted in, but not restricted to, the following Schemes.

**Scheme 1** depicts the method of Argentine et al (Org. Process R&D 2009, 13, 131-143). Substituted semicarbazides of the general structure **5** can be prepared by reacting commercially available *tert*-butylcarbazate **2** with bromide **3** to afford Boc-protected hydrazide **4.** Intermediate **4** can then be reacted directly with potassium cyanate in aqueous ethanol or alternatively with trimethylsilylisocyanate in THF followed by treatment with acid to deprotect the Boc protecting group and protodesilylate the trimethylsilyl protecting group to regioselectively afford semicarbazide **5.** Compound **3**, when not commercially available, may be prepared from the corresponding alcohol by bromination with reagents such as PBr₃ in a solvent such as ether. Alkyl hydrazines such as **6** can also be prepared by one skilled in the art by reacting alkyl halide **3** with hydrazine in polar solvent and subsequent reaction with potassium cyanate to directly afford semicarbazide **5.**

Compounds comprising examples of Formula I may then be synthesized by activating carboxylic acid **7** with a variety of amide coupling reagents (such as HATU, BOP, TPI, or conversion of the acid to an acid chloride) in DMF and a base such as triethylamine and then reacting it with intermediate semicarbazide **5** to form acyl semicarbazide **8** which can be cyclized to the desired triazolone **1** by heating under acidic conditions such as camphorsulfonic acid (Argentine et al) or under basic conditions (Deng et al Tetrahedron Lett. 2005, 7993-7996) such as NaOH in EtOH.

Many compounds of the general structure **7** are commercially available but may also be synthesized using the synthetic routes described in **Scheme 2.** Uracils of the general structure **9** are commercially available or synthetically accessible using literature procedures or to those skilled in the art of organic synthesis. Chlorination of uracil **9** using a reagent such as phosphorus oxychloride in an organic solvent such as THF or dichloroethane provides the dichloro intermediate **10.**

The 4-chloro substituent of intermediate **10** is generally more reactive than the 2-chloro substituent and can be chemoselectively displaced in a SNAr reaction with diverse nucleophiles (carbon-based, substituted amino, hydroxyl-containing, etc.) or with diverse nucleophiles using metal-assisted or organometallic reagent-mediated displacement (e.g., Suzuki reactions, Buchwald aminations, Sonogashira reactions, etc.) to give the mono-chloro intermediate **11.** The 2-chloro substituent of **11** can be transposed to ester **12a** via a carbonylation reaction using a palladium catalyst and carbon monoxide in an alcoholic solvent under basic conditions (WO2008/47201, US2012/245124, WO2008/9487). Alternatively, the chloride of **11** can be displaced by cyanide under refluxing alcoholic/aqueous conditions or by using palladium-mediated cross-coupling with zinc cyanide in a polar solvent such as DMF or NMP to give nitrile **12b** (Wada et al Tetrahedron Lett. 2012, 53(14), 1720-1724). Ester **12a** can be hydrolyzed using NaOH or by dealkylative deprotection (e.g., potassium trimethylsilyloxide heated in a polar aprotic solvent) and nitrile **12b** can be deprotected by hydrolysis in aqueous NaOH at 100 °C to give intermediate carboxylic acid **7.**

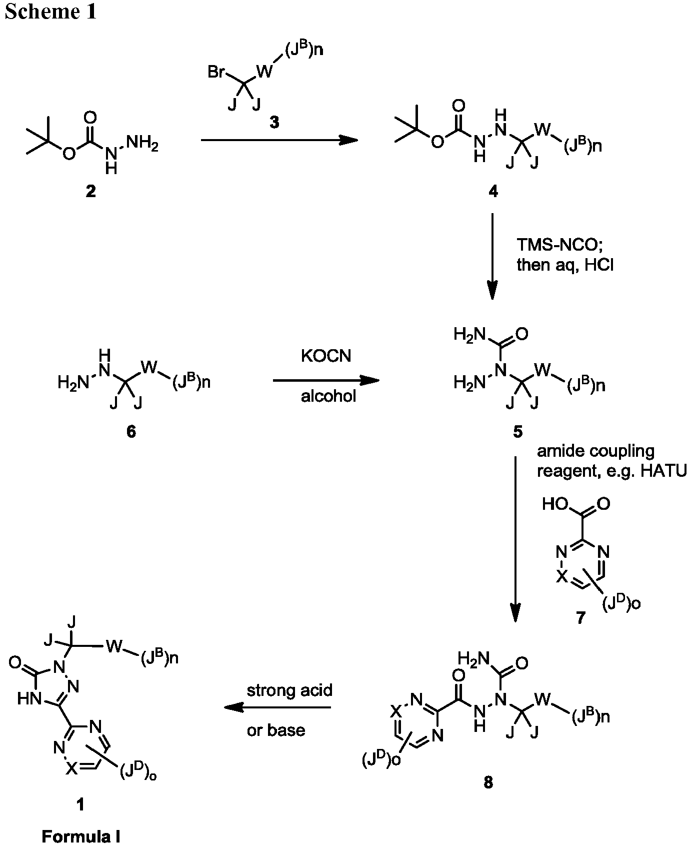

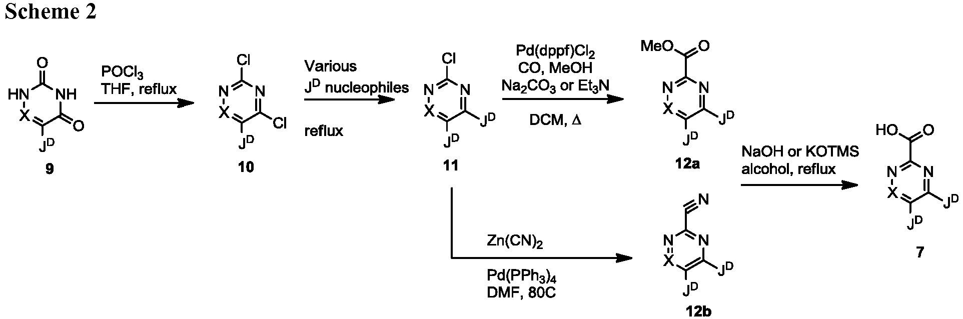

**Scheme 3** shows a complimentary method for making intermediate carboxylic acids **7** by condensation of an amidine such as commercially available methyl-2-amino-2-iminoacetate with a variety of 3-carbon electrophiles using reagents and conditions detailed in our earlier work (WO2013101830, WO2012003405) and others (WO2011149921, WO2013104703, WO2013030288, 2013004785, 2012004259) to form substituted pyrimidine carboxylates. Scheme 3, Reaction (1) shows the condensation of methyl-2-amino-2-iminoacetate with the anion of β-hydroxy ethylacrylate in refluxing ethanol to give a 2-carboxy-4-hydroxypyrimidine **7a** after ester deprotection using NaOH. This intermediate can be further derivatized by treatment with phosphorus oxychloride in DCM followed by SNAr displacement by various carbon, oxygen, and in this case an amino nucleophile HNR^{D}R^{D} to give a functionalized pyrimidine carboxylate **7b.** Scheme 3, Reactions (2) and (3) show an analogous example wherein the electrophile is a substituted malononitrile. Condensation of this reagent with methyl-2-amino-2-iminoacetate by refluxing in EtOH provides a 4,6-diaminopyrimidine **7c** that in the case of Reaction (3) can further cyclize under the reaction conditions to provide a functionalized lactam **7d** after ester hydrolysis to the carboxylic acid.

Condensation of appropriately substituted methyl 2-amino-2-hydrazonoacetate with diverse 2-carbon electrophiles followed by ester hydrolysis provides access to functionalized 3-carboxy-1,2,4-triazines (Palmer et al Bioorganic & Medicinal Chemistry 2007, 15(24), 7647-7660). Scheme 3, Reaction (4) illustrates this condensation with α-keto esters under refluxing EtOH and mild acid to give the 5-hydroxy-3-carboxy-1,2,4-triazine **7e** after ester deprotection. As in Reaction (1), intermediate **7e** can be further derivatized by treatment with phosphorus oxychloride in DCM followed by SNAr displacement by various carbon, oxygen, and in this case an amino nucleophile HNR^{D}R^{D} to give a functionalized 1,2,4-triazine carboxylate **7f.** Scheme 3, reaction (5) makes use of a diketone electrophile (Beard et al WO2008030843) to provide diverse 1,2,4-triazine-3-carboxylates of the general structure **7g** after ester hydrolysis.

**Scheme 4** illustrates a method for making alkoxytriazoles of Formula I by simply replacing the semicarbazide **5** used in Scheme 1, with thiosemicarbazide **5'** that can be prepared from **6** using potassium thiocyanate in alcoholic solvent. Coupling thiosemicarbazide **5'** with carboxylic acid **7** using a coupling reagent such as HATU provides acylthiosemicarbazide **13** that can be cyclized to the triazolethione **14** under basic or acidic conditions. Following the method of Kane et al (J. Med. Chem. 1994, 37(1), 125-132) and Courtemanche et al (WO 2002006272), triazolethione **14** reacts with methyl iodide under basic conditions such as K₂CO₃ in acetone to give S-methylation and subsequent oxidation using reagents such as MCPBA or Oxone can provide the triazole sulfone **15.** Displacement of this sulfone using an alcohol Rc-OH deprotonated with NaH in DMF and heating provides the desired alkoxytriazole **16** of Formula I.

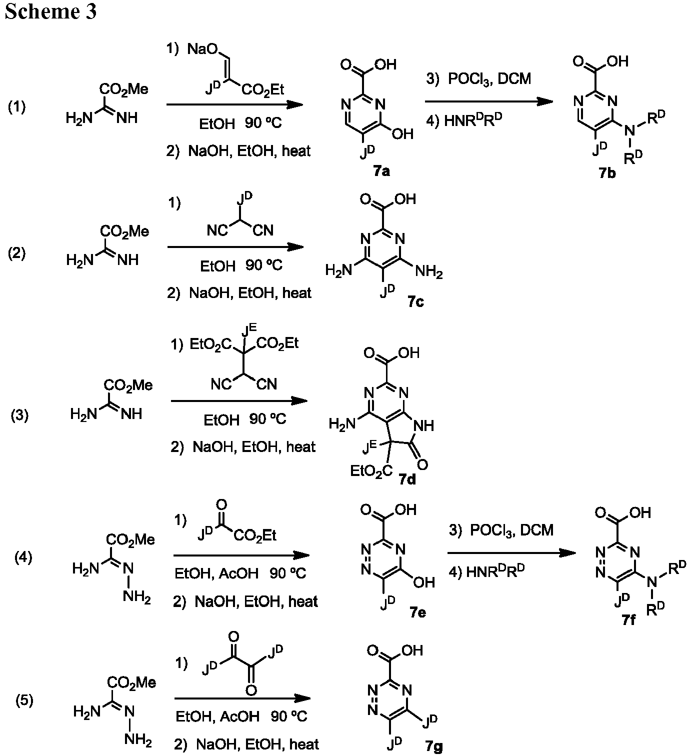

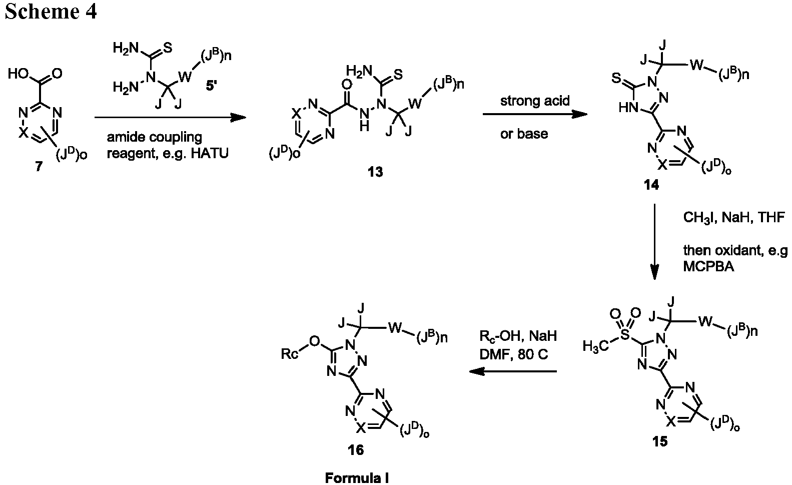

Synthesis of pyrazole variants of Formula I compounds is detailed in **Scheme 5.** The pyrazolone core structure can be synthesized by condensation of substituted hydrazine **6** with diethyl 2-oxosuccinate under acidic conditions to form hydroxypyrazole **17.** This intermediate may be O-alkylated with diverse electrophiles such as alkyl and aryl iodides and triflates under basic conditions to provide alkoxy pyrazole **18.** Treatment of **18** with ammonium chloride and trimethylaluminum gives the amidine **19** which can be condensed with 3-carbon electrophiles using the reagents, conditions, and references described above in Scheme 3, Reactions (1)-(3) to provide compounds of Formula I such as the substituted pyrimidines **20**. Amidine **19** can be treated with hydrazine in refluxing alcohol to form the 2-aminohydrazone 21 that can be condensed with 2-carbon electrophiles using the reagents, conditions, and references described above in Scheme 3, Reactions (4)-(5) to provide compounds of Formula I such as the substituted triazines **22.**

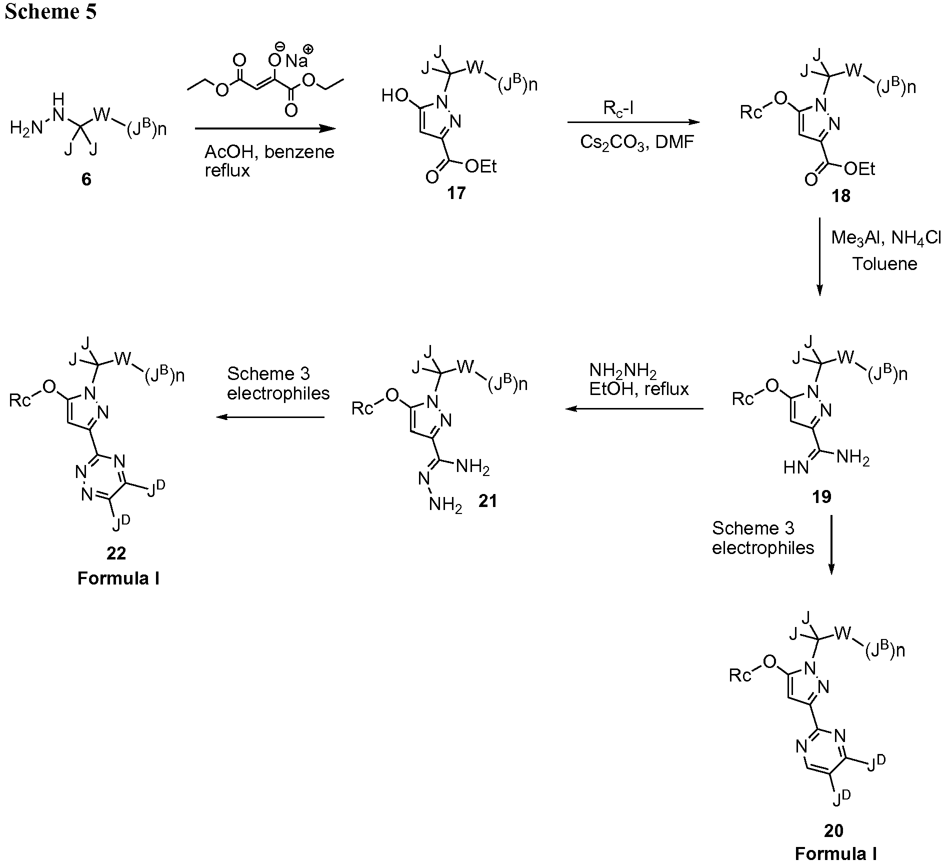

### Pharmaceutically acceptable salts of the invention.

In a second aspect, the invention relates to a pharmaceutical composition comprising the compound of any one of the above embodiments, or a pharmaceutically acceptable salt thereof, and at least one pharmaceutically acceptable excipient.

The phrase "pharmaceutically acceptable salt," as used herein, refers to pharmaceutically acceptable organic or inorganic salts of a compound of Formula I. The pharmaceutically acceptable salts of a compound of Formula I are used in medicine. Salts that are not pharmaceutically acceptable may, however, be useful in the preparation of a compound of Formula I or of their pharmaceutically acceptable salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

Pharmaceutically acceptable salts of the compounds described herein include those derived from the compounds with inorganic acids, organic acids or bases. In some embodiments, the salts can be prepared in situ during the final isolation and purification of the compounds. In other embodiments the salts can be prepared from the free form of the compound in a separate synthetic step.

When a compound of Formula I is acidic or contains a sufficiently acidic bioisostere, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particular embodiments include ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N, N.sup.1-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

When a compound of Formula I is basic or contains a sufficiently basic bioisostere, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particular embodiments include citric, hydrobromic, hydrochloric, maleic, phosphoric, sulfuric and tartaric acids. Other exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and palmoate (i.e., 1,1'-methylene-bis-(2-hydroxy-3-naphthoate)) salts.

The preparation of the pharmaceutically acceptable salts described above and other typical pharmaceutically acceptable salts is more fully described by Berg et al., "Pharmaceutical Salts," J. Pharm. Sci., 1977:66:1-19.

In addition to the compounds described herein, their pharmaceutically acceptable salts may also be employed in compositions to treat or prevent the herein identified disorders.

In all instances described herein, the term "compound" also includes a pharmaceutically acceptable salt of the compound, whether or not the phrase "pharmaceutically acceptable salt" is actually used.

### Pharmaceutical compositions and methods of administration.

The compounds herein disclosed, and their pharmaceutically acceptable salts thereof may be formulated as pharmaceutical compositions or "formulations".

A typical formulation is prepared by mixing a compound of Formula I, or a pharmaceutically acceptable salt thereof, and a carrier, diluent or excipient. Suitable carriers, diluents and excipients are well known to those skilled in the art and include materials such as carbohydrates, waxes, water soluble and/or swellable polymers, hydrophilic or hydrophobic materials, gelatin, oils, solvents, water, and the like. The particular carrier, diluent or excipient used will depend upon the means and purpose for which a compound of Formula I is being formulated. Solvents are generally selected based on solvents recognized by persons skilled in the art as safe (GRAS-Generally Regarded as Safe) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG400, PEG300), etc. and mixtures thereof. The formulations may also include other types of excipients such as one or more buffers, stabilizing agents, antiadherents, surfactants, wetting agents, lubricating agents, emulsifiers, binders, suspending agents, disintegrants, fillers, sorbents, coatings (e.g., enteric or slow release) preservatives, antioxidants, opaquing agents, glidants, processing aids, colorants, sweeteners, perfuming agents, flavoring agents and other known additives to provide an elegant presentation of the drug (i.e., a compound of Formula I or pharmaceutical composition thereof) or aid in the manufacturing of the pharmaceutical product (i.e., medicament).

The formulations may be prepared using conventional dissolution and mixing procedures. For example, the bulk drug substance (i.e., a compound of Formula I, a pharmaceutically acceptable salt thereof, or a stabilized form of the compound, such as a complex with a cyclodextrin derivative or other known complexation agent) is dissolved in a suitable solvent in the presence of one or more of the excipients described above. A compound having the desired degree of purity is optionally mixed with pharmaceutically acceptable diluents, carriers, excipients or stabilizers, in the form of a lyophilized formulation, milled powder, or an aqueous solution. Formulation may be conducted by mixing at ambient temperature at the appropriate pH, and at the desired degree of purity, with physiologically acceptable carriers. The pH of the formulation depends mainly on the particular use and the concentration of compound, but may range from about 3 to about 8. When the agent described herein is a solid amorphous dispersion formed by a solvent process, additives may be added directly to the spray-drying solution when forming the mixture such as the additive is dissolved or suspended in the solution as a slurry which can then be spray dried. Alternatively, the additives may be added following spray-drying process to aid in the forming of the final formulated product.

The compound of Formula I or a pharmaceutically acceptable salt thereof is typically formulated into pharmaceutical dosage forms to provide an easily controllable dosage of the drug and to enable patient compliance with the prescribed regimen. Pharmaceutical formulations of a compound of Formula I, or a pharmaceutically acceptable salt thereof, may be prepared for various routes and types of administration. Various dosage forms may exist for the same compound, since different medical conditions may warrant different routes of administration.

The amount of active ingredient that may be combined with the carrier material to produce a single dosage form will vary depending upon the subject treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight: weight). The pharmaceutical composition can be prepared to provide easily measurable amounts for administration. For example, an aqueous solution intended for intravenous infusion may contain from about 3 to 500 µg of the active ingredient per milliliter of solution in order that infusion of a suitable volume at a rate of about 30 mL/hr can occur. As a general proposition, the initial pharmaceutically effective amount of the inhibitor administered will be in the range of about 0.01-100 mg/kg per dose, namely about 0.1 to 20 mg/kg of patient body weight per day, with the typical initial range of compound used being 0.3 to 15 mg/kg/day.

The term "therapeutically effective amount" as used herein means that amount of active compound or pharmaceutical agent that elicits the biological or medicinal response in a tissue, system, animal or human that is being sought by a researcher, veterinarian, medical doctor or other clinician. The therapeutically or pharmaceutically effective amount of the compound to be administered will be governed by such considerations, and is the minimum amount necessary to ameliorate, cure or treat the disease or disorder or one or more of its symptoms.

The pharmaceutical compositions of Formula I will be formulated, dosed, and administered in a fashion, i.e., amounts, concentrations, schedules, course, vehicles, and route of administration, consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners, such as the age, weight, and response of the individual patient.

The term "prophylactically effective amount" refers to an amount effective in preventing or substantially lessening the chances of acquiring a disease or disorder or in reducing the severity of the disease or disorder before it is acquired or reducing the severity of one or more of its symptoms before the symptoms develop. Roughly, prophylactic measures are divided between *primary* prophylaxis (to prevent the development of a disease) and *secondary* prophylaxis (whereby the disease has already developed and the patient is protected against worsening of this process).

Acceptable diluents, carriers, excipients, and stabilizers are those that are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, tretralose or sorbitol; salt-forming counter-ions such as sodium; metal complexes (e.g., Zn-protein complexes); and/or non-ionic surfactants such as TWEEN™, PLURONICS™ or polyethylene glycol (PEG). The active pharmaceutical ingredients may also be entrapped in microcapsules prepared, for example, by coacervation techniques or by interfacial polymerization, e.g., hydroxymethylcellulose or gelatin-microcapsules and poly-(methylmethacylate) microcapsules, respectively; in colloidal drug delivery systems (for example, liposomes, albumin microspheres, microemulsions, nano-particles and nanocapsules) or in macroemulsions. Such techniques are disclosed in Remington's: The Science and Practice of Pharmacy, 21st Edition, University of the Sciences in Philadelphia, Eds., 2005 (hereafter "Remington's").

"Controlled drug delivery systems" supply the drug to the body in a manner precisely controlled to suit the drug and the conditions being treated. The primary aim is to achieve a therapeutic drug concentration at the site of action for the desired duration of time. The term "controlled release" is often used to refer to a variety of methods that modify release of drug from a dosage form. This term includes preparations labeled as "extended release", "delayed release", "modified release" or "sustained release". In general, one can provide for controlled release of the agents described herein through the use of a wide variety of polymeric carriers and controlled release systems including erodible and non-erodible matrices, osmotic control devices, various reservoir devices, enteric coatings and multiparticulate control devices.

"Sustained-release preparations" are the most common applications of controlled release. Suitable examples of sustained-release preparations include semipermeable matrices of solid hydrophobic polymers containing the compound, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and gamma-ethyl-L-glutamate, non-degradable ethylene-vinyl acetate, degradable lactic acid-glycolic acid copolymers, and poly-D-(-)-3-hydroxybutyric acid.

"Immediate-release preparations" may also be prepared. The objective of these formulations is to get the drug into the bloodstream and to the site of action as rapidly as possible. For instance, for rapid dissolution, most tablets are designed to undergo rapid disintegration to granules and subsequent deaggregation to fine particles. This provides a larger surface area exposed to the dissolution medium, resulting in a faster dissolution rate.

Agents described herein can be incorporated into an erodible or non-erodible polymeric matrix controlled release device. By an erodible matrix is meant aqueous-erodible or water-swellable or aqueous-soluble in the sense of being either erodible or swellable or dissolvable in pure water or requiring the presence of an acid or base to ionize the polymeric matrix sufficiently to cause erosion or dissolution. When contacted with the aqueous environment of use, the erodible polymeric matrix imbibes water and forms an aqueous-swollen gel or matrix that entraps the agent described herein. The aqueous-swollen matrix gradually erodes, swells, disintegrates or dissolves in the environment of use, thereby controlling the release of a compound described herein to the environment of use. One ingredient of this water-swollen matrix is the water-swellable, erodible, or soluble polymer, which may generally be described as an osmopolymer, hydrogel or water-swellable polymer. Such polymers may be linear, branched, or cross linked. The polymers may be homopolymers or copolymers. In certain embodiments, they may be synthetic polymers derived from vinyl, acrylate, methacrylate, urethane, ester and oxide monomers. In other embodiments, they can be derivatives of naturally occurring polymers such as polysaccharides (e.g., chitin, chitosan, dextran and pullulan; gum agar, gum arabic, gum karaya, locust bean gum, gum tragacanth, carrageenans, gum ghatti, guar gum, xanthan gum and scleroglucan), starches (e.g., dextrin and maltodextrin), hydrophilic colloids (e.g., pectin), phosphatides (e.g., lecithin), alginates (e.g., ammonium alginate, sodium, potassium or calcium alginate, propylene glycol alginate), gelatin, collagen, and cellulosics. Cellulosics are cellulose polymer that has been modified by reaction of at least a portion of the hydroxyl groups on the saccharide repeat units with a compound to form an ester-linked or an ether-linked substituent. For example, the cellulosic ethyl cellulose has an ether linked ethyl substituent attached to the saccharide repeat unit, while the cellulosic cellulose acetate has an ester linked acetate substituent. In certain embodiments, the cellulosics for the erodible matrix comprises aqueous-soluble and aqueous-erodible cellulosics can include, for example, ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC). In certain embodiments, the cellulosics comprises various grades of low viscosity (MW less than or equal to 50,000 Daltons, for example, the Dow Methocel™ series E5, E15LV, E50LV and K100LY) and high viscosity (MW greater than 50,000 Daltons, for example, E4MCR, E10MCR, K4M, K15M and K100M and the Methocel™ K series) HPMC. Other commercially available types of HPMC include the Shin Etsu Metolose 90SH series.

Other materials useful as the erodible matrix material include, but are not limited to, pullulan, polyvinyl pyrrolidone, polyvinyl alcohol, polyvinyl acetate, glycerol fatty acid esters, polyacrylamide, polyacrylic acid, copolymers of ethacrylic acid or methacrylic acid (EUDRAGIT®, Rohm America, Inc., Piscataway, New Jersey) and other acrylic acid derivatives such as homopolymers and copolymers of butylmethacrylate, methylmethacrylate, ethylmethacrylate, ethylacrylate, (2-dimethylaminoethyl) methacrylate, and (trimethylaminoethyl) methacrylate chloride.

Alternatively, the agents of the present invention may be administered by or incorporated into a non-erodible matrix device. In such devices, an agent described herein is distributed in an inert matrix. The agent is released by diffusion through the inert matrix. Examples of materials suitable for the inert matrix include insoluble plastics (e.g., methyl acrylate-methyl methacrylate copolymers, polyvinyl chloride, polyethylene), hydrophilic polymers (e.g., ethyl cellulose, cellulose acetate, cross linked polyvinylpyrrolidone (also known as crospovidone), and fatty compounds (e.g., carnauba wax, microcrystalline wax, and triglycerides). Such devices are described further in Remington: The Science and Practice of Pharmacy, 20th edition (2000).

As noted above, the agents described herein may also be incorporated into an osmotic control device. Such devices generally include a core containing one or more agents as described herein and a water permeable, non-dissolving and non-eroding coating surrounding the core which controls the influx of water into the core from an aqueous environment of use so as to cause drug release by extrusion of some or all of the core to the environment of use. In certain embodiments, the coating is polymeric, aqueous-permeable, and has at least one delivery port. The core of the osmotic device optionally includes an osmotic agent which acts to imbibe water from the surrounding environment via such a semi-permeable membrane. The osmotic agent contained in the core of this device may be an aqueous-swellable hydrophilic polymer or it may be an osmogen, also known as an osmagent. Pressure is generated within the device which forces the agent(s) out of the device via an orifice (of a size designed to minimize solute diffusion while preventing the build-up of a hydrostatic pressure head). Non limiting examples of osmotic control devices are disclosed in U. S. Patent Application Serial No. 09/495,061.

The amount of water-swellable hydrophilic polymers present in the core may range from about 5 to about 80 wt% (including for example, 10 to 50 wt%). Non limiting examples of core materials include hydrophilic vinyl and acrylic polymers, polysaccharides such as calcium alginate, polyethylene oxide (PEO), polyethylene glycol (PEG), polypropylene glycol (PPG), poly (2-hydroxyethyl methacrylate), poly (acrylic) acid, poly (methacrylic) acid, polyvinylpyrrolidone (PVP) and cross linked PVP, polyvinyl alcohol (PVA), PVA/PVP copolymers and PVA/PVP copolymers with hydrophobic monomers such as methyl methacrylate, vinyl acetate, and the like, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl cellulose (CEC), sodium alginate, polycarbophil, gelatin, xanthan gum, and sodium starch glycolate. Other materials include hydrogels comprising interpenetrating networks of polymers that may be formed by addition or by condensation polymerization, the components of which may comprise hydrophilic and hydrophobic monomers such as those just mentioned. Water-swellable hydrophilic polymers include but are not limited to PEO, PEG, PVP, sodium croscarmellose, HPMC, sodium starch glycolate, polyacrylic acid and cross linked versions or mixtures thereof.

The core may also include an osmogen (or osmagent). The amount of osmogen present in the core may range from about 2 to about 70 wt% (including, for example, from 10 to 50 wt%). Typical classes of suitable osmogens are water-soluble organic acids, salts and sugars that are capable of imbibing water to thereby effect an osmotic pressure gradient across the barrier of the surrounding coating. Typical useful osmogens include but are not limited to magnesium sulfate, magnesium chloride, calcium chloride, sodium chloride, lithium chloride, potassium sulfate, sodium carbonate, sodium sulfite, lithium sulfate, potassium chloride, sodium sulfate, mannitol, xylitol, urea, sorbitol, inositol, raffinose, sucrose, glucose, fructose, lactose, citric acid, succinic acid, tartaric acid, and mixtures thereof. In certain embodiments, the osmogen is glucose, lactose, sucrose, mannitol, xylitol, sodium chloride, including combinations thereof.

The rate of drug delivery is controlled by such factors as the permeability and thickness of the coating, the osmotic pressure of the drug-containing layer, the degree of hydrophilicity of the hydrogel layer, and the surface area of the device. Those skilled in the art will appreciate that increasing the thickness of the coating will reduce the release rate, while any of the following will increase the release rate: increasing the permeability of the coating; increasing the hydrophilicity of the hydrogel layer; increasing the osmotic pressure of the drug-containing layer; or increasing the device's surface area.

In certain embodiments, entrainment of particles of agents described herein in the extruding fluid during operation of such osmotic device is desirable. For the particles to be well entrained, the agent drug form is dispersed in the fluid before the particles have an opportunity to settle in the tablet core. One means of accomplishing this is by adding a disintegrant that serves to break up the compressed core into its particulate components. Non limiting examples of standard disintegrants include materials such as sodium starch glycolate (e. g., Explotab™ CLV), microcrystalline cellulose (e. g., Avicel™), microcrystalline silicified cellulose (e. g., ProSoIv™) and croscarmellose sodium (e. g., Ac-Di-Sol™), and other disintegrants known to those skilled in the art. Depending upon the particular formulation, some disintegrants work better than others. Several disintegrants tend to form gels as they swell with water, thus hindering drug delivery from the device. Non-gelling, non-swelling disintegrants provide a more rapid dispersion of the drug particles within the core as water enters the core. In certain embodiments, non-gelling, non-swelling disintegrants are resins, for example, ion-exchange resins. In one embodiment, the resin is Amberlite™ IRP 88 (available from Rohm and Haas, Philadelphia, PA). When used, the disintegrant is present in amounts ranging from about 1-25% of the core agent.

Another example of an osmotic device is an osmotic capsule. The capsule shell or portion of the capsule shell can be semipermeable. The capsule can be filled either by a powder or liquid consisting of an agent described herein, excipients that imbibe water to provide osmotic potential, and/or a water-swellable polymer, or optionally solubilizing excipients. The capsule core can also be made such that it has a bilayer or multilayer agent analogous to the bilayer, trilayer or concentric geometries described above.

Another class of osmotic device useful in this invention comprises coated swellable tablets, for example, as described in EP378404. Coated swellable tablets comprise a tablet core comprising an agent described herein and a swelling material, preferably a hydrophilic polymer, coated with a membrane, which contains holes, or pores through which, in the aqueous use environment, the hydrophilic polymer can extrude and carry out the agent. Alternatively, the membrane may contain polymeric or low molecular weight water-soluble porosigens. Porosigens dissolve in the aqueous use environment, providing pores through which the hydrophilic polymer and agent may extrude. Examples of porosigens are water-soluble polymers such as HPMC, PEG, and low molecular weight compounds such as glycerol, sucrose, glucose, and sodium chloride. In addition, pores may be formed in the coating by drilling holes in the coating using a laser or other mechanical means. In this class of osmotic devices, the membrane material may comprise any film-forming polymer, including polymers which are water permeable or impermeable, providing that the membrane deposited on the tablet core is porous or contains water-soluble porosigens or possesses a macroscopic hole for water ingress and drug release. Embodiments of this class of sustained release devices may also be multilayered, as described, for example, in EP378404.

When an agent described herein is a liquid or oil, such as a lipid vehicle formulation, for example as described in WO05/011634, the osmotic controlled-release device may comprise a soft-gel or gelatin capsule formed with a composite wall and comprising the liquid formulation where the wall comprises a barrier layer formed over the external surface of the capsule, an expandable layer formed over the barrier layer, and a semipermeable layer formed over the expandable layer. A delivery port connects the liquid formulation with the aqueous use environment. Such devices are described, for example, in US6419952, US6342249, US5324280, US4672850, US4627850, US4203440, and US3995631.

As further noted above, the agents described herein may be provided in the form of microparticulates, generally ranging in size from about 10µm to about 2mm (including, for example, from about 100µm to 1mm in diameter). Such multiparticulates may be packaged, for example, in a capsule such as a gelatin capsule or a capsule formed from an aqueous-soluble polymer such as HPMCAS, HPMC or starch; dosed as a suspension or slurry in a liquid ; or they may be formed into a tablet, caplet, or pill by compression or other processes known in the art. Such multiparticulates may be made by any known process, such as wet- and dry-granulation processes, extrusion/spheronization, roller-compaction, melt-congealing, or by spray-coating seed cores. For example, in wet-and dry-granulation processes, the agent described herein and optional excipients may be granulated to form multiparticulates of the desired size.

The agents can be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology, New York: Marcel Dekker, 1992, volume 9). For the preparation of microemulsions, surfactant (emulsifier), co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifier") is generally selected from the group of polyglycerol derivatives, glycerol derivatives and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono- di- and triglycerides, mono- and di-esters of PEG (e.g., oleoyl macrogol glycerides), etc.

The compounds described herein can be incorporated into pharmaceutically-acceptable nanoparticle, nanosphere, and nanocapsule formulations (Delie and Blanco-Prieto, 2005, Molecule 10:65-80). Nanocapsules can generally entrap compounds in a stable and reproducible way. To avoid side effects due to intracellular polymeric overloading, ultrafine particles (sized around 0.1 µm) can be designed using polymers able to be degraded in vivo (e.g., biodegradable polyalkyl-cyanoacrylate nanoparticles). Such particles are described in the prior art.

Implantable devices coated with a compound of this invention are another embodiment of the present invention. The compounds may also be coated on implantable medical devices, such as beads, or co-formulated with a polymer or other molecule, to provide a "drug depot", thus permitting the drug to be released over a longer time period than administration of an aqueous solution of the drug. Suitable coatings and the general preparation of coated implantable devices are described in U.S. Pat. Nos. 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition.

The formulations include those suitable for the administration routes detailed herein. The formulations may conveniently be presented in unit dosage form and may be prepared by any of the methods well known in the art of pharmacy. Techniques and formulations generally are found in Remington's. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

The terms "administer", "administering" or "administration" in reference to a compound, composition or formulation of the invention means introducing the compound into the system of the animal in need of treatment. When a compound of the invention is provided in combination with one or more other active agents, "administration" and its variants are each understood to include concurrent and/or sequential introduction of the compound and the other active agents.

The compositions described herein may be administered systemically or locally, e.g.: orally (e.g., using capsules, powders, solutions, suspensions, tablets, sublingual tablets and the like), by inhalation (e.g., with an aerosol, gas, inhaler, nebulizer or the like), to the ear (e.g., using ear drops), topically (e.g., using creams, gels, liniments, lotions, ointments, pastes, transdermal patches, etc), ophthalmically (e.g., with eye drops, ophthalmic gels, ophthalmic ointments), rectally (e.g., using enemas or suppositories), nasally, buccally, vaginally (e.g., using douches, intrauterine devices, vaginal suppositories, vaginal rings or tablets, etc), via an implanted reservoir or the like, or parenterally depending on the severity and type of the disease being treated. The term "parenteral" as used herein includes, but is not limited to, subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

The pharmaceutical compositions described herein may be orally administered in any orally acceptable dosage form including, but not limited to, capsules, tablets, aqueous suspensions or solutions. Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. Tablets may be uncoated or may be coated by known techniques including microencapsulation to mask an unpleasant taste or to delay disintegration and adsorption in the gastrointestinal tract and thereby provide a sustained action over a longer period. For example, a time delay material such as glyceryl monostearate or glyceryl distearate alone or with a wax may be employed. A water soluble taste masking material such as hydroxypropyl-methylcellulose or hydroxypropyl-cellulose may be employed.

Formulations of a compound of Formula I that are suitable for oral administration may be prepared as discrete units such as tablets, pills, troches, lozenges, aqueous or oil suspensions, dispersible powders or granules, emulsions, hard or soft capsules, e.g., gelatin capsules, syrups or elixirs. Formulations of a compound intended for oral use may be prepared according to any method known to the art for the manufacture of pharmaceutical compositions.

Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

The active compounds can also be in microencapsulated form with one or more excipients as noted above.

When aqueous suspensions are required for oral use, the active ingredient is combined with emulsifying and suspending agents. If desired, certain sweetening and/or flavoring agents may be added. Syrups and elixirs may be formulated with sweetening agents, for example glycerol, propylene glycol, sorbitol or sucrose. Such formulations may also contain a demulcent, a preservative, flavoring and coloring agents and antioxidant.

Sterile injectable forms of the compositions described herein (e.g., for parenteral administration) may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3 -butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of injectable formulations.

Oily suspensions may be formulated by suspending a compound of Formula I in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Aqueous suspensions of a compound of Formula I contain the active materials in admixture with excipients suitable for the manufacture of aqueous suspensions. Such excipients include a suspending agent, such as sodium carboxymethylcellulose, croscarmellose, povidone, methylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound described herein, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsulated matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

The injectable solutions or microemulsions may be introduced into a patient's bloodstream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

Compositions for rectal or vaginal administration are preferably suppositories which can be prepared by mixing the compounds described herein with suitable non-irritating excipients or carriers such as cocoa butter, beeswax, polyethylene glycol or a suppository wax which are solid at ambient temperature but liquid at body temperature and therefore melt in the rectum or vaginal cavity and release the active compound. Other formulations suitable for vaginal administration may be presented as pessaries, tampons, creams, gels, pastes, foams or sprays.

The pharmaceutical compositions described herein may also be administered topically, especially when the target of treatment includes areas or organs readily accessible by topical application, including diseases of the eye, the ear, the skin, or the lower intestinal tract. Suitable topical formulations are readily prepared for each of these areas or organs.

Dosage forms for topical or transdermal administration of a compound described herein include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel. Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2 octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum. For treatment of the eye or other external tissues, e.g., mouth and skin, the formulations may be applied as a topical ointment or cream containing the active ingredient(s) in an amount of, for example, 0.075 to 20% w/w. When formulated in an ointment, the active ingredients may be employed with either an oil-based, paraffinic or a water-miscible ointment base.

Alternatively, the active ingredients may be formulated in a cream with an oil-in-water cream base. If desired, the aqueous phase of the cream base may include a polyhydric alcohol, i.e., an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulfoxide and related analogs.

The oily phase of emulsions prepared using a compound of Formula I may be constituted from known ingredients in a known manner. While the phase may comprise merely an emulsifier (otherwise known as an emulgent), it desirably comprises a mixture of at least one emulsifier with a fat or an oil or with both a fat and an oil. A hydrophilic emulsifier may be included together with a lipophilic emulsifier which acts as a stabilizer. In some embodiments, the emulsifier includes both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations. Emulgents and emulsion stabilizers suitable for use in the formulation of a compound of Formula I include Tween™-60, Span™-80, cetostearyl alcohol, benzyl alcohol, myristyl alcohol, glyceryl mono-stearate and sodium lauryl sulfate.

The pharmaceutical compositions may also be administered by nasal aerosol or by inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. Formulations suitable for intrapulmonary or nasal administration have a particle size for example in the range of 0.1 to 500 micros (including particles in a range between 0.1 and 500 microns in increments microns such as 0.5, 1, 30, 35 microns, etc) which is administered by rapid inhalation through the nasal passage or by inhalation through the mouth so as to reach the alveolar sacs.

The pharmaceutical composition (or formulation) for use may be packaged in a variety of ways depending upon the method used for administering the drug. Generally, an article for distribution includes a container having deposited therein the pharmaceutical formulation in an appropriate form. Suitable containers are well-known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

The formulations may be packaged in unit-dose or multi-dose containers, for example sealed ampoules and vials, and may be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

In another aspect, a compound of Formula I or a pharmaceutically acceptable salt thereof may be formulated in a veterinary composition comprising a veterinary carrier. Veterinary carriers are materials useful for the purpose of administering the composition and may be solid, liquid or gaseous materials which are otherwise inert or accepted in the veterinary art and are compatible with the active ingredient. These veterinary compositions may be administered parenterally, orally or by any other desired route.

### Therapeutic methods

In another aspect, the disclosure relates to the treatment of certain disorders by using sGC stimulators, either alone or in combination, or their pharmaceutically acceptable salts or pharmaceutical compositions comprising them, in a patient in need thereof.

The present disclosure relates to stimulators of soluble guanylate cyclase (sGC), pharmaceutical formulations thereof and their use, alone or in combination with one or more additional agents, for treating and/or preventing various diseases, wherein an increase in the concentration of NO or an increase in the concentration of cGMP might be desirable.

Increased production of NO or increased concentration of cGMP in a tissue leads to vasodilation, inhibition of platelet aggregation and adhesion, anti-hypertensive effects, anti-remodeling effects, anti-fibrotic, anti-apoptotic effects, anti-inflammatory effects and neuronal signal transmission effects, among other effects.

In other embodiments, the compounds here disclosed are sGC stimulators that may be useful in the prevention and/or treatment of diseases and disorders characterized by undesirable reduced bioavailability of and/or sensitivity to NO in a biological system (e.g., in the human body), such as those associated with conditions of oxidative stress or nitrosative stress.

The term "cardiovascular disease" (or "cardiovascular disorder") as used herein, refers to a disease based on the abnormal symptoms of circulatory organs such as the heart, blood vessels (arteries, capillaries, and veins) or both. The term also includes any disease that affects the cardiovascular system in general, including cardiac disease, vascular diseases of the brain, vascular diseases of the kidney, liver and associated organs, or lung, and peripheral arterial disease, among others.

A "sGC-related cardiovascular disease" is one for which the NO/sGC/cGMP system is known or suspected to be involved and is a cardiovascular disease that can be treated or prevented by sGC activation/stimulation, by activation of a NO synthase, or by addition of NO or an NO-donor or an NO precursor such as L-Arginine or L-citruline, or by inhibition of a PDE (phosphodiesterase) enzyme responsible for the breakdown of cGMP, or a combination of the any of the above methods.

The term "vasodilation" as used herein, refers to the widening of blood vessels. It results from relaxation of smooth muscle cells within the vessel walls, in particular in the large veins, large arteries, and smaller arterioles. In essence, the process is the opposite of "vasoconstriction", which is the narrowing of blood vessels. When blood vessels dilate, the flow of blood is increased due to a decrease in vascular resistance. Therefore, dilation of arterial blood vessels (mainly the arterioles) decreases blood pressure. The response may be intrinsic (due to local processes in the surrounding tissue) or extrinsic (due to hormones or the nervous system). In addition, the response may be localized to a specific organ (depending on the metabolic needs of a particular tissue, as during strenuous exercise), or it may be systemic (seen throughout the entire systemic circulation).

The term "vasoconstriction" as used herein refers to the narrowing of a blood vessel due to muscle contraction. Vasoconstriction is one mechanism by which the body regulates and maintains mean arterial pressure (MAP). Generalized vasoconstriction usually results in an increase in systemic blood pressure, but it may also occur in specific tissues, causing a localized reduction in blood flow.

As used herein, the term "bronchoconstriction" is used to define the constriction of the airways in the lungs due to the tightening of surrounding smooth muscle, with consequent coughing, wheezing, and shortness of breath. The condition has a number of causes, the most common being asthma. Exercise and allergies can bring on the symptoms in an otherwise asymptomatic individual. Other conditions such as chronic obstructive pulmonary disease (COPD) can also present with bronchoconstriction.

Throughout this disclosure, the terms "hypertension", "arterial hypertension" or "high blood pressure (HBP)" are used interchangeably and refer to an extremely common and highly preventable chronic condition in which blood pressure (BP) in the arteries is higher than normal or desired. If not properly controlled, it represents a significant risk factor for several serious cardiovascular and renal conditions. Hypertension may be a primary disease, called "essential hypertension" or "idiopathic hypertension", or it may be caused by or related to other diseases, in which case it is classified as "secondary hypertension". Essential hypertension accounts for 90-95% of all cases.

As used herein, the term "resistant hypertension" refers to hypertension that remains above goal blood pressure (usually less than 140/90 mmHg, although a lower goal of less than 130/80 mmHg is recommended for patients with comorbid diabetes or kidney disease), in spite of concurrent use of three antihypertensive agents belonging to different antihypertensive drug classes. People who require four or more drugs to control their blood pressure are also considered to have resistant hypertension. Hypertension is an extremely common comorbid condition in diabetes, affecting ∼20-60% of patients with diabetes, depending on obesity, ethnicity, and age. This type of hypertension is herein refered to as "diabetic hypertension". In type 2 diabetes, hypertension is often present as part of the metabolic syndrome of insulin resistance also including central obesity and dyslipidemia. In type 1 diabetes, hypertension may reflect the onset of diabetic nephropathy.

"Pulmonary hypertension (PH)", as used herein, is a disease characterized by sustained elevations of blood pressure in the pulmonary vasculature (pulmonary artery, pulmonary vein and pulmonary capillaries), which results in right heart hypertrophy, eventually leading to right heart failure and death. Common symptoms of PH include shortness of breath, dizziness and fainting, all of which are exacerbated by exertion. Without treatment, median life expectancy following diagnosis is 2.8 years. PH exists in many different forms, which are categorized according to their etiology. Categories include pulmonary arterial hypertension (PAH), PH with left heart disease, PH associated with lung diseases and /or hypoxaemia, PH due to chronic thrombotic and/or embolic disease and miscellaneous PH. PAH is rare in the general population, but the prevalence increases in association with certain common conditions such as HIV infection, scleroderma and sickle cell disease. Other forms of PH are generally more common than PAH, and, for instance, the association of PH with chronic obstructive pulmonary disease (COPD) is of particular concern. Current treatment for pulmonary hypertension depends on the stage and the mechanism of the disease.

The term "coronary artery disease" refers to a condition in which the blood supply to the heart muscle is partially or completely blocked (ischemia of the heart muscle or myocardium). This reduced blood supply to the myocardium may result in a number of "acute myocardial syndromes": chest pain ("angina", also called "angina pectoris", stable or unstable) and different types of heart attacks ("myocardial infarction" or MI). One common cause of coronary artery disease is "atherosclerosis" which refers to hardening of the arteries, due to fatty deposits in the artery walls which then may progress through formation of atherosclerotic plaques, to narrowing and eventually blockage of blood flow to the in the artery. This process of atherosclerosis may affect other arteries as well, not just those of the heart. A blood clot is the most common cause of the blockage of the artery, as usually the artery is already partially blocked due to atherosclerotic plaque (atheroma), the atheroma may rupture or tear, leading to the formation of a clot. Occasionally, coronary artery disease is caused by spasm of a coronary artery, which can occur spontaneously or as a result of the use of certain drugs (e.g., cocaine, nicotine). Rarely, the cause of coronary artery disease is a birth defect, a viral infection (e.g., Kawasaki disease), systemic lupus erythematosus (lupus), inflammation of the arteries (arteritis), a blood clot that travelled from a heart chamber into one of the coronary arteries or physical damage (e.g., from injury or radiation therapy).

"Unstable angina", as used herein, refers to a change in the pattern of angina symptoms including prolonged or worsening angina and new onset of severe symptoms.

MI can be classified into two types: "Non-ST-segment elevation" MI and "ST-segment elevation" MI. The complications of acute coronary syndromes depend on how much, how long, and where the coronary artery is blocked. If the blockage affects a large amount of heart muscle, the heart will not pump effectively. If the blockage shuts off blood flow to the electrical system of the heart, the heart rhythm may be affected. When a heart attack occurs, part of the myocardium dies. Dead tissue and the scar tissue that replaces it, does not contract. The scar tissue sometimes even expands or bulges when the rest of the heart tries to contract. Consequently there is less muscle to pump blood. If enough muscle dies, the heart's pumping ability may be so reduced that the heart cannot meet the body's demands for oxygen and blood. Heart failure, low blood pressure or both then develop. If more than half of the myocardium is damaged or dies, the heart generally cannot function and severe disability or death is likely.

As used herein "Heart Failure" (HF) is a progressive disorder of left ventricular (LV) myocardial remodeling that culminates in a complex clinical syndrome in which impaired cardiac function and circulatory congestion are the defining features, and results in insufficient delivery of blood and nutrients to body tissues. The condition occurs when the heart is damaged or overworked and unable to pump out all the blood that returns to it from the systemic circulation. As less blood is pumped out, blood returning to the heart backs up and fluid builds up in other parts of the body. Heart failure also impairs the kidneys' ability to dispose of sodium and water, complicating fluid retention further. Heart failure is characterized by autonomic dysfunction, neuro-hormonal activation and overproduction of cytokines, which contribute to progressive circulatory failure. Symptoms of heart failure include: dyspnea (shortness of breath) while exercising or resting and waking at night due to sudden breathlessness, both indicative of pulmonary edema; general fatigue or weakness; edema of the feet, ankles and legs; rapid weight gain; or chronic cough, including that producing mucus or blood. Depending on its clinical presentation, heart failure is classified as *de novo,* transient, acute, post-acute or chronic. Acute heart failure, i.e., the rapid or gradual onset of symptoms requiring urgent therapy, may develop *de novo* or as a result of chronic heart failure becoming decompensated. The term "Heart failure" is often used to mean "chronic heart failure". The terms "congestive heart failure (CHF)" or "congestive cardiac failure (CCF)" are often used interchangeably with chronic heart failure. Common causes of heart failure include coronary artery disease including a previous myocardial infarction (heart attack), high blood pressure, atrial fibrillation, valvular heart disease, and cardiomyopathy. These cause heart failure by changing either the structure or the functioning of the heart.

There are two main types of heart failure: "heart failure due to reduced ejection fraction (HFREF)", also known as "heart failure due to left ventricular systolic dysfunction" or "systolic heart failure", and "heart failure with preserved ejection fraction (HFPEF)", also known as "diastolic heart failure" or "heart failure with normal ejection fraction (HFNEF)". Ejection fraction is the proportion of blood in the heart pumped out of the heart during a single contraction. It is a percentage with normal being between 50 and 75%.

The term "acute" (as in "acute HF") is used to mean rapid onset, and "chronic" refers to long duration. Chronic heart failure is a long term situation, usually with stable treated symptomatology. "Acute decompensated" heart failure is worsening or decompensated heart failure, referring to episodes in which a person can be characterized as having a change in heart failure signs and symptoms resulting in a need for urgent therapy or hospitalization. Heart failure may also occur in situations of high output (then it is termed "high output cardiac failure") where the ventricular systolic function is normal but the heart cannot deal with an important augmentation of blood volume.

In cardiovascular physiology, the term "Ejection Fraction (EF)" is defined as the fraction of blood in the left and right ventricles that is pumped out with each heartbeat or cardiac cycle. In finite mathematics allowed by medical imaging, EF is applied to both the right ventricle, which ejects blood via the pulmonary valve into the pulmonary circulation, or the left ventricle, which ejects blood via the aortic valve into the cerebral and systemic circulation.

The term "heart failure with preserved ejection fraction (HFPEF)" is commonly understood to refer to a manifestation of signs and symptoms of heart failure with an ejection fraction greater than 55%. It is characterized by a decrease in left ventricular compliance, leading to increased pressure in the left ventricle. Increased left atrial size is often seen with HFPEF as a result of the poor left ventricular function. There is an increased risk for congestive heart failure, atrial fibrillation, and pulmonary hypertension. Risk factors are hypertension, hyperlipidemia, diabetes, smoking, and obstructive sleep apnea. In this type of heart failure, the heart muscle contracts well but the ventricle does not fill with blood well in the relaxation phase.

The term "heart failure with reduced ejection fraction (HFREF)" refers to heart failure in which the ejection fraction is less than 40%.

Diabetes is a common comorbidity in patients with heart failure and is associated with poorer outcomes as well as potentially compromising the efficacy of treatments. Other important comorbidities include systemic hypertension, chronic airflow obstruction, sleep apnea, cognitive dysfunction, anemia, chronic kidney disease and arthritis. Chronic left heart failure is frequently associated with the development of pulmonary hypertension. The frequency of certain comorbidities varies by gender: among women, hypertension and thyroid disease are more common, while men more commonly suffer from chronic obstructive pulmonary disease (COPD), peripheral vascular disease, coronary artery disease and renal insufficiency. Depression is a frequent comorbidity of heart failure and the two conditions can and often do complicate one another. Cachexia has long been recognized as a serious and frequent complication of heart failure, affecting up to 15% of all heart failure patients and being associated with poor prognosis. Cardiac cachexia is defined as the nonedematous, non-voluntary loss of at least 6% of body weight over a period of six months.

The term "arrhythmias", as used herein, refers to abnormal heart rhythms that occur in more than 90 % of people who have had a heart attack. Sometimes the problem is with the part of the heart that triggers the heartbeat and the heart rate may be too slow, other times the problems may cause the heart to beat too rapidly or irregularly. Sometimes the signal to beat is not conducted from one part of the heart to the other and the heartbeat may slow or stop. In addition areas of the myocardium that have not died but have poor blood flow may be irritable. This causes heart rhythm problems such as ventricular tachycardia or ventricular fibrillation. This may lead to cardiac arrest if the heart stops pumping entirely.

The "pericardium" is the sack or membrane that surrounds the heart. "Pericarditis" or inflammation of this membrane may develop as a result of a heart attack and may result in fever, pericardial effusion, inflammation of the membranes covering the lungs (pleura), pleural effusion, and joint pain. Other complications after a heart attack may include malfunction of the mitral valve, rupture of the heart muscle, a bulge in the wall of the ventricle (ventricular aneurysm), blood clots, and low blood pressure.

The term "cardiomyopathy" refers to the progressive impairment of the structure and function of the muscular walls of the heart chambers. The main types of cardiomyopathies are dilated, hypertrophic and restrictive. Cardiomyophaties often cause symptoms of heart failure, and they may also cause chest pain, fainting and sudden death.

The terms "mitral valve regurgitation", "mitral regurgitation", "mitral insufficiency" or "mitral incompetence" refer to a situation in which the mitral valve of the heart doesn't close tightly, allowing blood to flow backward in the heart. As a result, blood can't move through the heart or to the rest of the body as efficiently, resulting in fatigue or shortness of breath.

The term "sleep apnea" refers to the most common of the sleep-disordered breathing disorders. It is a condition characterized by intermittent, cyclical reductions or total cessations of airflow, which may or may not involve obstruction of the upper airway. There are three types of sleep apnea: obstructive sleep apnea, the most common form, central sleep apnea and mixed sleep apnea.

"Central sleep apnea (CSA)", is caused by a malfunction in the brain's normal signal to breathe, rather than physical blockage of the airway. The lack of respiratory effort leads to an increase in carbon dioxide in the blood, which may rouse the patient. CSA is rare in the general population, but is a relatively common occurrence in patients with systolic heart failure.

As used herein, the term "metabolic syndrome", "insulin resistance syndrome" or "syndrome X", refers to a group or clustering of metabolic conditions (abdominal obesity, elevated fasting glucose, "dyslipidemia" (i.e,. elevated lipid levels) and elevated blood pressure (HBP)) which occur together more often than by chance alone and that together promote the development of type 2 diabetes and cardiovascular disease. Metabolic syndrome is characterized by a specific lipid profile of increased triglycerides, decreased high-density lipoprotein cholesterol (HDL-cholesterol) and in some cases moderately elevated low-density lipoprotein cholesterol (LDL-cholesterol) levels, as well as accelerated progression of "atherosclerotic disease" due to the pressure of the component risk factors. There are several types of dyslipidemias: "hypercholesterolemia" refers to elevated levels of cholesterol. Familial hypercholesterolemia is a specific form of hypercholesterolemia due to a defect on chromosome 19 (19p13.1-13.3). "Hyperglyceridemia" refers to elevated levels of glycerides (e.g., "hypertrigliceridemia" involves elevated levels of triglycerides). "Hyperlipoproteinemia" refers to elevated levels of lipoproteins (usually LDL unless otherwise specified).

The term "steatosis" refers to the abnormal retention of lipids within a cell. It usually reflects an impairment of the normal processes of synthesis and elimination of triglycerides. Excess fat accumulates in vesicles that displace the cytoplasm of the cell. In severe cases the cell may burst. Usually steatosis is observed in the liver as it is the organ mostly associated with fat metabolism. It can also be observed in the heart, kidneys and muscle tissue.

As used herein, the term "peripheral vascular disease (PVD)", also commonly referred to as "peripheral arterial disease (PAD)" or "peripheral artery occlusive disease (PAOD)", refers to the obstruction of large arteries *not* within the coronary, aortic arch vasculature, or the brain. PVD can result from atherosclerosis, inflammatory processes leading to stenosis, an embolism, thrombus formation or other types of occlusions. It causes either acute or chronic "ischemia (lack of blood supply)". Often PVD is a term used to refer to atherosclerotic blockages found in the lower extremity. PVD also includes a subset of diseases classified as microvascular diseases resulting from episodic narrowing of the arteries (e.g., "Raynaud's phenomenon"), or widening thereof (erythromelalgia), i.e., vascular spasms. Peripheral arterial diseases include occlusive thrombotic vasculitis, peripheral arterial occlusive disease, Raynaud's disease, and Raynaud's syndrome. Common symptoms are cold leg or feet, intermittent claudication, lower limb pain and critical limb ischemia (lower limb ulcers and necrosis). Diagnosis and treatment guidelines for peripheral arterial disease can be found in Eur. J. Vasco Endovasc. Surg, 2007, 33(1), S1.

The term "stenosis" as used herein refers to an abnormal narrowing in a blood vessel or other tubular organ or structure. It is also sometimes called a "stricture" (as in urethral stricture). The term "coarctation" is a synonym, but is commonly used only in the context of aortic coarctation. The term "restenosis" refers to the recurrence of stenosis after a procedure.

The term "thrombosis" refers to the formation of a blood clot ("thrombus") inside a blood vessel, obstructing the flow of blood through the circulatory system. When a blood vessel is injured, the body uses platelets (thrombocytes) and fibrin to form a blood clot to prevent blood loss. Alternatively, even when a blood vessel is not injured, blood clots may form in the body if the proper conditions present themselves. If the clotting is too severe and the clot breaks free, the traveling clot is now known as an "embolus". The term "thromboembolism" refers to the combination of thrombosis and its main complication, "embolism". When a thrombus occupies more than 75% of surface area of the lumen of an artery, blood flow to the tissue supplied is reduced enough to cause symptoms because of decreased oxygen (hypoxia) and accumulation of metabolic products like lactic acid ("gout"). More than 90% obstruction can result in anoxia, the complete deprivation of oxygen and "infarction", a mode of cell death.

An "embolism" (plural embolisms) is the event of lodging of an embolus (a detached intravascular mass capable of clogging arterial capillary beds at a site far from its origin) into a narrow capillary vessel of an arterial bed which causes a blockage (vascular occlusion) in a distant part of the body. This is not to be confused with a thrombus which blocks at the site of origin. The material that forms the embolism can have a number of different origins: if the material is blood the "embolus" is termed a "thrombus"; the solid material could also comprise fat, bacterial remains, infected tissue, etc.

"Ischemia" is a restriction in blood supply to tissues, causing a shortage of oxygen and glucose needed for cellular metabolism (to keep tissue alive). Ischemia is generally caused by problems with blood vessels, with resultant damage to or dysfunction of tissue. It also means local anemia in a given part of a body sometimes resulting from congestion (such as vasoconstriction, thrombosis or embolism). If the "ischemia" takes place in the heart muscle (or "myocardium") the ischemia is termed myocardial ischemia. Other types of ischemia are for instance cerebral ischemia, critical limb ischemia and the like.

"Reperfusion" occurs when blood supply returns to the tissue after a period of ischemia. Upon restoration of circulation to the tissue, inflammatory and oxidative stress processes may develop. One example of this chain of events is ischemia-reperfusion associated with organ transplants.

"Reperfusion injury" is the tissue damage caused when blood supply returns to the tissue after a period of ischemia and inflammation and oxidative damage ensue rather than restoration of normal function. Reperfusion of ischemic issues is often associated with microvascular injury, particularly due to the increased permeability of capillaries and arterioles that lead to an increase in diffusion and fluid filtration across the tissues. The activated endothelial cells produce more reactive oxygen species but less NO following reperfusion, and the imbalance results in an inflammatory response. White blood cells, carried to the area by the newly returned blood flow, release a host of inflammatory factors and free radicals in response to tissue damage. The restored blood flow brings with it oxygen that damages cellular proteins, DNA and plasma membranes. This process of ischemia-reperfusion is also thought to be responsible for formation and failure to heal of chronic wounds, (e.g., pressure sores or diabetic ulcers).

The term "angiopathy" as used herein is the generic term for a disease of the blood vessels (arteries, veins, and capillaries). The most common and most prevalent angiopathy is "diabetic angiopathy", a common complication of chronic diabetes. Another common type of angiopathy is "cerebral amyloid angiopathy" (CAA), also known as congophilic angiopathy, wherein amyloid deposits form in the walls of the blood vessels of the central nervous system. The term *congophilic* is used because the presence of the abnormal aggregations of amyloid can be demonstrated by microscopic examination of brain tissue after application of a special stain called Congo red. The amyloid material is only found in the brain and as such the disease is not related to other forms of amyloidosis.

A "stroke", or cerebrovascular accident (CVA), is the rapid loss of brain function(s) due to disturbance in the blood supply to the brain. This can be due to "ischemia" (lack of blood flow with resultant insufficient oxygen and glucose supply to the tissue) caused by blockage (thrombosis, arterial embolism, fat accumulation or a spasm), or a hemorrhage (leakage of blood). As a result, the affected area of the brain cannot function, which might result in an inability to move one or more limbs on one side of the body, inability to understand or formulate speech, or an inability to see one side of the visual field. Risk factors for stroke include old age, hypertension, previous stroke or transient ischemic attack (TIA), diabetes, high cholesterol, cigarette smoking and atrial fibrillation. High blood pressure is the most important modifiable risk factor of stroke. An "ischemic stroke" is occasionally treated in a hospital with thrombolysis (also known as a "clot buster"), and some hemorrhagic strokes benefit from neurosurgery. Prevention of recurrence may involve the administration of antiplatelet drugs such as aspirin and dipyridamole, control and reduction of hypertension, and the use of statins. Selected patients may benefit from carotid endarterectomy and the use of anticoagulants.

"Vascular dementia" is the 2nd most common cause of dementia among the elderly. It is more common among men and usually begins after age 70. It occurs more often in people who have vascular risk factors (e.g, hypertension, diabetes mellitus, hyperlipidemia, smoking) and in those who have had several strokes. Many people have both vascular dementia and Alzheimer disease. Vascular dementia typically occurs when multiple small cerebral infarcts (or sometimes hemorrhages) cause enough neuronal or axonal loss to impair brain function. Vascular dementias include the following types: multiple lacunar infarction (wherein small blood vessels are affected and infarcts occur deep within hemispheric white and gray matter); multi-infarct dementia (wherein medium-sized blood vessels are affected); strategic single-infarct dementia (wherein a single infarct occurs in a crucial area of the brain such as the angular gyrus or the thalamus; Binswanger dementia or subcortical arteriosclerotic encephalopathy (wherein small-vessel dementia is associated with severe, poorly controlled hypertension and systemic vascular disease and which causes diffuse and irregular loss of axons and myelin with widespread gliosis, tissue death due to an infarction, or loss of blood supply to the white matter of the brain).

The term "glioma" refers to a type of tumor that starts in the brain or spine. It is called a glioma because it arises from glial cells. The most common site of gliomas is the brain. Gliomas make up about 30% of all brain and central nervous system tumors and 80% of all malignant brain tumors.

According to the American Psychiatric Association's Diagnostic and Statistical Manual of Mental Disorders, Fourth Edition (DSM-IV), the term "sexual dysfunction" encompasses a series of conditions "characterized by disturbances in sexual desire and in the psychophysiological changes associated with the sexual response cycle"; while problems of this type are common, sexual dysfunction is only considered to exist when the problems cause distress for the patient. Sexual dysfunction can be either physical or psychological in origin. It can exist as a primary condition, generally hormonal in nature, although most often it is secondary to other medical conditions or to drug therapy for said conditions. All types of sexual dysfunction can be further classified as life-long, acquired, situational or generalized (or combinations thereof).

The DSM-IV-TR specifies five major categories of "female sexual dysfunction": sexual desire/interest disorders; "sexual arousal disorders (including genital, subjective and combined)"; orgasmic disorder; dyspareunia and vaginismus; and persistent sexual arousal disorder.

"Female sexual arousal disorder (FSAD)" is defined as a persistent or recurring inability to attain or maintain sufficient levels of sexual excitement, causing personal distress. FSAD encompasses both the lack of subjective feelings of excitement (i.e., subjective sexual arousal disorder) and the lack of somatic responses such as lubrication and swelling (i.e., genital/physical sexual arousal disorder). FSAD may be strictly psychological in origin, although it generally is caused or complicated by medical or physiological factors. Hypoestrogenism is the most common physiologic condition associated with FSAD, which leads to urogenital atrophy and a decrease in vaginal lubrication.

As used herein, "erectile dysfunction (ED)" is a male sexual dysfunction characterized by the inability to develop or maintain an erection of the penis during sexual performance. A penile erection is the hydraulic effect of blood entering and being retained in sponge-like bodies within the penis. The process is often initiated as a result of sexual arousal, when signals are transmitted from the brain to nerves in the penis. Erectile dysfunction is indicated when an erection is difficult to produce. The most important organic causes are cardiovascular disease and diabetes, neurological problems (for example, trauma from prostatectomy surgery), hormonal insufficiencies (hypogonadism) and drug side effects.

In one embodiment, compounds of **Formula I** that are stimulators of sGC, and their pharmaceutically acceptable salts thereof, are therefore useful in the prevention and/or treatment of the following types of cardiac, pulmonary, peripheral, hepatic, kidney, or cerebral vascular/endothelial disorders, conditions and diseases related to circulation:
- disorders related to high blood pressure and decreased coronary blood flow such as increased acute and chronic coronary blood pressure, arterial hypertension and vascular disorder resulting from cardiac and renal complications (e.g., heart disease, stroke, cerebral ischemia, renal failure); resistant hypertension; diabetic hypertension; essential hypertension; secondary hypertension;
- heart failure, HFPEF, HFREF; acute and chronic HF, and more specific forms of the disease (e.g., acute decompensated HF, right ventricular failure, left ventricular failure, total HF, ischemic cardiomyopathy, dilatated cardiomyopathy, congenital heart defects, HF with valvular defects, mitral valve stenosis, mitral valve insufficiency, aortic valve stenosis, aortic valve insufficiency, tricuspid stenosis, tricuspic insufficiency, pulmonary valve stenosis, pulmonar valve insufficiency, combined valvular defects; diabetic heart failure; alcoholic cardiomyopathy, storage cardiomyopathies; diastolic HF, systolic HF, acute phases of an existing chronic HF (worsening HF); diastolic or systolic dysfunction; coronary insufficiency; arrhythmias; reduction of ventricular preload; cardiac hypertrophy; heart failure/cardiorenal syndrome; portal hypertension; endothelial dysfunction or injury; disturbances of atrial and ventricular rhythm and conduction disturbances (e.g., atrioventricular blocks of degree I-III (AVB I-III), supraventricular tachyarrhythmia, atrial fibrillation, atrial flutter, ventricular fibrillation, ventricular flutter, ventricular tachyarrhythmia, torsade-de-pointes tachycardia, atrial and ventricular extrasystoles, AV-junction extrasystoles, sick-sinus syndrome, syncopes, AV-node reentry tachycardia; Wolff-Parkinson-White syndrome, acute coronary syndrome); Boxer cardiomyopathy; premature ventricular contraction;
- thromboembolic disorders and ischemias such as myocardial ischemia, infarction, heart attack, myocardial insufficiency, endothelial dysfunction, stroke, transient ischemic attacks (TIAs); obstructive thromboanginitis; stable or unstable angina pectoris; coronary spasms, spasms of the peripheral arteries; variant angina, Prinzmetal's angina; stroke; cardiac hypertrophy; preeclampsia; thrombogenic disorders; ischemia-reperfusion damage; ischemia-reperfusion associated with organ transplant (e.g., lung transplant, pulmonary transplant, cardiac transplant); conserving blood substituents in trauma patients;
- peripheral arterial disease, peripheral occlusive arterial disease; peripheral vascular disease; hypertonia; Raynaud's syndrome or phenomenon (primary and secondary); Raynaud's disease, critical limb ischemia; peripheral embolism; intermittent claudication; vaso-occlusive crisis; Duchenne and Becker muscular dystrophies; microcirculation abnormalities; control of vascular leakage or permeability; lumbar spinal canal stenosis; occlusive thrombotic vasculitis; thrombotic vasculitis; peripheral perfusion disturbances; arterial and venous thromboses; microalbuminuria; peripheral and autonomic neuropathies; diabetic microangiopathies;
- edema, for instance renal edema due to HF;
- Alzheimer's disease; Parkinson's disease; vascular dementias; vascular cognitive impairment; cerebral vasospasm; traumatic brain injury; improving perception, capacity for concentration, capacity for learning or memory performance after cognitive disturbances such as those occurring in mild cognitive impairment, age-related learning and memory disturbances, age-related memory loss, vascular dementia, head injury, stroke, post-stroke dementia, post-traumatic head injury, general disturbances of concentration and disturbances of concentration in children with learning and memory problems; Lewy body dementia, dementia with frontal lobe degeneration including Pick's syndrome; progressive nuclear palsy; dementia with corticobasal degeneration; Amyotropic Lateral Sclerosis (ALS); Huntington's disease; demyelination, Multiple Sclerosis, thalamic degeneration; Creutzfeldt-Jakob dementia, HIV-dementia, schizophrenia with dementia or Korsakoff psychosis; Multiple Systems atrophy and other forms of Parkinsonism Plus; movement disorders; neuroprotection; anxiety, tension and depression, post-traumatic stress disorder (PTSD); CNS-related sexual dysfunction and sleep disturbances; pathological eating disorders and use of luxury foods and addictive drugs; controlling cerebral perfusion, controlling migraines; prophylaxis and control of consequences of cerebral infarction (apoplexia cerebri) such as stroke, cerebral ischemias and head injury;
- Shock; cardiogenic shock; sepsis or septic shock or anaphylactic shock; aneurysm; control of leukocyte activation; inhibition or modulation of platelet aggregation; multiple organ dysfunction syndrome or multiple organ failure (MODS, MOF);
- pulmonary/respiratory conditions such as pulmonary hypertension (PH), pulmonary arterial hypertension (PAH), and associated pulmonary vascular remodeling (e.g., localized thrombosis and right heart hypertrophy); pulmonary hypertonia; primary pulmonary hypertension, secondary pulmonary hypertension, familial pulmonary hypertension, sporadic pulmonary hypertension, pre-capillary pulmonary hypertension, idiopathic pulmonary hypertension; thrombotic pulmonary arteriopathy, plexogenic pulmonary arteriopathy; cystic fibrosis; bronchoconstriction or pulmonary bronchoconstriction; acute respiratory distress syndrome; lung fibrosis, lung transplant; asthmatic diseases; other forms of PH (e.g., PH associated with left ventricular disease, HIV, SCD, thromboembolism (CTEPH), sarcoidosis, COPD or pulmonary fibrosis, acute respiratory distress syndrome (ARDS), acute lung injury, alpha-1-antitrypsin deficiency (AATD), pulmonary emphysema (e.g., smoking-induced) and cystic fibrosis (CF));
- pulmonary hypertension associated with or related to: left ventricular dysfunction, hypoxemia, WHO groups I, II, III, IV and V hypertensions, mitral valve disease, constrictive pericarditis, aortic stenosis, cardiomyopathy, mediastinal fibrosis, pulmonary fibrosis, anomalous pulmonary venous drainage, pulmonary veno-occlusive disease, pulmonary vasculitis, collagen vascular disease, congenital heart disease, pulmonary venous hypertension, interstitial lung disease, sleep-disordered breathing, sleep apnea, alveolar hypoventilation disorders, chronic exposure to high altitude, neonatal lung disease, alveolar-capillary dysplasia, sickle cell disease, other coagulation disorders, chronic thromboembolism, pulmonary embolism (due to tumor, parasites or foreign material), connective tissue disease, lupus, schistosomiasis, sarcoidosis, chronic obstructive pulmonary disease, asthma, emphysema, chronic bronchitis, pulmonary capillary hemangiomatosis, histiocytosis X, lymphangiomatosis and compressed pulmonary vessels (such as due to adenopathy, tumor or fibrosing mediastinitis);
- arterosclerotic diseases or conditions such as atherosclerosis (e.g., associated with endothelial injury, platelet and monocyte adhesion and aggregation, smooth muscle proliferation and migration); restenosis (e.g., developed after thrombolysis therapies, percutaneous transluminal angioplasties (PTAs), transluminal coronary angioplasties (PTCAs), heart transplant and bypass operations); inflammatory processes;
- micro and macrovascular damage (vasculitis), increased levels of fibrinogen and low density DLD, increased concentration of plasminogen activator inhibitor 1 (PA-1);
- diseases associated with metabolic syndrome (e.g., obesity, dyslipidemia, diabetes, high blood pressure); lipid related disorders such as dyslipidemia, hypercholesterolemias, decreased high-density lipoprotein cholesterol (HDL-cholesterol) and in some cases moderately elevated low-density lipoprotein cholesterol (LDL-cholesterol) levels, hypertriglyceridemias, hyperglyceridemia, hypolipoproteinanemias, sitosterolemia, fatty liver disease, and hepatitis; preeclampsia; polycystic kidney disease progression; subcutaneous fat; obesity; liver steatosis or abnormal lipid accumulation in the liver; steatosis of the heart, kidneys or muscle; abetalipoproteinemia; sitosterolemia; xanthomatosis; Tangier disease; adiposity; combined hyperlipidemias and metabolic syndrome; hyperammonemia and related diseases and disorders such as hepatic encephalopaties and other toxic encephalopaties and Reye syndrome;
- sexual, gynecological and urological disorders of conditions such as erectile dysfunction; impotence; premature ejaculation; female sexual dysfunction (e.g., female sexual arousal dysfunction, hypoactive sexual arousal disorder), vaginal atrophy, dyspaneuria, atrophic vaginitis; benign prostatic hyperplasia (BPH) or hypertrophy or enlargement; bladder outlet obstruction; bladder pain syndrome (BPS); interstitial cystitis (IC); overactive bladder; neurogenic bladder and incontinence; diabetic nephropathy; primary and secondary dysmenhorrea; lower urinary tract syndromes (LUTS); pelvic pains; benign and malignant diseases of the organs of the male and female urogenital system;
- acute and chronic renal insufficiency, acute and chronic renal failure, as well as underlying or related kidney diseases such as hypoperfusion, intradialytic hypotension, obstructive uropathy, glomerulopathies, glomerulonephritis, acute glomerulonephritis, glomerulosclerosis, tubulointerstitial diseases, nephropathic diseases (e.g., primary and congenital kidney diseases, nephritis); diseases characterized by abnormally reduced creatinine and or water excretion, abnormally increased blood concentrations of urea, nitrogen, potassium and/or creatinine, altered activity of renal enzymes (e.g., glutamyl synthetase), altered urine osmolarity or urine volume, increased microalbuminuria, macroalbuminuria, lesions of glomeruli and arterioles, tubular dilatation, hyperphosphatemia and/or need for dialysis; sequelae of renal insufficiency (e.g., pulmonary enema, HF, uremia, anemia, elecrolyte disturbances (herkalemia, hyponatremia) and disturbances of bone and carbohydrate metabolism;
- ocular diseases or disorders such as glaucoma, retinopathy and diabetic retinopathy;

The term "Inflammation" refers to the complex biological response of vascular tissues to harmful stimuli, such as pathogens, damaged cells, or irritants. The classical signs of acute inflammation are pain, heat, redness, swelling, and loss of function. Inflammation is a protective attempt by the organism to remove the injurious stimuli and to initiate the healing process. Inflammation is not a synonym for infection, even though the two are often correlated (the former often being a result of the latter). Inflammation can also occur in the absence of infection, although such types of inflammation are usually maladaptive (such as in atherosclerosis). Inflammation is a stereotyped response, and therefore it is considered as a mechanism of innate immunity, as compared to adaptive immunity, which is specific for each pathogen. Progressive destruction of tissue in the absence of inflammation would compromise the survival of the organism. On the other hand, chronic inflammation might lead to a host of diseases, such as hay fever, periodontitis, atherosclerosis, rheumatoid arthritis, and even cancer (e.g., gallbladder carcinoma). It is for that reason that inflammation is normally closely regulated by the body. Inflammation can be classified as either *acute* or *chronic.* "Acute inflammation" is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as "chronic inflammation", leads to a progressive shift in the type of cells present at the site of inflammation and is characterized by simultaneous destruction and healing of the tissue from the inflammatory process.

In another embodiment, compounds of **Formula I** that are stimulators of sGC, and their pharmaceutically acceptable salts thereof, are therefore useful in the prevention and/or treatment of the following types of cardiac, pulmonary, peripheral, hepatic, kidney, digestive or Central Nervous System disorders, conditions and diseases which may involve inflammation or an inflammatory process:
- heart muscle inflammation (myocarditis), chronic myocarditis, acute myocarditis, viral myocarditis;
- vasculitis; pancreatitis; peritonitis; rheumatoid diseases;
- inflammatory disease of the kidney; immunological kidney diseases (e.g., kidney transplant rejection, immune complex-induced kidney disease, nephropathy induced by toxins, constrast medium-induced nephropathy); diabetic and non-diabetic nephropathy, pyelonephritis, renal cysts, nephrosclerosis, hypertensive nephrosclerosis and nephrotic syndrome;
- chronic interstitial inflammations (Inflammatory bowel diseases (IBD) such as Crohn's, Ulcerative Colitis (UC));
- inflammatory skin diseases; and
- inflammatory diseases of the eye, such as blepharitis, dry eye syndrome and Sjögren's Syndrome.

The term "wound healing" refers to the intricate process where the skin (or another organ or tissue) repairs itself after injury. For instance, in normal skin, the epidermis (outermost layer) and dermis (inner or deeper layer) exist in a steady-state equilibrium, forming a protective barrier against the external environment. Once the protective barrier is broken, the normal (physiologic) process of wound healing is immediately set in motion. The classic model of wound healing is divided into three or four sequential, yet overlapping, phases: (1) hemostasis (not considered a phase by some authors), (2) inflammation, (3) proliferation and (4) remodeling. Upon injury to the skin, a set of complex biochemical events takes place in a closely orchestrated cascade to repair the damage. Within the first few minutes after the injury, platelets adhere to the site of injury, become activated, and aggregate (join together), followed by activation of the coagulation cascade which forms a clot of aggregated platelets in a mesh of cross-linked fibrin protein. This clot stops active bleeding ("hemostasis"). During the inflammation phase, bacteria and cell debris are phagocytosed and removed from the wound by white blood cells. Platelet-derived growth factors (stored in the alpha granules of the platelets) are released into the wound that cause the migration and division of cells during the proliferative phase. The proliferation phase is characterized by angiogenesis, collagen deposition, granulation tissue formation, epithelialization, and wound contraction. In "angiogenesis", vascular endothelial cells form new blood vessels. In "fibroplasia" and granulation tissue formation, fibroblasts grow and form a new, provisional extracellular matrix (ECM) by excreting collagen and fibronectin. Concurrently, "re-epithelialization" of the epidermis occurs, in which epithelial cells proliferate and 'crawl' atop the wound bed, providing cover for the new tissue. During wound contraction, myofibroblasts decrease the size of the wound by gripping the wound edges and contracting using a mechanism that resembles that in smooth muscle cells. When the cells' roles are close to complete, unneeded cells undergo apoptosis. During maturation and remodeling, collagen is remodeled and realigned along tension lines, and cells that are no longer needed are removed by apoptosis. However, this process is not only complex but fragile, and is susceptible to interruption or failure leading to the formation of non-healing chronic wounds (one example includes diabetic wounds or ulcers, and, in particular, diabetic foot ulcers). Factors that contribute to non-healing chronic wounds are diabetes, venous or arterial disease, infection, and metabolic deficiencies of old age.

The terms "bone healing", or "fracture healing" refers to a proliferative physiological process in which the body facilitates the repair of a bone fracture. In the process of fracture healing, several phases of recovery facilitate the proliferation and protection of the areas surrounding fractures and dislocations. The length of the process depends on the extent of the injury, and usual margins of two to three weeks are given for the reparation of most upper bodily fractures; anywhere above four weeks given for lower bodily injury. The healing process is mainly determined by the "periosteum" (the connective tissue membrane covering the bone). The periosteum is one source of precursor cells which develop into "chondroblasts" and osteoblasts that are essential to the healing of bone. The bone marrow (when present), endosteum, small blood vessels, and fibroblasts are other sources of precursor cells.

In another embodiment, compounds of Formula I, that are stimulators of sGC and their pharmaceutically acceptable salts thereof, are therefore useful in the treatment of the following types of diseases, disorders or conditions in which stimulation of the processes of wound or bone healing would be desirable:
- wound or ulcer healing in diabetics; microvascular perfusion improvement (e.g., following injury, to counteract the inflammatory response in perioperative care); anal fissures; diabetic ulcers (e.g., diabetic foot ulcers); bone healing; osteoclastic bone resorption and remodeling; and new bone formation.

The term "connective tissue" (CT) refers to a kind of animal tissue that supports, connects, or separates different types of tissues and organs of the body. It is one of the four general classes of animal tissues, the others being epithelial, muscle, and nervous tissues. Connective tissue is found everywhere, including in the central nervous system. It is located in between other tissues. All CT has three main components--ground substances, fibers and cells--and all these components are immersed in the body fluids.

The term "connective tissue disorder or condition" refers to any condition that involves abnormalities in connective tissue in one or more parts of the body. Certain disorders are characterized by over-activity of the immune system with resulting inflammation and systemic damage to the tissues, usually with replacement of normal tissue (e.g., normal tissue of a certain organ) with connective tissue. Other disorders involve biochemical abnormalities or structural defects of the connective tissue itself. Some of these disorders are inherited, and some are of unknown etiology.

When connective tissue diseases are of autoimmune origin they are classified as "rheumatic disorders", "autoimmune rheumatic disorders" or "autoimmune collagen-vascular disorders".

In an "autoimmune disorder", antibodies or other cells produced by the body attack the body's own tissues. Many autoimmune disorders affect connective tissue in a variety of organs. In autoimmune disorders, inflammation and the immune response may result in connective tissue damage, around the joints and also in other tissues, including vital organs, such as the kidneys or organs of the gastrointestinal tract. The sac that surrounds the heart (pericardium), the membrane that covers the lungs (pleura), the mediastinum (an undelineated group of structures in the thorax, surrounded by loose connective tissue, containing the heart, the great vessels of the heart, the esophagus, the trachea, the phrenic nerve, the cardiac nerve, the thoracic duct, the thymus, and the lymph nodes of the central chest) and even the brain may be affected.

The term "fibrosis" as used herein refers to the accumulation of connective tissue or fibrous tissue (scar tissue, collagen) in a certain organ or part of the body. If fibrosis arises from a single cell line it is called a "fibroma". Fibrosis occurs as the body attempts to repair and replace damaged cells, and thus can be a reactive, benign or a pathological state. Physiological fibrosis is similar to the process of scarring. A pathological state develops when the tissue in question is repeatedly and continuously damaged. A single episode of injury, even if severe, does not usually cause fibrosis. If injury is repeated or continuous (for instance as it occurs in chronic hepatitis) the body attempts to repair the damage, but the attempts result instead in excessive accumulation of scar tissue. Scar tissue starts to replace regular tissue of the organ which performs certain functions that the scar tissue is not able to perform; it can also interfere with blood flow and limit blood supply to other cells. As a result, these other functional cells start to die and more scar tissue is formed. When this occurs in the liver, blood pressure in the vein that carries blood from the intestine to the liver (portal vein) increases, giving rise to the condition known as "portal hypertension".

The term "sclerosis" refers to the hardening or stiffening of tissue or a structure or organ that would normally be flexible, usually by replacement of normal organ specific tissue with connective tissue.

There are many types of fibroses or fibrotic diseases including but not limited to pulmonary fibrosis (idiopathic pulmonary fibrosis, cystic fibrosis), fibrosis of the liver (or "cirrhosis"), endomyocardial fibrosis, old myocardial infarction, atrial fibrosis, mediastinal fibrosis, myelofibrosis (affecting the bone marrow), retroperitoneal fibrosis, progressive massive fibrosis (affects the lungs), nephrogenic fibrosis (affecting the skin), Crohn's disease, arthrofibrosis, Peyronie's disease (affecting the penis), Dupuytren's contracture (affecting the hands and fingers), some forms of adhesive capsulitis (affecting the shoulders).

There are many types of scleroses or "sclerotic diseases" including but not limited to Amyotropic Lateral Sclerosis (ALS); atherosclerosis; focal segmental glomerulosclerosis and nephrotic syndrome; hippocampal sclerosis (affecting the brain); lichen sclerosus (a disease that hardens connective tissue of the vagina and penis); liver sclerosis (chirrhosis); multiple sclerosis or focal sclerosis (diseases that affects coordination); osteosclerosis (a disease in which bone densitiy is significantly reduced); otosclerosis (disease affecting the ears); tuberous sclerosis (rare genetic disease affecting multiple systems); primary sclerosing cholanginitis (hardening of the bile duct); primary lateral sclerosis (progressive muscle weakness in the voluntary muscles); and keloids.

The term "scleroderma" or "systemic sclerosis" or "progressive systemic scleroderma" refers to a condition which involves scarring of the joints, skin and internal organs as well as blood vessel abnormalities. Systemic sclerosis can sometimes occur in limited forms, for examples sometimes affecting just the skin or mainly only certain parts of the skin or as CREST syndrome (wherein peripheral areas of the skin but not the trunk are involved). The usual initial symptom of systemic sclerosis is swelling, then thickening and tightening of the skin at the end of the fingers. "Raynaud's phenomenon", in which fingers suddenly and temporarily become very pale and tingle or become numb, painful or both, is common.

The term "polymyositis" refers to muscle inflammation. The term "dermatomyositis", refers to muscle inflammation that is accompanied by skin inflammation. The term "polychondritis" refers to cartilage inflammation.

The term "oesinophilic fasciitis" refers to a rare disorder in which oesinophilic immune cells are released and results in inflammation and hardening of the "fasciae" which is the layer of tough fibrous tissue beneath the skin, on top and between the muscles. The fasciae becomes painfully inflamed and swollen and gradually hardens in the arms and legs. As the skin of the arms and legs progressively hardens, they become difficult to move. Eventually the become stuck in unusual positions. Sometimes, if the arms are involved the person may develop carpal tunnel syndrome.

In another embodiment, specific diseases of disorders which may be treated and/or prevented by administering an sGC stimulator of **Formula I** that are stimulators of sGC, and their pharmaceutically acceptable salts thereof, include but are not limited to the following type of diseases involving inflammation, autoimmunity or fibrosis (i.e., fibrotic diseases):
- urogenital system disorders, such as diabetic nephropathy; renal fibrosis and renal failure resulting from chronic kidney diseases or insufficiency (e.g., due to accumulation/ deposition and tissue injury); progressive sclerosis; glomerulonephritis; focal segmental glomerulosclerosis and nephrotic syndrome; prostate hypertrophy; kidney fibrosis; interstitial renal fibrosis;
- pulmonary system disorders such as pulmonary fibrosis (idiopathic pulmonary fibrosis, cystic fibrosis); progressive massive fibrosis (affects the lungs);
- disorders affecting the heart, such as endomyocardial fibrosis; old myocardial infarction; atrial fibrosis; cardiac interstitial fibrosis; cardiac remodeling and fibrosis; cardiac hypertrophy;
- disorders of the liver and related organs, such as liver sclerosis or cirrhosis; liver cirrhosis associated with chronic liver disease; hepatic fibrosis; hepatic stellate cell activation; hepatic fibrous collagen and total collagen accumulation; liver disease of necro-inflammatory and/or of immunological origin; portal hypertension; primary biliary cirrhosis; primary sclerosing cholanginitis; other cholestatic liver diseases, for instance those associated with granulomatous liver diseases, liver malignancies, intrahepatic cholestasis of pregnancy, hepatitis, sepsis, drugs or toxins, graft-versus-host disease, post-liver transplantation, choledocholithiasis, bile duct tumors, pancreatic carcinoma, Mirizzi's syndrome, AIDS cholangiopathy or parasites; schistosomiasis;
- digestive diseases or disorders such as Crohn's disease; Ulcerative Colitis;
- diseases of the skin or the eyes, such as nephrogenic fibrosis; keloids; fibrotic topical or skin disorders or conditions; dermal fibrosis; scleroderma, skin fibrosis; morphea; hypertrophic scars; naevi; proliferative vitroretinopathy; sarcoids; granulomas;
- diseases affecting the nervous system, such as Amyotropic Lateral Sclerosis (ALS); hippocampal sclerosis, multiple sclerosis (MS) or focal sclerosis; primary lateral sclerosis;
- diseases of the bones, such as osteosclerosis;
- otosclerosis; other hearing diseases or disorders; hearing impairement, partial or total hearing loss; partial or total deafness; tinnitus; noise-induced hearing loss;
- other diseases involving autoimmunity, inflammation or fibrosis: scleroderma; localized scleroderma or circumscribed scleroderma; mediastinal fibrosis; fibrosis mediastinitis; myelofibrosis; retroperitoneal fibrosis; arthrofibrosis; Peyronie's disease; Dupuytren's contracture; lichen sclerosus; some forms of adhesive capsulitis; atherosclerosis; tuberous sclerosis; systemic sclerosis; polymyositis; dermatomyositis, polychondritis oesinophilic fasciitis; and Systemic Lupus Erythematosus or lupus; bone marrow fibrosis, myelofibrosis or osteomyelofibrosis; sarcoidosis; uterine fibroids; endometriosis.

In another embodiment, specific diseases of disorders which may be treated and/or prevented by administering an sGC stimulator of **Formula I** that are stimulators of sGC, and their pharmaceutically acceptable salts thereof, include but are not limited to: certain types of cancers; Sickle Cell Disease; Sickle Cell Anemia; cancer metastasis; osteoporosis; gastroparesis; functional dyspepsia; diabetic complications; alopecia or hair loss; diseases associated with endothelial dysfunction; and neurologic disorders associated with decreased nitric oxide production.

In some embodiments, the disclosure relates to a method of treating a disease, health condition or disorder in a subject, comprising administering a therapeutically effective amount of a compound of **Formula I**, or a pharmaceutically acceptable salt thereof, to the subject in need of treatment, wherein the disease, health condition or disorder is selected from one of the diseases listed above.

In another embodiment, compounds of the invention can be delivered in the form of implanted devices, such as stents. A stent is a mesh 'tube' inserted into a natural passage/conduit in the body to prevent or counteract a disease-induced, localized flow constriction. The term may also refer to a tube used to temporarily hold such a natural conduit open to allow access for surgery.

A drug-eluting stent (DES) is a peripheral or coronary stent (a scaffold) placed into narrowed, diseased peripheral or coronary arteries that slowly releases a drug to block cell proliferation, usually smooth muscle cell proliferation. This prevents fibrosis that, together with clots (thrombus), could otherwise block the stented artery, a process called restenosis. The stent is usually placed within the peripheral or coronary artery by an Interventional Cardiologist or Interventional Radiologist during an angioplasty procedure. Drugs commonly used in DES in order to block cell proliferation include paclitaxel or rapamycin analogues.

In some embodiments of the invention, a sGC stimulator of the invention can be delivered by means of a drug-eluting stent coated with said sGC stimulator. A drug-eluting stent coated with a sGC stimulator of the invention may be useful in the prevention of stent restenosis and thrombosis during percutaneous coronary interventions. A drug-eluting stent coated with a sGC stimulator of the invention may be able to prevent smooth cell proliferation as well as to assist re-vascularization and re-generation of the endothelial tissue of the artery in which the stent is inserted.

An alternative to percutaneous coronary intervention for the treatment of intractable angina due to coronary artery occlusive disease is the procedure named Coronary Artery Bypass Grafting (CABG). CABG provides only palliation of an ongoing process that is further complicated by the rapid development of graft atherosclerosis. The saphenous vein graft is the most commonly used conduit in CABG surgery. The long-term clinical success of venous CABG is hampered for three main reasons: accelerated graft atherosclerosis, incomplete endothelialization and thrombosis.

In some embodiments, a sGC stimulator of the invention can be used for the prevention of saphenous graft failure during CABG. Compounds of the invention may assist the process of endothelialization and help prevent thrombosis. In this indication, the sGC stimulator is delivered locally in the form of a gel.

The terms, "disease", "disorder" and "condition" may be used interchangeably here to refer to an sGC, cGMP and/or NO mediated medical or pathological condition.

As used herein, the terms "subject" and "patient" are used interchangeably. The terms "subject" and "patient" refer to an animal (e.g., a bird such as a chicken, quail or turkey, or a mammal), specifically a "mammal" including a non-primate (e.g., a cow, pig, horse, sheep, rabbit, guinea pig, rat, cat, dog, and mouse) and a primate (e.g., a monkey, chimpanzee and a human), and more specifically a human. In some embodiments, the subject is a non-human animal such as a farm animal (e.g., a horse, cow, pig or sheep), or a pet (e.g., a dog, cat, guinea pig or rabbit). In some embodiments, the subject is a human.

The disclosure also provides a method for treating one of the above diseases, conditions and disorders in a subject, comprising administering a therapeutically effective amount of a compound of Table I, or a pharmaceutically acceptable salt thereof, to the subject in need of the treatment. Alternatively, the invention provides the use of a compound of Table I, or a pharmaceutically acceptable salt thereof, in the treatment of one of these diseases, conditions and disorders in a subject in need of the treatment. The invention further provides a method of making or manufacturing a medicament useful for treating one of these diseases, conditions and disorders comprising using a compound of Table I, or a pharmaceutically acceptable salt thereof.

The term "biological sample", as used herein, refers to an *in vitro* or *ex vivo* sample, and includes, without limitation, cell cultures or extracts thereof; biopsied material obtained from a mammal or extracts thereof; blood, saliva, urine, faeces, semen, tears, lymphatic fluid, ocular fluid, vitreous humour, or other body fluids or extracts thereof.

"Treat", "treating" or "treatment" with regard to a disorder or disease refers to alleviating or abrogating the cause and/or the effects of the disorder or disease. As used herein, the terms "treat", "treatment" and "treating" refer to the reduction or amelioration of the progression, severity and/or duration of an sGC, cGMP and/or NO mediated condition, or the amelioration of one or more symptoms (preferably, one or more discernable symptoms) of said condition (i.e., "managing" without "curing" the condition), resulting from the administration of one or more therapies (e.g., one or more therapeutic agents such as a compound or composition of the invention). In specific embodiments, the terms "treat"; "treatment" and "treating" refer to the amelioration of at least one measurable physical parameter of an sGC, cGMP and/or NO mediated condition. In other embodiments the terms "treat", "treatment" and "treating" refer to the inhibition of the progression of an sGC, cGMP and/or NO mediated condition, either physically by, e.g., stabilization of a discernable symptom or physiologically by, e.g., stabilization of a physical parameter, or both.

The term "preventing" as used herein refers to administering a medicament beforehand to avert or forestall the appearance of one or more symptoms of a disease or disorder. The person of ordinary skill in the medical art recognizes that the term "prevent" is not an absolute term. In the medical art it is understood to refer to the prophylactic administration of a drug to substantially diminish the likelihood or seriousness of a condition, or symptom of the condition and this is the sense intended in this disclosure. The Physician's Desk Reference, a standard text in the field, uses the term "prevent" hundreds of times. As used therein, the terms "prevent", "preventing" and "prevention" with regard to a disorder or disease, refer to averting the cause, effects, symptoms or progression of a disease or disorder prior to the disease or disorder fully manifesting itself.

In one embodiment, the methods of the disclosure are a preventative or "pre-emptive" measure to a patient, specifically a human, having a predisposition (e.g., a genetic predisposition) to developing an sGC, cGMP and/or NO related disease, disorder or symptom.

In other embodiments, the methods of the disclosure are a preventative or "pre-emptive" measure to a patient, specifically a human, suffering from a disease, disorder or condition that makes him at risk of developing an sGC, cGMP or NO related disease, disorder or symptom.

The compounds and pharmaceutical compositions described herein can be used alone or in combination therapy for the treatment or prevention of a disease or disorder mediated, regulated or influenced by sGC, cGMP and/or NO.

Compounds and compositions here disclosed are also useful for veterinary treatment of companion animals, exotic animals and farm animals, including, without limitation, dogs, cats, mice, rats, hamsters, gerbils, guinea pigs, rabbits, horses, pigs and cattle.

In other embodiments, the disclosure provides a method of stimulating sGC activity in a biological sample, comprising contacting said biological sample with a compound or composition of the invention. Use of a sGC stimulator in a biological sample is useful for a variety of purposes known to one of skill in the art. Examples of such purposes include, without limitation, biological assays and biological specimen storage.

### Combination Therapies

The compounds and pharmaceutical compositions described herein can be used in combination therapy with one or more additional therapeutic agents. For combination treatment with more than one active agent, where the active agents are in separate dosage formulations, the active agents may be administered separately or in conjunction. In addition, the administration of one element may be prior to, concurrent to, or subsequent to the administration of the other agent.

When co-administered with other agents, e.g., when co-administered with another pain medication, an "effective amount" of the second agent will depend on the type of drug used. Suitable dosages are known for approved agents and can be adjusted by the skilled artisan according to the condition of the subject, the type of condition(s) being treated and the amount of a compound described herein being used. In cases where no amount is expressly noted, an effective amount should be assumed. For example, compounds described herein can be administered to a subject in a dosage range from between about 0.01 to about 10,000 mg/kg body weight/day, about 0.01 to about 5000 mg/kg body weight/day, about 0.01 to about 3000 mg/kg body weight/day, about 0.01 to about 1000 mg/kg body weight/day, about 0.01 to about 500 mg/kg body weight/day, about 0.01 to about 300 mg/kg body weight/day, about 0.01 to about 100 mg/kg body weight/day.

When "combination therapy" is employed, an effective amount can be achieved using a first amount of a compound of Table I or a pharmaceutically acceptable salt thereof and a second amount of an additional suitable therapeutic agent.

In one embodiment of this invention, a compound of Table I and the additional therapeutic agent are each administered in an effective amount (i.e., each in an amount which would be therapeutically effective if administered alone). In another embodiment, the compound of Table I and the additional therapeutic agent are each administered in an amount which alone does not provide a therapeutic effect (a sub-therapeutic dose). In yet another embodiment, the compound of Table I can be administered in an effective amount, while the additional therapeutic agent is administered in a sub-therapeutic dose. In still another embodiment, the compound of Table I can be administered in a sub-therapeutic dose, while the additional therapeutic agent, for example, a suitable cancer-therapeutic agent is administered in an effective amount.

As used herein, the terms "in combination" or "co-administration" can be used interchangeably to refer to the use of more than one therapy (e.g., one or more prophylactic and/or therapeutic agents). The use of the terms does not restrict the order in which therapies (e.g., prophylactic and/or therapeutic agents) are administered to a subject.

Co-administration encompasses administration of the first and second amounts of the compounds in an essentially simultaneous manner, such as in a single pharmaceutical composition, for example, capsule or tablet having a fixed ratio of first and second amounts, or in multiple, separate capsules or tablets for each. In addition, such co administration also encompasses use of each compound in a sequential manner in either order. When co-administration involves the separate administration of the first amount of a compound of Table I and a second amount of an additional therapeutic agent, the compounds are administered sufficiently close in time to have the desired therapeutic effect. For example, the period of time between each administration which can result in the desired therapeutic effect, can range from minutes to hours and can be determined taking into account the properties of each compound such as potency, solubility, bioavailability, plasma half-life and kinetic profile. For example, a compound of Table I and the second therapeutic agent can be administered in any order within about 24 hours of each other, within about 16 hours of each other, within about 8 hours of each other, within about 4 hours of each other, within about 1 hour of each other or within about 30 minutes of each other.

More, specifically, a first therapy (e.g., a prophylactic or therapeutic agent such as a compound described herein) can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (e.g., a prophylactic or therapeutic agent such as an anti-cancer agent) to a subject.

Examples of other therapeutic agents that may be combined with a compound of this disclosure, either administered separately or in the same pharmaceutical composition include, but are not limited to:
(1) Endothelium-derived releasing factor (EDRF);
(2) NO donors such as a nitrosothiol, a nitrite, a sydnonimine, a NONOate, a N-nitrosoamine, a N-hydroxyl nitrosamine, a nitrosimine, nitrotyrosine, a diazetine dioxide, an oxatriazole 5-imine, an oxime, a hydroxylamine, a N-hydroxyguanidine, a hydroxyurea or a furoxan. Some examples of these types of compounds include: glyceryl trinitrate (also known as GTN, nitroglycerin, nitroglycerine, and trinitrogylcerin), the nitrate ester of glycerol; sodium nitroprusside (SNP), wherein a molecule of nitric oxide is coordinated to iron metal forming a square bipyramidal complex; 3-morpholinosydnonimine (SIN-1), a zwitterionic compound formed by combination of a morpholine and a sydnonimine; S-nitroso-N-acetylpenicillamine (SNAP), an N-acetylated amino acid derivative with a nitrosothiol functional group; diethylenetriamine/NO (DETA/NO), a compound of nitric oxide covalently linked to diethylenetriamine; and NCX 4016, an m-nitroxymethyl phenyl ester of acetyl salicylic acid. More specific examples of some of these classes of NO donors include: the classic nitrovasodilators, such as organic nitrate and nitrite esters, including nitroglycerin, amyl nitrite, isosorbide dinitrate, isosorbide 5-mononitrate, and nicorandil; Isosorbide (Dilatrate®-SR , Imdur®, Ismo®, Isordil®, Isordil®, Titradose®, Monoket®), FK 409 (NOR-3); FR 144420 (NOR-4); 3-morpholinosydnonimine; Linsidomine chlorohydrate ("SIN-1"); S-nitroso-N-acetylpenicillamine ("SNAP"); AZD3582 (CINOD lead compound), NCX 4016, NCX 701, NCX 1022, HCT 1026, NCX 1015, NCX 950, NCX 1000, NCX 1020, AZD 4717, NCX 1510/NCX 1512, NCX 2216, and NCX 4040 (all available from NicOx S.A.), S-nitrosoglutathione (GSNO), Sodium Nitroprusside, S-nitrosoglutathione mono-ethyl-ester (GSNO-ester), 6-(2-hydroxy-1-methyl-nitrosohydrazino)-*N*-methyl-1-hexanamine (NOC-9) or diethylamine NONOate. Nitric oxide donors are also as disclosed in U.S. Pat. Nos. 5,155,137, 5,366,997, 5,405,919, 5,650,442, 5,700,830, 5,632,981, 6,290,981, 5,691,423 5,721,365, 5,714,511, 6,511,911, and 5,814,666, Chrysselis et al. (2002) J Med Chem. 45:5406-9 (such as NO donors 14 and 17), and Nitric Oxide Donors for Pharmaceutical and Biological Research, Eds: Peng George Wang, Tingwei Bill Cai, Naoyuki Taniguchi, Wiley, 2005;
(3) Other substances that enhance cGMP concentrations such as protoporphyrin IX, arachidonic acid and phenyl hydrazine derivatives;
(4) Nitric Oxide Synthase substrates: for example, n-hydroxyguanidine based analogs, such as N[G]-hydroxy-L-arginine (NOHA), 1-(3, 4-dimethoxy-2-chlorobenzylideneamino)-3-hydroxyguanidine, and PR5 (1-(3, 4-dimethoxy-2-chlorobenzylideneamino)-3-hydroxyguanidine); L-arginine derivatives (such as homo-Arg, homo-NOHA, N-tert-butyloxy- and N-(3-methyl-2-butenyl)oxy-L-arginine, canavanine, epsilon guanidine-carpoic acid, agmatine, hydroxyl-agmatine, and L-tyrosyl-L-arginine); N-alkyl-N'-hydroxyguanidines (such as N-cyclopropyl-N'-hydroxyguanidine and N-butyl-N'-hydroxyguanidine), N-aryl-N'-hydroxyguanidines (such as N-phenyl-N'-hydroxyguanidine and its para-substituted derivatives which bear -F, -Cl, -methyl, -OH substituents, respectively); guanidine derivatives such as 3-(trifluormethyl) propylguanidine; and others reviewed in Cali et al. (2005, Current Topics in Medicinal Chemistry 5:721-736) and disclosed in the references cited therein;
(5) Compounds which enhance eNOS transcription: for example those described in WO 02/064146, WO 02/064545, WO 02/064546 and WO 02/064565, and corresponding patent documents such as US2003/0008915, US2003/0022935, US2003/0022939 and US2003/0055093. Other eNOS transcriptional enhancers including those described in US20050101599 (e.g., 2,2-difluorobenzo[1,3]dioxol-5-carboxylic acid indan-2-ylamide, and 4-fluoro-N-(indan-2-yl)-benzamide), and Sanofi-Aventis compounds AVE3085 and AVE9488 (CA Registry NO. 916514-70-0; Schäfer et al., Journal of Thrombosis and Homeostasis 2005; Volume 3, Supplement 1: abstract number P1487);
(6) NO independent heme-independent sGC activators, including, but not limited to: BAY 58-2667 (see patent publication DE19943635) 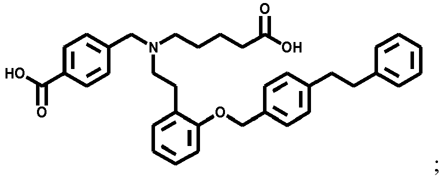 HMR-1766 (ataciguat sodium, see patent publication WO2000002851) 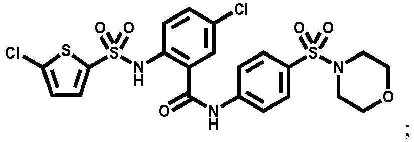 S 3448
(2-(4-chloro-phenylsulfonylamino)-4,5-dimethoxy-N-(4-(thiomorpholine-4-sulfonyl)-phenyl)-benzam ide (see patent publications DE19830430 and WO2000002851) 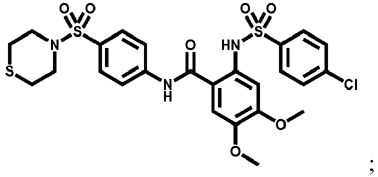 and HMR-1069 (Sanofi-Aventis).
(7) Heme-dependent sGC stimulators including, but not limited to:
YC-1 (see patent publications EP667345 and DE19744026) 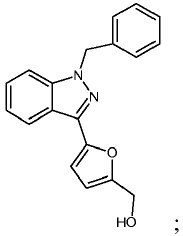
Riociguat (BAY 63-2521, Adempas, commercial product, described in DE19834044) 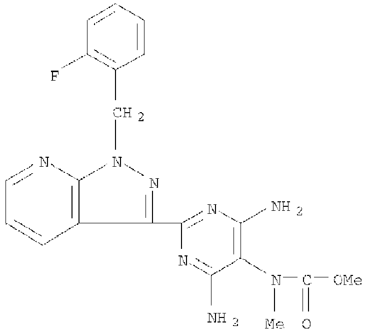
Neliciguat (BAY 60-4552, described in WO 2003095451) 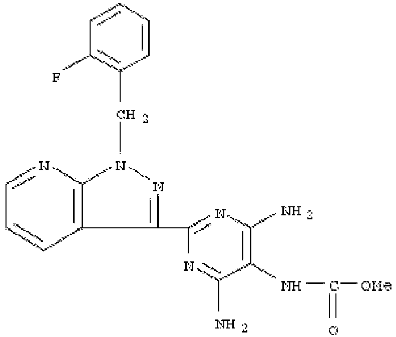
Vericiguat (BAY 1021189, clinical backup to Riociguat),
BAY 41-2272 (described in DE19834047 and DE19942809) 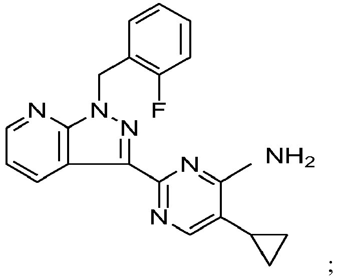
BAY 41-8543 (described in DE19834044) 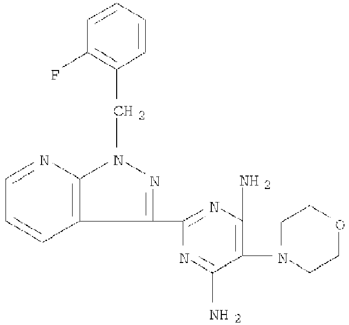
Etriciguat (described in WO 2003086407) 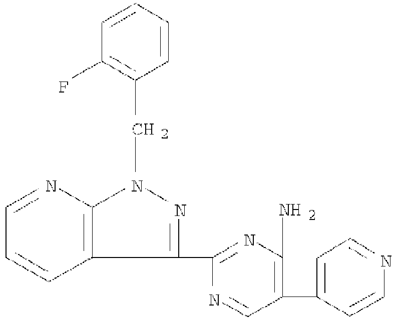
CFM-1571 (see patent publication WO2000027394) 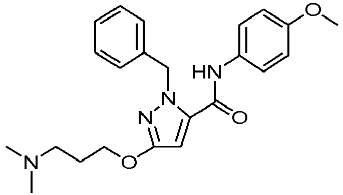
A-344905, its acrylamide analogue A-350619 and the aminopyrimidine analogue A-778935. 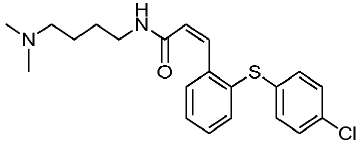
A350-619; 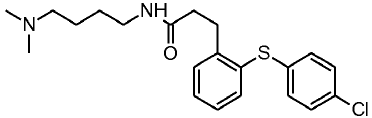
A-344905; 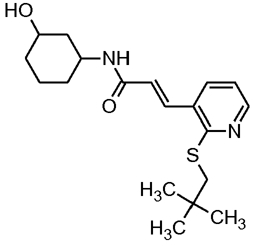
A-778935;
Compounds disclosed in one of publications: US20090209556, US8455638, US20110118282 (WO2009032249), US20100292192, US20110201621, US7947664, US8053455 (WO2009094242), US20100216764, US8507512, (WO2010099054) US20110218202 (WO2010065275), US20130012511 (WO2011119518), US20130072492 (WO2011149921), US20130210798 (WO2012058132)and other compounds disclosed in Tetrahedron Letters (2003), 44(48): 8661-8663.
(8) Compounds that inhibit the degradation of cGMP, such as:
PDE5 inhibitors, such as, for example, Sildenafil (Viagra®) and other related agents such as Avanafil, Lodenafil, Mirodenafil, Sildenafil citrate (Revatio®), Tadalafil (Cialis® or Adcirca®), Vardenafil (Levitra®) and Udenafil; Alprostadil; and Dipyridamole; PF-00489791
PDE9 inhibitors, such as, for example, PF-04447943;

(9) Calcium channel blockers such as:
Dihydropyridine calcium channel blockers: Amlodipine (Norvasc), Aranidipine (Sapresta), Azelnidipine (Calblock), Barnidipine (HypoCa), Benidipine (Coniel), Cilnidipine (Atelec, Cinalong, Siscard), Clevidipine (Cleviprex), Diltiazem, Efonidipine (Landel), Felodipine (Plendil), Lacidipine (Motens, Lacipil), Lercanidipine (Zanidip), Manidipine (Calslot, Madipine), Nicardipine (Cardene, Carden SR), Nifedipine (Procardia, Adalat), Nilvadipine (Nivadil), Nimodipine (Nimotop), Nisoldipine (Baymycard, Sular, Syscor), Nitrendipine (Cardif, Nitrepin, Baylotensin), Pranidipine (Acalas), Isradipine (Lomir);
Phenylalkylamine calcium channel blockers: Verapamil (Calan, Isoptin) 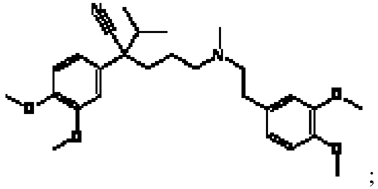
Gallopamil (Procorum, D600);
Benzothiazepines: Diltiazem (Cardizem); 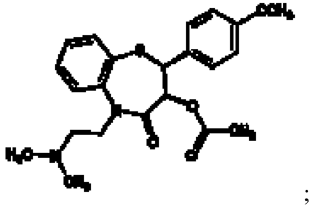
Nonselective calcium channel inhibitors such as: mibefradil, bepridil and fluspirilene, fendiline;

(10) Endothelin receptor antagonists (ERAs): for instance the dual (ET_{A} and ET_{B}) endothelin receptor antagonist Bosentan (marketed as Tracleer®); Sitaxentan, marketed under the name Thelin®; Ambrisentan is marketed as Letairis® in U.S; dual/nonselective endothelin antagonist Actelion-1, that entered clinical trials in 2008;
(11) Prostacyclin derivatives or analogues: for instance prostacyclin (prostaglandin I₂), Epoprostenol (synthetic prostacyclin, marketed as Flolan®); Treprostinil (Remodulin®), Iloprost (Ilomedin®), Iloprost (marketed as Ventavis®); oral and inhaled forms of Remodulin® that are under development; Beraprost, an oral prostanoid available in Japan and South Korea;
(12) Antihyperlipidemics such as: bile acid sequestrants (e.g., Cholestyramine, Colestipol, Colestilan and Colesevelam); statins such as Atorvastatin, Simvastatin, Lovastatin, Fluvastatin, Pitavastatin, Rosuvastatin and Pravastatin; ; cholesterol absorption inhibitors such as Ezetimibe; other lipid lowering agents such as Icosapent ethyl ester, Omega-3-acid ethyl esters, Reducol;; fibric acid derivatives such as Clofibrate, Bezafibrate, Clinofibrate, Gemfibrozil, Ronifibrate, Binifibrate, Fenofirate, Ciprofibrate, Choline fenofibrate; nicotinic acid derivatives such as Acipimox and Niacin; also combinations of statins, niacin, intestinal cholesterol absorption-inhibiting supplements (ezetimibe and others) and fibrates; antiplatelet therapies such as Clopidogrel bisulfate;
(13) Anticoagulants, such as the following types:
- Coumarines (Vitamin K antagonists): Warfarin® (Coumadin) mostly used in the US and UK; Acenocoumarol® and Phenprocoumon®, mainly used in other countries; Phenindione®;
- Heparin and derivative substances such as: Heparin; low molecular weight heparin, Fondaparinux and Idraparinux;
- Direct thrombin inhibitors such as: Argatroban, Lepirudin, Bivalirudin and Dabigatran; Ximelagatran (Exanta®), not approved in the US;
- Tissue plasminogen activators, used to dissolve clots and unblock arteries, such as Alteplase;

(14) Antiplatelet drugs: for instance thienopyridines such as Lopidogrel and Ticlopidine; Dipyridamole; Aspirin;
(15) ACE inhibitors, for example the following types:
- Sulfhydryl-containing agents such as Captopril (trade name Capoten®), the first ACE inhibitor and Zofenopril;
- Dicarboxylate-containing agents such as Enalapril (Vasotec/Renitec®); Ramipril (Altace/Tritace/Ramace/Ramiwin®); Quinapril (Accupril®), Perindopril (Coversyl/Aceon®); Lisinopril (Lisodur/Lopril/Novatec/Prinivil/Zestril®) and Benazepril (Lotensin®);
- Phosphonate-containing agents such as: Fosinopril;
- Naturally occurring ACE inhibitors such as: Casokinins and lactokinins, which are breakdown products of casein and whey that occur naturally after ingestion of milk products, especially cultured milk; The Lactotripeptides Val-Pro-Pro and Ile-Pro-Pro produced by the probiotic *Lactobacillus helveticus* or derived from casein also have ACE-inhibiting and antihypertensive functions;
- Other ACE inhibitors such as Alacepril, Delapril, Cilazapril, Imidapril, Trandolapril, Temocapril, Moexipril, Spirapril,

(16) Supplemental oxygen therapy;
(17) Beta blockers, such as the following types:
- Non-selective agents: Alprenolol®, Bucindolol®, Carteolol®, Carvedilol® (has additional α-blocking activity), Labetalol® (has additional α-blocking activity), Nadolol®, Penbutolol® (has intrinsic sympathomimetic activity), Pindolol® (has intrinsic sympathomimetic activity), Oxprenonol, Acebutolol, Sotalol, Mepindolol, Celiprolol, Arotinolol, Tertatolol, Amosulalol, Nipradilol, Propranolol® and Timolol®;
- β₁-Selective agents: Acebutolol® (has intrinsic sympathomimetic activity), Atenolol®, Betaxolol®, Bisoprolol®, Celiprolol®, Dobutamine hydrochloride, Irsogladine maleate, Carvedilol, Talinolol, Esmolol®, Metoprolol® and Nebivolol®;
- β₂-Selective agents: Butaxamine® (weak α-adrenergic agonist activity);

(18) Antiarrhythmic agents such as the following types:
- Type I (sodium channel blockers): Quinidine, Lidocaine, Phenytoin, Propafenone
- Type III (potassium channel blockers): Amiodarone, Dofetilide, Sotalol
- Type V: Adenosine, Digoxin

(19) Diuretics such as: Thiazide diuretics, e.g., Chlorothiazide, Chlorthalidone, and Hydrochlorothiazide, Bendroflumethiazide, Cyclopenthiazide, Methyclothiazide, Polythiazide , Quinethazone, Xipamide, Metolazone, Indapamide, Cicletanine; Loop diuretics, such as Furosemide and Toresamide; potassium-sparing diuretics such as Amiloride, Spironolactone, Canrenoate potassium, Eplerenone and Triamterene; combinations of these agents; other diuretics such as Acetazolamid and Carperitide
(20a) Direct-acting vasodilators such as Hydralazine hydrochloride, Diazoxide, Sodium nitroprusside, Cadralazine; other vasodilators such as Isosorbide dinitrate and Isosorbide 5-mononitrate;
(20b) Exogenous vasodilators such as:
- Adenocard®, an adenosine agonist, primarily used as an anti-arrhythmic;
- Alpha blockers (which block the vasoconstricting effect of adrenaline):
   Alpha-1-adrenoceptor antagonists such as Prazosin, Indoramin, Urapidil, Bunazosin, Terazosin, Doxazosin
- Atrial natriuretic peptide (ANP);
- Ethanol;
- Histamine-inducers, which complement proteins C3a, C4a and C5a work by triggering histamine release from mast cells and basophil granulocytes;
- Tetrahydrocannabinol (THC), major active chemical in marijuana which has minor vasodilatory effects;
- Papaverine, an alkaloid found in the opium poppy papaver somniferum;b

(21) Bronchodilators: there are two major types of bronchodilator, β₂ agonists and anticholinergics, exemplified below:
- β₂ agonists: Salbutamol® or albuterol (common brand name: Ventolin) and Terbutaline® are short acting β₂ agonists for rapid relief of COPD symptoms. Long acting β₂ agonists (LABAs) such as Salmeterol® and Formoterol®;
- anticholinergics: Ipratropium® is the most widely prescribed short acting anticholinergic drug. Tiotropium® is the most commonly prescribed long-acting anticholinergic drug in COPD;
- Theophylline®, a bronchodilator and phosphodiesterase inhibitor;

(22) Corticosteroids: such as beclomethasone, methylprednisolone, betamethasone, prednisone, prenisolone, triamcinolone, dexamethasone, fluticasone, flunisolide and hydrocortisone, and corticosteroid analogs such as budesonide
(23) Dietary supplements such as, for example: omega-3 oils; folid acid, niacin, zinc, copper, Korean red ginseng root, ginkgo, pine bark, *Tribulus terrestris,* arginine, *Avena sativa,* horny goat weed, maca root, muira puama, saw palmetto, and Swedish flower pollen; Vitamin C, Vitamin E, Vitamin K2; Testosterone supplements, Testosterone transdermal patch; Zoraxel, Naltrexone, Bremelanotide (formerly PT-141), Melanotan II, hMaxi-K; Prelox: a Proprietary mix/combination of naturally occurring ingredients, L-arginine aspartate and Pycnogenol;
(24) PGD2 receptor antagonists including, but not limited to, compounds described as having PGD2 antagonizing activity in United States Published Applications US20020022218, US20010051624, and US20030055077, PCT Published Applications WO9700853, WO9825919, WO03066046, WO03066047, WO03101961, WO03101981, WO04007451, WO0178697, WO04032848, WO03097042, WO03097598, WO03022814, WO03022813, and WO04058164, European Patent Applications EP945450 and EP944614, and those listed in: Torisu et al. 2004 Bioorg Med Chem Lett 14:4557, Torisu et al. 2004 Bioorg Med Chem Lett 2004 14:4891, and Torisu et al. 2004 Bioorg & Med Chem 2004 12:4685;
(25) Immunosuppressants such as cyclosporine (cyclosporine A, Sandimmune® Neoral®), tacrolimus (FK-506, Prograf®), rapamycin (sirolimus, Rapamune®) and other FK-506 type immunosuppressants, and mycophenolate, e.g., mycophenolate mofetil (CellCept®);
(26) Non-steroidal anti-asthmatics such as β2-agonists (e.g., terbutaline, metaproterenol, fenoterol, isoetharine, albuterol, salmeterol, bitolterol and pirbuterol) and β2-agonist-corticosteroid combinations (e.g., salmeterol-fluticasone (Advair®), formoterol-budesonid (Symbicort®)), theophylline, cromolyn, cromolyn sodium, nedocromil, atropine, ipratropium, ipratropium bromide, leukotriene biosynthesis inhibitors (zileuton, BAY1005);
(27) Non-steroidal anti-inflammatory agents (NSAIDs) such as propionic acid derivatives (e.g., alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid and tioxaprofen), acetic acid derivatives (e.g., indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin and zomepirac), fenamic acid derivatives (e.g., flufenamic acid, meclofenamic acid, mefenamic acid, niflumic acid and tolfenamic acid), biphenylcarboxylic acid derivatives (e.g., diflunisal and flufenisal), oxicams (e.g., isoxicam, piroxicam, sudoxicam and tenoxican), salicylates (e.g., acetyl salicylic acid and sulfasalazine) and the pyrazolones (e.g., apazone, bezpiperylon, feprazone, mofebutazone, oxyphenbutazone and phenylbutazone);
(28) Cyclooxygenase-2 (COX-2) inhibitors such as celecoxib (Celebrex®), rofecoxib (Vioxx®), valdecoxib, etoricoxib, parecoxib and lumiracoxib;
(opioid analgesics such as codeine, fentanyl, hydromorphone, levorphanol, meperidine, methadone, morphine, oxycodone, oxymorphone, propoxyphene, buprenorphine, butorphanol, dezocine, nalbuphine and pentazocine; and
(29) Anti-diabetic agents such as insulin and insulin mimetics, sulfonylureas (e.g., Glyburide, Glybenclamide, Glipizide, Gliclazide, Gliquidone, Glimepiride, Meglinatide, Tolbutamide, Chlorpropamide, Acetohexamide, Tolazamide), biguanides, e.g., metformin (Glucophage®), α-glucosidase inhibitors (such as Acarbose, Epalrestat, Voglibose, Miglitol), thiazolidinone compounds, e.g., rosiglitazone (Avandia®), troglitazone (Rezulin®), ciglitazone, pioglitazone (Actos®) and englitazone; insulin sensitizers such as Pioglitazone and Rosiglitazone; Insulin secretagogues such as Repaglinide, Nateglinide and Mitiglinide; Incretin mimetics such as Exanatide and Liraglutide; Amylin analogues such as Pramlintide; glucose lowering agents such as Chromiumm picolinate (optinally combined with biotin); dipeptidyl peptidase IV inhibitors such as Sitagliptin, Vildagliptin, Saxagliptin, Alogliptin and Linagliptin; vaccines currently being developed for the treatment of diabetes; AVE-0277, Alum-GAD, BHT-3021, IBC-VS01; cytokine targeted therapies in development for the treatment of diabetes such as Anakinra, Canakinumab, Diacerein,Gevokizumab, LY-2189102, MABP-1, GIT-027; drugs in development for the treatment of diabetes:

| **Drugs in development for the treatment of diabetes** | | | |
|---|---|---|---|
| Dapagliflozin | AstraZeneca/ Bristol-Myers Squibb | SGLT-2 Inhibitors | Recommended Approval |
| Alogliptin benzoate/metformin hydrochloride | Takeda | Insulin Sensitizers/ Dipeptidyl Peptidase IV (CD26; DPP-IV; DP-IV) Inhibitors | Pre-Registered |
| Anagliptin | Kowa/ Sanwa | Dipeptidyl Peptidase IV (CD26; DPP-IV; DP-IV) Inhibitors | Pre-Registered |
| Insulin degludec | Novo Nordisk | | Pre-Registered |
| Insulin degludec/insulin aspart | Novo Nordisk | | Pre-Registered |
| Insulin human (rDNA origin) inhalation powder | MannKind | | Pre-Registered |
| Lixisenatide | Sanofi | Insulin Secretagogues/ GLP-1 Receptor Agonists | Pre-Registered |
| Recombinant human insulin | Biodel | | Pre-Registered |
| Teneligliptin | Mitsubishi Tanabe Pharma | Dipeptidyl Peptidase IV (CD26; DPP-IV; DP-IV) Inhibitors | Pre-Registered |
| AVE-0277 | Andromeda Biotech/ Teva | | Phase III |
| Albiglutide | GlaxoSmithKline | GLP-1 Receptor Agonists | Phase III |
| Aleglitazar | Roche | PPARalpha Agonists/ PPARgamma Agonists | Phase III |
| Atorvastatin calcium/glimepiride | GlaxoSmithKline | K(ATP) Channel Blockers/ Dipeptidyl Peptidase IV (CD26; DPP-IV; DP-IV) Inhibitors/ HMG-CoA Reductase Inhibitors/ TNFSF6 Expression Inhibitors | Phase III |
| BYK-324677 | Nycomed | | Phase III |
| Balaglitazone | Dr. Reddy's Laboratories | Insulin Sensitizers/ PPARgamma Partial Agonists | Phase III |
| CSG-452 | Chugai Pharmaceutical | SGLT-2 Inhibitors | Phase III |
| Canagliflozin | Johnson & Johnson/ Mitsubishi Tanabe Pharma | SGLT-2 Inhibitors | Phase III |
| Canagliflozin/metformin hydrochloride | Johnson & Johnson | SGLT-2 Inhibitors/ Insulin Sensitizers | Phase III |
| Dapagliflozin/Metformin hydrochloride | AstraZeneca/ Bristol-Myers Squibb | SGLT-2 Inhibitors/ Insulin Sensitizers | Phase III |
| Dulaglutide | Lilly | Insulin Secretagogues/ GLP-1 Receptor Agonists | Phase III |
| Empagliflozin | Boehringer Ingelheim/ Lilly | SGLT-2 Inhibitors | Phase III |
| Empagliflozin/linagliptin | Boehringer Ingelheim/ Lilly | SGLT-2 Inhibitors/ Dipeptidyl Peptidase IV (CD26; DPP-IV; DP-IV) Inhibitors | Phase III |
| Gemigliptin | LG Life Sciences | Dipeptidyl Peptidase IV (CD26; DPP-IV; DP-IV) Inhibitors | Phase III |
| Hepatic-directed vesicle insulin | Diasome Pharmaceuticals | | Phase III |
| Human isophane insulin | Wockhardt | | Phase III |
| IN-105 | Biocon | | Phase III |
| Insulin degludec/liraglutide | Novo Nordisk | Insulin Secretagogues/ GLP-1 Receptor Agonists | Phase III |
| Insulin glargine | Sanofi | | Phase III |
| Ipragliflozin L-proline | Astellas Pharma/ Kotobuki | SGLT-2 Inhibitors | Phase III |
| LY-2605541 | Lilly | | Phase III |
| LY-2963016 | Lilly | | Phase III |
| Lixisenatide/Insulin glargine | Sanofi | Insulin Secretagogues/ GLP-1 Receptor Agonists | Phase III |
| Lobeglitazone sulfate | Chong Kun Dang Pharm (CKD Pharm) | PPARalpha Agonists/ PPARgamma Agonists/ Insulin Sensitizers | Phase III |
| Luseogliflozin | Taisho | SGLT-2 Inhibitors | Phase III |
| Otelixizumab | Tolerx | Anti-CD3 | Phase III |
| Ranolazine | Gilead | Sodium Channel Blockers | Phase III |
| Recombinant human insulin | National Institute of Health Sciences | | Phase III |
| Sitagliptin phosphate monohydrate/pioglitazone hydrochloride | Merck & Co. | PPARgamma Agonists/ Insulin Sensitizers/ Dipeptidyl Peptidase IV (CD26; DPP-IV; DP-IV) Inhibitors | Phase III |
| Sitagliptin/atorvastatin calcium | Merck & Co. | Dipeptidyl Peptidase IV (CD26; DPP-IV; DP-IV) Inhibitors/ HMG-CoA Reductase Inhibitors/ TNFSF6 Expression Inhibitors | Phase III |
| TAK-875 | Takeda | Free Fatty Acid Receptor 1 (FFAR1; GPR40) Agonists/ Insulin Secretagogues | Phase III |
| TT-401 | 7TM Pharma | Cannabinoid CB1 Antagonists | Phase I |
| TT-401 | Transition Therapeutics | | Phase I |
| ZYH-2 | Cadila Healthcare (d/b/a Zydus Cadila) | PPARalpha Ligands/ PPARgamma Ligands | Phase I |
| ZYO-1 | Cadila Healthcare (d/b/a Zydus Cadila) | Cannabinoid CB1 Antagonists | Phase I |
| 701645 | Cellonis Biotechnologies | | Phase I |
| 701499 | Cellonis Biotechnologies | | Phase I |
| 743300 | University of California, San Francisco | | Phase I |
| 448661 | University of Pittsburgh | | Phase I |
| AD-1 | National Institute Pharma Res Dev | | Clinical |
| Colesevelam hydrochloride | Daiichi Sankyo | Bile Acid Sequestrants | Clinical |
| DBPR-108 | National Health Research Institutes/ ScinoPharm | | IND Filed |
| Nodlin | Biolaxy | | IND Filed |
| PSN-491 | Prosidion | Glucose-Dependent Insulinotropic Receptor (GDIR, GPR119) Agonists/ Dipeptidyl Peptidase IV (CD26; DPP-IV; DP-IV) Inhibitors | IND Filed |
| Tolimidone | Melior Discovery | Lyn Kinase Activators | IND Filed |
| ZYD-1 | Cadila Healthcare (d/b/a Zydus Cadila) | GLP-1 Receptor Agonists | IND Filed |
| ZYOG-1 | Cadila Healthcare (d/b/a Zydus Cadila) | GLP-1 Receptor Agonists | IND Filed |

(30) HDL cholesterol-increasing agents such as Anacetrapib, MK-524A, CER-001, DRL-17822, Dalcetrapib, JTT-302, RVX-000222, TA-8995;
(31) Antiobesity drugs such as Methamphetamine hydrochloride, Amfepramone hydrochloride (Tenuate ®), Phentermine (Ionamin ®), Benzfetamine hydrochloride (Didrex ®), Phendimetrazine tartrate (Bontril®, Prelu-2 ®, Plegine ®), Mazindol (Sanorex ®), Orlistat (Xenical ®), Sibutramine hydrochloride monohydrate (Meridia ®, Reductil ®), Rimonabant (Acomplia ®), Amfepramone, Chromium picolinate, RM-493, TZP-301; combination such as Phentermine/Topiramate, Bupropion/Naltrexone, Sibutramine/Metformin, Bupropion SR/Zonisamide SR, Salmeterol, xinafoate/fluticasone propionate; Lorcaserin hydrochloride, Phentermine/topiramate, Bupropion/naltrexone, Cetilistat, Exenatide, KI-0803, Liraglutide, Metformin hydrochloride, Sibutramine/Metformin, 876167, ALS-L-1023, Bupropion SR/Zonisamide SR, CORT-108297, Canagliflozin, Chromium picolinate, GSK-1521498, LY-377604, Metreleptin, Obinepitide, P-57AS3, PSN-821, Salmeterol xinafoate/fluticasone propionate, Sodium tungstate, Somatropin (recombinant), TM-30339, TTP-435, Tesamorelin, Tesofensine, Velneperit, Zonisamide, BMS-830216, ALB-127158, AP-1030, ATHX-105, AZD-2820, AZD-8329, Beloranib hemioxalate, CP-404, HPP-404, ISIS-FGFR4Rx, Insulinotropin, KD-3010PF, 05212389, PP-1420, PSN-842, Peptide YY3-36, Resveratrol, S-234462; S-234462, Sobetirome, TM-38837, Tetrahydrocannabivarin, ZYO-1, beta-Lapachone;
(32) Angiotensin receptor blockers such as Losartan, Valsartan, Candesartan cilexetil, Eprosaran, Irbesartan, Telmisartan, Olmesartran medoxomil, Azilsartan medoxomil;
(33) Renin inhibitors such as Aliskiren hemifumirate;
(34) Centrally acting alpha-2-adrenoceptor agonists such as Methyldopa, Clonidine, Guanfacine;
(35) Adrenergic neuron blockers such as Guanethidine, Guanadrel;
(36) Imidazoline I-1 receptor agonists such as Rimenidine dihydrogen phosphate and Moxonidine hydrochloride hydrate;
(37) Aldosterone antagonists such as Spironolactone and Eplerenone
(38) Potassium channel activators such as Pinacidil
(39) Dopamine D1 agonists such as Fenoldopam mesilate; Other dopamine agonists such as Ibopamine, Dopexamine and Docarpamine;
(40) 5-HT2 antagonists such as Ketanserin;
(41) Drugs that are currently being developed for the treatment of arterial hypertension:

| **Drugs in development for the treatment of hypertension** | | | |
|---|---|---|---|
| Azilsartan | Takeda | Angiotensin AT1 Antagonists/ Angiotensin AT2 Antagonists/ Insulin Sensitizers | Registered |
| Amlodipine besylate/irbesartan | Dainippon Sumitomo Pharma | Angiotensin AT1 Antagonists/ Calcium Channel Blockers | Pre-Registered |
| Azilsartan/amlodipine besilate | Takeda | Angiotensin AT1 Antagonists/ Insulin Sensitizers/ Calcium Channel Blockers | Phase III |
| Cilnidipine/valsartan | Ajinomoto/ Mochida | Angiotensin AT1 Antagonists/ Calcium Channel Blockers | Phase III |
| Fimasartan | Boryung | Angiotensin AT1 Antagonists | Phase III |
| Irbesartan/atorvastatin | Hanmi | Angiotensin AT1 Antagonists/ Dipeptidyl Peptidase IV (CD26; DPP-IV; DP-IV) Inhibitors/ HMG-CoA Reductase Inhibitors/ TNFSF6 Expression Inhibitors | Phase III |
| Irbesartan/trichlormethiazide | Shionogi | Angiotensin AT1 Antagonists | Phase III |
| Losartan potassium/hydrochlorothiazide/amlodipine besylate | Merck & Co. | Angiotensin AT1 Antagonists/ Calcium Channel Blockers | Phase III |
| Pratosartan | Boryung | Angiotensin AT1 Antagonists | Phase III |
| ACT-280778 | Actelion | | Phase II |
| Amiloride hydrochloride/spironolactone | Hemodynamic Therapeutics | Mineralocorticoid Receptor (MR) Antagonists/ Na+/H+ Exchanger (NHE) Inhibitors/ Epithelial Sodium Channels (ENaC) Blockers/ K(V)1.5 Channel Blockers/ K(V)4.3 Channel Blockers | Phase II |
| Angiotensin vaccine/CoVaccine HT | BTG | | Phase II |
| CYT006-AngQb | Cytos Biotechnology | Anti-Angiotensin II | Phase II |
| Cholecalciferol | Emory University | | Phase II |
| Cobiprostone | Sucampo Pharmaceuticals | CIC-2 Channel Activators | Phase II |
| INT-001 | IntelGenx | | Phase II |
| LCZ-696 | Novartis | Angiotensin AT1 Antagonists/ Neprilysin (Enkephalinase, Neutral Endopeptidase, NEP) Inhibitors | Phase II |
| LFF-269 | Novartis | | Phase II |
| Octreotide acetate | Chiasma | Growth Hormone Release Inhibitors/ Somatostatin Agonists | Phase II |
| PL-3994 | Palatin Technologies | Atrial Natriuretic Peptide A (NPR1; Guanylate Cyclase A) Receptor Agonists | Phase II |
| Rostafuroxine | Sigma-Tau | | Phase II |
| SLx-2101 | NT Life Sciences | Phosphodiesterase V (PDE5A) Inhibitors | Phase II |
| TBC-3711 | Encysive Pharmaceuticals | Endothelin ETA Receptor Antagonists | Phase II |
| Udenafil | Dong-A/ Falk Pharma | Phosphodiesterase V (PDE5A) Inhibitors | Phase II |
| Atorvastatin calcium/losartan potassium | HanAII BioPharma | Angiotensin AT1 Antagonists/ Dipeptidyl Peptidase IV (CD26; DPP-IV; DP-IV) Inhibitors/ HMG-CoA Reductase Inhibitors/ TNFSF6 Expression Inhibitors | Phase I |
| BIA-5-1058 | BIAL | Dopamine beta-monooxygenase Inhibitors | Phase I |
| CS-3150 | Daiichi Sankyo | | Phase I |
| DSP-9599 | Dainippon Sumitomo Pharma | Renin Inhibitors | Phase I |
| MK-1597 | Actelion/ Merck & Co. | Renin Inhibitors | Phase I |
| MK-4618 | Merck & Co. | | Phase I |
| MK-5478 | Merck & Co. | | Phase I |
| MK-7145 | Merck & Co. | | Phase I |
| MK-8266 | Merck & Co. | | Phase I |
| MK-8457 | Merck & Co. | | Phase I |
| MP-157 | Mitsubishi Tanabe Pharma | Angiotensin AT2 Agonists | Phase I |
| MT-3995 | Mitsubishi Tanabe Pharma | Mineralocorticoid Receptor (MR) Antagonists | Phase I |
| Mirodenafil hydrochloride | SK Chemicals | Phosphodiesterase V (PDE5A) Inhibitors | Phase I |
| NV-04 | Novogen | Antioxidants | Phase I |
| Nifedipine/Candesartan cilexetil | Bayer | Angiotensin AT1 Antagonists/ Calcium Channel Blockers/ Antioxidants | Phase I |
| QGC-001 | Quantum Genomics | Glutamyl Aminopeptidase (Aminopeptidase A) Inhibitors | Phase I |
| RDX-5791 | Ardelyx | Na+/H+ Exchanger type 3 (NHE-3) Inhibitors | Phase I |
| TAK-272 | Takeda | Renin Inhibitors | Phase I |
| TAK-591 | Takeda | Angiotensin AT2 Antagonists | Phase I |
| VTP-27999 | Vitae Pharmaceuticals | Renin Inhibitors | Phase I |
| Vasomera | PhaseBio | VPAC2 (VIP2) Agonists | Phase I |

(42) Vasopressin antagonists such as Tolvaptan;
(43) Calcium channel sensitizers such as Levosimendan or activators such as Nicorandil;
(44) PDE-3 inhibitors such as Amrinone, Milrinone, Enoximone, Vesnarinone, Pimobendan, Olprinone;
(45) Adenylate cyclase activators such as Colforsin dapropate hydrochloride;
(46) Positive inotropic agents such as Digoxin and Metildigoxin; metabolic cardiotonic agents such as Ubidecarenone; brain naturetic peptides such as Nesiritide;
(47) Drugs that are currently in development for the treatment of heart failure:

| **Drugs in development for the treatment of heart failure** | | | |
|---|---|---|---|
| Bucindolol hydrochloride | ARCA | beta-Adrenoceptor Antagonists | Pre-Registered |
| Aliskiren hemifumarate | Novartis | Renin Inhibitors | Phase III |
| Ferric carboxymaltose | Vifor | | Phase III |
| LCZ-696 | Novartis | Angiotensin AT1 Antagonists/ Neprilysin (Enkephalinase, Neutral Endopeptidase, NEP) Inhibitors | Phase III |
| Neuregulin-1 | Zensun | | Phase III |
| Olmesartan medoxomil | Tohoku University | Angiotensin AT1 Antagonists | Phase III |
| C3BS-CQR-1 | Cardio3 BioSciences | | Phase II/III |
| MyoCell | Bioheart | | Phase II/III |
| Serelaxin | Novartis | | Phase II/III |
| AAV1/SERCA2a | AmpliPhi Biosciences/ Celladon/ Mount Sinai School of Medicine | | Phase II |
| Albiglutide | GlaxoSmithKline | GLP-1 Receptor Agonists | Phase II |
| Allogeneic mesenchymal precursor cells | Mesoblast | | Phase II |
| AlsterMACS | Miltenyi Biotec | | Phase II |
| BAY -94-8862 | Bayer | Mineralocorticoid Receptor (MR) Antagonists | Phase II |
| COR-1 | Corimmun | | Phase II |
| CXL-1020 | Cardioxyl Pharmaceuticals | Nitric Oxide Donors | Phase II |
| Cenderitide | Nile Therapeutics | Guanylate Cyclase Activators | Phase II |
| Endometrial regenerative cells | ERCell/ Medistem | | Phase II |
| JNJ-39588146 | Johnson & Johnson | | Phase II |
| Omecamtiv mecarbil | Amgen/ Cytokinetics | Cardiac Myosin Activators | Phase II |
| PL-3994 | Palatin Technologies | Atrial Natriuretic Peptide A (NPR1; Guanylate Cyclase A) Receptor Agonists | Phase II |
| Remestemcel-L | Osiris | | Phase II |
| TRV-120027 | Trevena | Angiotensin AT1 Receptor Ligands | Phase II |
| Urocortin 2 | Neurocrine Biosciences | CRF2 Agonists | Phase II |
| AAV6-CMV-SERCA2a | Imperial College | | Phase I/II |
| Anakinra | National Institutes of Health (NIH) | IL-1 Receptor Antagonists | Phase I/II |
| LipiCell | Bioheart/ Instituto de Medicina Regenerativa | | Phase I/II |
| ALD-201 | Cytomedix/ Texas Heart Institute | | Phase I |
| BAY-1021189/Vericiguat | Bayer | | Phase II |
| BAY-1067197 | Bayer | Adenine Receptor Agonists | Phase I |
| BAY -86-8050 | Bayer | Drugs Acting on Vasopressin (AVP) Receptors | Phase I |
| BIA-5-1058 | BIAL | Dopamine beta-monooxygenase Inhibitors | Phase I |
| CSCs | University of Louisville | | Phase I |
| Calcitonin gene related peptide | VasoGenix | | Phase I |
| JVS-100 | Juventas Therapeutics | | Phase I |
| MyoCell SDF-1 | Bioheart | | Phase I |
| Myoblast | Advanced Cell Technology (ACT) | | Phase I |
| RO-1160367 | Serodus | 5-HT4 Antagonists | Phase I |
| Recombinant human glial growth factor 2 | Acorda/ Vanderbilt University | | Phase I |
| [18F]LMI-1195 | Lantheus Medical Imaging | | Phase I |
| 677950 | Kyoto Prefectural University of Medicine | | Phase I |

(48) Drugs currently in development for the treatment of pulmonary hypertension:

| **Drugs in development for the treatment of pulmonary hypertension** | | | |
|---|---|---|---|
| Imatinib mesylate | Novartis | Breast Cancer-Resistant Protein (BCRP; ABCG2) Inhibitors/ Abl Kinase Inhibitors/ Angiogenesis Inhibitors/ Bcr-Abl Kinase Inhibitors/ CSF1R (c-FMS) Inhibitors/ KIT (C-KIT) Inhibitors/ Apoptosis Inducers/ PDGFRalpha Inhibitors/ PDGFRbeta Inhibitors/ Inhibitors of Signal Transduction Pathways | Pre-Registered |
| Treprostinil diethanolamine | United Therapeutics | Prostacyclin Analogs | Pre-Registered |
| GSK-1325760A | GlaxoSmithKline | | Phase III |
| Macitentan | Actelion | Endothelin ETA Receptor Antagonists/ Endothelin ETB Receptor Antagonists | Phase III |
| Riociguat/Adempas | Bayer | Guanylate Cyclase Activators | Approved 2013 |
| Selexipag | Actelion/ Nippon Shinyaku | Prostanoid IP Agonists | Phase III |
| Udenafil | Dong-A | Phosphodiesterase V (PDE5A) Inhibitors | Phase III |
| L-Citrulline | Nat Heart, Lung, and Blood Institute/ Vanderbilt University | | Phase II/III |
| BQ-123 | Brigham & Women's Hospital | Endothelin ETA Receptor Antagonists | Phase II |
| Cicletanine | Gilead | | Phase II |
| Fasudil hydrochloride | Asahi Kasei | Rho Kinase Inhibitors/ Calcium Sensitizers | Phase II |
| Nilotinib hydrochloride monohydrate | Novartis | Bcr-Abl Kinase Inhibitors/ Apoptosis Inducers/ Inhibitors of Signal Transduction Pathways | Phase II |
| PRX-08066 | Clinical Data | 5-HT2B Antagonists | Phase II |
| Terguride | ErgoNex Pharma | 5-HT2A Antagonists/ 5-HT2B Antagonists/ Dopamine Autoreceptor Agonists/ Dopamine D2 Receptor Partial Agonists/ Prolactin Secretion Inhibitors | Phase II |
| Tezosentan disodium | Actelion | Endothelin ETA Receptor Antagonists/ Endothelin ETB Receptor Antagonists | Phase II |
| Anakinra | Virginia Commonwealth University (VCU) | IL-1 Receptor Antagonists | Phase I/II |
| Simvastatin | Imperial College | HDL-Cholesterol Increasing Agents/ HMG-CoA Reductase Inhibitors | Phase I/II |
| 99mTC-PulmoBind | Montreal Heart Institute (MHI) | | Phase I |
| APD-811 | Arena | Prostanoid IP Agonists | Phase I |
| Sorafenib | Bayer | Raf kinase B Inhibitors/ Raf kinase C Inhibitors/ Angiogenesis Inhibitors/ Flt3 (FLK2/STK1) Inhibitors/ VEGFR-1 (Flt-1) Inhibitors/ KIT (C-KIT) Inhibitors/ VEGFR-2 (FLK-1/KDR) Inhibitors/ VEGFR-3 (FLT4) Inhibitors/ PDGFRbeta Inhibitors/ RET Inhibitors/ Inhibitors of Signal Transduction Pathways | Phase I |
| Triplelastat | Proteo Biotech | Elastase Inhibitors | Phase I |

(49) Drugs in current development for the treatment of female sexual dysfunction:

| **Drugs in active development for the treatment of female sexual dysfunction** | | | |
|---|---|---|---|
| Alprostadil | Apricus Biosciences/ VIVUS | | Phase III |
| Prasterone | EndoCeutics/ Monash University | HSD11B1 Expression Inhibitors | Phase III |
| Testosterone transdermal gel | BioSante | Androgen Receptor Agonists | Phase III |
| Bremelanotide | Palatin Technologies | Melanocortin MC3 Receptor Agonists/ Melanocortin MC4 Receptor Agonists | Phase II |
| Pill-Plus | Pantarhei Bioscience | | Phase II |
| Testosterone MDTS | Acrux | Androgen Receptor Agonists | Phase II |
| Estradiol/testosterone | BioSante | Estrogen Receptor (ER) Agonists/ Androgen Receptor Agonists | Phase I |
| LGD-2941 | Ligand | Selective Androgen Receptor Modulators (SARM) | Phase I |
| Lidocaine/heparin | Urigen | | Phase I |
| OnabotulinumtoxinA | Allergan | | Phase I |

(50) Drugs used for the treatment of erectile dysfunction such as Alprostadil, Aviptadil, Phentolamine mesilate, Weige, Alprostadil;
(51) Drugs currently in development for the treatment of male sexual dysfunction:

| **Drugs in active development for the treatment of erectile dysfunction** | | | |
|---|---|---|---|
| Fluvastatin sodium | Novartis | Apoptosis Inducers/ HMG-CoA Reductase Inhibitors | Phase III |
| Lodenafil carbonate | Cristalia | Phosphodiesterase V (PDE5A) Inhibitors | Phase III |
| EFLA-400 | Chonbuk National University Hospital | | Phase II/III |
| Apomorphine hydrochloride | Vectura | Dopamine D2 Agonists | Phase II |
| LY-900010 | Lilly | Phosphodiesterase V (PDE5A) Inhibitors/ Selective Androgen Receptor Modulators (SARM) | Phase II |
| Nitroglycerin | Futura Medical | | Phase II |
| RX-10100 | Rexahn | Drugs Acting on Dopaminergic Transmission/ Drugs Acting on Serotonergic Transmission | Phase II |
| YHD-1023 | Yuhan | | Phase II |
| I NT-007 | IntelGenx | | Phase I |
| LY-2452473 | Lilly | Selective Androgen Receptor Modulators (SARM) | Phase I |
| hMaxi-K | Albert Einstein College of Medicine/ Ion Channel Innovations/ Mount Sinai School of Medicine | | Phase I |
| KH-204 | KMSI | | Clinical |

(51) Drugs in development for the treatment of sleep apnea:

| **Drugs in development for the treatment of sleep apnea** | | | |
|---|---|---|---|
| CX-1739 | Cortex | AMPA Receptor Modulators | Phase II |
| Phentermine/topira mate | VIVUS | AMPA Antagonists/ Kainate Antagonists/ Sodium Channel Blockers/ Carbonic Anhydrase Type II Inhibitors | Phase II |
| AVE-0118 | Sanofi | Potassium Channel Blockers | Phase I |
| Suvorexant | Merck & Co. | Orexin Receptor Antagonists | Phase I |

(52) Drugs currently in development for the treatment of metabolic syndrome:

| **Antihyperlipidemic drugs under active development for the treatment of patients with metabolic syndrome** | | | |
|---|---|---|---|
| GFT-505 | Genfit | PPARalpha Agonists/ PPARdelta Agonists | Phase II |
| MBX-8025 | Metabolex | PPARdelta Agonists | Phase II |
| Pitavastatin calcium | Kowa | APOA1 Expression Enhancers/ HMG-CoA Reductase Inhibitors/ SPP1 (Osteopontin) Expression Inhibitors | Phase I |

(53) Antiobesity drugs:

| **Drugs marketed for the treatment of obesity** | | | |
|---|---|---|---|
| Methamphetamine hydrochloride (Desoxyn) | Abbott | Noradrenergic, alpha- and beta-adrenoceptor agonist | 1943 (U.S.) |
| Amfepramone hydrochloride (Tenuate) | Sanofi | Noradrenergic release stimulant | 1959 (U.S.) |
| Phentermine (lonamin) | UCB Celltech | Noradrenergic release stimulant | 1959 (U.S.) |
| Benzfetamine hydrochloride (Didrex) | Pfizer | Noradrenergic release stimulant | 1960 (U.S.) |
| Phendimetrazine tartrate (Bontril, Prelu-2, Plegine) | Pfizer | Noradrenergic release stimulant | 1961 (U.S.) |
| Mazindol (Sanorex) | Novartis | Noradrenergic reuptake inhibitor | 1973 (U.S.) |
| Orlistat (Xenical) | Roche | Pancreatic lipase inhibitor | 1998 (New Zealand) |

(54) Drugs used for the treatment of Alzheimer's disease: e.g., cholinesterase inhibitors prescribed for mild to moderate Alzheimer's disease, including Razadyne® (galantamine), Exelon® (rivastigmine), and Aricept® (donepezil), Cognex® (tacrine); Namenda® (memantine), an N-methyl D-aspartate (NMDA) antagonist, and Aricept®, prescribed to treat moderate to severe Alzheimer's disease; vitamin E (an anti-oxidant).
(55) Antidepressants: tricyclic antidepressants such as amitriptyline (Elavil®), desipramine (Norpramin®), imipramine (Tofranil®), amoxapine (Asendin®), nortriptyline; the selective serotonin reuptake inhibitors (SSRI's) such as paroxetine (Paxil®), fluoxetine (Prozac®), sertraline (Zoloft®), and citralopram (Celexa®); and others such as doxepin (Sinequan®) and trazodone (Desyrel®); SNRIs (e.g., venlafaxine and reboxetine); dopaminergic antidepressants (e.g., bupropion and amineptine).
(56) Neuroprotective agents: e.g., memantine, L-dopa, bromocriptine, pergolide, talipexol, pramipexol, cabergoline, neuroprotective agents currently under investigation including anti-apoptotic drugs (CEP 1347 and CTCT346), lazaroids, bioenergetics, antiglutamatergic agents and dopamine receptors. Other clinically evaluated neuroprotective agents are, e.g., the monoamine oxidase B inhibitors selegiline and rasagiline, dopamine agonists, and the complex I mitochondrial fortifier coenzyme Q10.
(57) Antipsychotic medications: e.g., ziprasidone (Geodon™), risperidone (Risperdal™), and olanzapine (Zyprexa™).
(58) NEP inhibitors such as Sacubitril, Omapatrilat.
(59) Methylene Blue (MB).

### Kits

The compounds and pharmaceutical formulations described herein may be contained in a kit. The kit may include single or multiple doses of two or more agents, each packaged or formulated individually, or single or multiple doses of two or more agents packaged or formulated in combination. Thus, one or more agents can be present in first container, and the kit can optionally include one or more agents in a second container. The container or containers are placed within a package, and the package can optionally include administration or dosage instructions. A kit can include additional components such as syringes or other means for administering the agents as well as diluents or other means for formulation. Thus, the kits can comprise: a) a pharmaceutical composition comprising a compound described herein and a pharmaceutically acceptable carrier, vehicle or diluent; and b) a container or packaging. The kits may optionally comprise instructions describing a method of using the pharmaceutical compositions in one or more of the methods described herein (e.g., preventing or treating one or more of the diseases and disorders described herein). The kit may optionally comprise a second pharmaceutical composition comprising one or more additional agents described herein for co therapy use, a pharmaceutically acceptable carrier, vehicle or diluent. The pharmaceutical composition comprising the compound described herein and the second pharmaceutical composition contained in the kit may be optionally combined in the same pharmaceutical composition.

A kit includes a container or packaging for containing the pharmaceutical compositions and may also include divided containers such as a divided bottle or a divided foil packet. The container can be, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. It is feasible that more than one container can be used together in a single package to market a single dosage form. For example, tablets may be contained in a bottle which is in turn contained within a box.

An example of a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide written memory aid containing information and/or instructions for the physician, pharmacist or subject regarding when the medication is to be taken. A "daily dose" can be a single tablet or capsule or several tablets or capsules to be taken on a given day. When the kit contains separate compositions, a daily dose of one or more compositions of the kit can consist of one tablet or capsule while a daily dose of another or more compositions of the kit can consist of several tablets or capsules. A kit can take the form of a dispenser designed to dispense the daily doses one at a time in the order of their intended use. The dispenser can be equipped with a memory-aid, so as to further facilitate compliance with the regimen. An example of such a memory-aid is a mechanical counter which indicates the number of daily doses that have been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

### EXAMPLES

As used herein, all abbreviations, symbols and conventions are consistent with those used in the contemporary scientific literature. See, e.g., Janet S. Dodd, ed., The ACS Style Guide: A Manual for Authors and Editors, 2nd Ed., Washington, D.C.: American Chemical Society, 1997.

### Example 1: Procedures for the synthesis of compounds of the invention

Compounds of the present invention embodied in Formula I may be synthesized by those skilled in the art of synthetic organic chemistry using a variety of synthetic routes such as those depicted in, but not restricted to, the following Schemes.

**Scheme 1** depicts the method of Argentine et al (Org. Process R&D 2009, 13, 131-143). Substituted semicarbazides of the general structure **5** can be prepared by reacting commercially available *tert*-butylcarbazate **2** with bromide **3** to afford Boc-protected hydrazide **4.** Intermediate **4** can then be reacted directly with potassium cyanate in aqueous ethanol or alternatively with trimethylsilylisocyanate in THF followed by treatment with acid to deprotect the Boc protecting group and protodesilylate the trimethylsilyl protecting group to regioselectively afford semicarbazide **5**. Compound **3**, when not commercially available, may be prepared from the corresponding alcohol by bromination with reagents such as PBr₃ in a solvent such as ether. Alkyl hydrazines such as **6** can also be prepared by one skilled in the art by reacting alkyl halide **3** with hydrazine in polar solvent and subsequent reaction with potassium cyanate to directly afford semicarbazide **5**.

Compounds comprising examples of Formula I may then be synthesized by activating carboxylic acid **7** with a variety of amide coupling reagents (such as HATU, BOP, TPI, or conversion of the acid to an acid chloride) in DMF and a base such as triethylamine and then reacting it with intermediate semicarbazide **5** to form acyl semicarbazide **8** which can be cyclized to the desired triazolone **1** by heating under acidic conditions such as camphorsulfonic acid (Argentine et al) or under basic conditions (Deng et al Tetrahedron Lett. 2005, 7993-7996) such as NaOH in EtOH.

Many compounds of the general structure **7** are commercially available but may also be synthesized using the synthetic routes described in **Scheme 2**. Uracils of the general structure **9** are commercially available or synthetically accessible using literature procedures or to those skilled in the art of organic synthesis. Chlorination of uracil **9** using a reagent such as phosphorus oxychloride in an organic solvent such as THF or dichloroethane provides the dichloro intermediate **10**.

The 4-chloro substituent of intermediate **10** is generally more reactive than the 2-chloro substituent and can be chemoselectively displaced in a SNAr reaction with diverse nucleophiles (carbon-based, substituted amino, hydroxyl-containing, etc.) or with diverse nucleophiles using metal-assisted or organometallic reagent-mediated displacement (e.g., Suzuki reactions, Buchwald aminations, Sonogashira reactions, etc.) to give the mono-chloro intermediate **11**. The 2-chloro substituent of **11** can be transposed to ester **12a** via a carbonylation reaction using a palladium catalyst and carbon monoxide in an alcoholic solvent under basic conditions (WO2008/47201, US2012/245124, WO2008/9487). Alternatively, the chloride of **11** can be displaced by cyanide under refluxing alcoholic/aqueous conditions or by using palladium-mediated cross-coupling with zinc cyanide in a polar solvent such as DMF or NMP to give nitrile **12b** (Wada et al Tetrahedron Lett. 2012, 53(14), 1720-1724). Ester **12a** can be hydrolyzed using NaOH or by dealkylative deprotection (e.g., potassium trimethylsilyloxide heated in a polar aprotic solvent) and nitrile **12b** can be deprotected by hydrolysis in aqueous NaOH at 100 °C to give intermediate carboxylic acid 7.

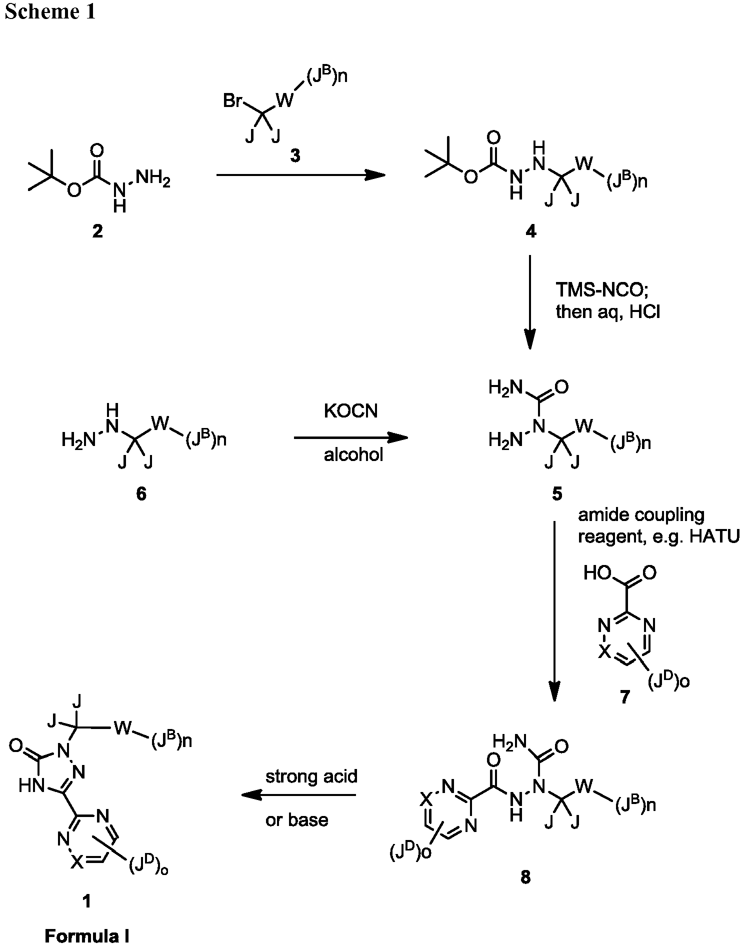

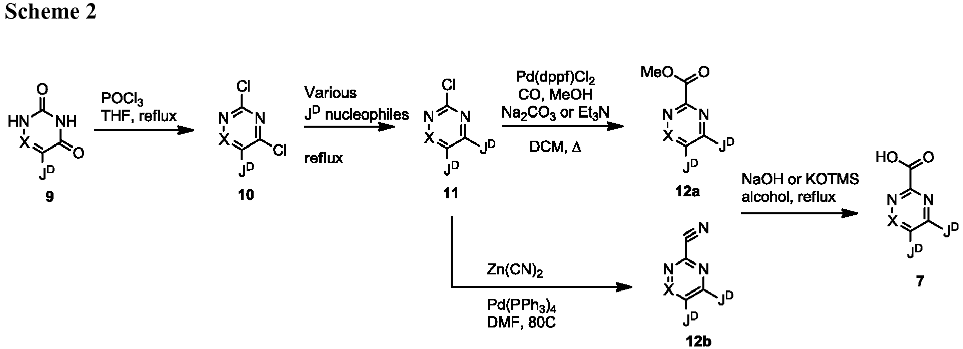

**Scheme 3** shows a complimentary method for making intermediate carboxylic acids **7** by condensation of an amidine such as commercially available methyl-2-amino-2-iminoacetate with a variety of 3-carbon electrophiles using reagents and conditions detailed in our earlier work (WO2013101830, WO2012003405) and others (WO2011149921, WO2013104703, WO2013030288, 2013004785, 2012004259) to form substituted pyrimidine carboxylates. Scheme 3, Reaction (1) shows the condensation of methyl-2-amino-2-iminoacetate with the anion of β-hydroxy ethylacrylate in refluxing ethanol to give a 2-carboxy-4-hydroxypyrimidine **7a** after ester deprotection using NaOH. This intermediate can be further derivatized by treatment with phosphorus oxychloride in DCM followed by SNAr displacement by various carbon, oxygen, and in this case an amino nucleophile HNR^{D}R^{D} to give a functionalized pyrimidine carboxylate **7b**. Scheme 3, Reactions (2) and (3) show an analogous example wherein the electrophile is a substituted malononitrile. Condensation of this reagent with methyl-2-amino-2-iminoacetate by refluxing in EtOH provides a 4,6-diaminopyrimidine **7c** that in the case of Reaction (3) can further cyclize under the reaction conditions to provide a functionalized lactam **7d** after ester hydrolysis to the carboxylic acid.

Condensation of appropriately substituted methyl 2-amino-2-hydrazonoacetate with diverse 2-carbon electrophiles followed by ester hydrolysis provides access to functionalized 3-carboxy-1,2,4-triazines (Palmer et al Bioorganic & Medicinal Chemistry 2007, 15(24), 7647-7660). Scheme 3, Reaction (4) illustrates this condensation with α-keto esters under refluxing EtOH and mild acid to give the 5-hydroxy-3-carboxy-1,2,4-triazine **7e** after ester deprotection. As in Reaction (1), intermediate **7e** can be further derivatized by treatment with phosphorus oxychloride in DCM followed by SNAr displacement by various carbon, oxygen, and in this case an amino nucleophile HNR^{D}R^{D} to give a functionalized 1,2,4-triazine carboxylate **7f**. Scheme 3, reaction (5) makes use of a diketone electrophile (Beard et al WO2008030843) to provide diverse 1,2,4-triazine-3-carboxylates of the general structure **7g** after ester hydrolysis.

**Scheme 4** illustrates a method for making alkoxytriazoles of Formula I by simply replacing the semicarbazide **5** used in Scheme 1, with thiosemicarbazide **5'** that can be prepared from **6** using potassium thiocyanate in alcoholic solvent. Coupling thiosemicarbazide **5'** with carboxylic acid **7** using a coupling reagent such as HATU provides acylthiosemicarbazide **13** that can be cyclized to the triazolethione **14** under basic or acidic conditions. Following the method of Kane et al (J. Med. Chem. 1994, 37(1), 125-132) and Courtemanche et al (WO 2002006272), triazolethione **14** reacts with methyl iodide under basic conditions such as K₂CO₃ in acetone to give S-methylation and subsequent oxidation using reagents such as MCPBA or Oxone can provide the triazole sulfone **15**. Displacement of this sulfone using an alcohol Rc-OH deprotonated with NaH in DMF and heating provides the desired alkoxytriazole **16** of Formula I.

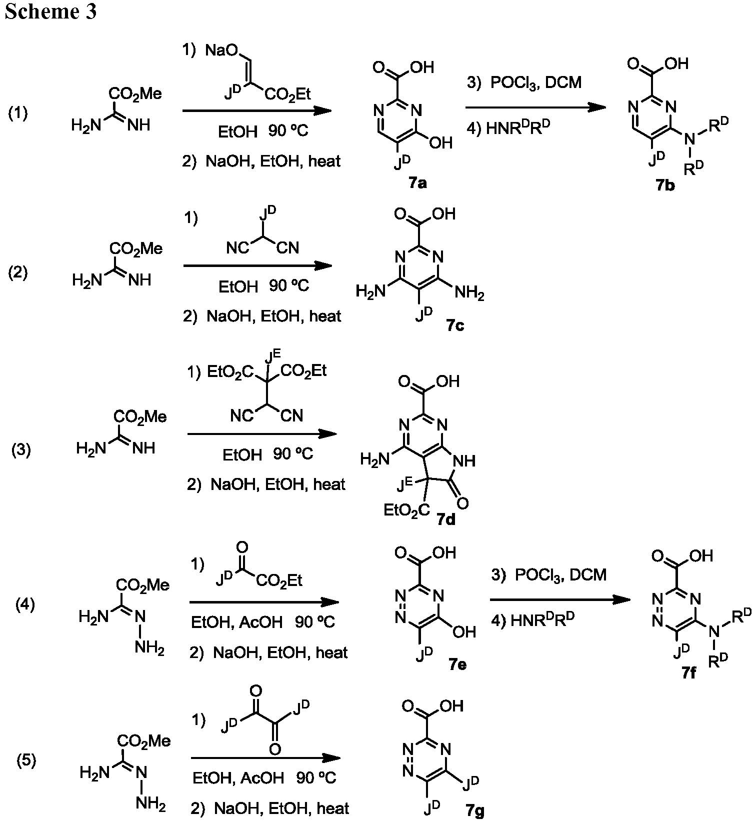

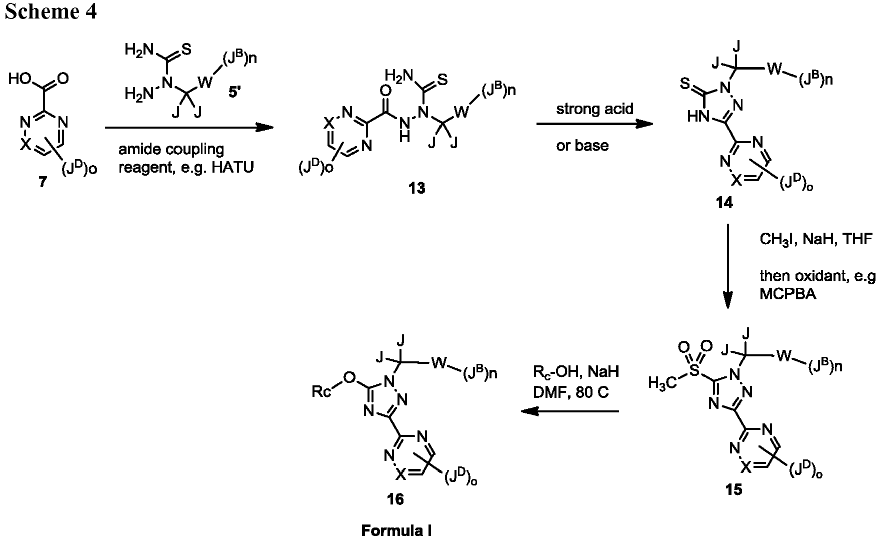

Synthesis of pyrazole variants of Formula I compounds is detailed in **Scheme 5**. The pyrazolone core structure can be synthesized by condensation of substituted hydrazine **6** with diethyl 2-oxosuccinate under acidic conditions to form hydroxypyrazole **17**. This intermediate may be O-alkylated with diverse electrophiles such as alkyl and aryl iodides and triflates under basic conditions to provide alkoxy pyrazole **18**. Treatment of **18** with ammonium chloride and trimethylaluminum gives the amidine **19** which can be condensed with 3-carbon electrophiles using the reagents, conditions, and references described above in Scheme 3, Reactions (1)-(3) to provide compounds of Formula I such as the substituted pyrimidines **20**. Amidine **19** can be treated with hydrazine in refluxing alcohol to form the 2-aminohydrazone 21 that can be condensed with 2-carbon electrophiles using the reagents, conditions, and references described above in Scheme 3, Reactions (4)-(5) to provide compounds of Formula I such as the substituted triazines **22**.

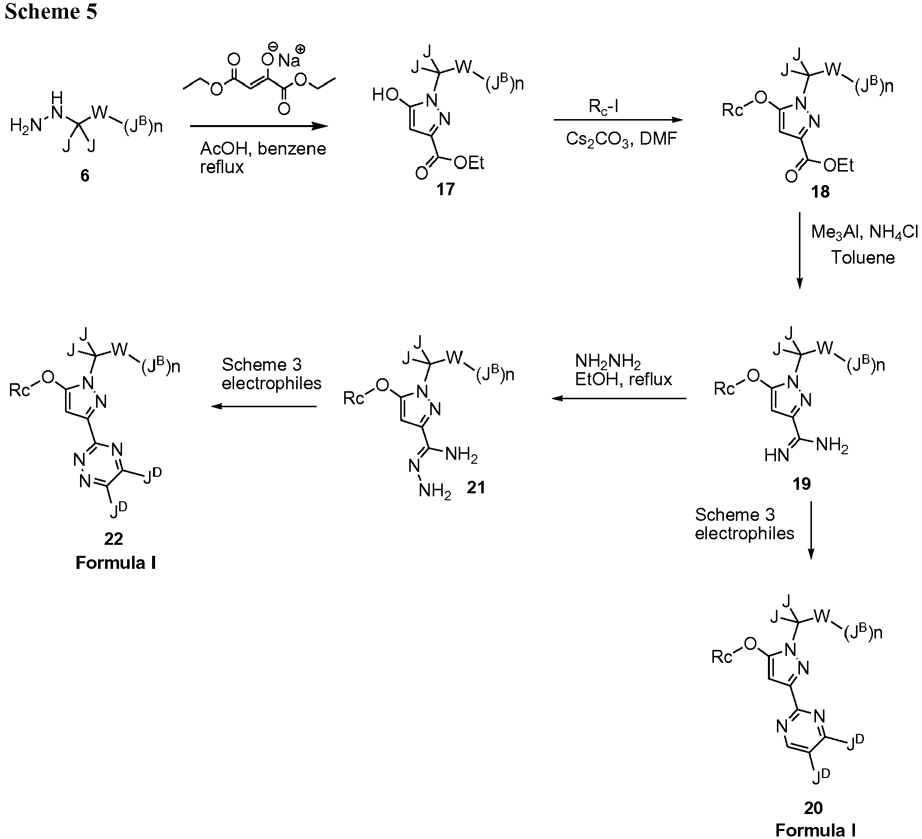

### General Procedure A

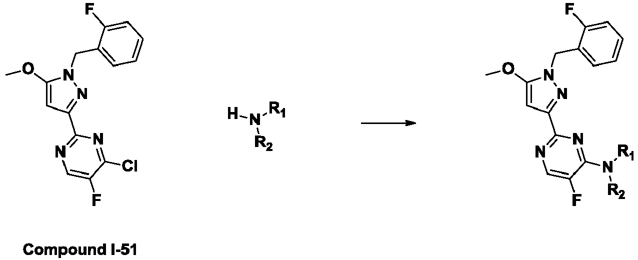

A solution of amino nucleophile (3 equiv.), triethylamine (10 equiv.), and **Compound I-51** (1 equiv.) was stirred in dioxane and water (3:1 ratio) at 90 °C until complete consumption of starting material was observed by LC/MS. The solution was diluted with aqueous IN hydrochloric acid and ethyl acetate. The layers were then separated and the aqueous layer was extracted with ethyl acetate. The organics were combined, dried over magnesium sulfate, filtered, and the solvent was removed in vacuo. Purification yielded the desired product.

### Compound I-1

The title compound was synthesized in 4 steps:

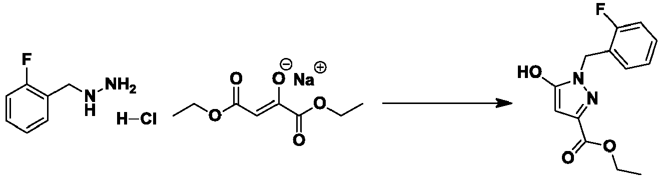

### Step 1: Synthesis of ethyl 1-(2-fluorobenzyl)-5-hydroxy-1H-pyrazole-3-carboxylate.

A mixture containing acetic acid (15 equiv.) and diethyl oxalacetate sodium salt (1 equiv.) in benzene was stirred at 25 °C for 30 min. To this mixture was added (2-fluorobenzyl)hydrazine hydrochloride (2 equiv.). The resulting mixture was heated to 100 °C for 2 h. The mixture was cooled to 23 °C and concentrated under vacuum. The precipitate formed was collected by filtration. The solid was dissolved in ethyl acetate and washed with IN HCl solution (x3). The organic layer was dried, filtered, and evaporated to give a solid containing the desired product. The solid was rinsed with a minimal amount of a methanol-diethyl ether mixture, dried under vacuum to deliver the desired intermediate, ethyl 1-(2-fluorobenzyl)-5-hydroxy--1H-pyrazole-3-carboxylate (11.8 g, 71 % yield) as a cream colored solid.
¹H NMR (500 MHz, CD₃OD) δ ppm 7.24 - 7.41 (m, 1 H), 7.09 - 7.16 (m, 2 H), 7.02 - 7.07 (m, 1 H), 5.95 (s, 1 H), 5.29 (s, 2 H), 4.33 (q, 2 H), 1.36 (t, 3 H).

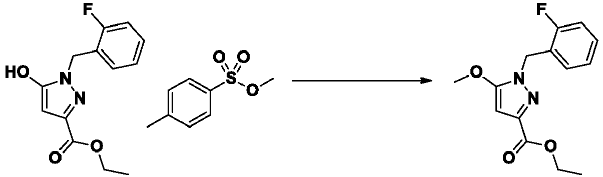

### Step 2: Synthesis of ethyl 1-(2-fluorobenzyl)-5-methoxy-1H-pyrazole-3-carboxylate.

A mixture of potassium carbonate (2.5 equiv.), methyl ester of *p*-toluenesulfonic acid (1.1 equiv.) and ethyl 1-(2-fluorobenzyl)-5-hydroxy-1H-pyrazole-3-carboxylate (1 equiv.) in DMF was stirred at 23 °C for 24 h. The mixture was diluted with ethyl acetate and washed with water. The organic layer was dried, filtered and evaporated to give an oil. The oil was purified by silica gel chromatography (0 to 100% ethyl acetate in hexanes) to deliver the desired intermediate, ethyl 1-(2-fluorobenzyl)-5-methoxy-1H-pyrazole-3-carboxylate (2.8 g, 88 % yield) as a clear oil.
¹H NMR (500 MHz, CDCl₃) δ ppm 7.22 - 7.29 (m, 1 H), 7.03 - 7.10 (m, 2 H), 6.98 (td, 1 H), 6.12 (s, 1 H), 5.33 (s, 2 H), 4.36 - 4.45 (m, 2 H), 3.88 - 3.92 (m, 3 H), 1.36 - 1.44 (m, 3 H).

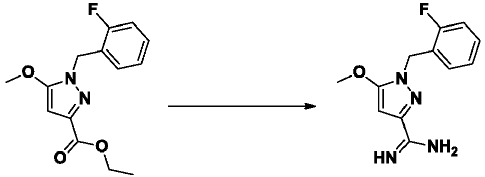

### Step 3: Synthesis of 1-(2-fluorobenzyl)-5-methoxy-1H-pyrazole-3-carboximidamide.

To a cold suspension of ammonium chloride (5.3 equiv.) in toluene at 0 °C was added, slowly, a 2.0 M solution of triemthylaluminum in toluene (5.3 equiv.). The mixture was removed from the ice bath and stirred at 23 °C until the bubbling ceased. To this mixture was added a solution of ethyl 1-(2-fluorobenzyl)-5-methoxy-1H-pyrazole-3-carboxylate (1 equiv.) in toluene. The mixture was heated to 90 °C for 24 h. The mixture was cooled to 23 °C and quenched with methanol (5.3 equiv.).

The white precipitate was removed by filtration. The filtrate was washed with water, dried, and concentrated under vacuum to deliver the desired intermediate, 1-(2-fluorobenzyl)-5-methoxy-1H-pyrazole-3-carboximidamide (110 mg, 5% yield) as a white solid.
¹H NMR (500 MHz, CD₃OD) δ ppm 7.25 - 7.35 (m, 1 H), 7.02 - 7.24 (m, 3 H), 6.17 - 6.22 (m, 1 H), 5.22 - 5.28 (m, 2 H), 4.92 (s, 3 H)

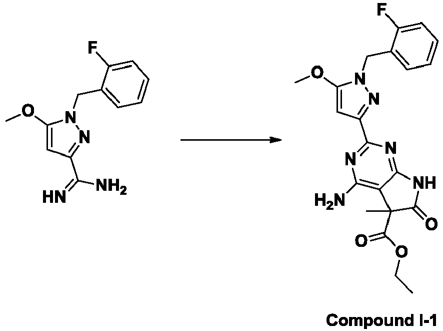

### Step 4: Synthesis of Compound I-1

A mixture containing diethyl 2-(dicyanomethyl)-2-methylmalonate (1.5 equiv.), potassium hydrogencarbonate (2 equiv.) and 1-(2-fluorobenzyl)-5-methoxy-1H-pyrazole-3--carboximidamide (1 equiv.) in *tert*-butanol was heated to 85 °C for 1 h. The mixture was diluted in ethyl acetate and washed with water. The organic layer was dried, filtered and evaporated to give an oil that was purified via silica gel chromatography (0 to 100% ethyl acetate in hexanes) to deliver the desired compound (125 mg, 53 % yield) as a white solid.
¹H NMR (500 MHz, DMSO-*d*₆) δ ppm 7.31 - 7.39 (m, 1 H), 7.19 - 7.24 (m, 1 H), 7.17 (td, 1 H), 7.07 (td, 1 H), 6.17 (s, 1 H), 5.22 (s, 2 H), 4.05 - 4.15 (m, 2 H), 3.93 (s, 3 H), 1.57 (s, 3 H), 1.10 (t, 3 H).

### Compound I-2

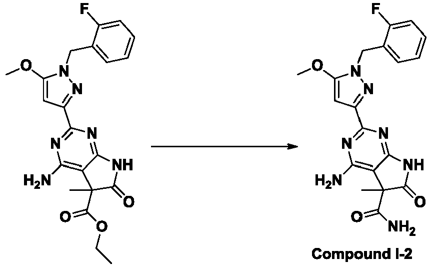

In a sealed vial, a mixture of 7N solution of ammonia in methanol (200 equiv.) and ethyl 4-amino-2-(1-(2-fluorobenzyl)-5-methoxy-1H-pyrazol-3-yl)-5-methyl-6-oxo-6,7-dihydro-5H-pyrrolo[ 2,3-d]pyrimidine-5-carboxylate (1 equiv.) was heated to 50 °C for 24 h. The mixture was cooled to 23 °C and concentrated under vacuum to give a solid. The residue was purified via silica gel chromatography (0 to 100% ethyl acetate in hexanes) to deliver the desired compound (30.1 mg, 43% yield) as a white solid.
¹H NMR (500 MHz, CD₃OD) δ ppm 7.27 - 7.34 (m, 1 H), 7.08 - 7.15 (m, 2 H), 6.98 - 7.06 (m, 1 H), 6.34 (s, 1 H), 5.31 (s, 2 H), 3.97 (s, 3 H), 1.70 (s, 3 H).

### Compound I-6

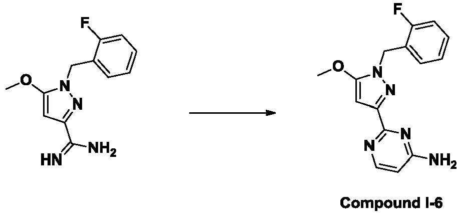

A solution of 3-methoxyacrylonitrile (3 equiv.), 1-(2-fluorobenzyl)-5-methoxy--1H-pyrazole-3-carboximidamide (described in step 3 towards the synthesis of **Compound I-1**) (1 equiv.), and DBU (1 equiv.) in pyridine was heated at 100 °C for 39 h. The solvent was removed in vacuo. Purification via silica gel chromatography (0-10% methanol in dichloromethane) delivered the desired compound (5 mg, 7% yield) as a brown film.
¹H-NMR (500 MHz, CD₃OD) δ 8.09 (d, 1 H), 7.32-7.28 (m, 1 H), 7.12-7.08 (m, 2 H), 7.06-7.03 (m, 1 H), 6.44 (d, 1 H), 6.32 (s, 1 H), 5.31 (s, 2 H), 3.97 (s, 3 H).

### Compound I-5

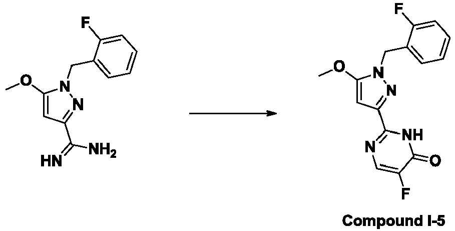

A solution of sodium 3-ethoxy-2-fluoro-3-oxoprop-1-en-1-olate (3 equiv.) and 1-(2-fluorobenzyl)-5-methoxy-1H-pyrazole-3-carboximidamide (described in step 3 towards the synthesis of **Compound I-1**) (1 equiv.) in ethanol was stirred at 90 °C for 2 h. The solvent was removed in vacuo and resulting residue was brought up in dichloromethane and aqueous 1N hydrochloric acid solution. The layers were separated and the aqueous layer was extracted with dichloromethane. The organics were dried over magnesium sulfate, filtered, and the solvent was removed in vacuo. Purification via silica gel chromatography (0-5% methanol in dichloromethane) delivered the desired compound (13 mg, 20% yield) as a white solid.
¹H-NMR (500 MHz, CDCl₃) δ 10.39 (br s, 1 H), 7.89 (d, 1 H), 7.34-7.29 (m, 1 H), 7.14-7.07 (m, 3 H), 6.21 (s, 1 H), 5.23 (s, 2 H), 3.97 (s, 3 H).

### Compound I-48

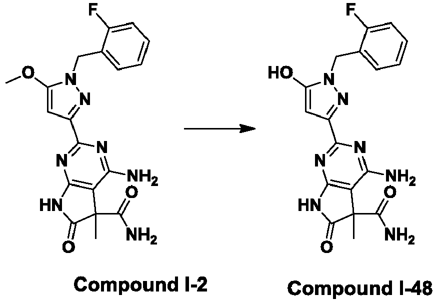

A mixture containing a solution of hydrogen bromide [33wt% in acetic acid] (25 equiv.) in acetic acid and **Compound I-2** (1 equiv.) was heated to 90 °C for 2 h. The mixture was cooled to 23 °C and diluted in ethyl acetate. The organic layer was washed with saturated solution of sodium bicarbonate, dried over MgSO₄, filtered and evaporated to give a crude solid. The crude solid was purified via silica gel chromatography utilizing a 0-100% ethyl acetate/hexanes gradient to deliver the desired compound (15 mg, 18% yield) as a light yellow solid.
¹H NMR (500 MHz, DMSO-*d*₆) δ ppm 7.30 - 7.38 (m, 1 H), 7.19 - 7.25 (m, 1 H), 7.13 - 7.18 (m, 1 H), 7.02 - 7.09 (m, 1 H), 5.91 (s, 1 H), 5.18 (s, 2 H), 1.52 (s, 3 H).

### Compound I-4

The title compound was synthesized in 3 steps:

### Step 1: Synthesis of ethyl 5-(difluoromethoxy)-1-(2-fluorobenzyl)-1H-pyrazole-3-carboxylate.

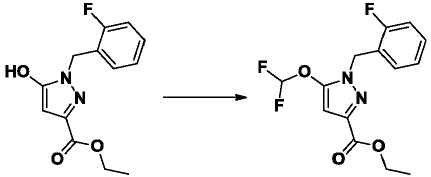

In a flask equipped with a reflux condenser and under argon, a mixture of ethyl 1-(2-fluorobenzyl)-5-hydroxy-1H-pyrazole-3-carboxylate (described in step 1 towards the synthesis of **Compound I-1**) (1 equiv.), potassium carbonate (1.1 equiv.), and sodium 2-chloro-2,2-difluoroacetate (1.4 equiv.) in DMF was heated to 100 °C for 1.5 h. During the course of the reaction, gas evolution was observed. The mixture was cooled to 23 °C and diluted with ethyl acetate and water. The organic layer was washed with water, dried, filtered and evaporated to give a crude oil. The oil was purified via silica gel chromatography (0 to 100% ethyl acetate in hexanes) to deliver the desired intermediate, ethyl 5-(difluoromethoxy)-1-(2-fluorobenzyl)-1H-pyrazole-3-carboxylate (3.5 g, 88 % yield) as a clear oil.
¹H NMR (500 MHz, CDCl₃) δ ppm 7.26 - 7.32 (m, 2 H), 7.04 - 7.13 (m, 3 H), 6.46 - 6.48 (m, 1 H), 5.39 (s, 2 H), 4.41 (q, 2 H), 1.40 (t, 3 H).

### Step 2: Synthesis of 5-(difluoromethoxy)-1-(2-fluorobenzyl)-1H-pyrazole-3-carboximidamide.

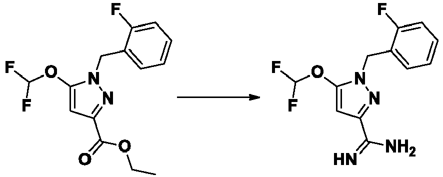

To a cold suspension of ammonium chloride (5.3 equiv.) in toluene at 0 °C was added, slowly, 2.0M solution of trimethylaluminum in toluene (5.3 equiv.). Once the addition was complete, the mixture was allowed to warm to 23 °C and stirred at this temperature until the bubbling ceased. To this mixture was added a solution of ethyl 5-(difluoromethoxy)-1-(2-fluorobenzyl)-1H-pyrazole-3-carboxylate (1 equiv.) in toluene. The mixture was heated to 90 °C for 24 h. The mixture was cooled to 0 °C and quenched with methanol (5.3 equiv). The white precipitate was collected by filtration and dried under vacuum. The desired product was washed away from the white solid with methanol (100 ml). The solid was removed by filtration. The filtrate was concentrated under vacuum to deliver the desired intermediate, 5-(difluoromethoxy)-1-(2-fluorobenzyl)-1H-pyrazole-3-carboximidamide (3.2 g, 100 % yield) as a white solid.
¹H NMR (500 MHz, CD₃OD) δ ppm 7.36 - 7.43 (m, 1 H), 7.28 (t, 1 H), 7.11 - 7.22 (m, 3 H), 6.80 (s, 1 H), 5.47 (s, 2 H).

### Step 3: Synthesis of Compound I-4

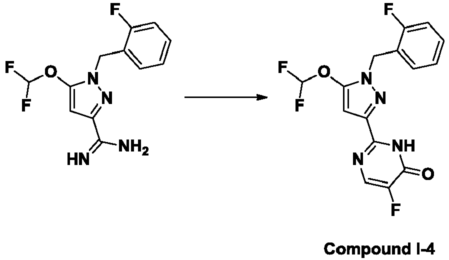

A mixture containing potassium hydrogencarbonate (1 equiv.), DBU (1 equiv.), 5-(difluoromethoxy)-1-(2-fluorobenzyl)-1H-pyrazole-3-carboximidamide (1 equiv.) and sodium (*E*)-3-ethoxy-2-fluoro-3-oxoprop-1-en-1-olate (3 equiv.) in *t*-BuOH was heated at 85 °C for 24 h. The mixture was diluted in ethyl acetate and washed with water. The organic layer was dried, filtered and evaporated to give a crude oil. The oil was purified via silica gel chromatography (0 to 100% ethyl acetate in hexanes) to deliver the desired compound (116 mg, 23% yield) as a white solid.
¹H NMR (500 MHz, DMSO-*d*₆) δ ppm 8.11 (br. s., 1 H), 7.37 - 7.55 (m, 2 H), 7.31 (d, 1 H), 7.18 - 7.27 (m, 2 H), 6.68 (br. s., 1 H), 5.37 (s, 2 H).

### Compound I-49

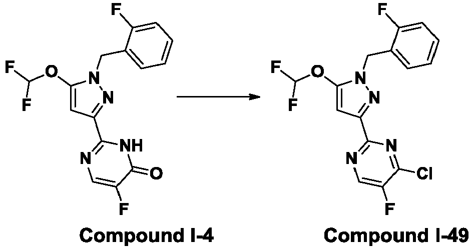

A mixture containing phosphorus oxychloride (100 equiv.) and **Compound I-4** (1 equiv.) was stirred at 23 °C for 24 h. The mixture was concentrated under vacuum to deliver the desired intermediate, 4-chloro-2-(5-(difluoromethoxy)-1-(2-fluorobenzyl)-1H-pyrazol-3-yl)-5-fluoropyrimidine (183 mg, quantitative yield) as a yellow oil.
¹H NMR (500 MHz, CDCl₃) δ ppm 8.72 (s, 1 H), 7.31 - 7.38 (m, 2 H), 7.12 - 7.17 (m, 1 H), 7.08 (t, 1 H), 6.82 (s, 1 H), 6.52 - 6.81 (m, 1 H), 5.53 (s, 2 H).

### Compound I-42

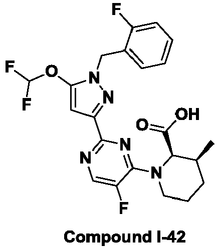

A mixture containing *cis*-3-methylpiperidine-2-carboxylic acid (3 equiv.), triethylamine (3 equiv.) and **Compound I-49** (1 equiv.) in a 3:1 mixture of 1,4-dioxane and water was heated at 70 °C for 24 h. The mixture was diluted in ethyl acetate. The organic layer was washed with 1N HCl solution, dried, filtered and evaporated to give a solid. The solid was purified by silica gel chromatography (0 to 10% methanol in DCM) to deliver the desired compound (11 mg, 16% yield) as a light yellow solid.
¹H NMR (500 MHz, CD₃OD) δ ppm 8.16 - 8.21 (m, 1 H), 7.30 - 7.38 (m, 1 H), 7.11 - 7.20 (m, 3 H), 6.94 - 7.11 (m, 1 H), 6.65 (s, 1 H), 5.40 (s, 2 H), 5.01 (d, 1 H), 4.34 (d, 1 H), 3.61 - 3.73 (m, 1 H), 2.04 - 2.13 (m, 1 H), 1.84 - 1.92 (m, 1 H), 1.68 - 1.80 (m, 2 H), 1.50 (qd, 1 H), 1.19 (d, 3 H).

### Compound I-14

The title compound was synthesized in 4 steps:

### Step 1: Synthesis of ethyl 5-hydroxy-1-(3,3,3-trifluoropropyl)-1H-pyrazole-3-carboxylate.

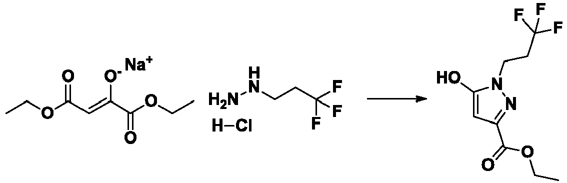

A mixture of acetic acid (1 equiv.), sodium diethyl oxalacetate (1 equiv.) and (3,3,3-trifluoropropyl)hydrazine hydrochloride (1 equiv.) in benzene was heated to 60 °C for 3 h. The mixture was diluted in ethyl acetate and washed with 1N HCl solution. The organic layer was dried, filtered and evaporated to give a crude oil. The oil was purified via silica gel chromatography (0 to 100% ethyl acetate in hexanes) to deliver the desired intermediate, ethyl 5-hydroxy-1-(3,3,3-trifluoropropyl)-1H-pyrazole-3-carboxylate (1.0 g, 17% yield) as a light yellow solid.
¹H NMR (500 MHz, CD₃OD) δ ppm 5.86 (s, 1 H), 4.32 (q, 2 H), 4.26 (t, 2 H), 2.66 - 2.80 (m, 2 H), 1.33 - 1.38 (m, 3 H).

### Step 2: Synthesis of ethyl 5-methoxy-1-(3,3,3-trifluoropropyl)-1H-pyrazole-3-carboxylate.

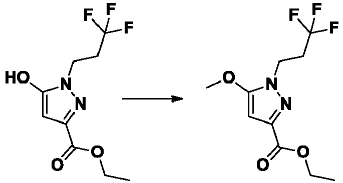

A mixture containing methyl *p*-toluenesulfonate (1 equiv.), potassium carbonate (1.4 equiv.) and ethyl 5-hydroxy-1-(3,3,3-trifluoropropyl)-1H-pyrazole-3-carboxylate (1 equiv.) in DMF was stirred at 23 °C for 24 h. The mixture was diluted in ethyl acetate and washed with water. The organic layer was dried, filtered and evaporated to give a crude oil. The oil was purified via silica gel chromatography (0 to 30% ethyl acetate in hexanes) to deliver the desired intermediate, ethyl 5-methoxy-1-(3,3,3-trifluoropropyl)-1H-pyrazole-3-carboxylate (612 mg, 58% yield) as a clear oil.
¹H NMR (500 MHz, CDCl₃) δ ppm 6.07 (s, 1 H), 4.40 (q, 2 H), 4.25 - 4.33 (m, 2 H), 3.94 (s, 3 H), 2.62 - 2.73 (m, 2 H), 1.40 (t, 3 H).

### Step 3: Synthesis of 5-methoxy-1-(3,3,3-trifluoropropyl)-1H-pyrazole-3-carboximidamide.

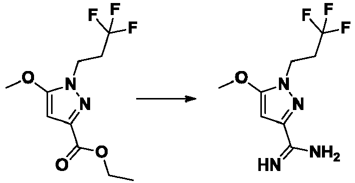

To a cold suspension of ammonium chloride (1 equiv.) in toluene at 0 °C, was added a 2M solution of trimethylaluminum in heptane (5.3 equiv.). The mixture was removed from the ice bath and stirred at 23 °C until the gas evolution ceased. To this mixture was added a solution of ethyl 5-methoxy-1-(3,3,3-trifluoropropyl)-1H-pyrazole-3-carboxylate (1 equiv.) in toluene. The mixture was heated to 100 °C for 24 h. The mixture was cooled to 0 °C and quenched with methanol (5.3 equiv.) and dichloromethane. The precipitate formed was removed by filtration. The filtrate was concentrated to deliver the desired intermediate, 5-methoxy-1-(3,3,3-trifluoropropyl)-1H-pyrazole-3-carboximidamide (172 mg, 32% yield) as a tan solid.
¹H NMR (500 MHz, DMSO-*d*₆) δ ppm 6.55 (s, 1 H), 4.27 (t, 2 H), 3.33 (s, 3 H), 2.82 - 2.95 (m, 2 H).

### Step 4: Synthesis of Compound I-14

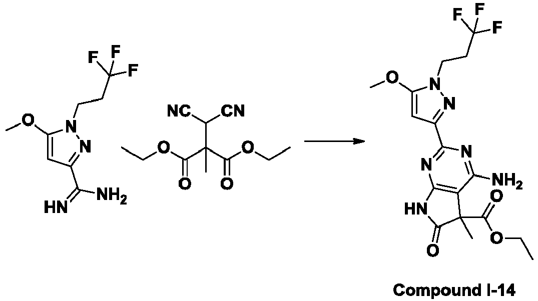

A mixture containing potassium bicarbonate (1.1 equiv.), diethyl 2-(dicyanomethyl)-2-methylmalonate (1 equiv.) and 5-methoxy-1-(3,3,3-trifluoropropyl)-1H-pyrazole-3-carboximidamide (1.1 equiv.) in *tert-BuOH* was heated at 85 °C for 1 h. The mixture was diluted in ethyl acetate and washed with water. The organic layer was dried, filtered and evaporated to give a crude oil. The oil was purified via silica gel chromatography (0 to 50% ethyl acetate in hexanes) to deliver the desired compound (54 mg, 17% yield) as a light yellow solid.
¹H NMR (500 MHz, CD₃OD) δ ppm 6.24 - 6.30 (m, 1 H), 4.24 - 4.34 (m, 2 H), 4.20 (q, 2 H), 4.04 - 4.14 (m, 2 H), 3.96 (s, 3 H), 1.68 (s, 3 H), 1.18 - 1.22 (m, 3 H).

### Compound I-7

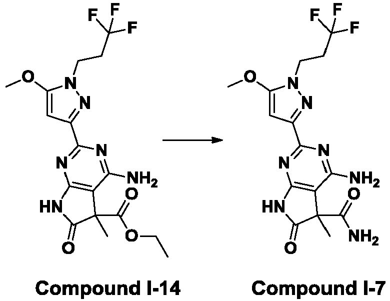

In a sealed vial, a mixture containing 7N solution of ammonia (30 equiv.) in methanol and Compound **I-14** (1 equiv.) was stirred at 23 °C for 24 h. Then, it was concentrated under vacuum. The solid obtained was rinsed with chloroform and dried under vacuum to deliver the desired compound (25 mg, 46% yield) as a white solid.
¹H NMR (500 MHz, DMSO-*d*₆) δ ppm 6.12 (s, 1 H), 4.19 (t, 2 H), 3.92 (s, 3 H), 2.72 - 2.83 (m, 2 H), 1.53 (s, 3 H).

### Compound I-44

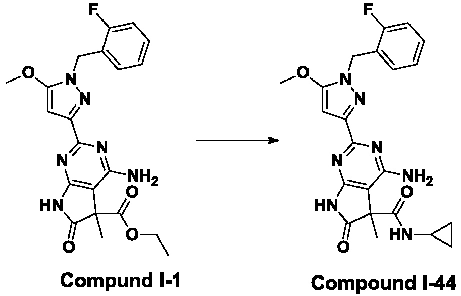

In a sealed vial, a mixture containing cyclopropylamine (100 equiv.) and **Compound I-1** (1 equiv.) was heated to 50 °C for 24 h. The mixture was concentrated under vacuum. The resulting residue was purified via silica gel chromatography (0 to 100% ethyl acetate in hexanes) to deliver the desired compound (44 mg, 52% yield) as a white solid.
¹H NMR (500 MHz, DMSO-*d*₆) δ ppm 7.35 (d, 1 H), 7.18 - 7.25 (m, 1 H), 7.16 (t, 1 H), 6.98 - 7.10 (m, 1 H), 6.16 (s, 1 H), 5.21 (s, 2 H), 3.82 - 4.03 (m, 3 H), 2.63 (dt, 1 H), 1.51 (s, 3 H), 0.51 - 0.64 (m, 2 H), 0.45 (d, 2 H).

### Compound I-35

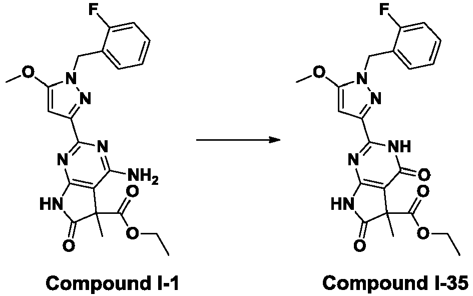

To a cold solution of **Compound I-1** (1 equiv.) in TFA (52 equiv.) at 0 °C was added a solution of sodium nitrite (1.5 equiv.) in water. The mixture was removed from the ice bath and stirred at 23 °C for 1 h. The mixture was diluted in ethyl acetate and washed with water. The organic layer was dried, filtered and evaporated to give a solid. The solid was rinsed with a minimal amount of methanol and pentane. The remaining solid was collected by filtration to deliver the desired compound (81 mg, 40% yield) as a white solid.
¹H NMR (500 MHz, DMSO-*d*₆) δ ppm 7.34 - 7.41 (m, 1 H), 7.15 - 7.28 (m, 4 H), 5.27 (s, 2 H), 4.07 (d, 2 H), 3.95 (s, 3 H), 1.50 (s, 3 H), 1.08 (t, 3 H).

### Compound I-43

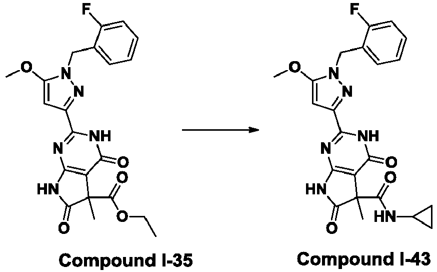

A mixture containing cyclopropylamine (200 equiv.) and **Compound I-35** (1 equiv.) was heated to 70 °C for 24 h. The mixture was concentrated under vacuum to give a crude oil. The crude oil was purified by column chromatography (0 to 100% ethyl acetate in hexanes). The concentrated fractions were rinsed with a minimal amount of methanol and diethyl ether, dried under vacuum to deliver the desired compound (15 mg, 36% yield) as a white solid.
¹H NMR (500 MHz, DMSO-*d*₆) δ ppm 7.34 - 7.42 (m, 1 H), 7.13 - 7.27 (m, 3 H), 6.42 (s, 1 H), 5.27 (s, 2 H), 3.95 (s, 3 H), 2.54 - 2.60 (m, 1 H), 1.44 (s, 3 H), 0.52 - 0.59 (m, 2 H), 0.35 (br. s., 2 H).

### Compound I-15

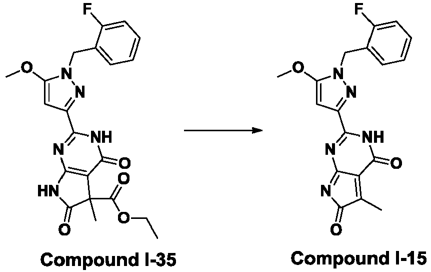

A mixture containing **Compound I-35** (1 equiv.) and 7N solution of ammonia in methanol (100 equiv.) was heated at 70 °C for 2 d. The mixture was concentrated under vacuum to give a white residue. The residue was purified via silica gel chromatography (0 to 10% methanol in dichloromethane) to deliver the desired compound (14 mg, 31% yield) as a white solid.
¹H NMR (500 MHz, DMSO-*d*₆) δ ppm 7.32 - 7.44 (m, 1 H), 7.13 - 7.27 (m, 4 H), 5.27 (s, 2 H), 3.91 - 3.98 (m, 3 H), 1.35 (s, 3 H).

### Compound I-26

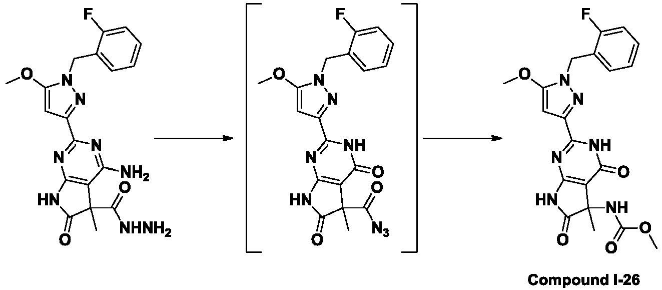

To a cold mixture of
4-amino-2-(1-(2-fluorobenzyl)-5-methoxy-1H-pyrazol-3-yl)-5-methyl-6-oxo-6,7-dihydro-5H-pyrrolo[ 2,3-d]pyrimidine-5-carbohydrazide (described in step 1 towards the synthesis of **Compound I-42** (1 equiv.) in TFA (20 equiv.) at 0 °C, was added a solution of sodium nitrite (2.5 equiv.) in water. The mixture was stirred at 0 °C for 30 min. The mixture was diluted in ethyl acetate and washed with brine. The organic layer was dried, filtered and evaporated to give the desired intermediate. It was dissolved in methanol (10 ml) and heated to 100 °C in a sealed vial for 3 h. The mixture was concentrated under vacuum. The resulting oil was purified via silica gel chromatography (0 to 100% ethyl acetate in hexanes and then 0 to 10% methanol in dichloromethane) to deliver the desired compound (35 mg, 13% yield) as a light orange solid.
¹H NMR (500 MHz, CD₃OD) δ ppm 7.86 (s, 1 H), 7.25 - 7.38 (m, 1 H), 7.07 - 7.17 (m, 3 H), 5.33 (s, 2 H), 3.98 (s, 3 H), 3.56 (s, 3 H), 1.51 - 1.59 (m, 3 H).

### Compound I-16

The title compound was synthesized in 3 steps:

### Step 1: Synthesis of 1-(2-fluorobenzyl)-5-methoxy-1H-pyrazole-3-carboximidhydrazide.

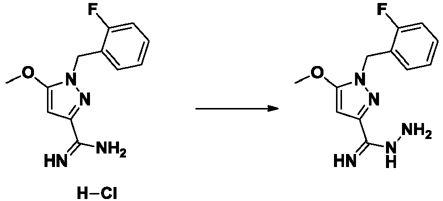

A mixture containing 1-(2-fluorobenzyl)-5-methoxy-1H-pyrazole-3-carboximidamide hydrochloride (described in step 3 towards the synthesis of **Compound I-1**) (1 equiv.), triethylamine (4 equiv.), and hydrazine monohydrate (1 equiv.) in ethanol was stirred at 23 °C for 24 h. The mixture was diluted in ethyl acetate and water. The precipitate was removed by filtration. The filtrate was dried, filtered and evaporated to deliver the desired intermediate, 1-(2-fluorobenzyl)-5-methoxy-1H-pyrazole-3-carboximidhydrazide (465 mg, 50% yield) as a light brown solid.
¹H NMR (500 MHz, CD₃OD) δ ppm 7.23 - 7.38 (m, 1 H), 7.06 - 7.15 (m, 2 H), 7.02 (td, 1 H), 5.98 (s, 1 H), 5.20 - 5.27 (m, 2 H), 3.92 - 3.95 (m, 3 H).

### Step 2: Synthesis of ethyl 2-(3-(1-(2-fluorobenzyl)-5-methoxy-1H-pyrazol-3-yl)-5-oxo-4,5-dihydro-1,2,4-triazin-6-yl)-2-methyl propanoate.

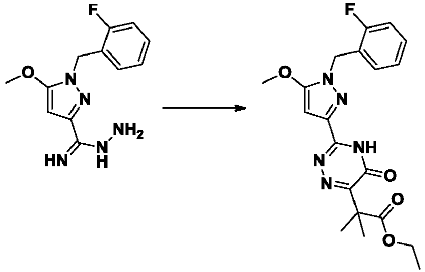

A mixture containing diethyl 2,2-dimethyl-4-oxopentanedioate (1.1 equiv.) and 1-(2-fluorobenzyl)-5-methoxy-1H-pyrazole-3-carboximidhydrazide (1 equiv.) in ethanol was heated to 85 °C for 24 h. The mixture was cooled to 23 °C and the precipitate was removed by filtration. The filtrate was concentrated under vacuum to give a crude oil. The oil was purified via silica gel chromatography (40% ethyl acetate in hexanes) to deliver the desired intermediate, ethyl 2-(3-(1-(2-fluorobenzyl)-5-methoxy-1H-pyrazol-3-yl)-5-oxo-4,5-dihydro-1,2,4-triazin-6-yl)-2-methyl propanoate (84 mg, 11% yield) as a white solid.
¹H NMR (400 MHz, CD₃OD) δ ppm 7.26 - 7.35 (m, 1 H), 7.04 - 7.15 (m, 4 H), 5.35 (s, 2 H), 4.08 - 4.15 (m, 2 H), 3.98 (s, 3 H), 1.50 (s, 6 H), 1.18 (t, 3 H).

### Step 3: Synthesis of Compound I-16

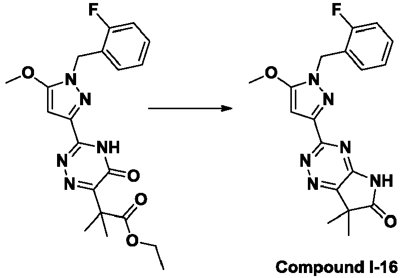

A mixture containing ethyl 2-(3-(1-(2-fluorobenzyl)-5-methoxy-1H-pyrazol-3-yl)-5-oxo-4,5-dihydro-1,2,4-triazin-6-yl)-2-methyl propanoate (1 equiv.) and POCl₃ (7 equiv.) was stirred at 23 °C for 2 h. The mixture was concentrated under vacuum. To the resulting residue, was added a 7N solution of ammonia in methanol] (10 equiv.). The mixture was heated to 100 °C for 24 h. The precipitate was removed by filtration. The filtrate was concentrated under vacuum. The resulting residue was purified via silica gel chromatography (0 to 80% ethyl acetate in hexanes) to deliver the desired compound (39 mg, 34% yield) as a white solid.
¹H NMR (500 MHz, CD₃OD) δ ppm 7.28 - 7.34 (m, 1 H), 7.04 - 7.14 (m, 4 H), 5.35 (s, 2 H), 4.00 (s, 3 H), 1.52 (s, 6 H).

### Compound I-47

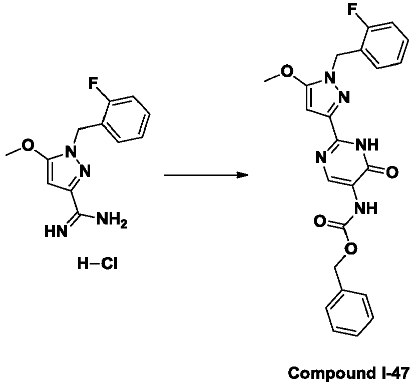

A mixture containing potassium bicarbonate (5 equiv.), 1-(2-fluorobenzyl)-5-methoxy-1H-pyrazole-3-carboximidamide hydrochloride (described in step 3 towards the synthesis of **Compound I-1**) (3 equiv.), and sodium 2-(((benzyloxy)carbonyl)amino)-3-methoxy-3-oxoprop-en-1-olate (1 equiv.) in ethanol was heated at 80 °C for 24 h. The mixture was concentrated under vacuum. The resulting residue was diluted in ethyl acetate and washed with water. The organic layer was dried, filtered and evaporated to give a crude oil. The oil was purified via silica gel column chromatography (0 to 100% ethyl aceate in hexanes). The resulting material was rinsed with a minimal amount of methanol and pentane to deliver the desired compound (107 mg, 36% yield) as a light yellow solid.
¹H NMR (500 MHz, DMSO-*d*₆) δ ppm 7.30 - 7.47 (m, 5 H) 7.14 - 7.28 (m, 3 H) 6.37 (s, 1 H) 5.23 - 5.32 (m, 2 H) 5.16 (s, 2 H) 3.90 - 3.99 (m, 3 H).

### Compound I-25

The title compound was synthesized in 2 steps:

### Step 1: Synthesis of ethyl 4-amino-2-(4-bromo-1-(2-fluorobenzyl)-5-methoxy-1H-pyrazol-3-yl)-5-methyl-6-oxo-6,7-dihydro-5 H-pyrrolo[2,3-d]pyrimidine-5-carboxylate.

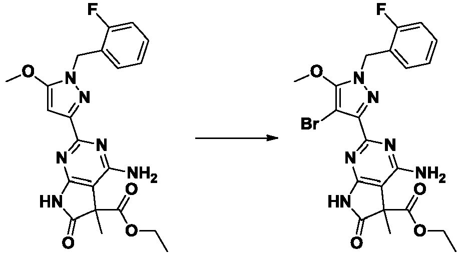

A mixture containing NBS (1.5 equiv.) and **Compound I-1** (1 equiv.) in DMF was stirred at 23 °C for 2 d. The mixture was diluted in ethyl acetate and washed with water. The organic layer was dried, filtered and evaporated to give a crude oil. The oil was purified by silica gel chromatography (0 to 100% ethyl acetate in hexanes) to deliver the desired intermediate, ethyl 4-amino-2-(4-bromo-1-(2-fluorobenzyl)-5-methoxy-1H-pyrazol-3-yl)-5-methyl-6-oxo-6,7-dihydro-5 H-pyrrolo[2,3-d]pyrimidine-5-carboxylate (200 mg, quantitative yield) as an orange oil. ¹H NMR (500 MHz, CD₃OD) δ ppm 7.31 - 7.40 (m, 1 H), 7.10 - 7.27 (m, 3 H), 5.37 (s, 2 H), 4.21 (q, 2 H), 4.03 (s, 3 H), 1.69 (s, 3 H), 1.21 - 1.26 (m, 3 H).

### Step 2: Synthesis of Compound I-25

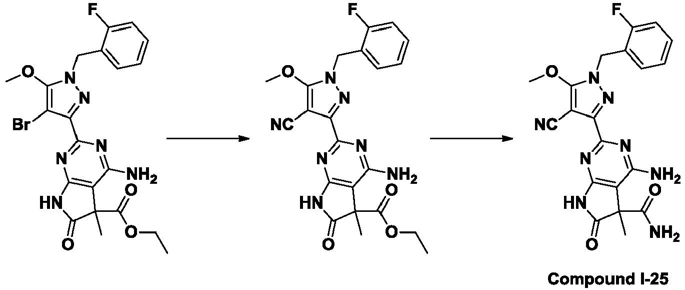

A mixture containing copper (I) cyanide (5.5 equiv.) and ethyl 4-amino-2-(4-bromo-1-(2-fluorobenzyl)-5-methoxy-1H-pyrazol-3-yl)-5-methyl-6-oxo-6,7-dihydro-5 H-pyrrolo[2,3-d]pyrimidine-5-carboxylate (1 equiv.) in DMF was heated to 120 °C in the microwave for 90 min. The mixture was cooled to 23 °C and treated with 10% aqueous solution of ammonium hydroxide and ethyl acetate. The organic layer was isolated, dried, filtered and concentrated to give a crude oil. The oil was purified via silica gel chromatography (0 to 80% ethyl acetate in hexanes) to deliver a yellow oil containing the desired intermediate ethyl 4-amino-2-(4-cyano-1-(2-fluorobenzyl)-5-methoxy-1H-pyrazol-3-yl)-5-methyl-6-oxo-6,7-dihydro-5H -pyrrolo[2,3-d]pyrimidine-5-carboxylate.

The isolated oil was combined with a 7N solution of ammonia in methanol (30 equiv.) and heated to 85 °C for 30 min. The mixture was cooled to 23 °C and concentrated under vacuum. The resulting residue was purified by column chromatography (0 to 10% methanol in dichloromethane) to deliver the desired compound (2.9 mg, 11% yield) as a tan solid.
¹H NMR (500 MHz, DMSO-*d*₆) δ ppm 7.40 (d, 1 H), 7.19 - 7.27 (m, 3 H), 5.27 (s, 2 H), 4.30 (s, 3 H), 1.53 (s, 3 H).

### Compound I-45

The title compound was synthesized in 4 steps:

### Step 1: Synthesis of ethyl 1-(2-fluorobenzyl)-5-hydroxy-4-methyl-1H-pyrazole-3-carboxylate.

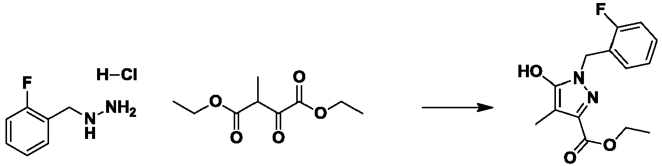

A mixture containing sodium acetate (1 equiv.), diethyl oxalpropionate (1 equiv.), and (2-fluorobenzyl)hydrazine hydrochloride (1 equiv.) in benzene was heated to 90 °C for 1 h. The mixture was diluted in ethyl acetate and washed with water. The organic layer was dried, filtered and evaporated to give a solid. The solid was purified via silica gel chromatography (0 to 100% ethyl acetate in hexanes) to deliver the desired intermediate, ethyl 1-(2-fluorobenzyl)-5-hydroxy-4-methyl-1H-pyrazole-3-carboxylate (800 mg, 29% yield) as a white solid.
¹H NMR (500 MHz, CD₃OD) δ ppm 7.29 (tdd, 1 H), 7.06 - 7.12 (m, 2 H), 6.94 - 6.99 (m, 1 H), 5.26 (s, 2 H), 4.28 - 4.34 (m, 2 H), 2.13 (s, 3 H), 1.35 (t, 3 H).

### Step 2: Synthesis of ethyl 1-(2-fluorobenzyl)-5-methoxy-4-methyl-1H-pyrazole-3-carboxylate.

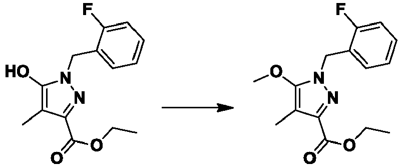

A mixture of potassium carbonate (1.1 equiv.), methyl ester of *p*-toluenesulfonic acid (1.1 equiv), and ethyl 1-(2-fluorobenzyl)-5-hydroxy-4-methyl-1H-pyrazole-3-carboxylate (1 equiv.) in DMF (14 ml) was stirred at 23 °C for 24 h. The mixture was diluted in ethyl acetate and washed with 1N HCl solution. The organic layer was dried, filtered and evaporated to give a crude oil. The oil was purified via silica gel chromatography (0 to 50% ethyl acetate in hexanes) to deliver the desired intermediate ethyl 1-(2-fluorobenzyl)-5-methoxy-4-methyl-1H-pyrazole-3-carboxylate (800 mg, 95% yield) as a clear oil. ¹H NMR (500 MHz, CDCl₃) δ ppm 7.22 - 7.30 (m, 1 H), 6.98 - 7.10 (m, 3 H), 5.33 (s, 2 H), 4.37 - 4.44 (m, 2 H), 3.79 (s, 3 H), 2.23 - 2.27 (m, 3 H), 1.41 (t, 3 H).

### Step 3: Synthesis of 1-(2-fluorobenzyl)-5-methoxy-4-methyl-1H-pyrazole-3-carboximidamide

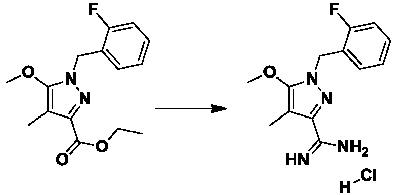

To a cold suspension of ammonium chloride (5.3 equiv.) in toluene at 0 °C, was added a 2M solution of trimethylaluminum in heptane (5.3 equiv.). The mixture was removed from the ice bath and stirred at 23 °C until the mixture ceased bubbling. Then, to this mixture, was added a solution of ethyl 1-(2-fluorobenzyl)-5-methoxy-4-methyl-1H-pyrazole-3-carboxylate (1 equiv.) in toluene (7 ml). The mixture was heated to 100 °C for 24 h. The contents were cooled to 0 °C, quenched with methanol (5.3 equiv.) and dichloromethane, and stirred at 23 °C for 30 min. The precipitate was removed by filtration. The filtrate was concentrated under vacuum to deliver the desired intermediate 1-(2-fluorobenzyl)-5-methoxy-4-methyl-1H-pyrazole-3-carboximidamide (263 mg, 37% yield) as a light brown gum.
¹H NMR (500 MHz, CD₃OD) δ ppm 7.31 - 7.38 (m, 1 H), 7.11 - 7.28 (m, 3 H), 5.33 (s, 2 H), 3.92 (s, 3 H), 2.21 (s, 3 H).

### Step 4: Synthesis of Compound I-45

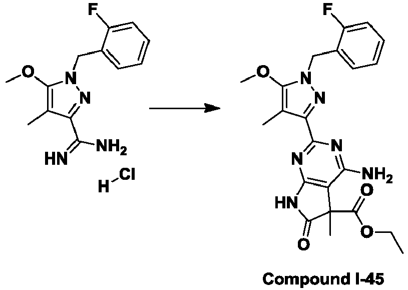

A mixture containing diethyl 2-(dicyanomethyl)-2-methylmalonate (1.1 equiv.), potassium bicarbonate (1.1 equiv.), and 1-(2-fluorobenzyl)-5-methoxy-4-methyl-1H-pyrazole-3-carboximidamide (1 equiv.) in *tert-BuOH* was heated to 80 °C for 3 h. The mixture was diluted in ethyl acetate and washed with water. The organic layer was dried, filtered and evaporated to give a crude oil. The oil was purified via silica gel chromatography (0 to 100% ethyl acetate in hexanes) to deliver the desired compound (139 mg, 31% yield) as a light yellow solid.
¹H NMR (500 MHz, CD₃OD) δ ppm 7.28 - 7.35 (m, 1 H), 7.09 - 7.15 (m, 2 H), 7.03 - 7.09 (m, 1 H), 5.33 (s, 2 H), 4.21 (q, 2 H), 3.87 (s, 3 H), 2.36 (s, 3 H), 1.68 (s, 3 H), 1.18 - 1.23 (m, 3 H).

### Compound I-27

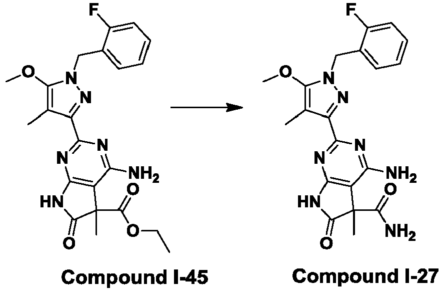

A mixture containing **Compound I-45** (1 equiv.) and a 7N solution of ammonia (10 equiv.) was stirred at 23 °C for 24 h. The precipitate formed was collected and dried under vacuum to deliver the desired compound (89 mg, 76% yield) as a white solid.
¹H NMR (500 MHz, DMSO-*d*₆) δ ppm 7.32 - 7.39 (m, 1 H), 7.20 - 7.25 (m, 1 H), 7.15 - 7.19 (m, 2 H), 5.23 (s, 2 H), 3.85 (s, 3 H), 2.28 (s, 3 H), 1.52 (s, 3 H).

### Compound I-46

The title compound was synthesized in 3 steps:

### Step 1: Synthesis of ethyl 5-ethoxy-1-(2-fluorobenzyl)-1H-pyrazole-3-carboxylate.

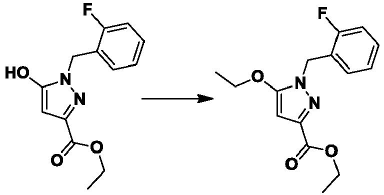

A mixture containing ethyl 1-(2-fluorobenzyl)-5-hydroxy-1H-pyrazole-3-carboxylate (described in step 1 towards the synthesis of **Compound I-1**) (1 equiv.), potassium carbonate (3 equiv.), and iodoethane (2 equiv.) was stirred at 23°C for 48 h. The mixture was diluted with ethyl acetate and washed with water. The organic layer was dried, filtered and evaporated to give a crude oil which was purified via silica gel chromatography to deliver the desired intermediate ethyl 5-ethoxy-1-(2-fluorobenzyl)-1H-pyrazole-3-carboxylate as a colorless oil.
¹H NMR (500 MHz, DMSO-*d*₆) δ ppm 7.37 (m, 1 H), 7.20 (m, 2 H), 7.11 (m, 1 H), 6.20 (m, 1 H), 5.23 (m, 2 H), 4.20 (m, 4 H), 1.28 (dt, 6 H).

### Step 2: Synthesis of 5-ethoxy-1-(2-fluorobenzyl)-1H-pyrazole-3-carboximidamide.

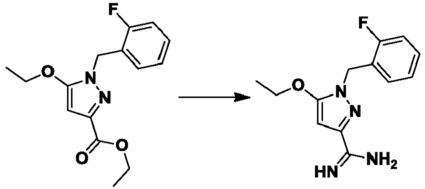

To a suspension of ammonia hydrochloride (5.3 equiv.) in toluene at 0 °C was added a solution of trimethylaluminum 2M in toluene (5.3 equiv.). The mixture was removed from the ice bath and stirred at 23°C until bubbling ceased. To this mixture was added a solution of ethyl 5-ethoxy-1-(2-fluorobenzyl)-1H-pyrazole-3-carboxylate (1 equiv.) in toluene and stirred at 80 °C for 24 h. The mixture was cooled in an ice bath and quenched slowly with methanol, and the resulting white precipitate was removed by filtration on celite pad. The filtrate was concentrated and dried under vacuum to deliver the desired intermediate 5-ethoxy-1-(2-fluorobenzyl)-1H-pyrazole-3-carboximidamide as an off-white solid.
¹H NMR (500 MHz, DMSO-*d*₆) δ ppm 9.20 (m, 3 H), 7.18 (m, 4 H), 6.71 (s, 1 H), 5.31 (s, 2 H), 4.19 (d, 2 H), 1.34 (t, 3 H).

### Step 3: Synthesis of Compound I-46

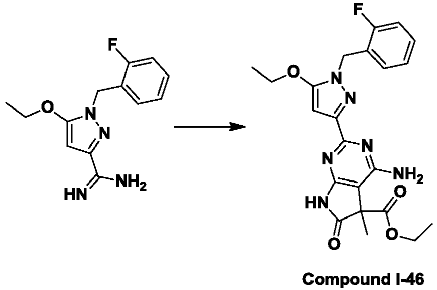

A mixture containing 5-ethoxy-1-(2-fluorobenzyl)-1H-pyrazole-3-carboximidamide (1 equiv.), diethyl 2-(dicyanomethyl)-2-methylmalonate (1.5 equiv.), and potassium hydrogencarbonate (2 equiv.) in tert-butanol was heated at 85°C for 5 h. The mixture was cooled, diluted with ethyl acetate, and washed with water. The organic layer was dried, filtered and evaporated to give a crude oil which was purified via silica gel chromatography to deliver the desired compound (17 mg, 5% yield) as an off white solid.
¹H NMR (500 MHz, CDCl₃) δ ppm 8.81 (m, 1 H), 7.14 (m, 1 H), 6.93 (m, 3 H), 6.21 (m, 1 H), 5.44 (m, 1 H), 5.29 (s, 2 H), 4.12 (m, 5 H), 1.64 (s, 3 H), 1.31 (t, 3 H), 1.15 (t, 3 H).

### Compound I-28

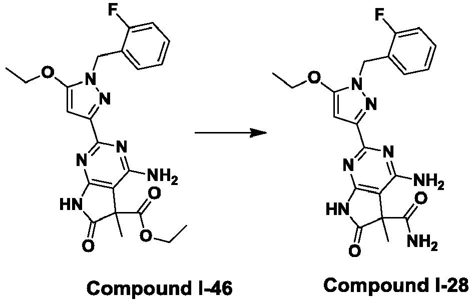

A solution of **Compound I-46** (1 equiv.) in 7N ammonia in methanol (excess) was stirred at 23 °C for 18 h. The solvent was removed in vacuo to deliver the desired compound (14 mg, 100 % yield) as an off white solid.
¹H-NMR (500 MHz, CD₃OD) δ ppm 7.19 (m, 1 H), 6.97 (m, 3 H), 6.19 (m, 1 H), 5.21 (s, 2 H), 4.10 (m, 2 H), 1.60 (s, 3 H), 1.28 (m, 3 H).

### Compound I-8

The title compound was synthesized in 3 steps:

### Step 1: Synthesis of ethyl 1-(2-fluorobenzyl)-5-isopropoxy-1H-pyrazole-3-carboxylate.

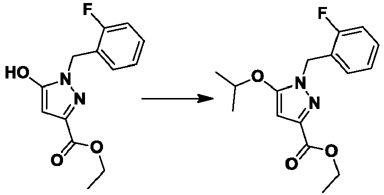

A mixture containing ethyl 1-(2-fluorobenzyl)-5-hydroxy-1H-pyrazole-3-carboxylate (described in step 1 towards the synthesis of **Compound I-1**) (1 equiv.), 2-iodopropane (2 equiv.), and potassium carbonate (3 equiv.) in DMF was stirred at 23 °C for 48 h. The mixture was diluted with ethyl acetate and washed with water. The organic layer was dried, filtered and evaporated to give a crude oil which was purified via silica gel chromatography to deliver the desired intermediate ethyl 1-(2-fluorobenzyl)-5-isopropoxy-1H-pyrazole-3-carboxylate as a colorless oil.
¹H NMR (500 MHz, DMSO-*d*₆) δ ppm 7.37 (m, 1 H), 7.21 (m, 2 H), 7.11 (m, 1 H), 6.21 (m, 1 H), 5.22 (s, 2 H), 4.57 (dt, 1 H), 4.23 (q, 2 H), 1.26 (m, 9 H).

### Step 2: Synthesis of 5-isopropoxy-1-(2-fluorobenzyl)-1H-pyrazole-3-carboximidamide.

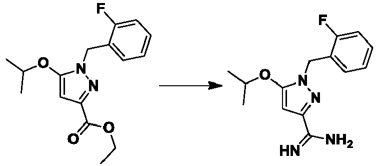

To a suspension of ammonia hydrochloride (5.3 equiv.) in toluene at 0 °C was added a solution of trimethylaluminum 2M in toluene (5.3 equiv.). The mixture was removed from the ice bath and stirred at 23 °C until bubbling ceased. To this mixture was added a solution of ethyl 1-(2-fluorobenzyl)-5-isopropoxy-1H-pyrazole-3-carboxylate (1 equiv.) in toluene and stirred at 80 °C for 24 h. The mixture was cooled in an ice bath and quenched slowly with methanol and the resulting white precipitate was removed by filtration on celite pad. The filtrate was concentrated and dried under vacuum to deliver the desired intermediate 5-isopropoxy-1-(2-fluorobenzyl)-1H-pyrazole-3-carboximidamide as an off-white solid.
¹H-NMR (500 MHz, DMSO-*d*₆) δ ppm 7.21 (m, 4 H), 6.75 (s, 1 H), 5.28 (m, 2 H), 4.48 (m, 1 H), 1.30 (d, 6 H).

### Step 3: Synthesis of Compound I-8

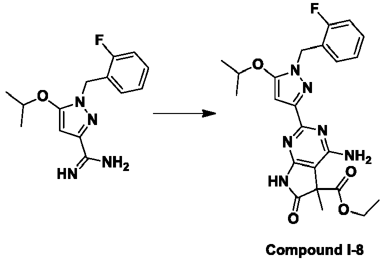

A mixture containing 5-isopropoxy-1-(2-fluorobenzyl)-1H-pyrazole-3-carboximidamide (1 equiv.), diethyl 2-(dicyanomethyl)-2-methylmalonate (1.5 equiv.), and potassium hydrogencarbonate (2 equiv.) in *tert*-butanol was heated at 85 °C for 5 h. The mixture was cooled, diluted with ethyl acetate, and washed with water. The organic layer was dried, filtered and evaporated to give a crude oil which was purified via silica gel chromatography to deliver the desired compound (89 mg, 18% yield) as an off white solid.
¹H-NMR (500 MHz, CD₃OD) δ 7.31 (m, 1 H), 7.09 (m, 3 H), 6.32 (s, 1 H), 5.31 (s, 2 H), 4.55 (m, 1 H), 4.21 (q, 2 H), 1.69 (s, 3 H), 1.33 (d, 6 H), 1.21 (s, 3 H).

### Compound I-39

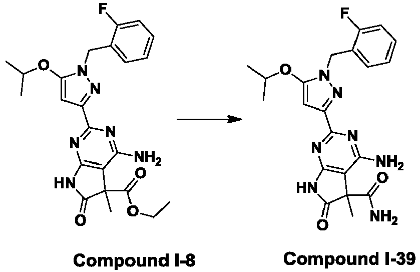

A solution of **Compound I-8** (1 equiv.) in 7N ammonia in methanol (excess) was stirred at 23°C for 18 h. The solvent was removed in vacuo to give 4-amino-2-(5-isopropoxy-1-(2-fluorobenzyl)-1H-pyrazol-3-yl)-5-methyl-6-oxo-6,7-dihydro-5H-pyrro lo[2,3-d]pyrimidine-5-carboxamide (46 mg, 61 % yield) as an off white solid.
¹H NMR (500 MHz, CD₃OD) δ ppm 7.31 (m, 1 H), 7.09 (m, 3 H), 6.32 (s, 1 H), 5.31 (s, 2 H), 4.56 (m, 1 H), 1.72 (s, 3 H), 1.35 (d, 6 H).

### Compound I-51

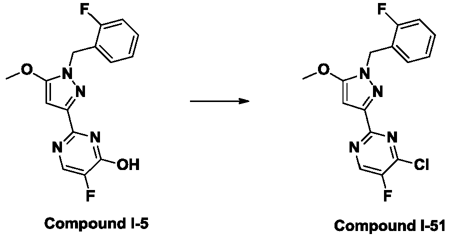

A suspension of **Compound I-5** (1 equiv.) in phosphoryl trichloride (50 equiv.) was heated to 65 °C for 2 h. The solution was cooled to 23 °C, and concentrated down to a thick brown syrup. The residue was reconstituted in dichloromethane and purified via silica gel chromatography (0-100% ethyl acetate in hexanes gradient) to yield the title compound (1.6 g, 95 % yield) as a brown solid.
¹H NMR (500 MHz, DMSO-*d*₆) δ ppm 8.92 (m, 1 H), 7.37 (m, 1 H), 7.21 (m, 2 H), 7.12 (m, 1 H), 6.40 (m, 1 H), 5.27 (m, 2 H), 3.97 (s, 3 H).

### Compound I-19

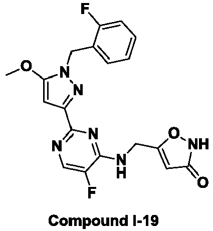

The title compound was prepared following general procedure A in library format, except muscimol was the amine reactant, 3 equivalents of triethylamine was used, and contents were stirred at 70 °C for 2 d. The resulting residue after workup was suspended in dichloromethane and a minimal amount of methanol. The precipitate was removed by filtration and the filtrate was concentrated under vacuum. The resulting residue was purified via silica gel chromatography (0 to 10% methanol in dichloromethane) to deliver the desired compound (87 mg, 96% yield) as a white solid.
¹H NMR (500 MHz, DMSO-*d*₆) δ ppm 8.34 (br. s., 1 H), 7.32 - 7.41 (m, 1 H), 7.23 (d, 1 H), 7.17 (t, 1 H), 7.06 - 7.12 (m, 1 H), 6.41 (br. s., 1 H), 5.77 (s, 1 H), 5.28 (s, 2 H), 4.72 (s, 2 H), 3.96 (s, 3 H).

### Compound I-20

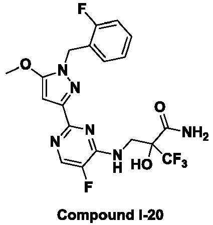

The title compound was prepared following general procedure A in library format, except 2-(aminomethyl)-3,3,3-trifluoro-2-hydroxypropanamide was the amine reactant, 3 equivalents of triethylamine was used, and contents were stirred at 100 °C for 24 h. The resulting solid after workup was rinsed with a minimal amount of methanol and diethyl ether. The remaining solid was dried under vacuum to deliver the desired compound, **Compound I-20** (40 mg, 29% yield) as a white solid.
¹H NMR (500 MHz, DMSO-*d*₆) δ ppm 8.27 (d, 1 H), 7.64 (br. s., 1 H), 7.12 - 7.25 (m, 3 H), 6.27 (s, 1 H), 5.24 (s, 2 H), 3.94 (s, 3 H).

### Compound I-9

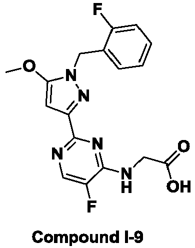

The title compound was prepared following general procedure A in library format, except 2-aminoacetic acid (4 equiv.) was the amine reactant, 13.5 equivalents of triethylamine was used, and contents were stirred at 100 °C for 3 d as a solution in THF:water (2:1). The reaction was cooled to 23 °C, concentrated in vacuo, and the resulting crude material was purified via reverse phase HPLC to deliver the desired compound (2.7 mg, 12% yield) as a white solid.
¹H-NMR (500 MHz, DMSO-d₆) δ ppm 8.52 (m, 1 H), 8.28 (m, 1 H), 7.35 (m, 1 H), 7.19 (m, 2 H), 7.05 (m, 1 H), 6.31 (s, 1 H), 5.26 (s, 2 H), 4.18 (m, 2 H), 3.94 (s, 3 H).

### Compound I-13

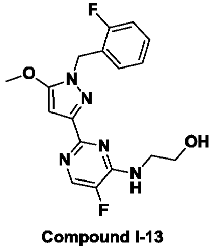

The title compound was prepared following general procedure A in library format, except 2-aminoethanol (4 equiv.) was the amine reactant, 13.5 equivalents of triethylamine was used, and contents were stirred at 100 °C for 3 d as a solution in THF:water (2:1). The reaction was cooled to 23 °C, concentrated in vacuo, and the resulting crude material was purified via reverse phase HPLC to deliver the desired compound (7.5 mg, 35% yield) as a white solid.
¹H-NMR (500 MHz, DMSO-d₆) δ ppm 8.69 (m, 1 H), 8.28 (m, 1 H), 7.38 (m, 1 H), 7.22 (m, 2 H), 7.07 (m, 1 H), 6.42 (s, 1 H), 5.30 (s, 3 H), 3.98 (s, 3 H), 3.62 (s, 4 H).

### Compound I-32

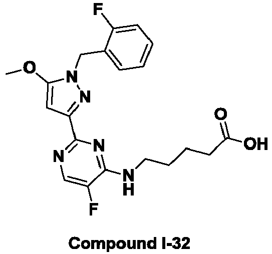

The title compound was prepared following general procedure A in library format, except 5-aminopentanoic acid (4 equiv.) was the amine reactant, 13.5 equivalents of triethylamine was used, and contents were stirred at 100 °C for 3 d as a solution in THF: water (2:1). The reaction was cooled to 23 °C, concentrated in vacuo, and the resulting crude material was purified via reverse phase HPLC to deliver the desired compound (2.5 mg, 10% yield) as a white solid.
¹H-NMR (500 MHz, DMSO-d₆) δ ppm 12.03 (m, 1 H), 8.57 (m, 1 H), 8.24 (m, 1 H), 7.36 (m, 1 H), 7.20 (m, 2 H), 7.08 (m, 1 H), 6.38 (m, 1 H), 5.28 (s, 2 H), 3.97 (s, 3 H), 3.53 (m, 2 H), 2.28 (m, 2 H), 1.60 (m, 4 H).

### Compound I-36

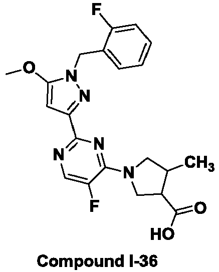

The title compound was prepared following general procedure A in library format, except 4-methylpyrrolidine-3-carboxylic acid (4 equiv.) was the amine reactant, 13.5 equivalents of triethylamine was used, and contents were stirred at 100 °C for 3 d as a solution in THF:water (2:1). The reaction was cooled to 23 °C, concentrated in vacuo, and the resulting crude material was purified via reverse phase HPLC to deliver the desired compound (6.3 mg, 25% yield) as a white solid.
¹H-NMR (500 MHz, DMSO-d₆) δ ppm 8.31 (m, 1 H), 7.37 (m, 1 H), 7.21 (m, 2 H), 7.06 (m, 1 H), 6.40 (s, 1 H), 5.28 (s, 2 H), 4.10 (m, 2 H), 3.96 (s, 3 H), 3.84 (m, 1 H), 3.35 (m, 1 H), 3.07 (m, 1 H), 2.79 (m, 1 H), 1.15 (m, 3 H).

### Compound I-11

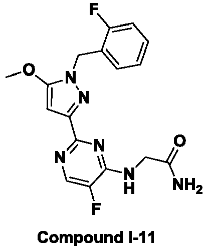

The title compound was prepared following general procedure A in library format, except 2-aminoacetamide (8 equiv.) was the amine reactant, 20 equivalents of triethylamine was used, and contents were stirred at 100 °C for 18 h as a solution in THF:water (2:1). The reaction was concentrated under a stream of nitrogen, and the resulting crude material was purified by reverse phase HPLC to deliver the desired compound (1.8 mg, 8% yield) as a white solid.
¹H-NMR (500 MHz, DMSO-d₆) δ ppm 8.26 (m, 1 H), 7.55 (m, 1 H), 7.35 (m, 1 H), 7.18 (m, 3 H), 7.04 (m, 1 H), 6.33 (s, 1 H), 5.27 (s, 2 H), 4.05 (d, 3 H), 3.95 (s, 3 H).

### Compound I-33

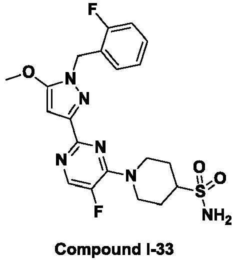

The title compound was prepared following general procedure A in library format, except piperidine-4-sulfonamide (4 equiv.) was the amine reactant, 13.5 equivalents of triethylamine was used, and contents were stirred at 100 °C for 3 d as a solution in THF:water (2:1). The reaction was cooled to 23 °C, concentrated in vacuo, and the resulting crude material was purified via reverse phase HPLC to deliver the desired compound (1.1 mg, 4% yield) as a white solid.
¹H-NMR (500 MHz, CD₃OD) δ ppm 8.26 (m, 1 H), 7.35 (m, 1 H), 7.13 (m, 3 H), 6.47 (m, 1 H), 5.37 (m, 2 H), 4.99 (m, 2 H), 4.04 (s, 3 H), 3.39 (m, 3 H), 2.36 (m, 2 H), 1.94 (m, 2 H).

### Compound I-12

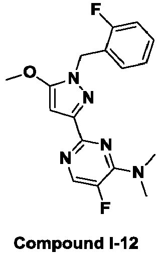

The title compound was prepared following general procedure A in library format, except dimethylamine (50 equiv.) was the amine reactant, no triethylamine was used, and contents were stirred at 23 °C for 1 h, then 90 °C for 12 h as a solution in dioxane:water (3:1). The resulting crude material after workup was purified via reverse phase HPLC to deliver the desired compound (6 mg, 29% yield) as a white solid.
¹H-NMR (500 MHz, CD₃OD) δ ppm 8.22 (m, 1 H), 7.35 (m, 1 H), 7.16 (m, 3 H), 6.50 (m, 1 H), 5.39 (s, 2 H), 4.04 (m, 3 H), 3.54 (m, 6 H).

### Compound I-31

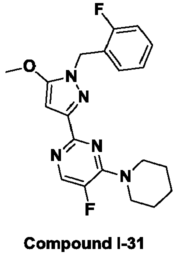

The title compound was prepared following general procedure A in library format, except piperidine (1 equiv.) was the amine reactant, no triethylamine was used, and contents were stirred at 23 °C for 1 h, then 90 °C for 12 h as a solution in dioxane:water (3:1). The resulting crude material after workup was purified via reverse phase HPLC to deliver the desired compound (6 mg, 26% yield) as a white solid.
¹H-NMR (500 MHz, CD₃OD) δ ppm 8.21 (m, 1 H), 7.36 (m, 1 H), 7.15 (m, 3 H), 6.49 (m, 1 H), 5.38 (m, 2 H), 4.13 (br. s., 4 H), 4.05 (s, 3H), 1.84 (br. s., 6 H).

### Compound I-23

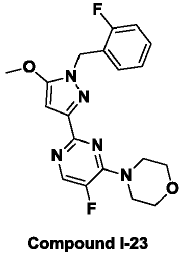

The title compound was prepared following general procedure A in library format, except morpholine (1 equiv.) was the amine reactant, no triethylamine was used, and contents were stirred at 23 °C for 1 h, then 90 °C for 12 h as a solution in dioxane:water (3:1). The mixture was worked up according to procedure to deliver the desired compound (7 mg, 30% yield) as a white solid.
¹H-NMR (500 MHz, CD₃OD) δ ppm 8.29 (m, 1 H), 7.35 (m, 1 H), 7.16 (m, 3 H), 6.52 (m, 1 H), 5.39 (m, 2 H), 4.17 (m, 4 H), 4.05 (s, 3 H), 3.87 (m, 4 H).

### Compound I-24

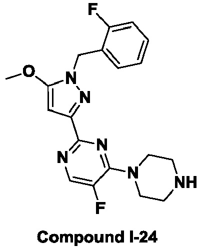

The title compound was prepared following general procedure A in library format, except piperazine (1 equiv.) was the amine reactant, no triethylamine was used, and contents were stirred at 23 °C for 1 h, then 90 °C for 12 h as a solution in dioxane:water (3:1). The mixture was worked up according to procedure to deliver the desired compound (5 mg, 22% yield) as a white solid.
¹H-NMR (500 MHz, CD₃OD) δ ppm 8.11 (m, 1 H), 7.30 (m, 1 H), 7.09 (m, 2 H), 7.00 (m, 1 H), 6.30 (m, 1 H), 5.31 (m, 2 H), 3.97 (s, 3 H), 3.88 (m, 4 H), 2.94 (m, 4 H).

### Compound I-22

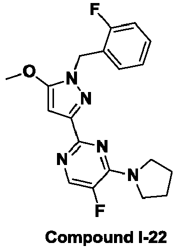

The title compound was prepared following general procedure A in library format, except pyrrolidine (1 equiv.) was the amine reactant, no triethylamine was used, and contents were stirred at 23 °C for 1 h, then 90 °C for 12 h as a solution in dioxane:water (3:1). The mixture was worked up according to procedure to deliver the desired compound (7 mg, 32% yield) as a white solid.
¹H-NMR (500 MHz, CD₃OD) δ 8.18 (m, 1 H), 7.36 (m, 1 H), 7.15 (m, 3 H), 6.48 (s, 1 H), 5.39 (s, 2 H), 4.05 (s, 7 H), 2.12 (m, 4 H).

### Compound I-52

The title compound was synthesized in 4 steps:

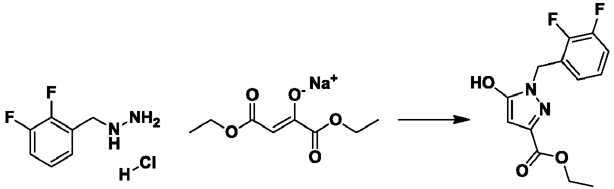

### Step 1: Synthesis of ethyl 1-(2,3-difluorobenzyl)-5-hydroxy-1H-pyrazole-3-carboxylate.

A mixture containing (2,3-difluorobenzyl)hydrazine hydrochloride (1.1 equiv.), acetic acid (10 equiv.) and sodium (*Z*)-1,4-diethoxy-1,4-dioxobut-2-en-2-olate (1 equiv.) in benzene was heated to reflux for 24 h. The mixture was cooled to 23 °C and concentrated under vacuum. The resulting solid was rinsed with a minimal amount of methanol and diethyl ether. The solid was collected by filtration and dried under vacuum to deliver the desired intermediate, ethyl 1-(2,3-difluorobenzyl)-5-hydroxy-1H-pyrazole-3-carboxylate (11.07 g, 92% yield) as a light yellow solid.
¹H NMR (500 MHz, DMSO-*d*₆) δ ppm 7.27 - 7.50 (m, 1 H), 7.06 - 7.27 (m, 1 H), 6.79 - 6.92 (m, 1 H), 5.75 - 5.93 (m, 1 H), 5.14 - 5.33 (m, 2 H), 4.14 - 4.33 (m, 2 H), 1.23 (dt, 3 H).

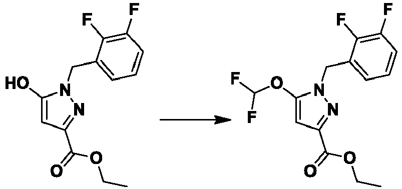

### Step 2: Synthesis of ethyl 1-(2,3-difluorobenzyl)-5-(difluoromethoxy)-1H-pyrazole-3-carboxylate.

A mixture containing ethyl 1-(2,3-difluorobenzyl)-5-hydroxy-1H-pyrazole-3-carboxylate (1 equiv.), potassium carbonate (1.1 equiv.), and sodium 2-chloro-2,2-difluoroacetate (1.4 equiv.) in DMF was heated to 100 °C for 3 h. The mixture was cooled to 23 °C and diluted with ethyl acetate. The organic layer was washed with water, dried, filtered and evaporated to give a crude oil. The oil was purified via silica gel chromatography (0 to 100% ethyl acetate in hexanes) to deliver the desired intermediate, ethyl 1-(2,3-difluorobenzyl)-5-(difluoromethoxy)-1H-pyrazole-3-carboxylate (4.72 g, 36% yield) as a yellow oil.
¹H NMR (500 MHz, CDCl₃) δ ppm 7.08 - 7.18 (m, 1 H), 7.01 - 7.07 (m, 1 H), 6.87 (t, 1 H), 6.51 (s, 1H), 6.48 (s, 1 H), 5.41 (s, 2 H), 4.41 (q, 2 H), 1.40 (t, 3 H).

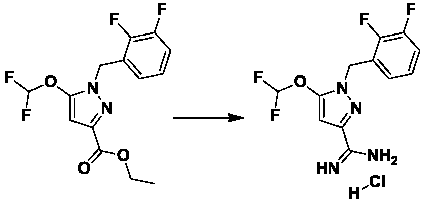

### Step 3: Synthesis of 1-(2,3-difluorobenzyl)-5-(difluoromethoxy)-1H-pyrazole-3-carboximidamide hydrochloride.

To a cold suspension of ammonium chloride (5.3 equiv.) in toluene at 0 °C, was added a 2 M solution of triemthylaluminum in heptane (5.3 equiv.). The mixture was removed from the ice bath and stirred at 23 °C for 1 h. To this mixture, was added a solution of ethyl 1-(2,3-difluorobenzyl)-5-(difluoromethoxy)-1H-pyrazole-3-carboxylate (1 equiv.) in toluene. The mixture was heated to reflux for 24 h. The mixture was cooled to 0 °C and quenched with methanol (1 equiv.) and a 6 N aqueous solution of HCl (16 equiv.). The mixture was heated to 90 °C for 10 min. The mixture was cooled to 23 °C and the precipitate formed was collected by filtration. The solid was further dried in vacuo to deliver the desired intermediate,
1-(2,3-difluorobenzyl)-5-(difluoromethoxy)-1H-pyrazole-3-carboximidamide hydrochloride (6.8 g, quantitative yield) as a light yellow solid.
¹H NMR (500 MHz, DMSO-*d*₆) δ ppm 7.42 - 7.56 (m, 1 H), 7.37 (s, 1 H), 7.17 - 7.27 (m, 1 H), 6.98 - 7.12 (m, 2 H), 5.49 (s, 2 H).

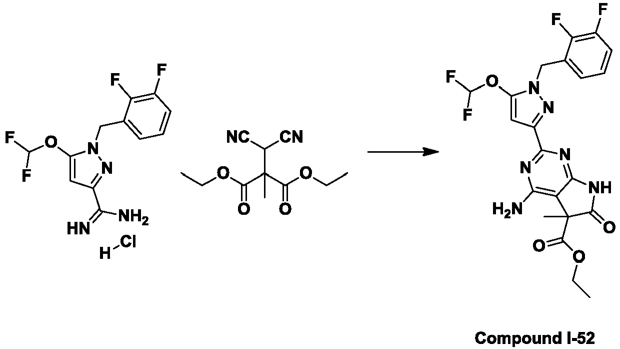

### Step 4: Synthesis of Compound I-52

A mixture containing diethyl 2-(dicyanomethyl)-2-methylmalonate (3 equiv.), potassium hydrogencarbonate (3 equiv.), and 1-(2,3-difluorobenzyl)-5-(difluoromethoxy)-1H-pyrazole-3-carboximidamide hydrochloride (1 equiv.) in *tert*-BuOH was heated to 80 °C for 3 h. The mixture was cooled to 23 °C and diluted in ethyl acetate. The organic layer was washed with water, dried, filtered and evaporated to give a crude oil which was purified via silica gel chromatography (0 to 10% ethyl acetate in hexanes) to deliver the desired compound (220 mg, 30% yield) as a light yellow solid.
¹H NMR (500 MHz, DMSO-*d*₆) δ ppm 7.54 (s, 1 H), 7.40 - 7.47 (m, 1 H), 7.24 - 7.40 (m, 1 H), 7.18 - 7.24 (m, 1 H), 7.02 (d, 1 H), 5.39 (s, 2 H), 4.00 - 4.15 (m, 2 H), 1.57 (s, 3 H), 1.10 (t, 3 H).

### Compound I-53

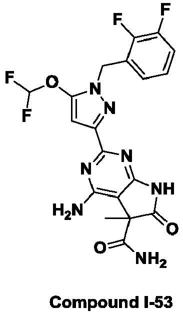

A mixture containing a 7 N solution of ammonia in methanol (50 equiv.) in methanol and **Compound I-52** (1 equiv.) was stirred at 23 °C for 24 h. The precipitate formed during the reaction was collected by filtration and dried in vacuo to deliver the desired compound (48 mg, 58% yield) as a white solid.
¹H NMR (500 MHz, DMSO-*d*₆) δ ppm 7.38 - 7.48 (m, 1 H), 7.12 - 7.28 (m, 2 H), 7.01 (t, 1 H), 6.42 - 6.52 (m, 1 H), 5.39 (s, 2 H), 1.49 - 1.55 (m, 3 H).

### Compound I-54

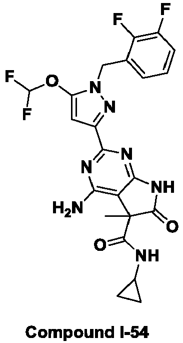

A mixture containing cyclopropylamine (60 equiv.) and **Compound I-52** (1 equiv.) was heated to 60 °C for 18 h. The mixture was in vacuo, and the resulting residue was purified via silica gel chromatography (0 to 100% ethyl acetate in hexanes) to deliver the desired compound (32 mg, 82% yield) as a white solid.
¹H NMR (500 MHz, CD₃OD) δ ppm 7.22 (q, 1 H), 6.82 - 7.15 (m, 3 H), 6.62 (s, 1 H), 5.44 (s, 2 H), 2.67 (tt, 1 H), 1.66 (s, 3 H), 0.66 - 0.78 (m, 2 H), 0.46 - 0.58 (m, 2 H).

### Compound I-55

The title compound was synthesized in 4 steps:

### Step 1: Synthesis of ethyl 1-(2,4-difluorobenzyl)-5-hydroxy-1H-pyrazole-3-carboxylate.

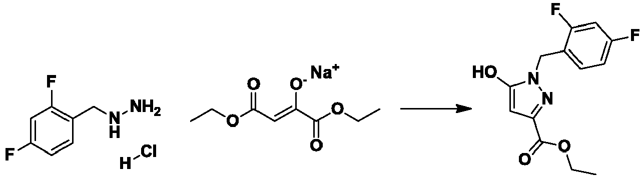

A mixture containing acetic acid (2 equiv.), (2,4-difluorobenzyl)hydrazine hydrochloride (1 equiv.) and sodium (*Z*)-1,4-diethoxy-1,4-dioxobut-2-en-2-olate (1 equiv.) in benzene was heated to 90 °C for 24 h. The mixture was diluted in ethyl acetate and washed with water. The organic layer was dried, filtered and evaporated to give a solid, which was purified via silica gel chromatography (0 to 100% ethyl acetate in hexanes) to deliver the desired intermediate, ethyl 1-(2,4-difluorobenzyl)-5-hydroxy-1H-pyrazole-3-carboxylate (1.06 g, 49% yield) as a light yellow solid.
¹H NMR (500 MHz, CD₃OD) δ ppm 7.09 - 7.23 (m, 1 H), 6.87 - 7.03 (m, 2 H), 5.91 (s, 1 H), 5.14 - 5.29 (m, 2 H), 4.31 (q, 2 H), 1.21 - 1.42 (m, 3 H).

### Step 2: Synthesis of ethyl 1-(2,4-difluorobenzyl)-5-(difluoromethoxy)-1H-pyrazole-3-carboxylate

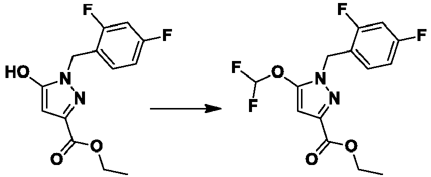

.In a flask equipped with a reflux condenser and under an atmosphere of argon, a mixture of ethyl 1-(2,4-difluorobenzyl)-5-hydroxy-1H-pyrazole-3-carboxylate (1 equiv.), potassium carbonate (1.1 equiv.), and sodium 2-chloro-2,2-difluoroacetate (1.4 equiv.) in DMF was heated to 100 °C for 1.5 h. The mixture was cooled to 23 °C and diluted with ethyl acetate and water. The organic layer was washed with water, dried, filtered and evaporated to give a crude oil which was purified via silica gel chromatography (0 to 50% ethyl acetate in hexanes) to deliver the desired intermediate, ethyl 1-(2,4-difluorobenzyl)-5-(difluoromethoxy)-1H-pyrazole-3-carboxylate (1.42 g, quantitative yield) as a light yellow solid.
¹H NMR (500 MHz, CD₃OD) δ ppm 7.24 - 7.32 (m, 1 H), 7.11 - 7.16 (m, 1 H), 6.93 - 7.03 (m, 1 H), 6.85 (s, 1 H), 6.47 (s, 1 H), 5.34 (s, 2 H), 4.30 - 4.38 (m, 2 H), 1.32 - 1.39 (m, 3 H).

### Step 3: Synthesis of 1-(2,4-difluorobenzyl)-5-(difluoromethoxy)-1H-pyrazole-3-carboximidamide hydrochloride.

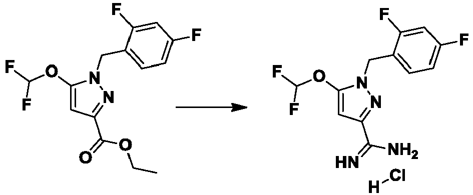

To a cold suspension of ammonium chloride (5.3 equiv.) in toluene at 0 °C, was added a 2 M solution of trimethylaluminum in heptane (5.3 equiv.). The mixture was removed from the ice bath and stirred at 23 °C for 1 h. To this mixture was added a solution of ethyl 1-(2,4-difluorobenzyl)-5-(difluoromethoxy)-1H-pyrazole-3-carboxylate (1 equiv.) in toluene. The mixture was heated to reflux for 24 h. The mixture was cooled to 0 °C and quenched with methanol (5.3 equiv.) and a 6 N aqueous solution of HCl (16 equiv.). The mixture was heated to 90 °C for 10 min. The mixture was cooled to 23 °C and the precipitate formed was collected by filtration. The solid was further dried under vacuum to deliver the desired intermediate, 1-(2,4-difluorobenzyl)-5-(difluoromethoxy)-1H-pyrazole-3-carboximidamide hydrochloride (1.2 g, 79% yield) as a light yellow solid.
¹H NMR (500 MHz, DMSO-*d*₆) δ ppm 7.28 - 7.42 (m, 2 H), 7.04 - 7.24 (m, 2 H), 6.98 (br. s., 1 H), 5.40 (s, 2 H).

### Step 4: Synthesis of Compound I-55

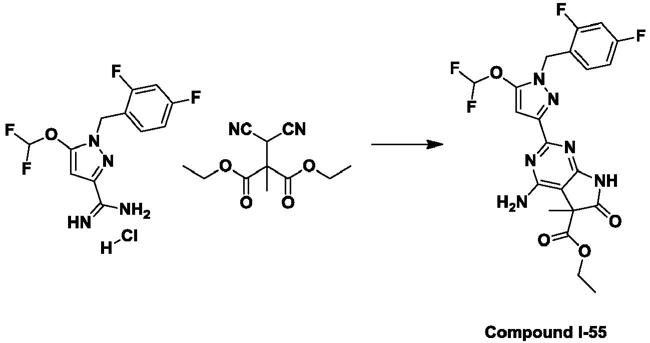

A mixture containing diethyl 2-(dicyanomethyl)-2-methylmalonate (3 equiv.), potassium hydrogencarbonate (3 equiv.), and 1-(2,4-difluorobenzyl)-5-(difluoromethoxy)-1H-pyrazole-3-carboximidamide hydrochloride (1 equiv.) in *tert*-buOH was heated to 85 °C for 3 h. The mixture was cooled to 23 °C and diluted in ethyl acetate. The organic layer was washed with water, dried, filtered and evaporated to give a crude oil. The oil was purified via silica gel chromatography (0 to 10% ethyl acetate in hexanes) to deliver the desired compound (404 mg, 64% yield) as a light yellow solid.
¹H NMR (500 MHz, DMSO-*d*₆) δ ppm 7.22 - 7.54 (m, 3 H), 7.12 (s, 1 H), 6.47 (s, 1 H), 5.31 (s, 2 H), 4.09 (t, 2 H), 1.57 (s, 3 H), 1.10 (t, 3 H).

### Compound I-58

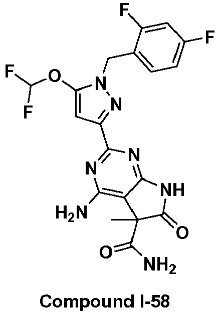

A mixture containing a 7N solution of ammonia in methanol (50 equiv.) in methanol and **Compound I-55** (1 equiv.) was stirred at 23 °C for 24 h. The mixture was concentrated in vacuo, and the resulting residue was treated with a minimal amount of methanol and diethyl ether. The precipitate formed was collected by filtration and dried under vacuum to deliver the desired compound (24 mg, 61% yield) as a white solid.
¹H NMR (500 MHz, DMSO-*d*₆) δ ppm 7.45 (br. s., 1 H), 7.23 - 7.35 (m, 2 H), 7.17 (s, 1 H), 7.12 (t, 1 H), 5.31 (s, 2 H), 1.44 - 1.59 (m, 3 H).

### Compound I-56

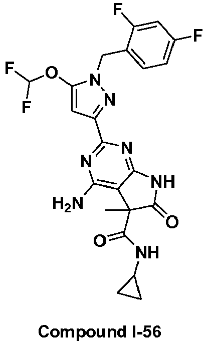

A mixture containing cyclopropylamine (50 equiv.) and **Compound I-55** (1 equiv.) was heated to 60 °C for 18 h. The mixture was concentrated in vacuo, and the resulting residue was purified via silica gel chromatography (0 to 100% ethyl acetate in hexanes) to deliver the desired compound (28 mg, 78% yield) as a tan solid.
¹H NMR (500 MHz, CD₃OD) δ ppm 7.22 - 7.31 (m, 1 H), 6.83 - 7.15 (m, 3 H), 6.62 (s, 1 H), 5.37 (s, 2 H), 2.62 - 2.74 (m, 1 H), 1.67 (s, 3 H), 0.73 (d, 2 H), 0.46 - 0.61 (m, 2 H).

### Compound I-57

The title compound was synthesized in 6 steps:

### Step 1: Synthesis of benzyl 2-(3,3,4,4,4-pentafluorobutyl)hydrazinecarboxylate.

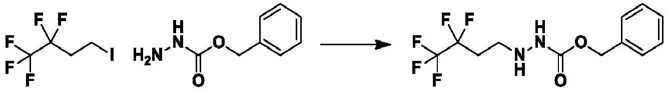

A mixture containing Hunig's base (3 equiv.), 3,3,4,4,4-pentafluoro-1-iodobutane (1.7 equiv.) and carbobenzoxyhydrazide (1 equiv.) in DMF was heated to 80 °C for 24 h. The mixture was diluted in ethyl acetate and washed with water. The organic layer was dried, filtered, and evaporated to give a crude oil which was purified via silica gel chromatography (0 to 100% ethyl acetate in hexanes) to deliver the desired intermediate, benzyl 2-(3,3,4,4,4-pentafluorobutyl)hydrazinecarboxylate (10.8 g, 58% yield) as a white solid.
¹H NMR (500 MHz, CD₃OD) δ ppm 7.28 - 7.41 (m, 5 H), 5.12 (s, 2 H), 2.98 - 3.17 (m, 2 H), 2.19 - 2.39 (m, 2 H).

### Step 2: Synthesis of (3,3,4,4,4-pentafluorobutyl)hydrazine hydrochloride.

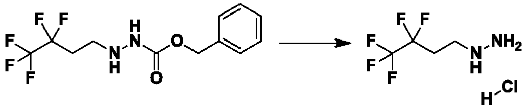

A mixture containing a 1.25M solution of HCl in ethanol (1 equiv.), 10 wt% Pd on carbon (0.1 equiv.), and benzyl 2-(3,3,4,4,4-pentafluorobutyl)hydrazinecarboxylate (1 equiv.) in methanol was subjected to an atmosphere of hydrogen with a balloon at 23 °C for 24 h. The mixture was filtered through an acro disk and the filtrate was concentrated in vacuo to deliver the desired intermediate, (3,3,4,4,4-pentafluorobutyl)hydrazine hydrochloride (7.1 g, 96% yield) as a light yellow oil.
¹H NMR (500 MHz, CD₃OD) δ ppm 3.27 (t, 2 H), 2.43 - 2.56 (m, 2 H).

### Step 3: Synthesis of ethyl 5-oxo-1-(3,3,4,4,4-pentafluorobutyl)-2,5-dihydro-1H-pyrazole-3-carboxylate.

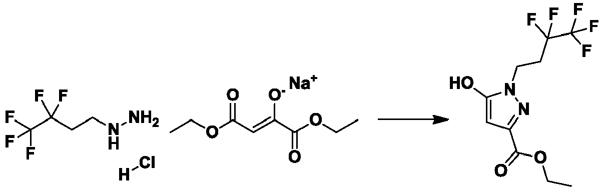

To a prestirred mixture of diethyl oxalacetate sodium salt (1 equiv.) and acetic acid (10 equiv.) in benzene was added (3,3,4,4,4-pentafluorobutyl)hydrazine hydrochloride (1 equiv.). The mixture was heated to 90 °C for 4 h. The solvent was removed in vacuo, and the resulting precipitate was rinsed with a minimal amount of diethyl ether, collected by filtration, and dried under vacuum to deliver the desired intermediate, ethyl 5-oxo-1-(3,3,4,4,4-pentafluorobutyl)-2,5-dihydro-1H-pyrazole-3-carboxylate (2.93 g, 24% yield) as a white solid.
¹H NMR (500 MHz, DMSO-*d*₆) δ ppm 5.78 (s, 1 H), 4.13 - 4.32 (m, 4 H), 2.64 - 2.83 (m, 2 H), 1.19 - 1.34 (m, 3 H).

### Step 4: Synthesis of ethyl 5-oxo-1-(3,3,4,4,4-pentafluorobutyl)-2,5-dihydro-1H-pyrazole-3-carboxylate.

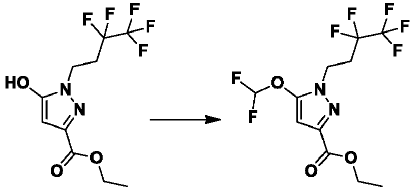

A mixture containing sodium 2-chloro-2,2-difluoroacetate (1,4 equiv.), potassium carbonate (1.1 equiv.), and ethyl 5-oxo-1-(3,3,4,4,4-pentafluorobutyl)-2,5-dihydro-1H-pyrazole-3-carboxylate (1 equiv.) in DMF was heated to 100 °C for 5 h. The mixture was diluted in ethyl acetate and washed with water. The organic layer was dried, filtered and evaporated to give an oil. The oil was purified via silica gel chromatography (0 to 100% ethyl acetate in hexanes) to deliver the desired intermediate, ethyl 5-oxo-1-(3,3,4,4,4-pentafluorobutyl)-2,5-dihydro-1H-pyrazole-3-carboxylate (2.9 g, 8% yield) as a light yellow oil.
¹H NMR (500 MHz, CDCl₃) δ ppm 6.55 - 6.75 (m, 1 H), 6.42 - 6.47 (m, 1 H), 4.34 - 4.48 (m, 4 H), 2.62 - 2.77 (m, 2 H), 1.40 (t, 3 H).

### Step 5: Synthesis of 5-(difluoromethoxy)-1-(3,3,4,4,4-pentafluorobutyl)-1H-pyrazole-3-carboximidamide hydrochloride.

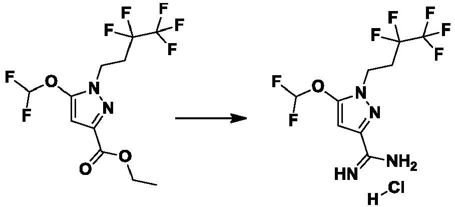

To a suspension of ammonium chloride (5.3 equiv.) in toluene (4.0 ml) at 0 °C, was added a 2.0M solution of trimethylaluminum in heptane. The mixture was heated to 90 °C for 24 h. Then, it was cooled to 0 °C. The mixture was quenched with methanol (5.3 equiv.) followed by a 6N aqueous solution of HCl (16 equiv.). The mixture was stirred at 23 °C for 30 min. The organic layer was removed in vacuo, and the resulting residue was dissolved in a minimal amount of water The precipitate was collected by filtration and dried under vacuum to deliver the desired intermediate, 5-(difluoromethoxy)-1-(3,3,4,4,4-pentafluorobutyl)-1H-pyrazole-3-carboximidamide hydrochloride (47 mg, 16% yield) as a tan solid.
¹H NMR (500 MHz, CD₃OD) δ ppm 6.90 - 7.27 (m, 1 H), 6.75 - 6.83 (m, 1 H), 4.52 (t, 2 H), 2.67 - 3.05 (m, 2 H).

### Step 6: Synthesis of Compound I-57

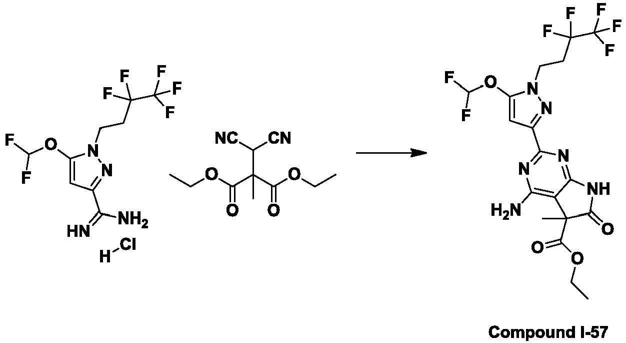

A mixture containing diethyl 2-(dicyanomethyl)-2-methylmalonate (4 equiv.), potassium hydrogencarbonate (4 equiv.), and 5-(difluoromethoxy)-1-(3,3,4,4,4-pentafluorobutyl)-1H-pyrazole-3-carboximidamide hydrochloride (1 equiv.) in *tert*-BuOH was heated to 85 °C for 4 h. The mixture was diluted in ethyl acetate and washed with water. The organic layer was dried, filtered and evaporated to give a dark oil which was purified via silica gel chromatography (0 to 100% ethyl acetate in hexanes) to deliver the desired compound (59 mg, 88% yield) as a light brown solid.
¹H NMR (500 MHz, CD₃OD) δ ppm 6.82 - 7.26 (m, 1 H), 6.51 - 6.67 (m, 1 H), 4.38 - 4.51 (m, 2 H), 4.13 - 4.26 (m, 2 H), 2.72 - 2.95 (m, 2 H), 1.65 - 1.72 (m, 3 H), 1.17 - 1.22 (m, 3 H).

### Compound I-50

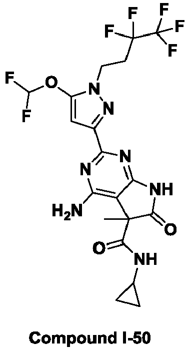

A mixture containing cyclopropylamine (50 equiv.) and **Compound I-57** (1 equiv.) was heated to 60 °C for 24 h. The mixture was concentrated under vacuum to give a crude oil which was purified via silica gel chromatography (0 to 100% ethyl acetate in hexanes) to deliver the desired compound (19 mg, 66% yield) as a light yellow solid.
¹H NMR (500 MHz, CD₃OD) δ ppm 7.02 (s, 1 H), 6.58 (s, 1 H), 4.39 - 4.50 (m, 2 H), 2.76 - 2.89 (m, 2 H), 2.68 (dt, 1 H), 1.68 (s, 3 H), 0.69 - 0.77 (m, 2 H), 0.51 - 0.56 (m, 2 H).

### Compound I-41

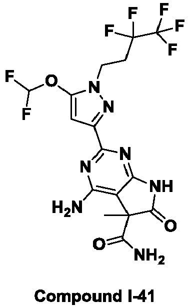

A 7N solution of ammonia in methanol (50 equiv.) and **Compound I-57** (1 equiv.) was stirred at 23 °C for 24 h. The mixture was concentrated in vacuo, and the resulting residue was purified via silica gel chromatography (0 to 100% ethyl acetate in hexanes) to deliver the desired compound (15.4 mg, 56% yield) as a light yellow solid.
¹H NMR (500 MHz, CD₃OD) δ ppm 6.84 - 7.25 (m, 1 H) 6.58 (s, 1 H) 4.45 (t, 2 H) 2.82 (tt, 2 H) 1.71 (s, 3 H).

### Compound I-34

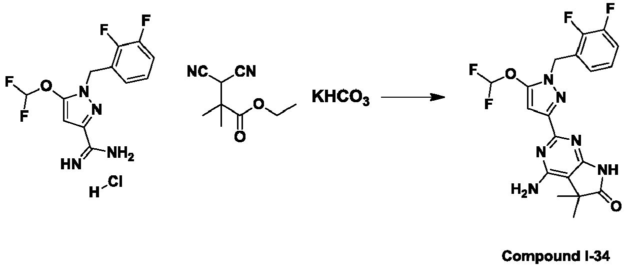

A mixture containing potassium hydrogencarbonate (3 equiv.), 1-(2,3-difluorobenzyl)-5-(difluoromethoxy)-1H-pyrazole-3-carboximidamide hydrochloride (Described in step 3 towards the synthesis of **Compound I-52** (1 equiv.), and methyl 3,3-dicyano-2,2-dimethylpropanoate (3 equiv.) in *tert*-BuOH was heated to 80 °C for 24 h. The mixture was diluted in ethyl acetate and washed with water. The organic layer was dried, filtered and evaporated to give an oil that was purified via silica gel chromatography (0 to 100% ethyl acetate in hexanes) to deliver the desired compound (230 mg, 45% yield) as a tan solid.
¹H NMR (500 MHz, CD₃OD) δ ppm 7.20 - 7.31 (m, 1 H), 6.82 - 7.18 (m, 3 H), 6.62 (s, 1 H), 5.38 - 5.50 (m, 2 H), 1.43 (s, 6 H).

### Compound I-40

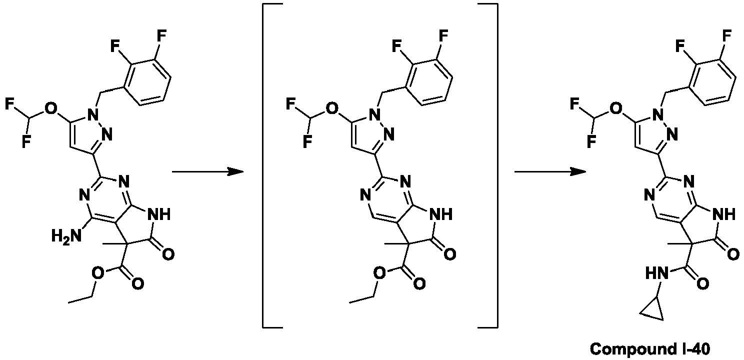

A mixture containing **Compound I-52** (1 equiv.) and isopentyl nitrite (3 equiv.) in THF was heated to 70 °C for 24 h. An additional isopentyl nitrite (3 equiv.) was added to the mixture heated to 70 °C for 2 d. The mixture was concentrated in vacuo, and the resulting yellow residue was purified via silica gel chromatography (0 to 100% ethyl acetate in hexanes) to deliver the desired intermediate, ethyl 2-(1-(2,3-difluorobenzyl)-5-(difluoromethoxy)-1H-pyrazol-3-yl)-5-methyl-6-oxo-6,7-dihydro-5H-pyr rolo[2,3-d]pyrimidine-5-carboxylate as a yellow oil. This intermediate product was combined with cyclopropyl amine (4.5 ml, large excess), and the mixture was heated to 70 °C for 24 h. The mixture was cooled to 23 °C and concentrated in vacuo. The resulting residue was purified via silica gel chromatography (0 to 100% ethyl acetate in hexanes) to deliver the desired compound (36 mg, 5% yield over 2 steps) as a light yellow solid.
¹H NMR (500 MHz, CDCl₃) δ ppm 8.81 - 8.89 (m, 1 H), 7.18 (d, 1 H), 7.09 (q, 1 H), 6.94 - 7.03 (m, 1 H), 6.82 (t, 1 H), 6.38 - 6.77 (m, 1 H), 5.46 (s, 2 H), 2.73 (tq, 1 H), 1.75 (s, 3 H), 0.71 - 0.88 (m, 2 H), 0.47 - 0.61 (m, 2 H).

### Compound 1-21

The title compound was prepared in 4 steps:

### Step 1: Synthesis of ethyl 2-(2-(2-fluorobenzyl)hydrazinyl)-2-iminoacetate

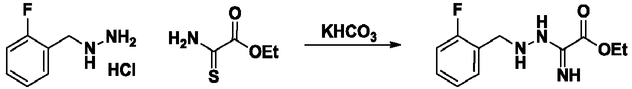

A mixture of (2-fluorobenzyl)hydrazine hydrochloride (1 equiv.), ethyl 2-amino-2-thioxoacetate (1 equiv.), and potassium bicarbonate (1 equiv.) in ethanol was stirred at 23 °C for 15 h. Smell of rotten eggs (H₂S) emanating from the reaction mixture indicated the progress of the reaction. The mixture was then diluted with ethyl acetate and saturated aqueous sodium bicarbonate solution and the phases were separated. The aqueous phase was extracted twice with ethyl acetate. The combined organic phase was washed with brine, dried over anhydrous magnesium sulfate, filtered and concentrated to give an oil. The crude oil was carried forward to the next step without any purification.

### Step 2: Synthesis of ethyl 1-(2-fluorobenzyl)-5-oxo-4,5-dihydro-1H-1,2,4--triazole-3-carboxylate

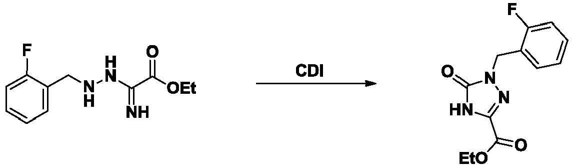

To a solution of ethyl 2-(2-(2-fluorobenzyl)hydrazinyl)-2-iminoacetate (1 equiv.) in dichloromethane at 0 °C was added CDI (2 equiv.) in small portions. The reaction mixture was stirred at 23 °C for 15 h. The mixture was then quenched with saturated aqueous ammonium chloride solution and the phases were separated. The aqueous phase was extracted twice with dichloromethane. The combined organic phase was dried over anhydrous magnesium sulfate, filtered, and concentrated. The residue was purified via silica gel chromatography (20 to 80% ethyl acetate in hexanes) to deliver the desired intermediate, ethyl 1-(2-fluorobenzyl)-5-oxo-4,5-dihydro-1H-1,2,4-triazole-3-carboxylate (9.32 g, 47% yield) as a white solid.
¹H NMR (500 MHz, DMSO-*d*₆) δ ppm 12.69 (br. s., 1 H), 7.33 - 7.45 (m, 1 H), 7.29 (td, 1 H), 7.16 - 7.25 (m, 2 H), 4.99 (s, 2 H), 4.29 (q, 2 H), 1.26 (t, 3 H).

### Step 3: Synthesis of 1-(2-fluorobenzyl)-5-oxo-4,5-dihydro-1H-1,2,4-triazole-3-carboximidamide

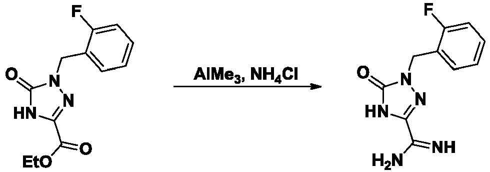

A 2M solution of AlMe₃ (5.5 equiv.) in toluene was added over 10 min to an ice cold suspension of dry NH₄Cl (5.5 equiv.) in toluene, and the mixture was stirred for 1h at 23 C. To it was added ethyl 1-(2-fluorobenzyl)-5-oxo-4,5-dihydro-1H-1,2,4-triazole-3-carboxylate (1 equiv.) and the reaction mixture was stirred at 80 °C overnight. Reaction mixture was then cooled to 0 °C and carefully treated with methanol and stirred for 30 min at 23 °C. The solid was filtered off and washed with methanol. The filterate was concentrated in vacuo to deliver the desired intermediate, 1-(2-fluorobenzyl)-5-oxo-4,5-dihydro-1H-1,2,4-triazole-3-carboximidamide which was directly used in the next step.

### Step 4: Synthesis of Compound I-21

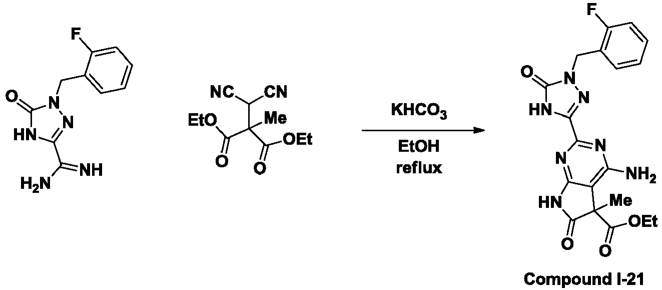

A mixture of 1-(2-fluorobenzyl)-5-oxo-4,5-dihydro-1H-1,2,4-triazole-3-carboximidamide (1 equiv.), diethyl 2-(dicyanomethyl)-2-methylmalonate (1.15 equiv.), and potassium bicarbonate (3 equiv.) in ethanol was heated to reflux overnight. The resultant solution was then cooled to 23 °C, diluted with water and ethyl acetate. Phases were separated and the aqueous layer was extracted twice with ethyl acetate. The combined organic phase was dried over anhydrous sodium sulfate, filtered and concentrated. The crude material was purified via reverse phase HPLC (30 % to 60 % acetonitrile in water with 0.1 % TFA) to deliver the desired compound (1.5mg, 0.1% yield) as a thin film.

### Compound I-10

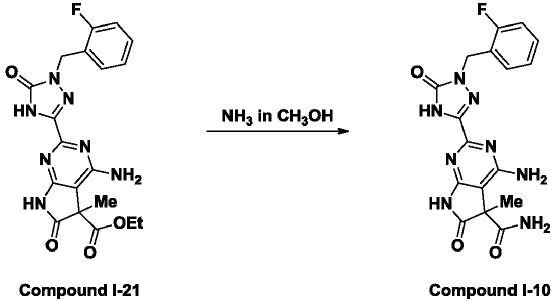

Ammonia (7.0M in MeOH) (200 equiv.) was added to Compound 1-21 (1 equiv.), and the reaction mixture was heated at 50 °C for 5 h. The resultant solution was then concentrated *in vacuo* to obtain the desired compound (3.6 mg, 26% yield) as a white solid.

### Compound I-30

The title compound was prepared in 3 steps:

### Step 1: Synthesis of ethyl 1-(2-fluorobenzyl)-5-methoxy-1H-1,2,4-triazole-3-carboxylate

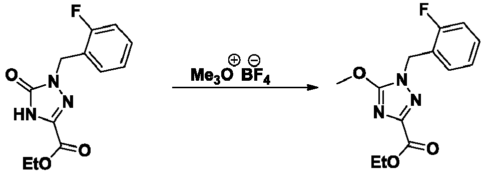

A mixture of ethyl 1-(2-fluorobenzyl)-5-oxo-4,5-dihydro-1H-1,2,4-triazole-3-carboxylate (Described in step 2 towards the synthesis of **Compound I-21** (1 equiv.), trimethyloxonium tetrafluoroborate (2 equiv.) in dichloromethane was stirred at 23 °C for 24 h. The solution was then diluted with water and dichloromethane. Phases were separated and the aqueous phase was extracted with dichloromethane (2x). The combined organic phase was dried over anhydrous magnesium sulfate, filtered, and concentrated. The residue was purified via silica gel chromatography (30 to 100 % ethyl acetate in hexane) to deliver the desired intermediate, ethyl 1-(2-fluorobenzyl)-5-methoxy-1H-1,2,4-triazole-3-carboxylate (0.23 g, 22% yield) as a colorless oil.
¹H NMR (500 MHz, DMSO-*d*₆) δ ppm 7.37 - 7.44 (m, 1 H), 7.26 - 7.32 (m, 1 H), 7.18 - 7.26 (m, 2 H), 5.25 (s, 2 H), 4.27 (q, 2 H), 4.10 (s, 3 H), 1.26 (t, 3 H).

### Step 2: Synthesis of 1-(2-fluorobenzyl)-5-methoxy-1H-1,2,4-triazole-3-carboximidamide

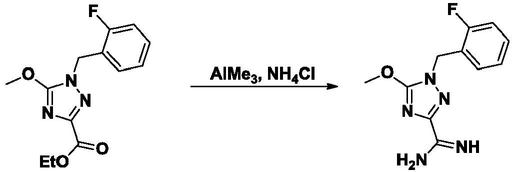

A 2M solution of AlMe₃ (5.5 equiv.) in toluene was added over 10 min to an ice cold suspension of dry NH₄Cl (5.5 equiv.) in toluene, and the mixture was stirred for 1 h at 23 C. To it was added a solution of ethyl 1-(2-fluorobenzyl)-5-methoxy-1H-1,2,4-triazole-3-carboxylate (1 equiv.) in toluene and the reaction mixture was stirred at 80 °C overnight. The teaction mixture was cooled to 0 °C, carefully treated with methanol, and stirred for 30 min at 23 °C. The solid was filtered off and washed with methanol. The filtrate was concentrated in vacuo to deliver the desired intermediate, 1-(2-fluorobenzyl)-5-methoxy-1H-1,2,4-triazole-3-carboximidamide which was directly used in the next step.

### Step 3: Synthesis of Compound I-30

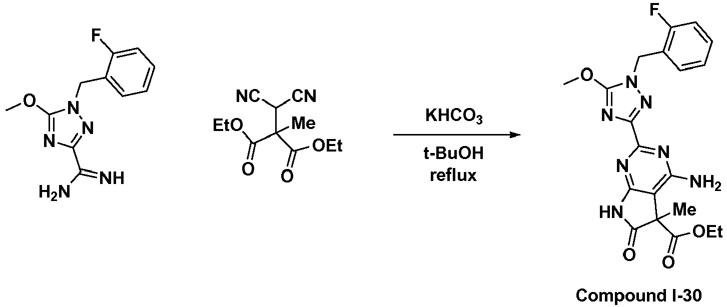

A mixture of 1-(2-fluorobenzyl)-5-methoxy-1H-1,2,4-triazole-3-carboximidamide (1 equiv.), diethyl 2-(dicyanomethyl)-2-methylmalonate (1.15 equiv.), and potassium bicarbonate (3 equiv.) in *t*-BuOH was heated to reflux for 4 h. After cooling, the reaction mixture was added with water and stirred for 30 min. The precipitate was filtered, washed with a minimum amount of water and diethyl ether, and dried overnight under high vacuum to deliver the desired compound (194 mg, 72% yield) as a white solid.
1H NMR (500 MHz, DMSO-d₆) δ ppm 11.35 (s, 1 H), 7.36 - 7.43 (m, 1 H), 7.26 - 7.32 (m, 1 H), 7.18 - 7.26 (m, 2 H), 6.75 (br. s., 2 H), 5.22 (s, 2 H), 4.02 - 4.15 (m, 5 H), 1.57 (s, 3 H), 1.09 (t, 3 H).

### Compound I-17

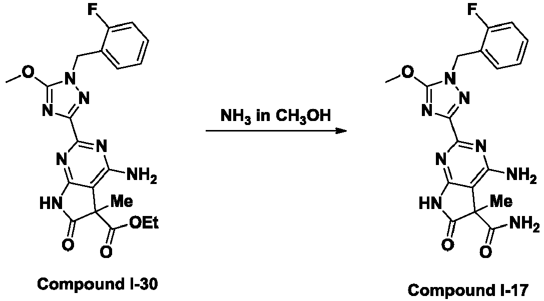

Ammonia (7.0M in MeOH) (200 equiv.) was added to **Compound I-30** (1 equiv.), and the reaction mixture was heated at 50 °C for 4 h. The resulting solution was concentrated in vacuo to deliver the desired compound (88 mg, 98% yield) as a white solid.
¹H NMR (500 MHz, DMSO-*d*₆) δ ppm 11.26 (s, 1 H), 7.46 (s, 1 H), 7.34 - 7.43 (m, 1 H), 7.14 - 7.32 (m, 4 H), 6.79 (br. s., 2 H), 5.22 (s, 2 H), 4.09 (s, 3 H),1.53 (s, 3 H).

### Compound I-37

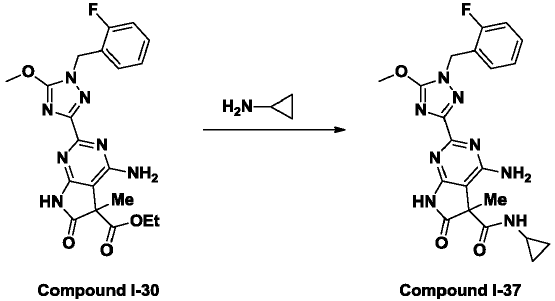

Cyclopropyl amine (150 equiv.) was added to **Compound I-30** (1 equiv.), and the reaction mixture was heated at 50 °C for 3 h. The resulting solution was concentrated in vacuo to deliver the desired compound (78 mg, 83% yield) as a yellow solid.
¹H NMR (500 MHz, DMSO-*d*₆) δ ppm 11.27 (s, 1 H), 7.60 (d, 1 H), 7.35 - 7.44 (m, 1 H), 7.16 - 7.32 (m, 3 H), 6.78 (br. s., 2 H), 5.23 (s, 2 H), 4.10 (s, 3 H), 2.58 - 2.67 (m, 1 H), 1.52 (s, 3 H), 0.55 - 0.62 (m, 2 H), 0.41 - 0.48 (m, 2 H).

### Compound I-29

The title compound was prepared in 3 steps:

### Step 1: ethyl 1-(2-fluorobenzyl)-4-methyl-5-oxo-4,5-dihydro-1H-1,2,4-triazole-3-carboxylate

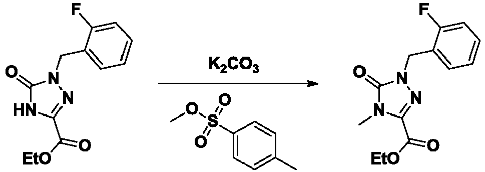

A mixture of ethyl 1-(2-fluorobenzyl)-5-oxo-4,5-dihydro-1H-1,2,4-triazole-3-carboxylate (1 equiv.), methyl 4-methylbenzenesulfonate (1.1 equiv.), and potassium carbonate (2 equiv.) in DMF was stirred at 23 °C for 24 h. The solution was then diluted with water and ethyl acetate. Phases were separated and the aqueous phase was extracted with ethyl acetate (2x). The combined organic phase was dried over anhydrous magnesium sulfate, filtered, and concentrated. The residue was purified via silica gel chromatography (20 to 50 % ethyl acetate in hexane) to deliver the desired intermediate, ethyl 1-(2-fluorobenzyl)-4-methyl-5-oxo-4,5-dihydro-1H-1,2,4-triazole-3-carboxylate (0.32 g, 61% yield) as a colorless oil.
¹H NMR (500 MHz, CDCl₃) δ ppm 7.27 - 7.33 (m, 2 H), 7.03 - 7.15 (m, 2 H), 5.15 - 5.18 (m, 2 H), 4.43 (q, 2 H), 3.58 (s, 3 H), 1.42 (t, 3 H).

### Step 2: Synthesis of 1-(2-fluorobenzyl)-4-methyl-5-oxo-4,5-dihydro-1H-1,2,4-triazole-3-carboximidamide

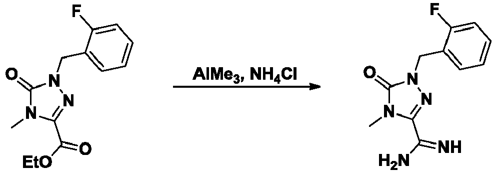

A 2M solution of AlMe₃ (5.5 equiv.) in toluene was added over 10 min to an ice cold suspension of dry NH₄Cl (5.5 equiv.) in toluene, and the mixture was stirred for 1 h at 23 °C. A solution of ethyl 1-(2-fluorobenzyl)-4-methyl-5-oxo-4,5-dihydro-1H-1,2,4-triazole-3-carboxylate (1 equiv.) in toluene was added to the reaction mixture and the contents were stirred at 80 °C overnight. Reaction mixture was then cooled to 0 °C, carefully treated with methanol, and stirred for 30 min at 23 C. The solid was filtered off and washed with methanol. The filtrate was concentrated in vacuo to deliver the desired intermediate, 1-(2-fluorobenzyl)-4-methyl-5-oxo-4,5-dihydro-1H-1,2,4-triazole-3-carboximidamide which was directly used in the next step.

### Step 3: Synthesis of Compound I-29

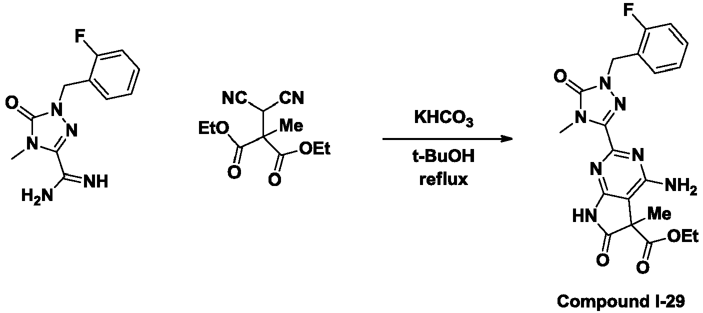

A mixture of 1-(2-fluorobenzyl)-4-methyl-5-oxo-4,5-dihydro-1H-1,2,4-triazole-3-carboximidamide (1 equiv.), diethyl 2-(dicyanomethyl)-2-methylmalonate (1.15 equiv.), and potassium bicarbonate (3 equiv.) in *t*-BuOH was heated to reflux for 4 h. After cooling, the reaction mixture was diluted with water and ethyl acetate. Phases were separated and the aqueous layer was extracted with ethyl acetate (2x). The combined organic phase was dried over anhydrous magnesium sulfate, filtered and concentrated. The residue was purified via silica gel chromatography (50 to 100 % ethyl acetate in hexane) to deliver the desired compound (123 mg, 28% yield) as a white solid.
1H NMR (500 MHz, DMSO-*d*₆) δ ppm 11.42 (s, 1 H), 7.33 - 7.43 (m, 1 H), 7.26 - 7.33 (m, 1 H), 7.15 - 7.25 (m, 2 H), 6.90 (br. s., 2 H), 5.04 (s, 2 H), 4.04 - 4.15 (m, 2 H), 3.57 (s, 3 H), 1.58 (s, 3 H), 1.10 (t, 3 H).

### Compound I-18

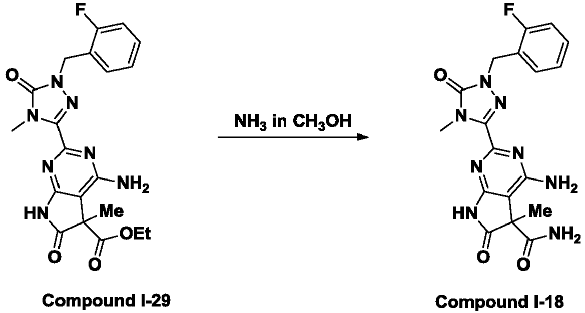

Ammonia (7.0M in MeOH) (100 equiv.) was added to **Compound I-29** (1 equiv.), and the reaction mixture was heated at 50 °C for 4 h. The resulting solution was concentrated in vacuo to deliver the desired compound, **Compound I-18** (44 mg, 93 %) as a white solid.
¹H NMR (500 MHz, DMSO-*d*₆) δ ppm 11.30 (s, 1 H), 7.43 (s, 1 H), 7.34 - 7.40 (m, 1 H), 7.26 - 7.33 (m, 1 H), 7.13 - 7.26 (m, 3 H), 6.90 (br. s., 2 H), 5.04 (s, 2 H), 3.57 (s, 3 H), 1.53 (s, 3 H).

### Compound I-38

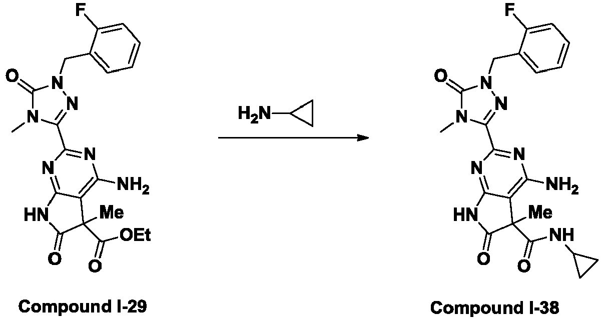

Cyclopropyl amine (150 equiv.) was added to **Compound I-29** (1 equiv.), and the reaction mixture was heated at 50 °C for 5 h. The resulting solution was concentrated in vacuo to deliver the desired compound (46 mg, 88 %) as a white solid.
¹H NMR (500 MHz, DMSO-*d*₆) δ ppm 11.26 (s, 1 H), 7.59 (d, 1 H), 7.33 - 7.42 (m, 1 H), 7.26 - 7.32 (m, 1 H), 7.16 - 7.25 (m, 2 H), 6.84 (br. s., 2 H), 5.04 (s, 2 H), 3.57 (s, 3 H), 2.58 - 2.66 (m, 1 H), 1.52 (s, 3 H), 0.53 - 0.62 (m, 2 H), 0.39 - 0.49 (m, 2 H).

### Compound I-3

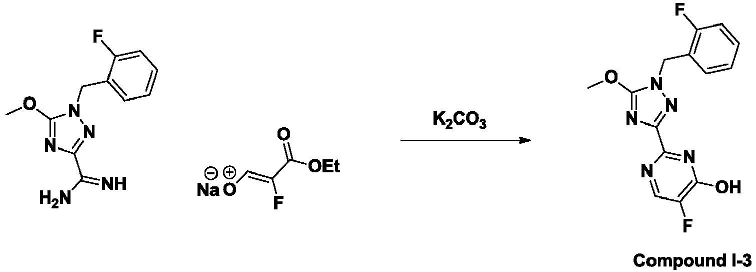

A mixture of 1-(2-fluorobenzyl)-5-methoxy-1H-1,2,4-triazole-3-carboximidamide (1 equiv.) (Described in step 2 towards the synthesis of **Compound I-30,** diethyl sodium (Z)-3-ethoxy-2-fluoro-3-oxoprop-1-en-1-olate (3 equiv.), and potassium carbonate (3 equiv.), in *t*-BuOH was heated to reflux for 15 h. After cooling, the volatiles were removed in vacuo and the residue was diluted with ethyl acetate and water. Phases were separated and the aqueous layer was extraced with dichloromethane (3x). The combined organic phase was dried over anhydrous magnesium sulfate, filtered, and concentrated to deliver the desired compound (216 mg, 15% yield) as a yellow solid.
¹H NMR (500 MHz, DMSO-*d*₆) δ ppm 13.11 (br. s., 1 H), 8.10 (br. s., 1 H), 7.31 - 7.45 (m, 2 H), 7.19 - 7.27 (m, 2 H), 5.26 (s, 2 H), 4.14 (s, 3 H).

### Compound I-70

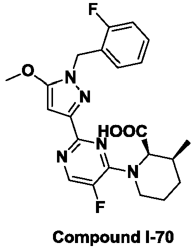

The title compound was prepared following general procedure A in library format, except (2R,3S)-3-methylpiperidine-2-carboxylic acid (10 equiv.) was the amine reactant, 20 equivalents of triethylamine was used, and contents were stirred as a solution in THF : water (2:1) at 100 °C for 18 h. The resulting residue after workup was purified via reverse phase HPLC to deliver the desired compound (2.5 mg, 10% yield) as a white solid.
¹H NMR (500 MHz, CD₃OD) δ ppm 8.16 (d, 1 H), 7.29 (m, 1 H), 7.10 (m, 2 H), 7.01 (t, 1 H), 6.38 (s, 1 H), 5.30 (s, 2 H), 5.03 (d, 1 H), 4.34 (d, 1 H), 3.97 (s, 3 H), 3.67 (m, 1 H), 2.08 (d, 1 H), 1.88 (m, 1 H), 1.73 (m, 2 H), 1.51 (dd, 1 H), 1.19 (d, 3 H)

### Compound I-69

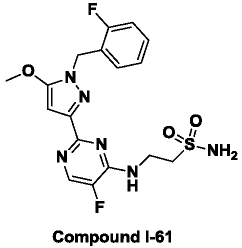

The title compound was prepared following general procedure A in library format, except 2-aminoethanesulfonamide (2 equiv.) was the amine reactant, and the contents were stirred as a solution in dioxane. Contents were concentrated under a stream of nitrogen, and the resulting residue was purified via reverse phase HPLC to deliver the desired compound (4.3 mg, 23% yield) as a white solid.
¹H NMR (500 MHz, CD₃OD) δ 8.25 (m, 1 H), 7.35 (m, 1 H), 7.15 (m, 3 H), 6.57 (m, 1 H), 5.39 (s, 2 H), 4.19 (m, 2 H), 4.05 (s, 3 H), 3.51 (m, 2 H).

### Compound I-68

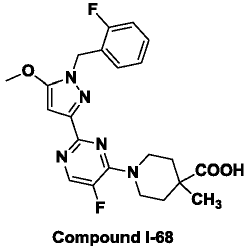

The title compound was prepared following general procedure A in library format, except 4-methylpiperidine-4-carboxylic acid (2 equiv.) was the amine reactant, and contents were stirred as a solution in dioxane. Contents were concentrated under a stream of nitrogen, and the resulting residue was purified via reverse phase HPLC to deliver the desired compound (2.1 mg, 11% yield) as a white solid.
¹H NMR (500 MHz, CD₃OD) δ 8.25 (m, 1 H), 7.36 (m, 1 H), 7.16 (s, 3 H), 6.51 (s, 1 H), 5.39 (s, 2 H), 4.66 (m, 2 H), 4.05 (s, 3 H), 3.62 (m, 2 H), 2.34 (m, 2 H), 1.69 (m, 2 H), 1.32 (s, 3 H).

### Compound I-67

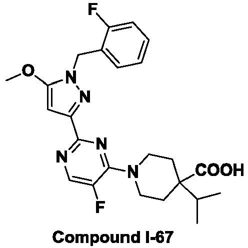

The title compound was prepared following general procedure A in library format, except 4-isopropylpiperidine-4-carboxylic acid (2 equiv.) was the amine reactant, and contents were stirred as a solution in dioxane. Contents were concentrated under a stream of nitrogen, and the resulting residue was purified via reverse phase HPLC to deliver the desired compound (6.3 mg, 30% yield) as a white solid.
¹H NMR (500 MHz, CD₃OD) δ 8.19 (m, 1 H), 7.35 (m, 1 H), 7.14 (m, 3 H), 6.47 (m, 1 H), 5.37 (m, 2 H), 4.85 (m, 2 H), 4.04 (s, 3 H), 3.29 (m, 2 H), 2.34 (m, 2 H), 1.82 (m, 1 H), 1.65 (m, 2 H), 0.98 (d, 6 H).

### Compound I-66

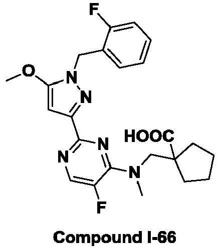

The title compound was prepared following general procedure A in library format, except 1-((methylamino)methyl)cyclopentanecarboxylic acid (2 equiv.) was the amine reactant, and contents were stirred as a solution in dixoane. Contents were concentrated under a stream of nitrogen, and the resulting residue was purified via reverse phase HPLC to deliver the desired compound (4.2 mg, 21% yield) as a white solid.
¹H NMR (500 MHz, CD₃OD) δ 8.27 (m, 1 H), 7.36 (m, 1 H), 7.15 (m, 3 H), 6.46 (m, 1 H), 5.38 (m, 2 H), 4.32 (m, 2 H), 4.05 (s, 3 H), 3.55 (m, 3 H), 2.27 (m, 2 H), 1.72 (m, 6 H).

### Compound I-65

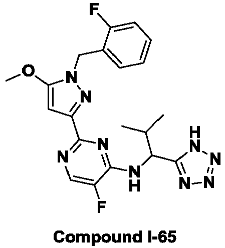

The title compound was prepared following general procedure A in library format, except 2-methyl-1-(1H-tetrazol-5-yl)propan-1-amine was the amine reactant, and contents were stirred as a solution in dioxane. Contents were concentrated under a stream of nitrogen, and the resulting residue was purified via reverse phase HPLC to deliver the desired compound (6.5 mg, 33% yield) as a white solid.
¹H NMR (500 MHz, CD₃OD) δ 8.27 (d, 1 H), 7.33 (d, 1 H), 7.11 (m, 3 H), 6.37 (s, 1 H), 5.61 (d, 1 H), 5.37 (m, 2 H), 4.04 (s, 3 H), 2.61 (m, 1 H), 1.16 (d, 3 H), 0.96 (d, 3 H).

### Compound I-64

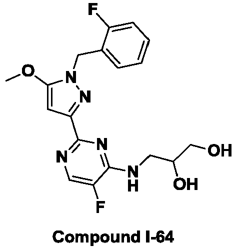

The title compound was prepared following general procedure A in library format, except 3-aminopropane-1,2-diol (4 equiv.) was the amine reactant, and contents were stirred as a solution in dixoane. Contents were concentrated in vacuo, and the resulting residue was purified via reverse phase HPLC to deliver the desired compound (6.5 mg, 37% yield) as a white solid.
¹H NMR (500 MHz, CD₃OD) δ 8.23 (m, 1 H), 7.35 (m, 1 H), 7.16 (m, 3 H), 6.55 (m, 1 H), 5.40 (m, 2 H), 4.06 (s, 3 H), 3.88 (m, 3 H), 3.64 (m, 1 H), 2.68 (s, 1 H).

### Compound I-63

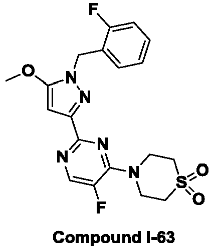

The title compound was prepared following general procedure A in library format, except thiomorpholine 1,1-dioxide was the amine reactant, contents were stirred as a solution in dioxane at 85 °C, and ethyl acetate was used during workup. The resulting residue was purified via reverse phase HPLC to deliver the desired compound (6.8 mg, 35% yield) as a white solid.
¹H NMR (500 MHz, CD₃OD) δ 8.44 (m, 1 H), 7.35 (m, 1 H), 7.10 (m, 3 H), 6.51 (m, 1 H), 5.38 (m, 2 H), 4.53 (m, 4 H), 4.05 (s, 3 H), 3.40 (m, 4 H).

### Compound I-62

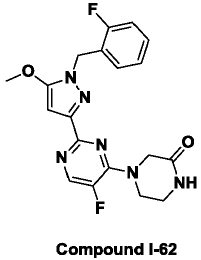

The title compound was prepared following general procedure A in library format, except piperazin-2-one was the amine reactant, and contents were stirred as a solution in dioxane at 85 °C.

Contents were concentrated under a stream of nitrogen, and the resulting residue was purified via reverse phase HPLC to deliver the desired compound (7.4 mg, 42% yield) as a white solid.
¹H NMR (500 MHz, CD₃OD) δ 8.34 (m, 1 H), 7.36 (m, 1 H), 7.14 (m, 3 H), 6.53 (m, 1 H), 5.39 (s, 2 H), 4.69 (s, 2 H), 4.32 (m, 2 H), 4.06 (s, 3 H), 3.56 (m, 2 H).

### Compound I-61

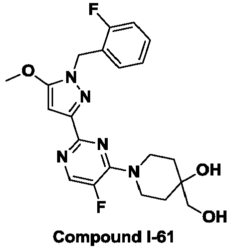

The title compound was prepared following general procedure A in library format, except 4-(hydroxymethyl)piperidin-4-ol was the amine reactant, and contents were stirred as a solution in dioxane at 85 °C. Contents were concentrated under a stream of nitrogen, and the resulting residue was purified via reverse phase HPLC to deliver the desired compound (8.6 mg, 45% yield) as a white solid.
¹H NMR (500 MHz, CD₃OD) δ 8.27 (m, 1 H), 7.35 (m, 1 H), 7.15 (m, 3 H), 6.52 (m, 1 H), 5.40 (m, 2 H), 4.76 (m, 2 H), 4.33 (m, 1H), 4.05 (s, 3 H), 3.72 (m, 2 H), 3.44 (m, 1 H), 1.83 (m, 4 H).

### Compound I-60

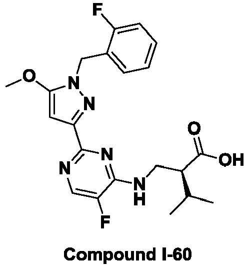

The title compound was prepared following general procedure A in library format, except (S)-3-amino-4-methylpentanoic acid hydrochloride was the amine reactant, 3 equivalents of triethylamine was used, and contents were stirred at 110 °C. The resulting residue after workup was purified via reverse phase HPLC to deliver the desired compound as a solid (11.8 mg, 43.7 % yield).
¹H NMR (500 MHz, CDCl₃) δ ppm 8.32 (br. s., 1 H), 8.22 (br. s., 1 H), 7.11 - 7.13 (m, 2 H), 6.99 (t, 1 H), 6.94 (t, 1 H), 6.28 (s, 1 H), 5.18 (s, 2 H), 3.89 (s, 4 H), 3.81 (d, 1 H), 2.58 (br. s., 1 H), 1.95 - 2.04 (m, 1 H), 0.93 - 0.98 (m, 6 H).

### Compound I-59

### Step 1: Synthesis of 2-(1-(2,3-difluorobenzyl)-5-(difluoromethoxy)-1H-pyrazol-3-yl)-5-fluoropyrimidine-4,6-diol

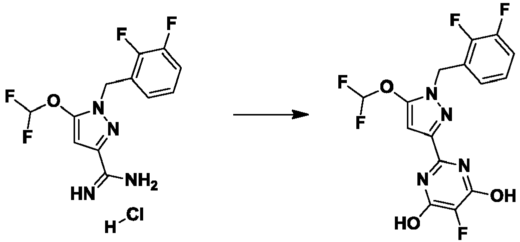

To a mixture containing diethyl 2-fluoromalonate (2 equiv.) and 1-(2,3-difluorobenzyl)-5-(difluoromethoxy)-1H-pyrazole-3-carboximidamide hydrochloride (Described in step 3 towards the synthesis of **Compound I-52** (1 equiv.) in methanol was added a 25 wt% solution of sodium methoxide in methanol (5 equiv.). The mixture was stirred at 23 °C for 1 h. To this mixture, was added HCl (5 equiv.). The mixture was stirred vigorously for 15 min. The precipitate formed was collected by filtration and dried under vacuum to deliver the desired intermediate, 2-(1-(2,3-difluorobenzyl)-5-(difluoromethoxy)-1H-pyrazol-3-yl)-5-fluoropyrimidine-4,6-diol (475 mg, 44 % yield) as a cream colored solid.
¹H NMR (500 MHz, DMSO-*d*₆) δ ppm 7.19 - 7.57 (m, 3 H), 7.11 - 7.16 (m, 1 H), 6.71 (s, 1 H), 5.41 (s, 2 H).

### Step 2: Synthesis of 4,6-dichloro-2-(1-(2,3-difluorobenzyl)-5-(difluoromethoxy)-1H-pyrazol-3-yl)-5-fluoropyrimidine

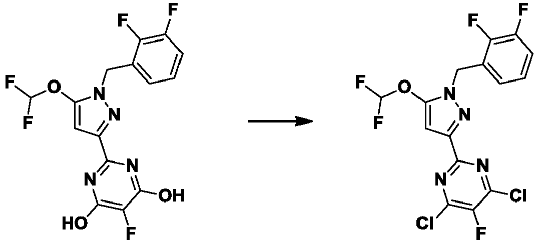

A mixture containig *N,N*-dimethylaniline (1.7 equiv.), POCl₃ (10 equiv.) and 2-(1-(2,3-difluorobenzyl)-5-(difluoromethoxy)-1H-pyrazol-3-yl)-5-fluoropyrimidine-4,6-diol (1 equiv.) in acetonitrile was heated to 60 °C for 24 h. The mixture was concentrated under vacuum, diluted with ethyl acetate, and washed with water. The organic layer was dried, filtered, and evaporated to give an oil, which was purified via silica gel chromatography (0-10% ethyl acetate in hexanes) to deliver the desired intermediate 4,6-dichloro-2-(1-(2,3-difluorobenzyl)-5-(difluoromethoxy)-1H-pyrazol-3-yl)-5-fluoropyrimidine (239 mg, 46 % yield) as a light yellow solid.
¹H NMR (500 MHz, CDCl₃) δ ppm 7.13 (q, 1 H), 7.01 - 7.07 (m, 1 H), 6.89 (t, 1 H), 6.72 (s, 1 H), 6.37 - 6.71 (m, 1 H), 5.47 (s, 2 H).

### Step 3: Synthesis of Compound I-59

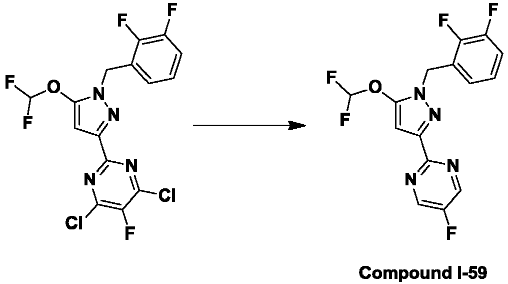

A mixture containing triethylamine (1.1 equiv.), 10 wt% palladium on carbon (0.1 equiv.) and 4,6-dichloro-2-(1-(2,3-difluorobenzyl)-5-(difluoromethoxy)-1H-pyrazol-3-yl)-5-fluoropyrimidine (1 equiv.) in ethyl acetate was placed under hydrogen (80 psi) and 50 °C for 4 h. The mixture was cooled to 23 °C and passed through an acro disk filter to remove inorganic materials. The filtrate was concentrated under vacuum to give an oil, which was purified via silica gel chromatography (0-40% ethyl acetate in hexanes) to deliver the desired compound (10 mg, 24 % yield) as a white solid.
¹H NMR (500 MHz, CDCl₃) δ ppm 8.67 (s, 2 H), 7.11 (q, 1 H), 6.97 - 7.06 (m, 1 H), 6.81 - 6.96 (m, 1 H), 6.38 - 6.73 (m, 2 H), 5.43 - 5.51 (m, 2 H).

### Compound I-72

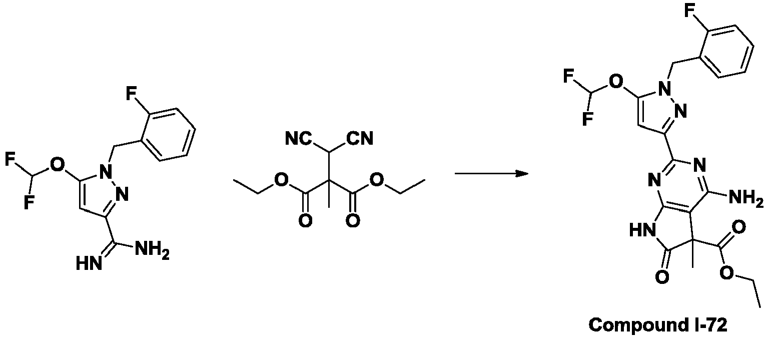

A mixture of 5-(difluoromethoxy)-1-(2-fluorobenzyl)-1H-pyrazole-3-carboximidamide (Described in step 2 towards the synthesis of **Compound I-4,** 1 equiv.), potassium hydrogencarbonate (2 equiv.), and diethyl 2-(dicyanomethyl)-2-methylmalonate (2 equiv.) in tert-butanol was heated in a sealed vial at 80 °C for 2 h. The mixture was cooled to 23 °C and diluted in ethyl acteate. The organic layer was washed with water, dried, filtered and concentrated in vacuo to give a crude oil. The oil was purified via silica gel chromatography utilizing a 0 to 50% ethyl acetate in hexanes gradient to deliver the desired compound (70 mg, 17% yield) as a white solid.
¹H NMR (500 MHz, CD₃OD) δ ppm 7.34 (dd, 1 H), 7.12 - 7.19 (m, 3 H), 6.82 - 7.12 (m, 1 H), 6.64 (s, 1 H), 5.42 (s, 2 H), 4.21 (q, 2 H), 1.69 (s, 3 H), 1.21 (t, 3 H).

### Compound I-71

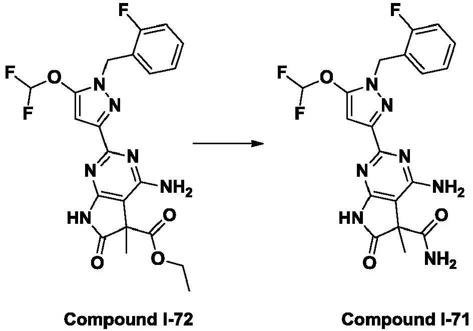

A mixture of **Compound I-72** (1 equiv.) and a 7N solution of ammonia in methanol (100 equiv.) in methanol was heated to 50 °C for 3 h. The mixture was concentrated in vacuo to give a white solid. The solid was rinsed with a minimal amount of diethyl ether, collected by filtration, and dried under high vacuum to deliver the desired compound (105 mg, 62% yield) as a white solid.
¹H NMR (500 MHz, DMSO-*d*₆) δ ppm 7.35 - 7.41 (m, 1 H), 7.12 - 7.27 (m, 4 H), 6.47 (s, 1 H), 5.33 (s, 2 H), 1.53 (s, 3 H).

### Example 2: Biological activity measurement by the sGC-HEK-cGMP assay, with LC/MS detection

Human embryonic kidney cells (HEK293), endogenously expressing soluble guanylate cyclase (sGC), were used to evaluate the activity of test compounds. Compounds stimulating the sGC enzyme should cause an increase in the intracellular concentration of cGMP. HEK 293 cells were seeded in Dulbecco's Modification of Eagle's Medium supplemented with fetal bovine serum (10 % final) and penicillin (100U/mL) / streptomycin (100µg/mL) in a 50µE volume at a density of 1.5x10⁴ cells/well in a poly-D-lysine coated 384 well flat bottom plate. Cells were incubated overnight at 37°C in a humidified chamber with 5% CO₂. Medium was aspirated and cells were washed with 1x Hank's Buffered Saline Salt Solution (50µL). Cells were then incubated for 15 minutes at 37°C with 50µE of a 0.5mM 3-isobutyl-1-methylxanthine (IBMX) solution. Test article and Diethylenetriamine NONOate (DETA-NONOate) solutions (x µM concentration for test article solution and 10 µM concentration for DETA-NONOate solution; wherein x is one of the following concentrations);
30000 nM
7500 nM
1875 nM
468.75 nM
117.19 nM
29.29 nM
7.32 nM
1.83 nM
0.46 nM
0.114 nM
0.029 nM
were then added to the assay mixture and the resulting mixture incubated at 37°C for 20 minutes. After the 20 minute incubation, the assay mixture was aspirated and 10% acetic acid containing 150ng/mL + 3-cGMP (internal standard for LCMS) (50µL) was added to the cells. The plate was incubated at 4°C for 30 minutes in the acetic acid solution to stop the reaction and lyse the cells. The plates were then centrifuged at 1,000g for 3 minutes at 4°C and the supernatant transferred to a clean reaction plate for LCMS analysis.

cGMP concentrations were determined from each sample using the LCMS conditions below (**Table 2**) and calculated standard curve. The standard curve was prepared in 10% acetic acid with 150ng/mL +3cGMP (isotopically labelled cGMP with a weight 3 units higher than wild type) with the following final concentrations of cGMP in ng/mL: 1, 5, 10, 50, 100, 250, 500, 1000, 2000.

**Table 2: LC/MS conditions, Example 2**

| | | | | | | |
|---|---|---|---|---|---|---|
| **MS:** | Thermo Vantage | | | | | |
| **Ion Mode:** | ESI⁺ | | | | | |
| **Scan Type:** | MRM | | | | | |

| **Compound:** | **Transition** | **Dwell Time (msec)** | **Collision Energy (V)** | **S Lens** | **Retention Time (min)** | |
|---|---|---|---|---|---|---|
| cGMP | 346 > 152 | 100 | 32 | 75 | 0.6 | |
| (+3) cGMP IS | 349 > 155 | 100 | 32 | 75 | 0.6 | |
| **HPLC:** | Waters Acquity UPLC | | | | | |
| **Column:** | Thermo Hypersil Gold 2.1 x 50 mm 1.9 micron particle size | | | | | |
| **Flow Rate:** | 750 uL/min | | | | | |
| **Column Temperature:** | RT | | | | | |
| **Autosampler Temperature:** | 6 °C | | | | | |
| **Injection Volume:** | 20 uL | | | | | |
| **Mobile Phases:** | A = 100% Water + 0.1% Formic Acid | | | | | |
| | B = 100% Acetonitrile + 0.1% Formic Acid | | | | | |

| **Gradient:** | **Time (min)** | | **% A** | **% B** | | |
|---|---|---|---|---|---|---|
| | 0 | | 100 | 0 | | |
| | 0.2 | | 100 | 0 | | |
| | 0.3 | | 50 | 50 | | |
| | 0.7 | | 50 | 50 | | |
| | 0.8 | | 100 | 0 | | |

Data were normalized to a high control using the following equation: 100*(Sample - Low Control)/(High Control - Low Control), where the low control is the average of 16 samples treated with 1% DMSO, and the high control is the average of 16 samples treated with 30µM of **Reference Compound Y depicted below.** Data were fit using a 4-parameter fit (log(agonist) vs. response - variable slope) using GraphPad Prism Software v.5. n=2 for all compounds. The Absolute EC₅₀ was interpolated from the curve fit and is defined as the concentration at which a given compound elicits 50% of the high control response. Compounds failing to elicit a minimum response of 50% are reported as >30µM. For compounds run in duplicate or n higher than 2, the result herein given is the geometric mean of the several results obtained. Table 3 summarizes results obtained for selected compounds of the invention in this assay.

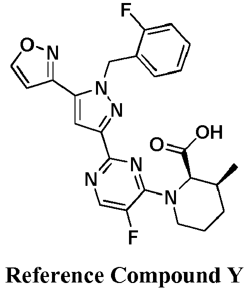

**Table 3. Whole cell activity in the HEK assay with LC/MS detection**

| **sGC_HEK_LC MS EC₅₀ Abs (nM)** | **compound number** | **sGC_HEK_LC MS EC₅₀ Abs (nM)** | **compound number** | **sGC_HEK_LC MS EC₅₀ Abs (nM)** | **compound number** |
|---|---|---|---|---|---|
| A | I-1 | B | I-2 | | |
| * | I-3 | C | I-27 | C | I-57 |
| C | I-4 | C | I-28 | C | I-58 |
| C | I-5 | C | I-29 | C | I-50 |
| C | I-6 | C | I-30 | C | I-41 |
| C | I-7 | C | I-31 | B | I-34 |
| C | I-8 | B | I-32 | B | I-40 |
| C | I-9 | C | I-33 | B | I-34 |
| C | I-10 | B | I-35 | B | I-40 |
| C | I-11 | C | I-36 | C | I-41 |
| C | I-12 | C | I-37 | C | I-50 |
| C | I-13 | C | I-38 | C | I-57 |
| C | I-14 | * | I-39 | C | I-58 |
| C | I-15 | B | I-42 | A | I-72 |
| B | I-16 | C | I-43 | B | I-71 |
| C | I-17 | A | I-44 | B | I-70 |
| C | I-18 | B | I-45 | | |
| C | I-19 | B | I-46 | | |
| B | I-20 | C | I-47 | | |
| C | I-21 | C | I-48 | | |
| C | I-22 | B | I-52 | | |
| C | I-23 | B | I-53 | | |
| C | I-24 | B | I-54 | | |
| B | I-25 | B | I-55 | | |
| C | I-26 | C | I-56 | | |
| C | I-69 | * | I-63 | | |
| B | I-68 | C | I-62 | | |
| B | I-67 | C | I-61 | | |
| B | I-66 | B | I-60 | | |
| C | I-65 | C | I-59 | | |
| C | I-64 | | | | |

| | | | | | |
|---|---|---|---|---|---|
| *Compound does not reach 50% Emax, so no EC₅₀ Abs value was calculated | | | | | |

Code definitions for the whole cell HEK assay values, expressed as Absolute EC₅₀ which is defined as the concentration at which a given compound elicits 50% of the high control response (**Reference Compound Y**). Compounds failing to elicit a minimum response of 50% are reported as *. EC₅₀Abs < 100 nM = A; 101 nM ≤ EC₅₀Abs < 1000 nM = B; 1001 nM ≤ EC₅₀Abs = C.

### Example 3A: Biological activity measurement by the thoracic aortic rings assay

Thoracic aortic rings are dissected from anesthetized (isoflurane) male Sprague-Dawley rats weighing 275-299g. Tissues are immediately transferred to ice-cold Krebs-Henseleit solution, which has been aerated with 95% O₂ and 5% CO₂ for 30 minutes. Following removal of connective tissue, aortic sections are cut into 4 rings (∼2 mm each) and suspended on 2 L-shaped hooks, with one hook fixed at the bottom of the tissue bath (Schuler Organ Bath, Harvard Apparatus) and the other connected to a force transducer (F30 Force Transducer, Harvard Apparatus). Baths containing Krebs Henseleit solution (10 mL) are heated to 37 °C and aerated with 95% O₂ and 5% CO₂. Rings are brought to an initial tension of 0.3-0.5 g and gradually raised to a resting tension of 1.0 g over 60 minutes. Rings are rinsed with Krebs Henseleit solution (heated to 37°C and aerated with 95% O2 and 5% CO2) at 15 minute intervals until a stable baseline is obtained. Rings are considered to be stable after a resting tension of 1.0 g is maintained (for approximately 10 minutes) without need for adjustment. Rings are then contracted with 100 ng/mL phenylephrine by adding 100 uL of a 10µg/mL phenylephrine stock solution. Tissues achieving a stable contraction are then treated in a cumulative, dose dependent manner with test compounds prepared in dimethylsulfoxide (DMSO). In some cases, tissues are rinsed three times over a 5 minute period with Krebs-Heinseleit's solution (heated to 37°C and aerated with 95% O2 and 5% CO2), allowed to stabilize at baseline, and then used for characterization of other test articles or DMSO effects. All data are collected using the HSE-ACAD software provided by Harvard Apparatus. Percent relaxation effects are calculated in Microsoft Excel using the recorded tension value of 100ng/mL phenylephrine treatment as 0% inhibition and treatment with 100 µM 3-isobutyl-1-methylxanthine as 100% inhibition. EC50 values are calculated from concentration-response curves generated with GraphPad Prism Software.

### Example 3B: Biological activity measurement by the thoracic aortic rings assay

As an alternative thoracic aortic rings assay, the procedure of Example 3 is used except that percent relaxation effects are calculated in Microsoft Excel using the recorded tension value of 100ng/mL phenylephrine treatment as 0 % inhibition and, after washing the tissue with buffer, the original resting tension of the tissue is used as 100% inhibition.

### Example 4: Blood pressure change in Sprague-Dawley rats

Male rats (250-350g body weight, supplied by Harlan Laboratories) were anesthetized with ketamine/xylazine and a heparinized saline fluid filled catheter implanted into the right femoral artery. The catheter was exteriorized between the scapula, capped, and the animal allowed to recover for at least 7 days post surgery prior to any compound testing. Prior to testing animals were maintained on normal diet, with free access to drinking water, under a 12 hour light-dark cycle.

On the day of experimentation, under inhaled isoflurane anesthesia, the catheter was uncapped and connected to a tether (Instech Labs) and pressure transducer (Harvard Apparatus). Blood pressure and heart rate were subsequently captured and analyzed with a dedicated data capture system (PowerLab, ADInstruments). Data sampling rates were set at 1 cycle per second. Once connected, each rat was allowed to recover from anesthesia and baseline blood pressure and heart rate levels were established in these conscious, freely-moving animals. Once baseline was established either vehicle (0.5% methylcellulose or 100% PEG400) or test article was administered orally (PO, 10 mg/kg) and the effects on blood pressure and heart rate monitored for up to 24 hours.

### Example 5: Purified human recombinant sGC α1β1 enzyme assay performed in the presence of Diethylenetriamine NONOate (DETA-NONOate), a nitric oxide donor.

Purified human recombinant soluble guanylate cyclase enzyme α1β1(h sGC) obtained from Enzo Life Sciences (P/N: ALX-201-177) was used to evaluate the activity of test compounds. The assay reactions contained 0.1 M Tris (pH 8.0), 0.5 mg/mL BSA, 2 mM DTT, 4 mM MgCl₂, 30uM DETA NONOate (Enzo Life Science P/N: ALX-430-014), and 12.5 ng/ml human soluble guanylate cyclase enzyme. Test compounds in DMSO were then added (in a 3-fold titration of compound over a 10-point curve starting at 30uM final concentration, all samples had a 3% DMSO final concentration). Guanosine 5'-triphosphate (Sigma-Aldrich P/N: G8877) was added to a final concentration of 300 µM and enzyme reactions were incubated (100 µL, 384-well plate format) at 37°C for 20 minutes. The controls contained 3% DMSO (low control), or 30uM of **Reference Compound Y** (high control). After the 20 minute incubation, the reaction was stopped with the addition of 100 µL of ice cold 20% acetic acid.

cGMP concentrations in all samples were determined using the cGMP HTRF (Cisbio P/N: 62GM2PEC) assay per manufacturer's instructions. A cGMP standard curve was fit using a 4-parameter fit (log(inhibitor) vs. response - variable slope) using GraphPad Prism Software v.6. Samples were diluted appropriately to ensure that values fell within the linear range of the standard curve. The EC₅₀ was interpolated from the curve fit and is defined as the concentration at which the compound elicits 50% of the maximal response of the 30uM of **Reference Compound Y,** the high control compound.

**Table 5. Enzyme data**

| Compound Number | sGC_Enz_HTRF_a1b1 EC₅₀ Abs (nM) |
|---|---|
| I-1 | A |
| I-3 | * |
| I-4 | C |
| I-5 | C |
| I-6 | C |
| I-9 | C |
| I-10 | * |
| I-13 | B |
| I-17 | C |
| I-18 | C |
| I-19 | B |
| I-20 | A |
| I-21 | C |
| I-29 | B |
| I-30 | B |
| I-37 | B |
| I-38 | A |
| I-39 | C |
| I-48 | C |
| I-59 | C |
| I-64 | B |
| I-70 | B |

Code definitions for the enzyme assay values, expressed as Absolute EC₅₀ (EC₅₀ Abs) which is defined as the concentration at which a given compound elicits 50% of the high control response (**Reference Compound Y**). Compounds failing to elicit a minimum response of 50% are reported as *.
EC₅₀Abs < 2000 nM = A
<= 2000 nM < EC₅₀Abs <5000 nM = B
<= 5000 nM = C

### Example 6: Animal model descriptions.

### Lamb model of pulmonary hemodynamics using inhaled sGC stimulator

It is possible to test whether inhalation of novel dry-powder microparticle formulations containing sGC stimulators would produce selective pulmonary vasodilation in lambs with acute pulmonary hypertension by following a published procedure ("Inhaled Agonists of Soluble Guanylate Cyclase Induce Selective Pulmonary Vasodilation", Oleg V. et al, American J of Resp and Critical Care Medicine, Vol 176, 2007, p 1138).

It is also possible to evaluate the combined administration of the microparticles of sGC stimulator and inhaled nitric oxide (iNO) in this system. Finally, it is possible to examine whether inhaling microparticles of an sGC stimulator would produce pulmonary vasodilation when the response to iNO (inducible nitric oxide synthase) is impaired.

Protocol: In awake, spontaneously breathing lambs instrumented with vascular catheters and a tracheostomy tube, U-46619 is infused intravenously to increase mean pulmonary arterial pressure to 35 mm Hg. Inhalation of microparticles composed of either BAY 41-2272, BAY 41-8543, or BAY 58-2667 and excipients (dipalmitoylphosphatidylcholine, albumin, lactose) produced dose dependent pulmonary vasodilation and increased transpulmonary cGMP release without significant effect on mean arterial pressure. Inhalation of microparticles containing BAY 41-8543 or BAY 58-2667 increased systemic arterial oxygenation. The magnitude and duration of pulmonary vasodilation induced by iNO were augmented after inhaling BAY 41-8543 microparticles. Intravenous administration of 1H-[1,2,4]oxadiazolo[4,3-a]quinoxalin-1-one (ODQ), which oxidizes the prosthetic heme group of sGC, markedly reduced the pulmonary vasodilator effect of iNO. In contrast, pulmonary vasodilation and transpulmonary cGMP release induced by inhaling BAY 58-2667 microparticles were greatly enhanced after treatment with ODQ. Thus, inhalation of microparticles containing agonists of sGC may provide an effective novel treatment for patients with pulmonary hypertension, particularly when responsiveness to iNO is impaired by oxidation of sGC. Note: BAY 41-2272, BAY 41-8543 are sGC stimulators whereas BAY 58-2667 is an sGC activator.

### Electrical Field Stimulated Guinea Pig Tracheal Smooth Muscle In Vitro (ex vivo) model for the assessment of bronchodilation.

It is possible to assess the bronchodilating effects of sGC stimulators by using the system described below. This system allows us to determine potency, efficacy and duration of action of several sGC stimulators, as well as to assess potential side effects such as blood pressure, or heart rate changes (see "Novel and Versatile Superfusion System. Its use in the Evaluation of Some Spasmogenic and Spasmolytic Agents Using Guinea pig isolated Tracheal Smooth Muscle.", R. A. Coleman et al., J. Pharmacol. Methods, 21, 71-86, 1989. See also "The role of soluble guanylyl cyclase in Chronic Obstructive Pulmonary Disease"; C Glynos et al.; AJRCCM Articles in Press; published on 10-July-2013 as 10.1164/rccm/201210-1884OC.

Animals: Guinea pig, Dunkin Hartley, male, Full barrier-bred and certified free of specific micro-organisms on receipt 525-609g on the experimental day, Harlan UK Ltd. Guinea pigs are housed in a group of 4 in solid-bottomed cages with Gold Flake bedding in a controlled environment (airflow, temperature and humidity). Food (FD1, Special Diet Services) and water are provided ad libitum.

### Guinea Pig Tracheal Smooth Muscle Contraction in Response to EFS. Assessment of Compound Potency and Efficacy:

On each experimental day, a guinea pig is killed by exposure to a rising concentration of CO2 and the trachea removed. The trachea is cleaned of extraneous tissue and cut open longitudinally in a line opposite the muscle, opened out and cut into strips 2 -3 cartilage rings wide. A cotton loop is attached to one end of each tracheal strip and a length of cotton to the other end. Tracheal strips are then suspended between two platinum electrodes, using tissue holders, in a Myobath system (World Precision Instruments Stevenage, UK). The loop is attached over the hook at the bottom of the tissue holder and the other end attached to the arm of a FORT10 force transducer (World Precision Instruments Stevenage, UK) ensuring that the tissue is positioned between the two platinum electrodes. The whole assembly is then lowered into a 10ml tissue bath containing modified Kreb's-Henseleit buffer, at 37 °C, bubbled with Carbogen. A 1 g tension is applied to each piece of tissue and the tissue washed, followed by a 1 hour stabilization period. Once the tissues has been allowed to stabilize, the apparatus for electrical field stimulation is set to deliver a stimulation of frequency 80Hz pulse width 0.1 ms, with a gated, uni-polar pulse, every 2 minutes using a DS8000 8 channel digital stimulator (World Precision Instruments Stevenage, UK). A voltage response curve is carried out on each tracheal strip at 2, 4, 6, 7, 8, 10, 12 V and a sub-maximal voltage then selected to apply to each tissue during the remainder of the experiment. Guinea pig tracheal smooth muscle (GPTSM) contraction is induced using sub-maximal Electrical Field Stimulation (EFS) (It is also possible to induce contraction by using a spasmogen substance, such as methacholine or histamine as described in Coleman et al.*). Compounds are dissolved in 100% DMSO at 3x10-2M and aliquots stored at -200 C. A separate aliquot is used for each experiment. Tissues are washed with Kreb's buffer and stimulated using the previously determined sub-maximal voltage for 1 hour to establish a stable baseline contraction prior to assessment of compound activity.

A cumulative dose response curve (DRC) to each test substance is then performed and changes in smooth muscle contraction measured. The effect of each test substance in each experiment is expressed as a percentage inhibition of the baseline contraction, normalized to the relevant vehicle controls. The experiment is performed three times, using tissue from three different animals. The data from all three experiments are pooled, the DRC plotted, and the test substance potency and efficacy determined. The potency of Ipratropium bromide is assessed alongside the test compounds and the IC50 determined to be 0.86nM (95% Cl, 0.78-0.94), in agreement with data previously produced in the system.

### Mouse model for diseases in which altered CFTR-function is causally involved

These diseases comprise cystic fibrosis, pancreatic disorders, gastrointestinal disorders, liver disorders, cystic fibrosis-related diabetes (CFRO), dry eye, dry mouth and Sjoegren's syndrome.

By using transgenic mice expressing or not expressing the delta F508CFTR channel it is possible to measure differences on nasal potential difference and salivation in the presence of a test sGC stimulator by using the literature protocol described below (see WO2011095534).

### Salivary Secretion Assay in delta(.6.)50S-CFTR mice

15 Male and female homozygous, heterozygous .6.50S-CFTR (backcrossed on the FVB genetic background for more than 12 generations, originally obtained from Erasmus University, Rotterdam; 10-14 weeks old and weighing 1S-36g of both sexes were used in this assay. Solutions of Vardenafil in concentrations of 0.07,0.14 and 0.42 mg/kg BW were 20 prepared in sterile saline, whereas the sGC stimulator BAY 41-2272 was dissolved to 0.01, 0.03, 0.1 and 0.3 mg/kg BW in a solvent containing 50% ddH20, 40% PEG 400 (polyethylene glycol 400) and 10% ethanol. The substances or the appropriate vehicles were administered to mice via intraperitoneal injection (5 ml/kg BW) 60 min prior to the salivary secretion assay. After 60 min, mice were anaesthetized with a combination of 25 ketamine and diazepam. The solution was prepared to contain 1 ml of 5 mg/ml diazepam. and 1 ml of 100 mg/ml ketamine in 8 ml sterile saline. Anesthesia was induced by intraperitoneal injection of the solution (10 ml/kg BW). After anesthesia, mice were pretreated with a subcutaneous injection of 1 mM atropine (50 1-11) into the left cheek in order to avoid a cross-stimulation of cholinergic receptors. Small strips of Whatman filter 5 paper were placed inside the previously injected cheek for 4 min to absorb any saliva secreted after the injection of atropine. This first piece of filter paper was removed and replaced with a second pre-weighed filter paper. Thereafter, 50 1-11 of a solution containing 100 I-IM isoprenaline and 1 mM atropine was injected into the left cheek at the same site to induce the salivary secretion by adrenergic mechanisms. The time of the 10 isoprenaline injection was taken as time zero, and filter paper stripes were replaced every 10 minutes for a total collection period of 30 minutes. Each piece of filter paper was immediately placed and sealed in a pre-weighed vial. After all samples had been collected, each vial was re-measured and the weights of all samples were recorded. The difference in total weight of vial plus paper measured before and after collecting saliva 15 was taken as the net weight of saliva secreted during the collection period. The total amounts of salivary secretion were calculated as the weight of saliva divided by the number of minutes required for each collection and then normalized to the mass of the mouse in grams. Results are expressed in table 1 as the mean percentage increase of n mice compared to placebo treatment. Statistics was analyzed by one way ANOVA test 20 followed by post-hoc Bonferoni analysis; */**/*** means statistical significant with p values <0.05/<0.01/0.001 and n.s. means non-significant.

These animal studies were carried out with a number of sGC stimulators, sGC activators and PDE5 inhibitors. The results suggests that compounds of the invention are useful for the treatment of cystic fibrosis, pancreatic disorders, gastrointestinal disorders, liver disorders, Cystic Fibrosis-related diabetes (CFRO), dry eye, dry mouth and Sjoegren's syndrome.

### Neuromuscular disorders

It has previously been shown that neuronal Nitric Oxide Synthase (nNOS) mislocalization from the sarcolemmal membrane to the sarcoplasm is observed in a broad range of non-dystrophic neuromuscular conditions associated with impaired motility status and catabolic stress. One tool for the evaluation of muscle biopsies of patients with a variety of inherited and acquired forms of neuromuscular disorders is the assessment of sarcolemal localization of nNOS. It was found that the level of nNOS at the sarcolemma correlates with mobility and functional status.

An analogous assessment can be used to determine nNOS localization in animal models of nondystrophic myopathy following the literature protocols described below ("Loss of sarcolemmal nNOS is common in acquired and inherited neuromuscular disorders"; E.L. Finanger Hedderick et al., Neurology, 2011, 76(11), 960-967).

### nNOS mislocalization in mouse models of acquired muscle atrophy

Two mouse models have been described that demonstrate muscle atrophy without compromised mobility: high-dose corticosteroids therapy and short-term starvation. Mice treated with steroids or starved for 48 hours showed significant decreases in overall body mass and in normalized wet skeletal muscle mass. Morphometric analysis of skeletal muscle specimens of both models demonstrated muscle atrophy, as defined by a significant decrease in mean minimal Feret fiber diameter as compared to age-matched controls (n = 5 for each group). Immunofluorescence staining for dystrophin, α-sarcoglycan, and α-1- syntrophin showed normal dystrophin localization suggestive of an intact DGC complex However, both steroid-treated and starved mice showed absent or severely reduced sarcolemmal nNOS staining. Real-time PCR for NOS family proteins (nNOS, eNOS, iNOS) revealed no significant differences in expression levels of any of the 3 transcripts in steroid-treated mice (n = 8 for each group). Moreover, Western blot analysis for nNOS, iNOS, and eNOS showed no differences in protein levels.

These murine animal models could be used to assess the effects of sGC stimulators (for example an sGC stimulator of the invention) in the symptoms of muscle atrophy and related disease states.

Starved mice exhibited a 1-fold decrease of nNOS and iNOS transcript expression as compared to wild type mice (n = 9 for controls, n = 7 for starved). However, the protein level of nNOS, iNOS, and eNOS revealed no differences between control and starved mice (n = 4 for each group). These data demonstrate that abnormal localization of nNOS occurs in mice with severe muscle atrophy even if overall mobility is preserved, supporting the notion that, in addition to impaired mobility, other triggers such as catabolic stress may be associated with sarcolemmal loss of nNOS.

### Skeletal muscle nNOS localization is maintained during hibernation (studies with squirrels)

Skeletal muscle specimens from hibernating 13-lined ground squirrels have been used to evaluate the impact of immobility and catabolic stress on nNOS localization in the context of maintained muscle homeostasis and integrity. These animals are obligate hibernating mammals that are protected against skeletal muscle atrophy during hibernation. Despite hibernating for 5 months with almost complete immobility and no caloric intake, sarcolemmal expression of nNOS is preserved. These data together with patient and mouse data indicate that biochemical control of nNOS localization is complex and, importantly, that preserved sarcolemmal nNOS may be significant in maintaining muscle homeostasis.

These results also suggest that targeting aberrant NO signaling (for instance with sGC stimulators such as the ones here described) may prove beneficial for a broad group of patients with neuromuscular disorders.

### Mouse models of Muscular Dystrophy (BMD and DMD)

Becker muscular dystrophy (BMD), characterized by progressive skeletal muscle wasting, is caused by mutations of the muscle protein dystrophin. In a human study, Martin et al. (see "Tadalafil Alleviates Muscle Ischemia in Patients with Becker Muscular Dystrophy"; Elizabeth A. Martin et al., Sci. Transl. Med. 4, 162ra155 (2012); "Vascular-targeted therapies for Duchenne muscular dystrophy"; Ennen et al., Skeletal Muscle, 2013, 3:9) assessed exercise-induced attenuation of reflex sympathetic vasoconstriction in the muscles of 10 patients with BMD and 7-age matched healthy male controls. This is a protective mechanism that optimizes perfusion of skeletal muscle to meet the metabolic demands of exercise. Reflex vasoconstriction was induced by simulated orthostatic stress and was measured as the forearm muscles were rested or lightly exercised in the form of rhythmic handgrip. First, the investigators showed that exercise-induced attenuation of reflex vasoconstriction was defective in 9 out of 10 patients with BMD in whom the common dystrophin mutations disrupt targeting of neuronal NO synthase (nNOS) to the muscle sarcolemma. Then, in a double-blind randomized placebo-controlled crossover trial, the authors showed that normal blood flow regulation was restored in eight of nine patients by a single oral dose of 20 mg of tadalafil, a specific PDE5 inhibitor.

It is possible to assess the effects of drugs acting on the NO pathway by using a dystrophin-deficient *mdx* mouse model of related disease Duchenne muscular dystrophy (DMD). This model has also shown that inhibitors of phosphodiesterase 5 (PDE5) alleviate some features of the dystrophic phenotype including vasospasm of skeletal muscle micro-vessels that can lead to muscle injury and fatigue.

With exercise of healthy skeletal muscle, sarcolemmal nNOS derived NO attenuates local α-adrenergic vasoconstriction, thereby optimizing perfusion to meet the metabolic demands of the active muscle. This protective mechanism (termed functional sympatholysis) is lost in *mdx* mice (a model of BMD and DMD), nNOS null mice, and boys with DMD causing functional muscle ischemia. Repeated bouts of functional ischemia could accelerate use-dependent injury of muscle fibers already weakened by dystrophin deficiency

In the *mdx* mouse, many features of the dystrophic phenotype can be improved by multiple strategies that boost NO signaling, including transgenic expression of nNOS, transgenic expression of dystrophin minigenes that restore sarcolemmal nNOS (and thereby restore functional sympatholysis), administration of the NOS substrate L-arginine (24, 25), treatment with NO-donating drugs, and phosphodiesterase 5A (PDE5A) inhibition with the drug tadalafil or sildenafil. These PDE5A inhibitors, which prolong the half-life of guanosine 3',5'-monophosphate (cGMP)- the downstream target of NO in vascular smooth muscle- were shown in the *mdx* mouse to alleviate muscle ischemia, as well as injury and fatigue, after a brief bout of exercise. Also, these drugs were shown to improve cardiac dynamics in *mdx* mice and to rescue dystrophic skeletal muscle and prolong survival in dystrophin-deficient zebra fish.

These findings support an essential role for sarcolemmal nNOS in modulating sympathetic vasoconstriction in exercising human skeletal muscles and suggests that targeting the aberrant NO pathway (for instance by using an sGC stimulator of the invention) may be a useful therapeutic approach for treating BMD and DMD in humans.

### Sickle Cell Disease

Sickle-cell disease (SCD), or sickle-cell anemia (SCA) or drepanocytosis, is a hereditary blood disorder, characterized by red blood cells that assume an abnormal, rigid, sickle shape. Sickling decreases the cells' flexibility and results in a risk of various complications. The sickling occurs because of a mutation in the hemoglobin gene. Individuals with one copy of the defunct gene display both normal and abnormal hemoglobin. This is an example of co-dominance. In 1994, in the US, the average life expectancy of persons with this condition was estimated to be 42 years in males and 48 years in females, but today, thanks to better management of the disease, patients can live into their 70s or beyond.

Sickle-cell anemia is a form of sickle-cell disease in which there is homozygosity for the mutation that causes HbS. Sickle-cell anemia is also referred to as "HbSS", "SS disease", "hemoglobin S" or permutations of those names. In heterozygous people, that is, those who have only one sickle gene and one normal adult hemoglobin gene, the condition is referred to as "HbAS" or "sickle cell trait". Other, rarer forms of sickle-cell disease are compound heterozygous states in which the person has only one copy of the mutation that causes HbS and one copy of another abnormal hemoglobin allele. They include sickle-hemoglobin C disease (HbSC), sickle beta-plus-thalassemia (HbS/β⁺) and sickle beta-zero-thalassemia (HbS/β⁰).

Although red blood cell (RBC) sickling and rheological abnormalities are central to the pathophysiology of sickle cell disease, vascular dysfunction resulting from complex interactions between sickled red blood cells (sRBC), endothelial cells, platelets and leukocytes play an equally important role. In sickle cell disease, endothelial activation is associated with sickle cell-mediated hypoxia-reperfusion events (see for example "Advances in understanding of the pathogenesis of cerebrovascular vasculopathy in sickle cell anemia", P. Connes et al., Br. J. Haematol. 2013, 161, 484-98). Red blood cell sickling and adhesion to endothelium initiate vaso-occlusion by impairing blood flow. The subsequent surge of inflammatory mediators and endothelial activation trigger a cascade of events leading to vascular damage. Pathophysiological responses to intermittent hypoxia-reperfusion from these vaso-occlusive events are demonstrated by an increased production of cytokines, leukocyte up-regulation and activation of pro-coagulant and adhesion molecules, with simultaneous inhibition of cytoprotective mediators.

Leukocytosis is correlated with nearly every manifestation of sickle cell disease, emphasizing the influential role of inflammation in the pathophysiology of sickle vasculopathy. Even at baseline, sickle cell disease patients exhibit elevations in pro-inflammatory cytokines, including C-reactive protein (CRP), tumor necrosis factor (TNF), interleukin-1 (IL-1) and interleukin-8 (IL-8). *In vitro* studies have shown that sRBC promote endothelial up-regulation of TNF-α and IL-1-β (8-10). Microarray studies of activated monocytes have shown differential expression of genes involved in inflammation, heme metabolism, cell cycle regulation, anti-oxidant responses, and angiogenesis. More recently, it was shown that differential expression of nuclear factor κ-light-chain-enhancer of activated B cells (NPκB/p65), Kruppel-like factor 2 (KLF2), and other transcription factors that regulate pathways of inflammation in sickle cell disease children at increased risk for stroke.

In transgenic mouse models (see "Novel Therapies Targeting the Endothelium in sickle cell disease", C.C Hoppe, Hemoglobin, 35(5-6):530-546 (2011) and references cited therein), sickling inducing oxidative stress has been shown to affect microvascular regulatory mechanisms leading to endothelial activation and exaggerated inflammatory and pro-adhesive responses. Oxidative stress occurs through formation of reactive oxygen species (ROS). Depletion of NO occurs through hemoglobin (Hb) mediated scavenging, consumption by ROS and arginase-mediated substrate depletion. In sickle cell disease, the scavenger systems that normally remove circulating free Hb are saturated. Free Hb depletes NO, leading to endothelial dysfunction. Consequently, the normal balance of vasoconstriction and vasodilation is skewed towards vasoconstriction, endothelial activation, oxidative stress and proliferative vasculopathy.

Therapies directed at restoring NO homeostasis have shown promise in preliminary studies in patients with sickle cell disease. Previous *in vitro* studies and studies in other patient populations showed NO-mediated down-regulation of endothelial adhesion molecule expression. Following these observations, the use of inhaled NO was studied in sickle cell disease children presenting with VOE and found associated trends toward lower pain scores, decreased analgesic requirements and a shorter hospital stay.

These findings were reproduced in a recent randomized placebo controlled trial evaluating inhaled NO for the treatment of acute VOE in adult patients with sickle cell disease, showing that inhaled NO significantly reduced pain scores and was associated with a trend towards decreased use of parenteral morphine compared with placebos. Results from a completed phase II trial of adult sickle cell disease patients treated with inhaled NO for acute VOE have not yet been made available (clinicaltrials. gov NCT00023296). Another phase II trial of inhaled NO for VOE treatment in children with sickle cell disease is expected to be completed (clinicaitrials.gov NCT00094887). The possible therapeutic role of inhaled NO for ACS in sickle cell disease is currently being assessed in both children and adults in two separate French phase II/III trials comparing the use of inhaled NO to placebo or standard care in children with ACS (clinicaltrials.gov NCT01089439 and NCT00748423).

Dietary supplementation of the NO synthase substrate, L-arginine, has been studied extensively in sickle cell disease as a means of increase NO bioavailability. In sickle mice, oral L-arginine at high doses has been shown to decrease Gardos channel activity, dense cell formation and hemolysis, as well as functional improvements in vascular reactivity.

Sildenafil, an agent aimed at amplifying the effect of endogenous NO by inhibiting PDE5, a downstream mediator of NO, is used widely in the general population to treat primary PHT. Preliminary studies in sickle cell disease patients with severe PHT reported improvements in PAP and exercise capacity after treatment with sildenafil. A multicenter trial (Treatment of Pulmonary Hypertension and Sickle Cell Disease with Sildenafil Therapy, Walk-PHaSST) testing the safety and efficacy of sildenafil in sickle cell disease patients with Doppler-defined PHT was stopped prematurely due to a higher frequency of serious side effects, including increased rates of VOE, headache, and visual disturbance in the treatment group.

Nitrite and niacin have also been investigated for their direct NO donor properties. In a pilot phase I/II clinical trial, sodium nitrite infusions in adult sickle cell disease patients enhanced forearm blood flow, consistent with a NO donor mechanism of action. A larger phase I/II trial is now investigating whether nitrite infusions administered as adjunctive therapy during acute VOE will improve microvascular blood flow and tissue oxygenation (clinicaltrials.gov NCT01033227). The effect of niacin on improvement in endothelial-dependent vasodilation is also being assessed in a phase II randomized, controlled trial (clinicaltrials.gov NCT 00508989).

The above results suggest that targeting the aberrant NO pathway in sickle cell disease (for instance by using an sGC stimulator of the invention) may be a useful therapy for the treatment of the disease. Murine models of sickle cell anemia that could be used to assess the effect of sGC stimulators (e.g., an sGC stimulator of the invention) in this disease state, are described in Blood, 2001, 98(5), 1577-84; J. Clin. Invest. 2004, 114(8), 1136-45; and Br. J. Haematol, 2004, 124(3), 391-402.

### Bladder dysfunction

It has been shown that the sGC activator BAY 60-2770 ameliorates overactive bladder in obese mice (see "The Soluble Guanylyl Cyclase Activator BAY 60-2770 ameliorates overactive bladder in obese mice", Luiz O Leiria et al., The Journal of Urology, 2013, doi:10.1016/j.juro.2013.09.020.). The animal model described in this publication can analogously be used to assess the effect of an sGC stimulator (for example, an sGC stimulator of the invention) on overactive bladder.

The same group of researchers have also described a rat model of bladder dysfunction (NO-deficient rats, F Z Monica et al., Neurology and Urodynamics, 30, 456-60, 2011) and have shown the protective effects of BAY-2272 (an sGC activator) in this model. The animal model described in this publication can analogously be used to assess the effect of an sGC stimulator (for example, an sGC stimulator of the invention) on bladder dysfunction related to detrusor smooth muscle overactivity.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has" and "having"), "include" (and any form of include, such as "includes" and "including"), "contain" (and any form contain, such as "contains" and "containing"), and any other grammatical variant thereof, are open-ended linking verbs. As a result, a method or device that "comprises", "has", "includes" or "contains" one or more steps or elements possesses those one or more steps or elements, but is not limited to possessing only those one or more steps or elements. Likewise, a step of a method or an element of a device that "comprises", "has", "includes" or "contains" one or more features possesses those one or more features, but is not limited to possessing only those one or more features. Furthermore, a device or structure that is configured in a certain way is configured in at least that way, but may also be configured in ways that are not listed.

As used herein, the terms "comprising," "has," "including," "containing," and other grammatical variants thereof encompass the terms "consisting of" and "consisting essentially of."

The phrase "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Where one or more ranges are referred to throughout this specification, each range is intended to be a shorthand format for presenting information, where the range is understood to encompass each discrete point within the range as if the same were fully set forth herein.

While several aspects and embodiments of the present invention have been described and depicted herein, alternative aspects and embodiments may be affected by those skilled in the art to accomplish the same objectives. Accordingly, this disclosure and the appended claims are intended to cover all such further and alternative aspects and embodiments as fall within the scope of the invention.

## Claims

1. A compound according to **Formula I,** or a pharmaceutically acceptable salt thereof, 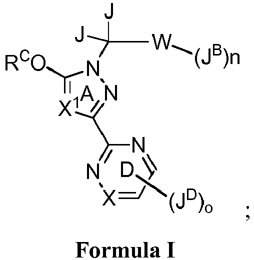
wherein X is selected from N or C;
X¹ is selected from N, CH, C(C₁₋₄ alkyl), C(C₁₋₄ fluoroalkyl), C(Cl), and CF;
W is either
i) absent, with J^{B} connected directly to the carbon atom bearing two J groups, each J is independently selected from hydrogen, methyl or fluorine, n is 1 and J^{B} is a C₁₋₆ alkyl chain optionally substituted by up to 6 instances of fluorine; or
ii) a ring B selected from phenyl or a 5 or 6-membered heteroaryl ring, containing 1 or 2 ring heteroatoms selected from N, O or S; wherein when W is ring B:
each J is hydrogen;
n is 0 or an integer selected from 1 to 3;
and each J^{B} is independently selected from halogen, -CN, a C₁₋₆ aliphatic, -OR^{B} or a C₃₋₈ cycloaliphatic group; wherein each said C₁₋₆ aliphatic and each said C₃₋₈ cycloaliphatic group is optionally and independently substituted with up to 3 instances of R³;
each R^{B} is independently selected from hydrogen, a C₁₋₆ aliphatic or a C₃₋₈ cycloaliphatic; wherein each said R^{B} that is a C₁₋₆ aliphatic and each said R^{B} that is a C₃₋₈ cycloaliphatic ring is optionally and independently substituted with up to 3 instances of R^{3a};
each R³ is independently selected from halogen, -CN, C₁₋₄ alkyl, C₁₋₄haloalkyl, -O(C₁₋₄ alkyl) or -O(C₁₋₄ haloalkyl);
each R^{3a} is independently selected from halogen, -CN, C₁₋₄ alkyl, C₁₋₄ haloalkyl, -O(C₁₋₄ alkyl) or -O(C₁₋₄ haloalkyl);
o is 0 or an integer selected from 1 to 3;
each J^{D} is either absent or independently selected from hydrogen, halogen, -CN, -NO₂, -OR^{D}, -SR^{D}, -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂, -N(R^{d})SO₂R^{D}, a C₁₋₆aliphatic, -(C₁₋₆ aliphatic)-R^{D}, a C₃₋₈ cycloaliphatic ring, a 6 to 10-membered aryl ring, a 4 to 8-membered heterocyclic ring or a 5 to 10-membered heteroaryl ring; wherein each said 4 to 8-membered heterocyclic ring and each said 5 to 10-membered heteroaryl ring contains between 1 and 3 heteroatoms independently selected from O, N or S; and wherein each of said C₁₋₆ aliphatic chains, each said C₃₋₈ cycloaliphatic ring, each said 6 to 10-membered aryl ring, each said 4 to 8-membered heterocyclic ring and each said 5 to 10-membered heteroaryl ring is optionally and independently substituted with up to 5 instances of R⁵;
each R^{D} is independently selected from hydrogen, a C₁₋₆ aliphatic, -(C₁₋₆aliphatic)-R^{f}, a C₃₋₈ cycloaliphatic ring, a 4 to 8-membered heterocyclic ring and phenyl; wherein each said 4 to 8-membered heterocyclic ring contains between 1 and 3 heteroatoms independently selected from O, N and S; and wherein each of said C₁₋₆ aliphatic chains, each said C₃₋₈ cycloaliphatic ring, each said 4 to 8-membered heterocyclic ring and each said phenyl is optionally and independently substituted with up to 5 instances of R^{5a}; wherein when any R^{D} is one of a C₁₋₆ aliphatic or a -(C₁₋₆ aliphatic)-R^{f} group, one or two -CH₂- units that form said C₁₋₆ aliphatic chains may, optionally, be replaced by a group independently selected from -C(O)-, -N(R^{d}) - or -O-;
each R^{d} is independently selected from hydrogen, a C₁₋₆ aliphatic, -(C₁₋₆aliphatic)-R^{f}, a C₃₋₈ cycloaliphatic ring, a 4 to 8-membered heterocyclic ring, phenyl or a 5 to 6-membered heteroaryl ring; wherein each said 4 to 8-membered heterocyclic ring and each said 5 or 6-membered heteroaryl ring contains between 1 and 3 heteroatoms independently selected from O, N or S; and wherein each of said C₁₋₆ aliphatic chains, each said C₃₋₈ cycloaliphatic ring, each said 4 to 8-membered heterocyclic ring, each said phenyl and each said 5 to 6-membered heteroaryl ring is optionally and independently substituted by up to 5 instances of R^{5b};
each R^{f} is independently selected from a C₁₋₃ alkyl, a C₃₋₈ cycloaliphatic ring, a 4 to 8-membered heterocyclic ring, phenyl or a 5 to 6-membered heteroaryl ring; wherein each said 4 to 8-membered heterocyclic ring and each said 5 to 6-membered heteroaryl ring contains between 1 and 4 heteroatoms independently selected from O, N or S; and wherein each said C₃₋₈ cycloaliphatic ring, each said 4 to 8-membered heterocyclic ring, each said phenyl and each said 5 to 6-membered heteroaryl ring is optionally and independently substituted by up to 5 instances of R^{5c};
when J^{D} is -C(O)N(R^{D})₂, -N(R^{D})₂, -N(R^{d})C(O)N(R^{D})₂, -OC(O)N(R^{D})₂ or -SO₂N(R^{D})₂, the two R^{D} groups together with the nitrogen atom attached to the two R^{D} groups may form a 4 to 8-membered heterocyclic ring or a 5-membered heteroaryl ring; wherein each said 4 to 8-membered heterocyclic ring and each said 5-membered heteroaryl ring optionally contains up to 3 additional heteroatoms independently selected from N, O or S, in addition to the nitrogen atom to which the two R^{D} groups are attached; and wherein each said 4 to 8-membered heterocyclic ring and each said 5-membered heteroaryl ring is optionally and independently substituted by up to 5 instances of R^{5d};
when J^{D} is -N(R^{d})C(O)R^{D}, the R^{D} group together with the carbon atom attached to the R^{D} group, with the nitrogen atom attached to the R^{d} group, and with the R^{d} group may form a 4 to 8-membered heterocyclic ring or a 5-membered heteroaryl ring; wherein each said 4 to 8-membered heterocyclic ring and each said 5-membered heteroaryl ring optionally contains up to 2 additional heteroatoms independently selected from N, O or S, in addition to the nitrogen atom to which the R^{d} group is attached; and wherein each said 4 to 8-membered heterocyclic ring and each said 5-membered heteroaryl ring is optionally and independently substituted by up to 5 instances of R^{5d};
when J^{D} is -N(R^{d})C(O)OR^{D}, the R^{D} group together with the oxygen atom attached to the R^{D} group, with the carbon atom of the -C(O)- portion of the -N(R^{d})C(O)OR^{D} group, with the nitrogen atom attached to the R^{d} group, and with said R^{d} group, may form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
when J^{D} is -N(R^{d})C(O)N(R^{D})₂, one of the R^{D} groups attached to the nitrogen atom, together with said nitrogen atom, and with the N atom attached to the R^{d} group and said R^{d} group may form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
when J^{D} is -N(R^{d})SO₂R^{D}, the R^{D} group together with the sulfur atom attached to the R^{D} group, with the nitrogen atom attached to the R^{d} group, and with said R^{d} group may combine to form a 4 to 8-membered heterocyclic ring; wherein said 4 to 8-membered heterocyclic ring optionally contains up to 2 additional heteroatoms independently selected from N, O or S, and is optionally and independently substituted by up to 5 instances of R^{5d};
each R⁵ is independently selected from halogen, -CN, C₁₋₆ alkyl, -(C₁₋₆alkyl)-R⁶, -OR⁶, -SR⁶, -COR⁶, -OC(O)R⁶, -C(O)OR⁶, -C(O)N(R⁶)₂, -N(R⁶)C(O)R⁶-N(R⁶)C(O)OR⁶, -N(R⁶)C(O)N(R⁶)₂, -N(R⁶)₂, -SO₂R⁶, -SO₂OH, -SO₂NHOH, -SO₂N(R⁶)₂, -SO₂N(R⁶)(CO)-R⁶, -N(R⁶)SO₂R⁶, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, said C₇₋₁₂ aralkyl, said C₃₋₈ cycloalkyl ring, said 4 to 7-membered heterocyclic ring, said 5 or 6-membered heteroaryl ring or said phenyl group is optionally and independently substituted with up to 3 instances of halogen, C₁₋₄ alkyl, C₁₋₄ (haloalkyl), -OH, -NH₂, -NH(C₁₋₄ alkyl), -N(C₁₋₄alkyl)₂, -CN, -COOH, -CONH₂, -COO(C₁₋₄ alkyl), -O(C₁₋₄ alkyl), -O(C₁₋₄ haloalkyl) or oxo;
alternatively, two instances of R⁵ attached to the same or different atoms of J^{D}, together with said atom or atoms of J^{D} to which they are attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -NR(CO)O(C₁₋₄ alkyl), -CONH₂, -OH or halogen; wherein R is hydrogen or a C₁₋₂ alkyl;
each R^{5a} is independently selected from halogen, -CN, C₁₋₆ alkyl, -(C₁₋₆alkyl)-R^{6a}, -OR^{6a}, -SR^{6a}, -COR^{6a}, -OC(O)R^{6a}, -C(O)OR^{6a}, -C(O)N(R^{6a})₂, -N(R^{6a})C(O)R^{6a}, -N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, each said C₇₋₁₂ aralkyl, said C₃₋₈ cycloalkyl ring, said 4 to 7-membered heterocyclic ring, said 5 or 6-membered heteroaryl ring or phenyl group is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -COO(C₁₋₄ alkyl), -CONH₂, -O(C₁₋₄ alkyl), -O(C₁₋₄ haloalkyl) or oxo;
each R^{5b} is independently selected from halogen, -CN, C₁₋₆ alkyl, -(C₁₋₆alkyl)-R^{6a}, -OR^{6a}, -SR^{6a}, -COR^{6a}, -OC(O)R^{6a}, -C(O)OR^{6a}, -C(O)N(R^{6a})₂, -N(R^{6a})C(O)R^{6a}, -N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, each said C₇₋₁₂ aralkyl, said C₃₋₈ cycloalkyl ring, said 4 to 7-membered heterocyclic ring, said 5 or 6-membered heteroaryl ring or phenyl group is optionally and independently substituted with up to 3 instances of halogen, C₁₋₄ alkyl, C₁₋₄ (haloalkyl), -OH, -NH₂, -NH(C₁₋₄alkyl), -N(C₁₋₄ alkyl)₂, -CN, -COOH, -COO(C₁₋₄ alkyl), -CONH₂, -O(C₁₋₄ alkyl), -O(C₁₋₄ haloalkyl) or oxo;
alternatively, two instances of R^{5a} or two instances of R^{5b} attached to the same or different atoms of R^{D} or R^{d}, respectively, together with said atom or atoms to which they are attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -NR(CO)O(C₁₋₄ alkyl), -CONH₂, -OH or halogen; wherein R is hydrogen or a C₁₋₂ alkyl;
each R^{5c} is independently selected from halogen, -CN, C₁₋₆ alkyl, -(C₁₋₆alkyl)-R⁶¹, -OR^{6b}, -SR^{6b}, -COR^{6b}, -OC(O)R^{6b}, -C(O)OR^{6b}, -C(O)N(R^{6b})₂, -N(R^{6b})C(O)R^{6b}, -N(R^{6b})C(O)OR^{6b}, -N(R^{6b})C(O)N(R^{6b})₂, -N(R^{6b})₂, -SO₂R^{6b}, -SO₂OH, -SO₂NHOH, -SO₂N(R^{6b})(CO)-R^{6b}, -SO₂N(R^{6b})₂, -N(R^{6b})SO₂R^{6b}, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, said C₇₋₁₂ aralkyl, said C₃₋₈ cycloalkyl ring, said 4 to 7-membered heterocyclic ring, said 5 or 6-membered heteroaryl ring or said phenyl groups is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -CONH₂, -COO(C₁₋₄ alkyl), -O(C₁₋₄ alkyl), -O(C₁₋₄ haloalkyl) or oxo;
alternatively, two instances of R^{5c} attached to the same or different atoms of R^{f}, together with said atom or atoms to which it is attached, may form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship with respect to each other; wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -CONH₂, -NR(CO)O(C₁₋₄ alkyl), -OH or halogen; wherein R is hydrogen or a C₁₋₂ alkyl;
each R^{5d} is independently selected from halogen, -CN, C₁₋₆ alkyl, -(C₁₋₆alkyl)-R⁶, -OR⁶, -SR⁶, -COR⁶, -OC(O)R⁶, -C(O)OR⁶, -C(O)N(R⁶)₂, -N(R⁶)C(O)R6-N(R⁶)C(O)OR⁶, -N(R⁶)C(O)N(R⁶)₂, -N(R⁶)₂, -SO₂OH, -SO₂NHOH, -SO₂N(R⁶)(CO)-R⁶, -SO₂R⁶, -SO₂N(R⁶)₂, -N(R⁶)SO₂R⁶, a C₇₋₁₂ aralkyl, a C₃₋₈ cycloalkyl ring, a 4 to 7-membered heterocyclic ring, a 5 or 6-membered heteroaryl ring, phenyl or an oxo group; wherein each 5 or 6-membered heteroaryl ring or 4 to 7-membered heterocyclic ring contains up to four ring heteroatoms independently selected from N, O and S, wherein each of said C₁₋₆ alkyl chains, said C₇₋₁₂ aralkyl, said C₃₋₈ cycloalkyl ring, said 4 to 7-membered heterocyclic ring, said 5 or 6-membered heteroaryl ring or said phenyl groups is optionally and independently substituted with up to 3 instances of halogen, C₁₋₄ alkyl, C₁₋₄ (haloalkyl), -OH, -NH₂, -NH(C₁₋₄ alkyl), -N(C₁₋₄ alkyl)₂, -CN, -COOH, -CONH₂, -COO(C₁₋₄ alkyl), -O(C₁₋₄ alkyl), -O(C₁₋₄ haloalkyl) or oxo;
two instances of R⁵ or two instances of R^{5d}, attached to the same or different atoms of J^{D}, together with said atom or atoms to which they are attached, may optionally form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship, wherein said 4 to 6-membered heterocycle or said 5 or 6-membered heteroaryl ring contains up to four ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -C(O)OH, -CONH₂, -NR(CO)O(C₁₋₄ alkyl), -OH or halogen; wherein R is hydrogen or a C₁₋₂ alkyl;
each R⁶ is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S;
each R^{6a} is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S;
each R^{6b} is independently selected from hydrogen, a C₁₋₆ aliphatic, phenyl, benzyl, a C₃₋₈ cycloalkyl ring, a 4 to 7-membered heterocyclic ring or a 5 or 6-membered heteroaryl ring, wherein each of said C₁₋₆ aliphatic, each of said phenyl, each of said benzyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo, wherein each of said 5 or 6-membered heteroaryl ring or 4 to 7-membered heterocyclic ring contains up to 4 ring heteroatoms independently selected from N, O and S; wherein
two instances of R⁶ linked to the same nitrogen atom of R⁵ or R^{5d}, together with said nitrogen atom of R⁵ or R^{5d}, respectively, may form a 5 to 8-membered heterocyclic ring or a 5-membered heteroaryl ring; wherein each said 5 to 8-membered heterocyclic ring and each said 5-membered heteroaryl ring optionally contains up to 2 additional heteroatoms independently selected from N, O or S;
two instances of R^{6a} linked to a nitrogen atom of R^{5a} or R^{5b}, together with said nitrogen, may form a 5 to 8-membered heterocyclic ring or a 5-membered heteroaryl ring; wherein each said 5 to 8-membered heterocyclic ring and each said 5-membered heteroaryl ring optionally contains up to 2 additional heteroatoms independently selected from N, O or S;
two instances of R^{6b} linked to a nitrogen atom of R^{5c}, together with said nitrogen, may form a 5 to 8-membered heterocyclic ring or a 5-membered heteroaryl ring; wherein each said 5 to 8-membered heterocyclic ring and each said 5-membered heteroaryl ring optionally contains up to 2 additional heteroatoms independently selected from N, O or S;
alternatively, two J^{D} groups attached to two vicinal ring D atoms, taken together with said two vicinal ring D atoms, may form a 5 to 7-membered heterocycle or a 5-membered heteroaryl ring that is fused to ring D; wherein said 5 to 7-membered heterocycle or said 5-membered ring heteroaryl contains from 1 to 3 heteroatoms independently selected from N, O or S; and wherein said 5 to 7-membered heterocycle or said 5-membered heteroaryl ring is optionally and independently substituted by up to 3 instances of oxo or -(Y)-R⁹;
wherein Y is either absent or is a C₁₋₆ alkyl chain, optionally substituted by up to 6 instances of fluoro; and wherein when Y is said C₁₋₆ alkyl chain, up to 3 methylene units of this alkyl chain, can be replaced by a group selected from -O-, -C(O) - or -N((Y¹)-R⁹⁹)-;
Y¹ is either absent or a C₁₋₆ alkyl chain, optionally substituted by up to 6 instances of fluoro;
when Y¹ is absent, each R⁹⁹ is independently selected from hydrogen, C₁₋₆ alkyl optionally substituted with up to 9 fluorine atoms, -COR¹⁰, -C(O)OR¹⁰,-C(O)N(R¹⁰)₂, -C(O)N(R¹⁰)SO₂R¹⁰, -SO₂R¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, a C₃₋₆ cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
when Y¹ is present, each R⁹⁹ is independently selected from hydrogen, halogen, -CN, C₁₋₆ alkyl optionally substituted with up to 9 fluorine atoms, -COR¹⁰, -OR¹⁰, -OC(O)R¹⁰, -C(O)OR¹⁰,-C(O)N(R¹⁰)₂, -C(O)N(R¹⁰)SO₂R¹⁰, -SO₂R¹⁰, -SOR¹⁰, -SR¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, a C₃₋₆ cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
each R⁹ is independently selected from hydrogen, -CN, -OR¹⁰, -COR¹⁰, -OC(O)R¹⁰, -C(O)OR¹⁰, -C(O)N(R¹⁰)₂, -C(O)N(R¹⁰)SO₂R¹⁰, -N(R¹⁰)C(O)R¹⁰, -N(R¹⁰)C(O)OR¹⁰, -N(R¹⁰)C(O)N(R¹⁰)₂, -N(R¹⁰)₂, -SO₂,R¹⁰, -SO₂N(R¹⁰)₂, -SO₂N(R¹⁰)COOR¹⁰, -SO₂N(R¹⁰)C(O)R¹⁰, -N(R¹⁰)SO₂R¹⁰, -SO₂OH, -SO₂NHOH, -SO₂N(R¹⁰)(CO)-R¹⁰, a C₃₋₆cycloalkyl ring, a 4-8-membered heterocyclic ring, a phenyl ring or a 5-6 membered heteroaryl ring; wherein each said 4 to 8-membered heterocyclic ring or 5 to 6-membered heteroaryl ring contains up to 4 ring heteroatoms independently selected from N, O or S; and wherein each of said C₃₋₆ cycloalkyl rings, each of said 4 to 8-membered heterocyclic rings, each of said phenyl and each of said 5 to 6-membered heteroaryl rings is optionally and independently substituted with up to 3 instances of R^{11a};
each R¹⁰ is independently selected from hydrogen, a C₁₋₆alkyl, -(C₁-₆alkyl)-R¹³, phenyl, benzyl, a C₃₋₆ cycloalkyl ring, a 4 to 7-membered heterocyclic ring or a 5 or 6-membered heteroaryl ring, wherein each 5 or 6-membered heteroaryl ring or 4 to 7-membered heterocyclic ring contains up to 4 ring heteroatoms independently selected from N, O and S; and wherein each of said C₁₋₆ alkyl, each said phenyl, each said benzyl, each said C₃₋₈ cycloalkyl group, each said 4 to 7-membered heterocyclic ring and each 5 or 6-membered heteroaryl ring is optionally and independently substituted with up to 3 instances of R^{11b};
each R¹³ is independently selected from a phenyl, a benzyl, a C₃₋₆ cycloalkyl ring, a 4 to 7-membered heterocyclic ring or a 5 or 6-membered heteroaryl ring, wherein each 5 or 6-membered heteroaryl ring or 4 to 7-membered heterocyclic ring contains up to 4 ring heteroatoms independently selected from N, O and S; and wherein each said phenyl, each of said benzyl, each said C₃₋₈ cycloalkyl group, each said 4 to 7-membered heterocyclic ring and each 5 or 6-membered heteroaryl ring is optionally and independently substituted with up to 3 instances of R^{11c};
each R^{11a} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹;
each R^{11b} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹; and
each R^{11c} is independently selected from halogen, C₁₋₆ alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ or -N(R¹²)SO₂R¹²; wherein each of said C₁₋₆ alkyl is optionally and independently substituted by up to 6 instances of fluoro and/or 3 instances of R¹²¹;
each R¹² is selected from hydrogen, C₁₋₄ alkyl, C₁₋₄ (fluoroalkyl), -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ fluoroalkyl) or oxo;
each R¹²¹ is selected from C₁₋₄ alkyl, C₁₋₄ (fluoroalkyl), -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ fluoroalkyl) or oxo;
R^{C} is selected from hydrogen, C₁₋₆ aliphatic, -(C₁₋₆ alkyl)-R^{N}, a 5 or 6-membered heteroaryl, phenyl, a 4 to 7 membered heterocyclic, a C₃₋₈ cycloaliphatic, -C(O)R⁷, -C(O)OR⁷, -C(O)N(R⁷)₂, and -C(O)N(R⁷)SO₂R⁷ ; wherein each of said 5 or 6-membered heteroaryl ring and 4 to 7-membered heterocyclic ring contains up to 4 ring heteroatoms independently selected from N, O and S; wherein each said C₁₋₆ aliphatic and each C₁₋₆ alkyl portion of said -(C₁₋₆ alkyl)-R^{N}, is optionally and independently substituted with up to 6 instances of halogen and up to 2 instances of -CN, -COOR⁸, -OR⁸, oxo, -N(R⁸)₂, -C(O)N(R⁸)₂, -N(R⁸)C(O)R⁸, -N(R⁸)C(O)OR⁸, -N(R⁸)C(O)N(R⁸)₂, -SO₂R⁸, -SO₂N(R⁸)₂, -NHOR⁸, -SO₂N(R⁸)COOR⁸, -SO₂N(R⁸)C(O)R⁸ and -N(R⁸)SO₂R⁸;
wherein each R⁷ is independently selected from hydrogen, C₁₋₆ alkyl, C₁₋₆ fluoroalkyl, a C₃₋₈ cycloalkyl ring, phenyl, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄haloalkyl) or oxo;
each R⁸ is independently selected from hydrogen, C₁₋₆ alkyl, C₁₋₆ fluoroalkyl, a C₃₋₈ cycloalkyl ring, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄ haloalkyl) or oxo;
each R^{N} is independently selected from a phenyl ring, a monocyclic 5 or 6-membered heteroaryl ring, a monocyclic C₃₋₆ cycloaliphatic ring, or a monocyclic 4 to 6-membered heterocycle; wherein said monocyclic 5 or 6-membered heteroaryl ring or said monocyclic 4 to 6-membered heterocycle contain between 1 and 4 heteroatoms selected from N, O or S; wherein said monocyclic 5 or 6-membered heteroaryl ring is not a 1,3,5-triazinyl ring; and wherein said phenyl, said monocyclic 5 to 6-membered heteroaryl ring, said monocyclic C₃₋₆ cycloaliphatic ring, or said monocyclic 4 to 6-membered heterocycle is optionally and independently substituted with up to 6 instances of fluoro and/or up to 3 instances of J^{M};
each J^{M} is independently selected from -CN, a C₁₋₆ aliphatic, -OR^{M}, -SR^{M}, -N(R^{M})₂, a C₃₋₈ cycloaliphatic ring or a 4 to 8-membered heterocyclic ring; wherein said 4 to 8-membered heterocyclic ring contains 1 or 2 heteroatoms independently selected from N, O or S; wherein each said C₁₋₆ aliphatic, each said C₃₋₈ cycloaliphatic ring and each said 4 to 8-membered heterocyclic ring, is optionally and independently substituted with up to 3 instances of R^{7c}; and
each R^{M} is independently selected from hydrogen, a C₁₋₆ aliphatic, a C₃₋₈ cycloaliphatic ring or a 4 to 8-membered heterocyclic ring; wherein each said 4 to 8-membered heterocylic ring contains between 1 and 3 heteroatoms independently selected from O, N or S;
each R^{7c} is independently selected from halogen, -CN, -NO₂, C₁₋₄ alkyl, C₁₋₄ haloalkyl, C₃₋₈ cycloalkyl ring, -OR^{8b}, -SR^{8b}, -N(R^{8b})₂, -C(O)O(C₁₋₄ alkyl), -C(O)OH, -NR(CO)CO(C₁₋₄ alkyl) or an oxo group; wherein each said cycloalkyl group is optionally and independently substituted with up to 3 instances of halogen;
each R^{8b} is independently selected from hydrogen, C₁₋₆ alkyl, C₁₋₆ fluoroalkyl, a C₃₋₈ cycloalkyl ring, a 4 to 7-membered heterocyclic ring or a 5 or 6-membered heteroaryl ring; wherein each of said 5 or 6-membered heteroaryl ring or 4 to 7-membered heterocyclic ring contains up to 4 ring heteroatoms independently selected from N, O and S; and wherein each of said C₁₋₆ alkyl, each of said phenyl, each of said C₃₋₈ cycloalkyl group, each of said 4 to 7-membered heterocyclic ring and each of said 5 or 6-membered heteroaryl ring is optionally and independently substituted with up to 3 instances of halogen, C₁₋₄ alkyl, -OH, -NH₂, -NH(C₁₋₄ alkyl), -N(C₁₋₄ alkyl)₂, -CN, -COOH, -COO(C₁₋₄ alkyl), -O(C₁₋₄ alkyl), -O(C₁₋₄haloalkyl) or oxo;
wherein the heterocyclic ring described above is completely saturated or contains one or more units of unsaturation but is not aromatic;
provided that the compound is not one represented by the general structure: 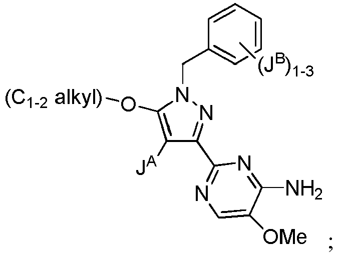
wherein J^{A} is either hydrogen or C₁₋₄ alkyl; and J^{B} is either halogen or C₁₋₄ (alkoxy).

2. The compound of claim 1, or a pharmaceutically acceptable salt thereof, wherein the compound is represented by **Formula IIa:** 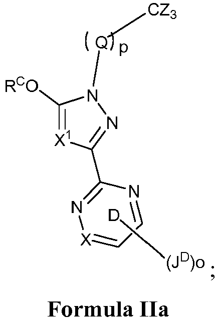 wherein Q represents a -CZ₂- group, each Z is independently selected from hydrogen or fluorine and p is an integer selected from 1, 2, 3, 4 and 5.

3. The compound according to claims 1 or 2, or a pharmaceutically acceptable salt thereof, represented by **Formula IIIa:** 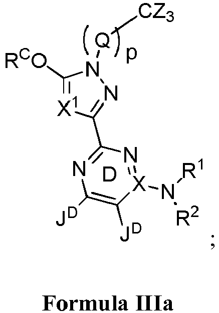 wherein,
when X is N, the moiety -N(R¹)(R²) is absent;
when X is C, the moiety -N(R¹)(R²) is present;
R¹ and R², together with the nitrogen atom to which they are attached, form a 4 to 8-membered heterocyclic ring or 5-membered heteroaryl ring; wherein said 4 to 8-membered heterocyclic ring or 5-membered heteroaryl ring optionally contains, in addition to the nitrogen atom to which both R¹ and R² are attached, up to 3 ring heteroatoms independently selected from N, O or S, and is optionally substituted by up to 5 instances of R^{5e};
each R^{5e} is independently selected from halogen, -CN, C₁₋₆ alkyl, -(C₁₋₄ alkyl)-R⁶, a C₃₋₈ cycloalkyl ring, C₁₋₄ (cyanoalkyl), -OR⁶, -SR⁶, -OCOR⁶, -COR⁶, -C(O)OR⁶, -C(O)N(R⁶)₂, -N(R⁶)C(O)R⁶, -N(R⁶)₂, -SO₂R⁶, -SO₂N(R⁶)₂, -N(R⁶)SO₂R⁶, -SO₂OH, -SO₂NHOH, -SO₂N(R⁶)(CO)-R⁶, benzyl, phenyl or an oxo group; wherein each said phenyl ring and each said benzyl group, is optionally and independently substituted with up to 3 instances of halogen, -OH, -NH₂, -NH(C₁₋₄ alkyl), -N(C₁₋₄ alkyl)₂, -CN, C₁₋₄ alkyl, C₁₋₄ haloalkyl, -CONH₂, -O(C₁₋₄ alkyl) or -O(C₁₋₄ haloalkyl); and wherein each said C₁₋₆ alkyl or C₁₋₄ alkyl chains and each said C₃₋₈ cycloalkyl ring is optionally and independently substituted with up to 3 instances of halogen; wherein
each R⁶ is independently selected from hydrogen, a C₁₋₆ alkyl, a C₂₋₄ alkenyl, phenyl, benzyl, or a C₃₋₈ cycloalkyl ring; wherein each said C₁₋₆ alkyl, each said C₂₋₄ alkenyl, each said phenyl, each said benzyl and each said C₃₋₈ cycloalkyl group is optionally and independently substituted with up to 3 instances of halogen;
alternatively, two of the instances of R^{5e} attached to the same or different atoms of said ring formed by R¹, R² and the nitrogen to which R¹ and R² are attached, together with said atom or atoms, optionally form a C₃₋₈ cycloalkyl ring, a 4 to 6-membered heterocyclic ring; a phenyl or a 5 or 6-membered heteroaryl ring, resulting in a bicyclic system wherein the two rings of the bicyclic system are in a spiro, fused or bridged relationship, wherein said 4 to 6-membered heterocycle or said 5 or 6-membered heteroaryl ring contains up to three ring heteroatoms independently selected from N, O or S; and wherein said C₃₋₈ cycloalkyl ring, 4 to 6-membered heterocyclic ring, phenyl or 5 or 6-membered heteroaryl ring is optionally and independently substituted by up to 3 instances of C₁₋₄ alkyl, C₁₋₄ haloalkyl, C₁₋₄ alkoxy, C₁₋₄ haloalkoxy, oxo, -C(O)O(C₁₋₄ alkyl), -CONH₂, -C(O)OH, -NR(CO)O(C₁₋₄ alkyl), -OH or halogen; wherein R is hydrogen or a C₁₋₂ alkyl;
alternatively, R¹ and R² are each independently selected from hydrogen, C₁₋₆ alkyl, a C₃₋₈ cycloalkyl ring, a 4 to 8-membered heterocyclic ring, a 5 or 6-membered heteroaryl ring, phenyl or a C₁₋₆ alkyl-R^{Y}; wherein each of said 4 to 8-membered heterocyclic ring and each of said 5 or 6-membered heteroaryl ring contains up to 3 ring heteroatoms independently selected from N, O and S; and wherein each of said C₁₋₆ alkyl, C₃₋₈ cycloalkyl ring, 4 to 8-membered heterocyclic ring, 5 or 6-membered heteroaryl ring, phenyl and the C₁₋₆ alkyl portion of the C₁₋₆ alkyl-R^{Y} moiety, is optionally and independently substituted with up to 5 instances of R^{5f};
R^{Y} is selected from a C₃₋₈ cycloalkyl ring, a 4 to 8-membered heterocyclic ring, phenyl, or a 5 to 6-membered heteroaryl ring; wherein each of said 4 to 8-membered heterocyclic ring or 5 to 6-membered heteroaromatic ring contains between 1 and 4 ring heteroatoms independently selected from N, O or S; and wherein each of said C₃₋₈ cycloalkyl ring, each of said 4 to 8-membered heterocyclic ring, each of said phenyl, and each of said 5 to 6-membered heteroaryl ring is optionally substituted with up to 5 instances of R^{5g};
each R^{5f} is independently selected from halogen, -CN, C₁₋₆ alkyl, -(C₁₋₄ alkyl)-R^{6a}, a C₇₋₁₂ aralkyl, C₃₋₈ cycloalkyl ring, C₁₋₄ (cyanoalkyl), -OR^{6a}, -SR^{6a}, -OCOR^{6a}, -COR^{6a}, -C(O)OR^{6a}, -C(O)N(R^{6a})₂, -N(R^{6a})C(O)R^{6a}, -N(R^{6a})₂, -SO₂OH, -SO₂NHOH, -SO₂N(R^{6a})(CO)-R^{6a}, -SO₂R^{6a}, -SO₂N(R^{6a})₂, -N(R^{6a})SO₂R^{6a}, phenyl or an oxo group; wherein each said phenyl group is optionally and independently substituted with up to 3 instances of halogen, -OH, -NH₂, -NH(C₁₋₄ alkyl), -N(C₁₋₄ alkyl)₂, -CN, -CONH₂, C₁₋₄ alkyl, C₁₋₄ haloalkyl, -O(C₁₋₄ alkyl) or -O(C₁₋₄ haloalkyl); and wherein each said C₇₋₁₂ aralkyl, each said C₁₋₆ alkyl, each said C₁₋₄ alkyl chain and each said C₃₋₈ cycloalkyl ring is optionally and independently substituted with up to three instances of halogen;
each R^{6a} is independently selected from hydrogen, a C₁₋₆ alkyl, a C₂₋₄ alkenyl, phenyl, benzyl, or a C₃₋₈ cycloalkyl ring; wherein each said C₁₋₆ alkyl, each said C₂₋₄ alkenyl, each said phenyl, each said benzyl and each said C₃₋₈ cycloalkyl ring is optionally and independently substituted with up to 3 instances of halogen;
when one of R¹ or R² is the C₃₋₈ cycloalkyl ring, 4 to 8-membered heterocyclic ring or 5 or 6-membered heteroaryl substituted with up to 5 instances of R^{5f}, two of the instances of R^{5f} attached to the same or different ring atoms of said R¹ or R², together with said atom or atoms, form a C₃₋₈ cycloalkyl ring, a 4 to 6-membered heterocyclic ring, a phenyl or a 5 or 6-membered heterocyclic ring, resulting in a bicyclic system wherein the two rings are in a spiro, fused or bridged relationship, wherein said 4 to 6-membered heterocycle or said 5 or 6-membered heterocyclic ring contains up to two ring heteroatoms independently selected from N, O or S; and wherein said C₃₋₈ cycloalkyl ring, 4 to 6-membered heterocyclic ring, phenyl or 5 or 6-membered heterocyclic ring is optionally substituted by up to 2 instances of C₁₋₄ alkyl, C₁₋₄haloalkyl, oxo, -(CO)O(C₁₋₄ alkyl), -NR'(CO)O(C₁₋₄ alkyl) or halogen; wherein R' is hydrogen or a C₁₋₂ alkyl;
each R^{5g} is independently selected from halogen, -CN, C₁₋₆ alkyl, -(C₁₋₄alkyl)-R^{6b}, a benzyl, C₃₋₈ cycloalkyl ring, C₁₋₄(cyanoalkyl), -OR^{6b}, -SR^{6b}, -OCOR^{6b}, -COR^{6b}, -C(O)OR^{6b}, -C(O)N(R^{6b})₂, -N(R^{6b})C(O)R^{6b}, -N(R^{6b})₂, -SO₂R^{6b}, -SO₂OH, -SO₂NHOH, -SO₂N(R^{6b})(CO)-R^{6b}, -SO₂N(R^{6b})₂, -N(R^{6b})SO₂R^{6b}, phenyl or an oxo group; wherein each said phenyl and each said benzyl group is optionally and independently substituted with up to 3 instances of halogen, -OH, -NH₂, -NH(C₁₋₄ alkyl), -N(C₁₋₄alkyl)₂, -CN, -CONH₂, C₁₋₄ alkyl, C₁₋₄haloalkyl, -O(C₁₋₄ alkyl) or -O(C₁₋₄haloalkyl); and wherein each said C₁₋₄ alkyl chain and each said C₃₋₈ cycloalkyl group is optionally and independently substituted with up to 3 instances of halogen;
each R^{6b} is independently selected from hydrogen, a C₁₋₆alkyl, a C₂₋₄ alkenyl, phenyl, benzyl, or a C₃₋₈ cycloalkyl ring; wherein each said C₁₋₆ alkyl, each said C₂₋₄ alkenyl, each said phenyl, each said benzyl and each said C₃₋₈ cycloalkyl group is optionally and independently substituted with up to 3 instances of halogen;
alternatively, two instances of R^{5g} attached to the same or different ring atoms of R^{Y}, together with said ring atom or atoms, form a C₃₋₈ cycloalkyl ring, a 4 to 6-membered heterocyclic ring; a phenyl or a 5 or 6-membered heteroaryl ring, resulting in a bicyclic system wherein the two rings are in a spiro, fused or bridged relationship, wherein said 4 to 6-membered heterocycle or said 5 or 6-membered heteroaryl ring contains up to three heteroatoms independently selected from N, O or S; and wherein said C₃₋₈ cycloalkyl ring, 4 to 6-membered heterocyclic ring, phenyl or a 5 or 6-membered heteroaryl ring is optionally and independently substituted by up to 3 instances of C₁₋₄ alkyl, C₁₋₄ haloalkyl, C₁₋₄ alkoxy, C₁₋₄haloalkoxy, oxo, -C(O)O(C₁₋₄ alkyl), -C(O)OH, -NR"(CO)O(C₁₋₄ alkyl), -OH or halogen; and
R" is hydrogen or a C₁₋₂ alkyl.

4. The compound according to claim 1, represented by **Formula IIb**, or a pharmaceutically acceptable salt thereof: 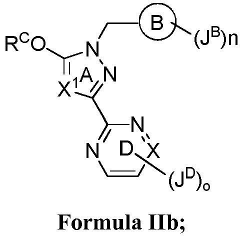 wherein, ring B is a phenyl or a 5 or 6-membered heteroaryl ring, containing 1 or 2 ring heteroatoms selected from N, O or S.

5. The compound of claim 4, or a pharmaceutically acceptable salt thereof, wherein each J^{D} is independently selected from halogen, a C₁₋₆ aliphatic, C₁₋₆ haloaliphatic, -N(R^{D})₂, -N(R^{d})COR^{D}, -N(R^{d})COOR^{D}, -OR^{D}, -N(R^{d})SO₂R^{D},or an optionally substituted C₃₋₈ cycloaliphatic ring.

6. The compound of claims 4 or 5, or a pharmaceutically acceptable salt thereof, wherein the compound is represented by **Formula IIIb:**

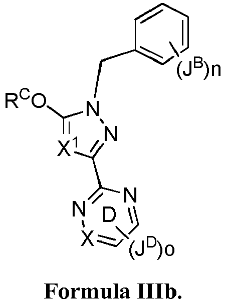

7. The compound according to claims 4 or 6, or a pharmaceutically acceptable salt thereof, represented by **Formula IVb:**

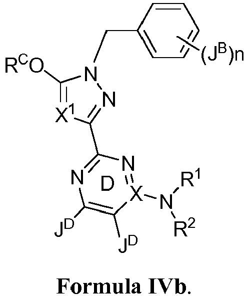

8. The compound according to any one of claims 4 to 6 , or a pharmaceutically acceptable salt thereof, represented by **Formula Vb:** 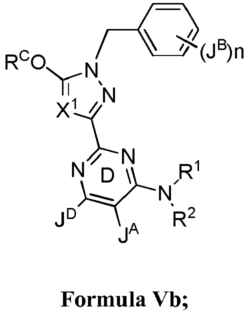
wherein, J^{D} is absent or selected from halogen, methyl, hydroxyl, methoxy, trifluoromethyl, trifluoromethoxy or -NR^{a}R^{b}; wherein R^{a} and R^{b} are each independently selected from hydrogen, C₁₋₆ alkyl or a 3-6 cycloalkyl ring; or wherein R^{a} and R^{b}, together with the nitrogen atom to which they are both attached, form a 4-8 membered heterocyclic ring, or a 5-membered heteroaryl ring optionally containing up to two additional heteroatoms selected from N, O and S; wherein each of said 4-8 membered heterocyclic ring and 5-membered heteroaryl ring is optionally and independently substituted by up to 5 instances of fluorine;
and J^{A} is selected from hydrogen or fluorine.

9. The compound of any one of claims 1, 2, 4 and 6, or a pharmaceutically acceptable salt thereof, wherein each J^{D} is independently selected from halogen, a C₁₋₆ aliphatic, a C₁₋₆ haloaliphatic -N(R^{D})₂, -N(R^{d})C(O)R^{D}, -N(R^{d})C(O)OR^{D}, -N(R^{d})C(O)N(R^{D})₂, -SO₂R^{D}, -SO₂N(R^{D})₂, -N(R^{d})SO₂R^{D}, -SR^{D}, -OR^{D} or an optionally substituted C₃₋₈ cycloaliphatic ring, and wherein o is an integer selected between 1 and 3.

10. The compound of claim 9, or a pharmaceutically acceptable salt thereof, wherein each J^{D} is independently selected from methyl, trifluoromethyl, chloro, fluoro, -N(R^{D})₂, N(R^{d})C(O)R^{D}, -N(R^{d})SO₂R^{D} or -OR^{D}.

11. The compound according to claim 1, or a pharmaceutically acceptable salt thereof, represented by one of **Formulae VIIa** or **VIIIb:**

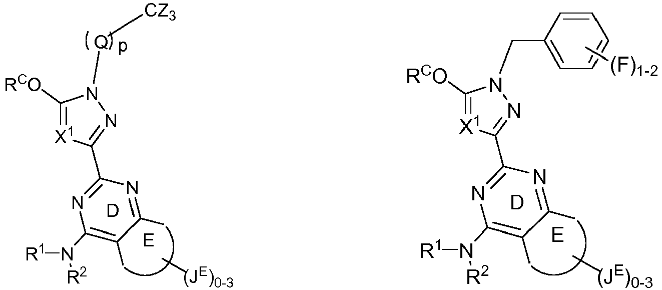

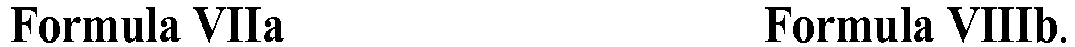

12. The compound according to any one of claims 1, 4, or 6, or a pharmaceutically acceptable salt thereof, represented by one of **Formulae Xb or XIb:** 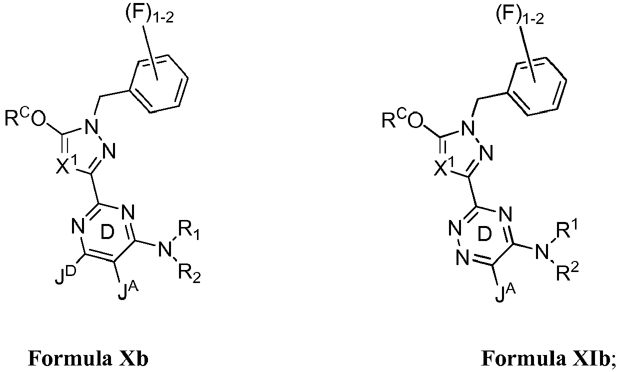
wherein each J^{D} is independently selected from -NH₂ or is absent; and
each J^{A} is alternatively:
i) when R¹ and R² are not simultaneously hydrogen, each J^{A} is independently selected from hydrogen or halogen; or
ii) when R¹ and R² are both simultaneously hydrogen, each J^{A} is independently selected from -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂ or -N(R^{d})SO₂R^{D}

13. The compound according to either of claims 1 or 2, or a pharmaceutically acceptable salt thereof, represented by one of **Formulae XIXa or Xa:** 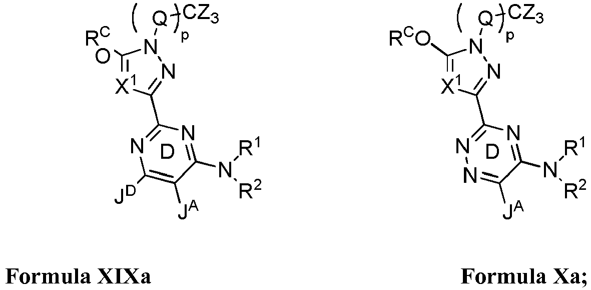 wherein
each J^{D} is independently selected from -NH₂ or is absent; and
each J^{A} is alternatively:
i) when R¹ and R² are not simultaneously hydrogen, each J^{A} is independently selected from hydrogen or halogen; or
ii) when R¹ and R² are both simultaneously hydrogen, each J^{A} is independently selected from -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂ or -N(R^{d})SO₂R^{D}

14. A compound according to claim 1, wherein the compound is selected from any of the following compounds: 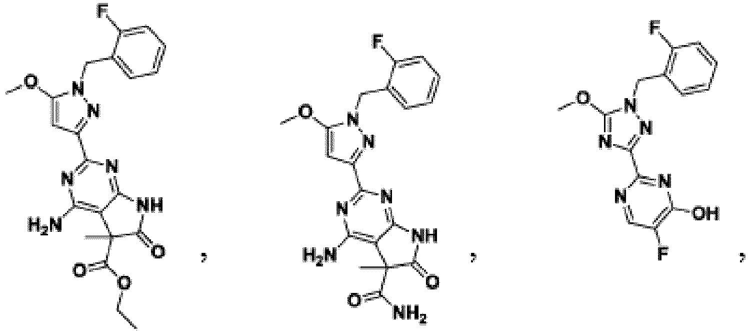 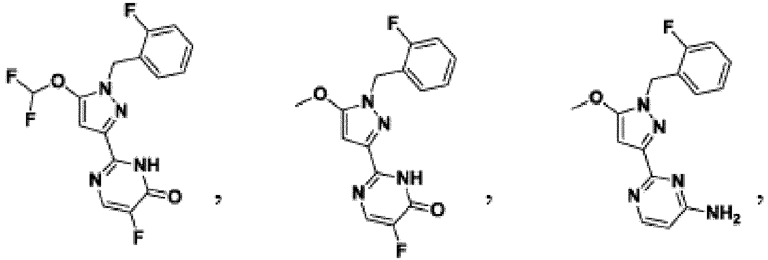 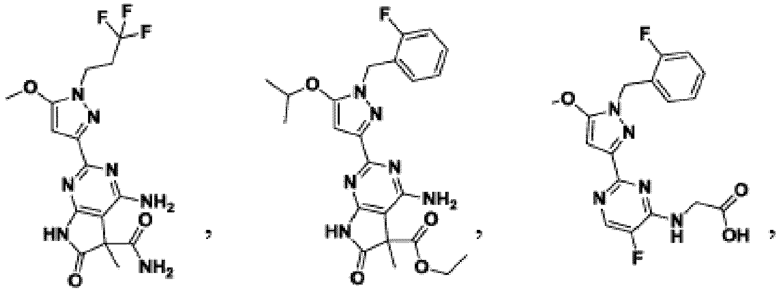 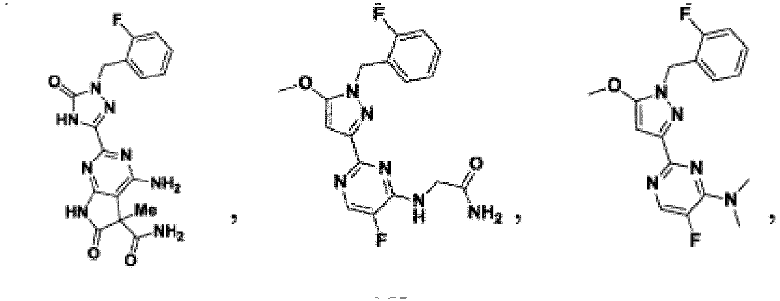 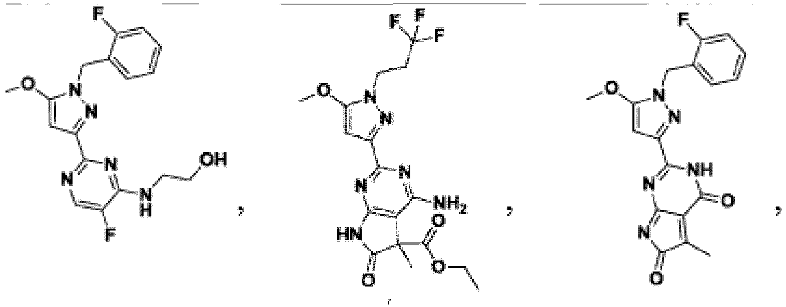 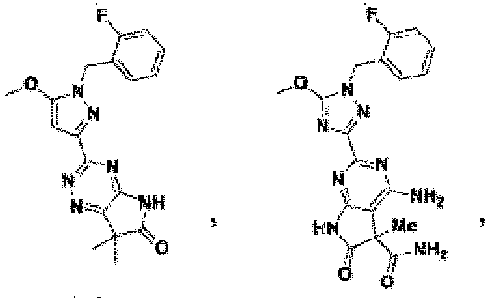 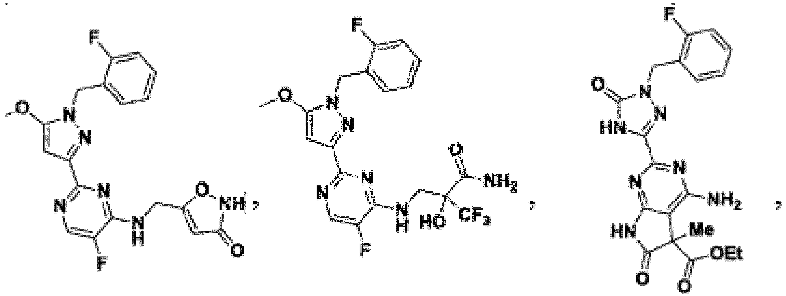 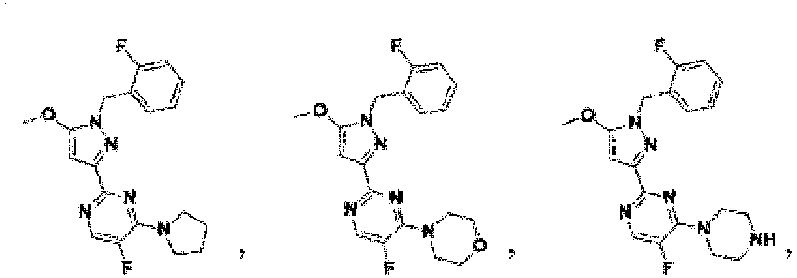 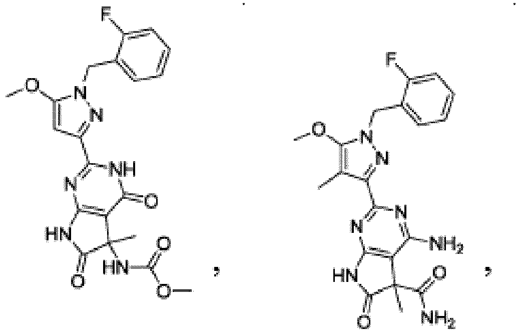 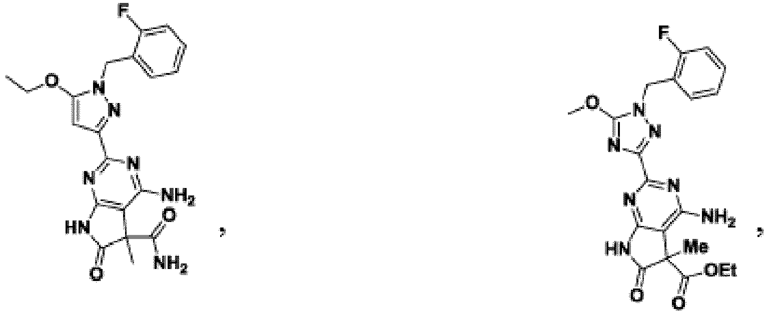 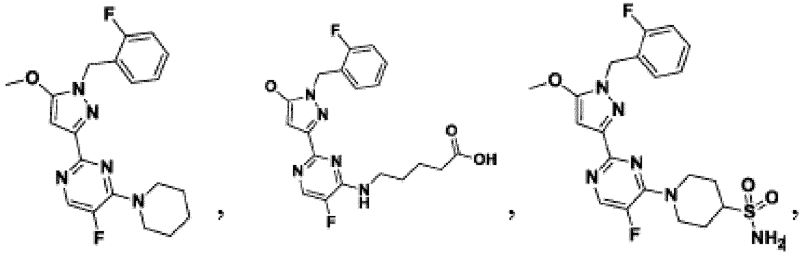 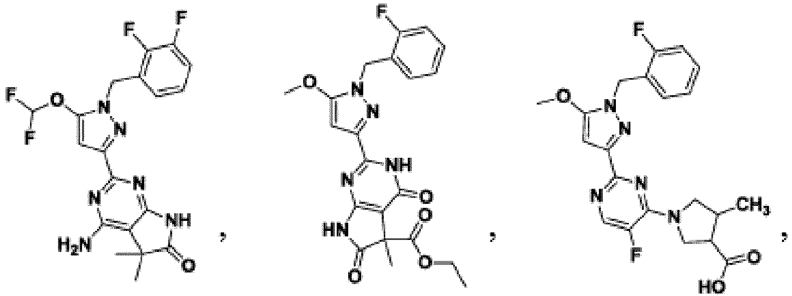 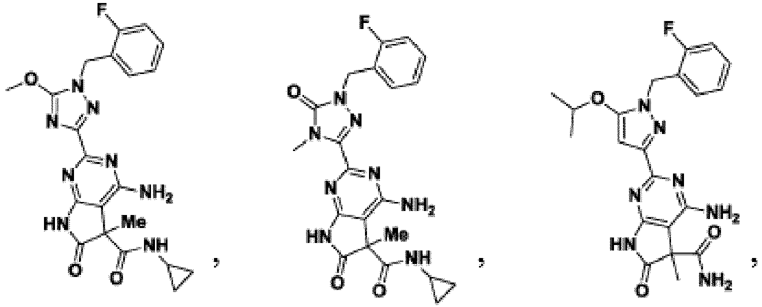 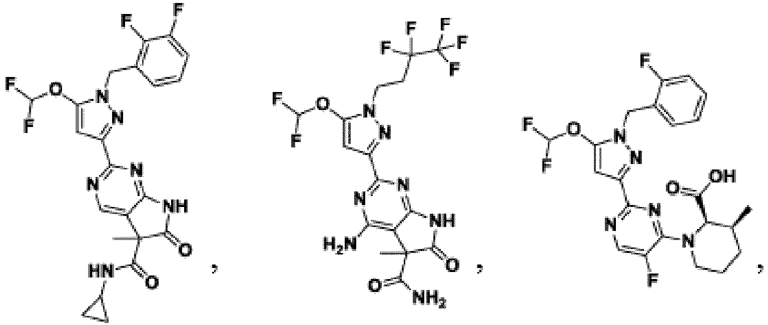 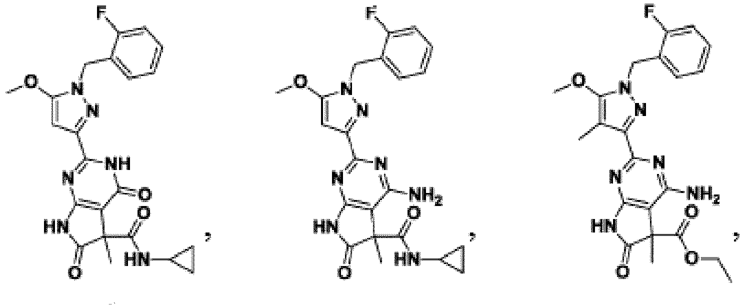 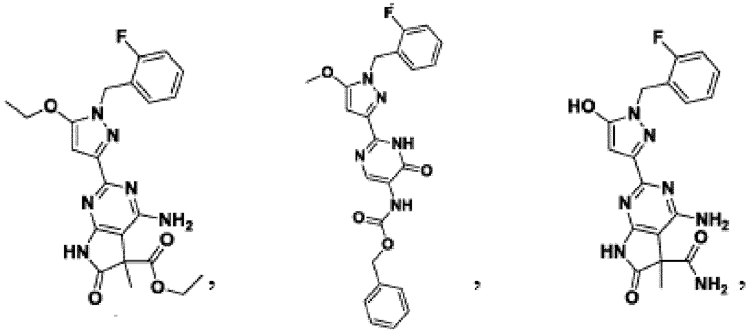 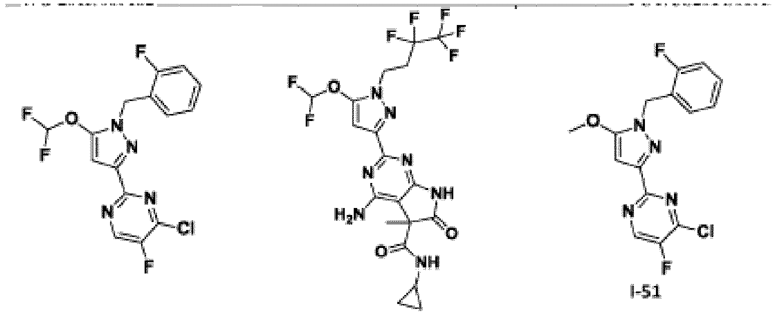 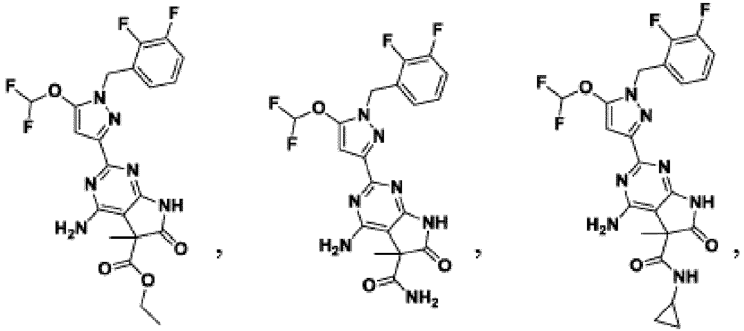 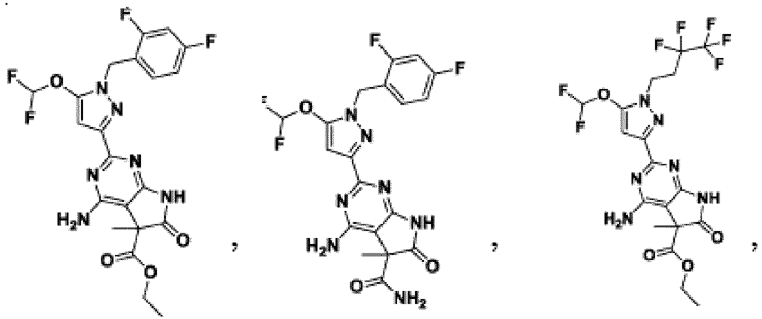 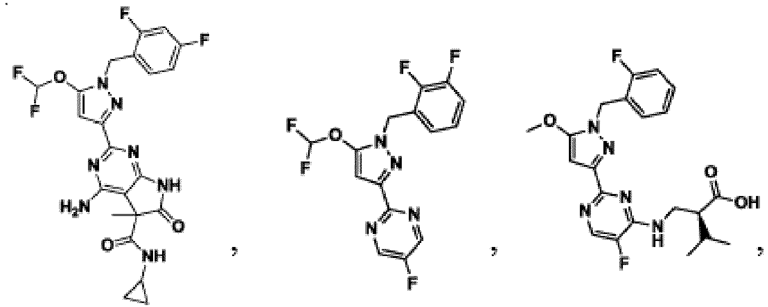 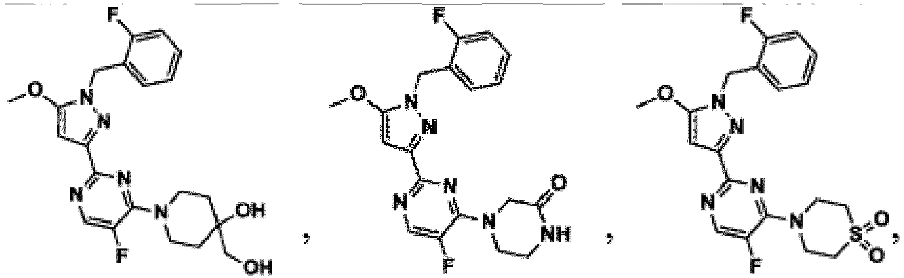 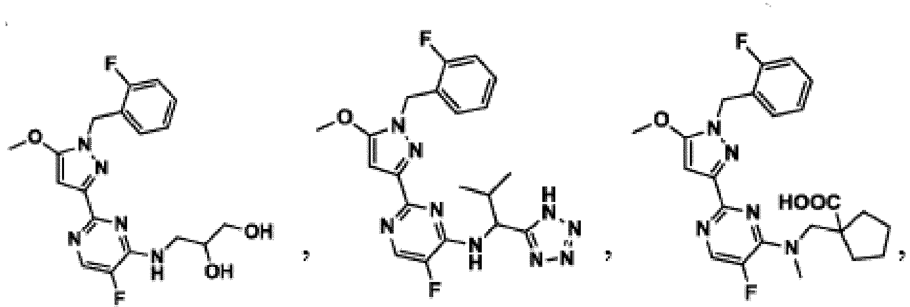 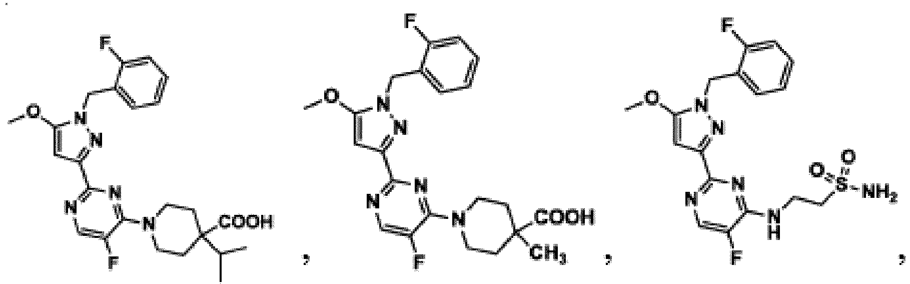 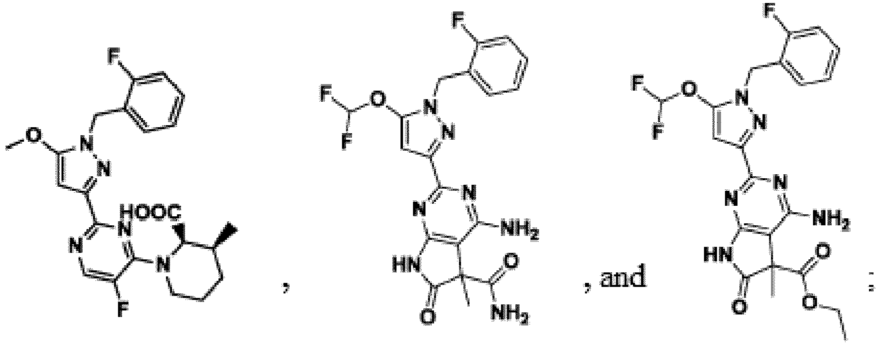 or a pharmaceutically acceptable salt thereof.

15. The compound of any one of claims 1 to 14 for use in the treatment of a disease, health condition or disorder selected from:
• disorders related to high blood pressure and decreased coronary blood flow; increased acute and chronic coronary blood pressure; arterial hypertension and vascular disorder resulting from cardiac and renal complications; arterial hypertension and vascular disorder resulting from heart disease, stroke, cerebral ischemis, renal failure orresistant hypertension; diabetic hypertension; essential hypertension; secondary hypertension;
• heart failure; HFPEF; HFREF; acute and chronic HF, and more specific forms of the disease; acute decompensated HF, right ventricular failure, left ventricular failure, total HF, ischemic cardiomyopathy, dilatated cardiomyopathy, congenital heart defects, HF with valvular defects, mitral valve stenosis, mitral valve insufficiency, aortic valve stenosis, aortic valve insufficiency, tricuspid stenosis, tricuspic insufficiency, pulmonary valve stenosis, pulmonar valve insufficiency, combined valvular defects; diabetic heart failure; alcoholic cardiomyopathy, storage cardiomyopathies; diastolic HF, systolic HF, acute phases of an existing chronic HF, worsening HF; diastolic or systolic dysfunction; coronary insufficiency; arrhythmias; reduction of ventricular preload; cardiac hypertrophy; heart failure/cardiorenal syndrome; portal hypertension; endothelial dysfunction or injury; disturbances of atrial and ventricular rhythm and conduction disturbances; atrioventricular blocks of degree I-III (AVB I-III); supraventricular tachyarrhythmia, atrial fibrillation, atrial flutter, ventricular fibrillation, ventricular flutter, ventricular tachyarrhythmia, torsade-de-pointes tachycardia, atrial and ventricular extrasystoles, AV-junction extrasystoles, sick-sinus syndrome, syncopes, AV-node reentry tachycardia; Wolff-Parkinson-White syndrome, acute coronary syndrome; Boxer cardiomyopathy; premature ventricular contraction;
• thromboembolic disorders and ischemias, myocardial ischemia, infarction, heart attack, myocardial insufficiency, endothelial dysfunction, stroke, transient ischemic attacks (TIAs); obstructive thromboanginitis; stable or unstable angina pectoris; coronary spasms, spasms of the peripheral arteries; variant angina, Prinzmetal's angina; stroke; cardiac hypertrophy; preeclampsia; thrombogenic disorders; ischemia-reperfusion damage; ischemia-reperfusion associated with organ transplant; ischemia-reperfusion associated with lung transplant, pulmonary transplant or cardiac transplant; conserving blood substituents in trauma patients;
• peripheral arterial disease; peripheral occlusive arterial disease; peripheral vascular disease; hypertonia; Raynaud's syndrome or Raynaud's phenomenon; primary and secondary Raynaud's phenomena; Raynaud's disease, critical limb ischemia; peripheral embolism; intermittent claudication; vaso-occlusive crisis; Duchenne and Becker muscular dystrophies; microcirculation abnormalities; control of vascular leakage or permeability; lumbar spinal canal stenosis; occlusive thrombotic vasculitis; thrombotic vasculitis; peripheral perfusion disturbances; arterial and venous thromboses; microalbuminuria; peripheral and autonomic neuropathies; diabetic microangiopathies;
• edema; renal edema due to heart failure;
• Alzheimer's disease; Parkinson's disease; vascular dementias; vascular cognitive impairment; cerebral vasospasm; traumatic brain injury; improving perception, capacity for concentration, capacity for learning or memory performance after cognitive disturbances such as those occurring in mild cognitive impairment, age-related learning and memory disturbances, age-related memory loss, vascular dementia, head injury, stroke, post-stroke dementia, post-traumatic head injury, general disturbances of concentration and disturbances of concentration in children with learning and memory problems; Lewy body dementia, dementia with frontal lobe degeneration including Pick's syndrome; progressive nuclear palsy; dementia with corticobasal degeneration; Amyotropic Lateral Sclerosis (ALS); Huntington's disease; demyelination, Multiple Sclerosis, thalamic degeneration; Creutzfeldt-Jakob dementia, HIV-dementia, schizophrenia with dementia or Korsakoff psychosis; Multiple Systems atrophy and other forms of Parkinsonism Plus; movement disorders; neuroprotection; anxiety, tension and depression, post-traumatic stress disorder (PTSD); CNS-related sexual dysfunction and sleep disturbances; pathological eating disorders and use of luxury foods and addictive drugs; controlling cerebral perfusion, controlling migraines; prophylaxis and control of consequences of cerebral infarction (apoplexia cerebri) such as stroke, cerebral ischemias and head injury;
• shock; cardiogenic shock; sepsis or septic shock or anaphylactic shock; aneurysm; control of leukocyte activation; inhibition or modulation of platelet aggregation; multiple organ failure (MODS, MOF);
• pulmonary/respiratory conditions; pulmonary hypertension (PH), pulmonary arterial hypertension (PAH), and associated pulmonary vascular remodeling; localized thrombosis and right heart hypertrophy; pulmonary hypertonia; primary pulmonary hypertension, secondary pulmonary hypertension, familial pulmonary hypertension, sporadic pulmonary hypertension, pre-capillary pulmonary hypertension, idiopathic pulmonary hypertension; thrombotic pulmonary arteriopathy, plexogenic pulmonary arteriopathy; cystic fibrosis; bronchoconstriction or pulmonary bronchoconstriction; acute respiratory distress syndrome; lung fibrosis, lung transplant; asthmatic diseases; other forms of PH; PH associated with left ventricular disease, HIV, SCD, thromboembolism (CTEPH), sarcoidosis, COPD or pulmonary fibrosis; acute respiratory distress syndrome (ARDS), acute lung injury, alpha-1-antitrypsin deficiency (AATD), pulmonary emphysema; smoking-induced emphysema and CF;
• pulmonary hypertension associated with or related to: left ventricular dysfunction, hypoxemia, WHO groups I, II, III, IV and V hypertensions, mitral valve disease, constrictive pericarditis, aortic stenosis, cardiomyopathy, mediastinal fibrosis, pulmonary fibrosis, anomalous pulmonary venous drainage, pulmonary veno-occlusive disease, pulmonary vasculitis, collagen vascular disease, congenital heart disease, pulmonary venous hypertension, interstitial lung disease, sleep-disordered breathing, sleep apnea, alveolar hypoventilation disorders, chronic exposure to high altitude, neonatal lung disease, alveolar-capillary dysplasia, sickle cell disease, other coagulation disorders, chronic thromboembolism, pulmonary embolism, embolism due to tumor, parasites or foreign material; pulmonary hypertension associated or related to: connective tissue disease, lupus, schistosomiasis, sarcoidosis, chronic obstructive pulmonary disease, asthma, emphysema, chronic bronchitis or pulmonary capillary hemangiomatosis; histiocytosis X, lymphangiomatosis and compressed pulmonary vessels; compressed vessels due to adenopathy, tumor or fibrosing mediastinitis;
• arterosclerotic diseases or conditions; atherosclerosis, atherosclerosis associated with endothelial injury, platelet and monocyte adhesion and aggregation, smooth muscle proliferation and migration; restenosis; restenosis developed after thrombolysis therapies, percutaneous transluminal angioplasties (PTAs), transluminal coronary angioplasties (PTCAs), heart transplant and bypass operations; inflammatory processes;
• micro and macrovascular damage; vasculitis; increased levels of fibrinogen and low density DLD, increased concentration of plasminogen activator inhibitor 1 (PA-1);
• diseases associated with metabolic syndrome: obesity, dyslipidemia, diabetes, high blood pressure; lipid related disorders: dyslipidemia, hypercholesterolemias, decreased high-density lipoprotein cholesterol (HDL-cholesterol) and in some cases moderately elevated low-density lipoprotein cholesterol (LDL-cholesterol) levels, hypertriglyceridemias, hyperglyceridemia, hypolipoproteinanemias, sitosterolemia, fatty liver disease, and hepatitis; preeclampsia; polycystic kidney disease progression; subcutaneous fat; obesity; liver steatosis or abnormal lipid accumulation in the liver; steatosis of the heart, kidneys or muscle; abetalipoproteinemia; sitosterolemia; xanthomatosis; Tangier disease; adiposity; combined hyperlipidemias and metabolic syndrome; hyperammonemia and related diseases and disorders; hepatic encephalopaties and other toxic encephalopaties and Reye syndrome;
• sexual, gynecological and urological disorders of conditions; erectile dysfunction; impotence; premature ejaculation; female sexual dysfunction; female sexual arousal dysfunction, hypoactive sexual arousal disorder, vaginal atrophy, dyspaneuria, atrophic vaginitis; benign prostatic hyperplasia (BPH) or hypertrophy or enlargement; bladder outlet obstruction; bladder pain syndrome (BPS); interstitial cystitis (IC); overactive bladder; neurogenic bladder and incontinence; diabetic nephropathy; primary and secondary dysmenhorrea; lower urinary tract syndromes (LUTS); pelvic pains; benign and malignant diseases of the organs of the male and female urogenital system;
• acute and chronic renal insufficiency, acute and chronic renal failure, as well as underlying or related kidney diseases such as hypoperfusion, intradialytic hypotension, obstructive uropathy, glomerulopathies, glomerulonephritis, acute glomerulonephritis, glomerulosclerosis, tubulointerstitial diseases, nephropathic diseases; primary and congenital kidney diseases; nephritis; diseases **characterized by** abnormally reduced creatinine and or water excretion, abnormally increased blood concentrations of urea, nitrogen, potassium and/or creatinine, altered activity of renal enzymes, altered urine osmolarity or urine volume, increased microalbuminuria, macroalbuminuria, lesions of glomeruli and arterioles, tubular dilatation, hyperphosphatemia and/or need for dialysis; sequelae of renal insufficiency; pulmonary enema, HF, uremia, anemia, elecrolyte disturbances; herkalemia, hyponatremia; disturbances of bone and carbohydrate metabolism;
• ocular diseases or disorders; glaucoma, retinopathy or diabetic retinopathy.

## Patentansprüche

1. Verbindung nach **Formel I,** oder ein oder ein pharmazeutisch annehmbares Salz davon, 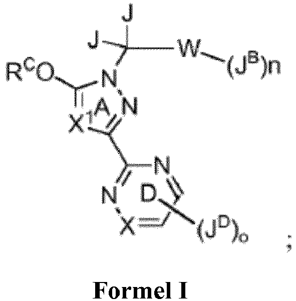
wobei X ausgewählt ist aus N oder C;
X¹ ausgewählt ist aus N, CH, C(C₁₋₄-Alkyl), C(C₁₋₄-Fluoralkyl), C(Cl) und CF;
W entweder wie folgt ist
i) es fehlt, wobei J^{B} direkt mit den zwei Kohlenstoffatom-tragenden J-Gruppen verbunden ist, jedes J unabhängig ausgewählt ist aus Wasserstoff, Methyl oder Fluor, n 1 ist und J^{B} eine C₁₋₆-Alkyl-Kette ist, optional substituiert durch bis zu 6 Vorkommen von Fluor; oder
ii) ein Ring B ausgewählt aus Phenyl oder ein 5- oder 6-gliedriger Heteroaryl-Ring, der 1 oder 2 Ring-Heteroatome enthält, ausgewählt aus N, O oder S; wobei, wenn W Ring B ist:
jedes J Wasserstoff ist;
n 0 oder eine Ganzzahl ausgewählt aus 1 bis 3 ist;
und jedes J^{B} unabhängig ausgewählt ist aus Halogen, -CN, einer C₁₋₆ aliphatischen, -OR^{B} oder einer C₃₋₈ cycloaliphatischen Gruppe; wobei jede der C₁₋₆ aliphatischen und jede der C₃₋₈ cycloaliphatischen Gruppe optional und unabhängig substituiert ist mit bis zu 3 Vorkommen von R³;
jedes R^{B} unabhängig ausgewählt ist aus Wasserstoff, einem C₁₋₆ aliphatischen oder einem C₃₋₈ cycloaliphatischen; wobei jedes von dem R^{B}, das ein C₁₋₆ aliphatisches ist, und jedes von dem R^{B}, das ein C₃₋₈ cycloaliphatischer Ring ist, optional und unabhängig substituiert ist mit bis zu 3 Vorkommen von R^{3a};
jedes R³ unabhängig ausgewählt ist aus Halogen, -CN, C₁₋₄-Alkyl, C₁₋₄-Haloalkyl, -O(C₁₋₄-Alkyl) oder -O(C₁₋₄-Haloalkyl);
jedes R^{3a} unabhängig ausgewählt ist aus Halogen, -CN, C₁₋₄-Alkyl, C₁₋₄-Haloalkyl, -O(C₁₋₄-Alkyl) oder -O(C₁₋₄-Haloalkyl);
o 0 oder eine Ganzzahl ausgewählt aus 1 bis 3 ist;
jedes J^{D} entweder fehlt oder unabhängig ausgewählt ist aus Wasserstoff, Halogen, -CN, -NO₂, -OR^{D}, -SR^{D}, -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂, -N(R^{d})SO₂R^{D}, einem C₁₋₆ aliphatischen, -(C₁₋₆ aliphatischen)-R^{D}, einem C₃₋₈ cycloaliphatischen Ring, einem 6- bis 10-gliedrigen Aryl-Ring, einem 4- bis 8-gliedrigen heterocyclischen Ring oder einem 5- bis 10-gliedrigen Heteroaryl-Ring; wobei jedes von dem 4- bis 8-gliedrigen heterocyclischen Ring und jedes von dem 5- bis 10-gliedrigen Heteroaryl-Ring zwischen 1 und 3 Heteroatome enthält, unabhängig ausgewählt aus O, N oder S; und wobei jedes von den C₁₋₆ aliphatischen Ketten, jedes von dem C₃₋₈ cycloaliphatischen Ring, jedes von dem 6- bis 10-gliedrigen Aryl-Ring, jedes von dem 4- bis 8-gliedrigen heterocyclischen Ring und jedes von dem 5- bis 10-gliedrigen Heteroaryl-Ring optional und unabhängig substituiert ist mit bis zu 5 Vorkommen von R⁵;
jedes R^{D} unabhängig ausgewählt ist aus Wasserstoff, einem C₁₋₆ aliphatischen, -(C₁₋₆ aliphatischen)-R^{f}, einem C₃₋₈ cycloaliphatischen Ring, einem 4- bis 8-gliedrigen heterocyclischen Ring und Phenyl; wobei jedes von dem 4- bis 8-gliedrigen heterocyclischen Ring zwischen 1 und 3 Heteroatome enthält, unabhängig ausgewählt aus O, N und S; und wobei jede von den C₁₋₆ aliphatischen Ketten, jedes von dem C₃₋₈ cycloaliphatischen Ring, jedes von dem 4- bis 8-gliedrigen heterocyclischen Ring und jedes von dem Phenyl optional und unabhängig substituiert ist mit bis zu 5 Vorkommen von R^{5a}; wobei, wenn ein R^{D} eine von einer C₁₋₆ aliphatischen oder einer -(C₁₋₆ aliphatischen)-R^{f} Gruppe ist, eine oder zwei Einheiten -CH₂-, die die C₁₋₆ aliphatischen Ketten bilden, optional durch eine Gruppe ersetzt sein kann, unabhängig ausgewählt aus -C(O)-, -N(R^{d}) - oder -O-;
jedes R^{d} unabhängig ausgewählt ist aus Wasserstoff, einem C₁₋₆ aliphatischen, -(C₁₋₆ aliphatischen)-R^{f}, einem C₃₋₈ cycloaliphatischen Ring, einem 4- bis 8-gliedrigen heterocyclischen Ring, Phenyl oder einem 5 bis 6-gliedrigen Heteroaryl-Ring; wobei jedes von dem 4- bis 8-gliedrigen heterocyclischen Ring und jedes von dem 5- oder 6-gliedriger Heteroaryl-Ring zwischen 1 und 3 Heteroatome enthält, unabhängig ausgewählt aus O, N oder S; und wobei jede von den C₁₋₆ aliphatischen Ketten, jedes von dem C₃₋₈ cycloaliphatischen Ring, jedes von dem 4-bis 8-gliedrigen heterocyclischen Ring, jedes von dem Phenyl und jedes von dem 5 bis 6-gliedrigen Heteroaryl-Ring optional und unabhängig substituiert ist mit bis zu 5 Vorkommen von R^{5b};
jedes R^{f} unabhängig ausgewählt ist aus einem C₁₋₃-Alkyl, einem C₃₋₈ cycloaliphatischen Ring, einem 4- bis 8-gliedrigen heterocyclischen Ring, Phenyl oder einem 5 bis 6-gliedrigen Heteroaryl-Ring; wobei jedes von dem 4- bis 8-gliedrigen heterocyclischen Ring und jedes von dem 5 bis 6-gliedrigen Heteroaryl-Ring zwischen 1 und 4 Heteroatome enthält, unabhängig ausgewählt aus O, N oder S; und wobei jedes von dem C₃₋₈ cycloaliphatischen Ring, jedes von dem 4- bis 8-gliedrigen heterocyclischen Ring, jedes von dem Phenyl und jedes von dem 5 bis 6-gliedrigen Heteroaryl-Ring optional und unabhängig substituiert ist mit bis zu 5 Vorkommen von R^{5c};
wenn J^{D} -C(O)N(R^{D})₂, -N(R^{D})₂, -N(R^{d})C(O)N(R^{D})₂, -OC(O)N(R^{D})₂ oder -SO₂N(R^{D})₂ ist, können die zwei R^{D}-Gruppen zusammen mit dem Stickstoffatom, das an die zwei R^{D}-Gruppen angelagert ist, einen 4- bis 8-gliedrigen heterocyclischen Ring oder einen 5-gliedrigen Heteroaryl-Ring bilden; wobei jedes von dem 4- bis 8-gliedrigen heterocyclischen Ring und jedes von dem 5-gliedriger Heteroaryl-Ring optional bis zu 3 zusätzliche Heteroatome enthält, unabhängig ausgewählt aus N, O oder S, zusätzlich zu dem Stickstoffatom, an das die zwei R^{D}-Gruppen angelagert sind; und wobei jedes von dem 4- bis 8-gliedrigen heterocyclischen Ring und jedes von dem 5-gliedriger Heteroaryl-Ring optional und unabhängig substituiert ist mit bis zu 5 Vorkommen von R^{5d};
wenn J^{D} -N(R^{d})C(O)R^{D} ist, die R^{D}-Gruppe zusammen mit dem Kohlenstoffatom, das an die R^{D}-Gruppe angelagert ist, mit dem Stickstoffatom, das an die R^{d}-Gruppe angelagert ist, und mit der R^{d}-Gruppe einen 4- bis 8-gliedrigen heterocyclischen Ring oder einen 5-gliedrigen Heteroaryl-Ring bilden kann; wobei jedes von dem 4- bis 8-gliedrigen heterocyclischen Ring und jedes von dem 5-gliedriger Heteroaryl-Ring optional bis zu 2 zusätzliche Heteroatome enthält, unabhängig ausgewählt aus N, O oder S, zusätzlich zu dem Stickstoffatom, an das die R^{d}-Gruppe angelagert ist; und wobei jedes von dem 4- bis 8-gliedrigen heterocyclischen Ring und jedes von dem 5-gliedriger Heteroaryl-Ring optional und unabhängig substituiert ist mit bis zu 5 Vorkommen von R^{5d};
wenn J^{D} -N(R^{d})C(O)OR^{D} ist, die R^{D}-Gruppe zusammen mit dem Sauerstoffatom, das an die R^{D}-Gruppe angelagert ist, mit dem Kohlenstoffatom des Abschnitts -C(O)- der Gruppe -N(R^{d})C(O)OR^{D} Gruppe, wobei das Stickstoffatom an die R^{d}-Gruppe angelagert ist, mit der R^{d}-Gruppe einen 4- bis 8-gliedrigen heterocyclischen Ring bilden kann; wobei der 4- bis 8-gliedrige heterocyclischen Ring optional bis zu 2 zusätzliche Heteroatome enthält, unabhängig ausgewählt aus N, O oder S und optional und unabhängig substituiert ist mit bis zu 5 Vorkommen von R^{5d};
wenn J^{D} -N(R^{d})C(O)N(R^{D})₂ ist, eine der R^{D}-Gruppen, die an das Stickstoffatom angelagert sind, zusammen mit dem Stickstoffatom und mit dem N-Atom, die an die R^{d}-Gruppe angelagert sind, und die R^{d}-Gruppe einen 4- bis 8-gliedrigen heterocyclischen Ring bilden kann; wobei der 4-bis 8-gliedrige heterocyclische Ring optional bis zu 2 zusätzliche Heteroatome enthält, unabhängig ausgewählt aus N, O oder S und optional und unabhängig substituiert ist mit bis zu 5 Vorkommen von R^{5d};
wenn J^{D} -N(R^{d})SO₂R^{D} ist, die R^{D}-Gruppe zusammen mit dem Schwefelatom, das an die R^{D}-Gruppe angelagert ist, mit dem Stickstoffatom, das an die R^{d}-Gruppe angelagert ist und mit der R^{d}-Gruppe kombinieren kann, um einen 4- bis 8-gliedrigen heterocyclischen Ring zu bilden; wobei der 4- bis 8-gliedrige heterocyclische Ring optional bis zu 2 zusätzliche Heteroatome enthält, unabhängig ausgewählt aus N, O oder S und optional und unabhängig substituiert ist mit bis zu 5 Vorkommen von R^{5d};
jedes R⁵ unabhängig ausgewählt ist aus Halogen, -CN, C₁₋₆-Alkyl, -(C₁₋₆-Alkyl)-R⁶, -OR⁶, -SR⁶, -COR⁶, -OC(O)R⁶, -C(O)OR⁶, -C(O)N(R⁶)₂, -N(R⁶)C(O)R⁶, -N(R⁶)C(O)OR⁶, -N(R⁶)C(O)N(R⁶)₂, -N(R⁶)₂, -SO₂R⁶, -SO₂OH, -SO₂NHOH, -SO₂N(R⁶)₂, -SO₂N(R⁶)(CO)-R⁶, -N(R⁶)SO₂R⁶, einem C₇₋₁₂-Aralkyl-, einem C₃₋₈-Cycloalkyl-Ring, einem 4- bis 7-gliedrigen heterocyclischen Ring, einem 5- oder 6-gliedrigen Heteroaryl-Ring, Phenyl oder einer Oxo-Gruppe; wobei jeder 5- oder 6-gliedrige Heteroaryl-Ring oder 4- bis 7-gliedrige heterocyclische Ring bis zu vier Ring-Heteroatome enthält, unabhängig ausgewählt aus N, O und S, wobei jede von den C₁₋₆-Alkyl-Ketten, dem C₇₋₁₂-Aralkyl-, demC₃₋₈-Cycloalkyl-Ring, dem 4-bis 7-gliedrigen heterocyclischen Ring, dem 5- oder 6-gliedrigen Heteroaryl-Ring oder der Phenyl-Gruppe optional und unabhängig substituiert ist mit bis zu 3 Vorkommen von Halogen, C₁₋₄-Alkyl, C₁₋₄ (Haloalkyl), -OH, -NH₂, -NH(C₁₋₄-Alkyl), -N(C₁₋₄-Alkyl)₂, -CN, -COOH, -CONH₂, -COO(C₁₋₄-Alkyl), -O(C₁₋₄-Alkyl), -O(C₁₋₄-Haloalkyl) oder Oxo;
alternativ zwei Vorkommen von R⁵, die an das gleiche oder an verschiedene Atome von J^{D} angelagert sind, zusammen mit dem Atom oder den Atomen von J^{D}, an die sie angelagert sind, einen C₃₋₈-Cycloalkyl-Ring, einen 4- bis 6-gliedrigen heterocyclischen Ring; einen Phenyl- oder einen 5- oder 6-gliedrigen Heteroaryl-Ring bilden können, was in einem bicylischen System resultiert, wobei die zwei Ringe des bicyclischen Systems in einem Spiro-, kondensierten oder überbrückten Verhältnis in Bezug aufeinander sind; wobei der 4- bis 6-gliedrige Heterocyclus oder der 5- oder 6-gliedriger Heteroaryl-Ring bis zu vier Ring-Heteroatome enthält, die unabhängig ausgewählt sind aus N, O oder S; und wobei der C₃₋₈-Cycloalkyl-Ring, 4- bis 6-gliedrige heterocyclische Ring, Phenyl oder 5- oder 6-gliedrige Heteroaryl-Ring optional und unabhängig substituiert ist mit bis zu 3 Vorkommen von C₁₋₄-Alkyl, C₁₋₄-Haloalkyl, C₁₋₄-Alkoxy, C₁₋₄-Haloalkoxy, Oxo, -C(O)O(C₁₋₄-Alkyl), -C(O)OH, -NR(CO)O(C₁₋₄-Alkyl), -CONH₂, -OH oder Halogen; wobei R Wasserstoff oder ein C₁₋₂-Alkyl ist;
jedes R^{5a} unabhängig ausgewählt ist aus Halogen, -CN, C₁₋₆-Alkyl, -(C₁₋₆-Alkyl)-R^{6a}, -OR^{6a}, -SR^{6a}, -COR^{6a}, -OC(O)R^{6a}, -C(O)OR^{6a}, -C(O)N(R^{6a})₂, -N(R^{6a})C(O)R^{6a}, -N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, einem C₇₋₁₂-Aralkyl, einem C₃₋₈-Cycloalkyl-Ring, einem 4-bis 7-gliedrigen heterocyclischen Ring, einem 5- oder 6-gliedrigen Heteroaryl-Ring, einer Phenyl- oder einer Oxo-Gruppe; wobei jeder 5-oder 6-gliedrige Heteroaryl-Ring oder 4- bis 7-gliedrige heterocyclische Ring bis zu vier Ring-Heteroatome enthält, unabhängig ausgewählt aus N, O und S, wobei jede von den C₁₋₆-Alkyl-Ketten, jedes von dem C₇₋₁₂-Aralkyl, der C₃₋₈-Cycloalkyl-Ring, der 4- bis 7-gliedrige heterocyclische Ring, der 5- oder 6-gliedrige Heteroaryl-Ring oder die Phenyl-Gruppe optional und unabhängig substituiert ist mit bis zu 3 Vorkommen von Halogen, C₁₋₄-Alkyl, C₁₋₄ (Haloalkyl), -OH, -NH₂, -NH(C₁₋₄-Alkyl), -N(C₁₋₄-Alkyl)₂, -CN, -COOH, -COO(C₁₋₄-Alkyl), -CONH₂, -O(C₁₋₄-Alkyl), -O(C₁₋₄-Haloalkyl) oder Oxo;
jedes R^{5b} unabhängig ausgewählt ist aus Halogen, -CN, C₁₋₆-Alkyl, -(C₁₋₆-Alkyl)-R^{6a}, -OR^{6a}, -SR^{6a}, -COR^{6a}, -OC(O)R^{6a}, -C(O)OR^{6a}, -C(O)N(R^{6a})₂, -N(R^{6a})C(O)R^{6a}, -N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, einem C₇₋₁₂-Aralkyl, einem C₃₋₈-Cycloalkyl-Ring, einem 4-bis 7-gliedrigen heterocyclischen Ring, einem 5- oder 6-gliedrigen Heteroaryl-Ring, einer Phenyl- oder einer Oxo-Gruppe; wobei jeder 5-oder 6-gliedrige Heteroaryl-Ring oder 4- bis 7-gliedrige heterocyclische Ring bis zu vier Ring-Heteroatome enthält, unabhängig ausgewählt aus N, O und S, wobei jede von den C₁₋₆-Alkyl-Ketten, jedes von dem C₇₋₁₂-Aralkyl, der C₃₋₈-Cycloalkyl-Ring, der 4- bis 7-gliedrige heterocyclische Ring, der 5- oder 6-gliedrige Heteroaryl-Ring oder die Phenyl-Gruppe optional und unabhängig substituiert ist mit bis zu 3 Vorkommen von Halogen, C₁₋₄-Alkyl, C₁₋₄ (Haloalkyl), -OH, -NH₂, -NH(C₁₋₄-Alkyl), -N(C₁₋₄-Alkyl)₂, -CN, -COOH, -COO(C₁₋₄-Alkyl), -CONH₂, -O(C₁₋₄-Alkyl), -O(C₁₋₄-Haloalkyl) oder Oxo;
alternativ zwei Vorkommen von R^{5a} oder zwei Vorkommen von R^{5b}, die an das gleiche oder an verschiedene Atome von R^{D} bzw. R^{d} angelagert sind, zusammen mit dem Atom oder den Atomen an die sie angelagert sind, einen C₃₋₈-Cycloalkyl-Ring, einen 4- bis 6-gliedrigen heterocyclischen Ring; einen Phenyl- oder einen 5- oder 6-gliedrigen Heteroaryl-Ring bilden können, was in einem bicylischen System resultiert, wobei die zwei Ringe des bicyclischen Systems in einem Spiro-, kondensierten oder überbrückten Verhältnis in Bezug aufeinander sind; wobei der 4- bis 6-gliedrige Heterocyclus oder der 5- oder 6-gliedriger Heteroaryl-Ring bis zu vier Ring-Heteroatome enthält, die unabhängig ausgewählt sind aus N, O oder S; und wobei der C₃₋₈-Cycloalkyl-Ring, 4- bis 6-gliedrige heterocyclische Ring, Phenyl oder 5- oder 6-gliedrige Heteroaryl-Ring optional und unabhängig substituiert ist mit bis zu 3 Vorkommen von C₁₋₄-Alkyl, C₁₋₄-Haloalkyl, C₁₋₄-Alkoxy, C₁₋₄-Haloalkoxy, Oxo, -C(O)O(C₁₋₄-Alkyl), -C(O)OH, -NR(CO)O(C₁₋₄-Alkyl), -CONH₂, -OH oder Halogen; wobei R Wasserstoff oder ein C₁₋₂-Alkyl ist;
jedes R^{5c} unabhängig ausgewählt ist aus Halogen, -CN, C₁₋₆-Alkyl, -(C₁₋₆-Alkyl)-R^{6b}, -OR^{6b}, -SR^{6b}, -COR^{6b}, -OC(O)R^{6b}, -C(O)OR^{6b}, -C(O)N(R^{6b})₂, -N(R^{6b})C(O)R^{6b}, -N(R^{6b})C(O)OR^{6b}, -N(R^{6b})C(O)N(R^{6b})₂, -N(R^{6b})₂, -SO₂R^{6b}, -SO₂OH, -SO₂NHOH, -SO₂N(R^{6b})(CO)-R^{6b}, -SO₂N(R^{6b})₂, -N(R^{6b})SO₂R^{6b}, einem C₇₋₁₂-Aralkyl, einem C₃₋₈-Cycloalkyl-Ring, einem 4- bis 7-gliedrigen heterocyclischen Ring, einem 5- oder 6-gliedrigen Heteroaryl-Ring, Phenyl oder einer Oxo-Gruppe; wobei jeder 5- oder 6-gliedrige Heteroaryl-Ring oder 4- bis 7-gliedrige heterocyclische- Ring bis zu vier Ring-Heteroatome enthält, unabhängig ausgewählt aus N, O und S, wobei jede von den C₁₋₆-Alkyl-Ketten, das C₇₋₁₂-Aralkyl, der C₃₋₈-Cycloalkyl-Ring, der 4- bis 7-gliedrige heterocyclische Ring, der 5- oder 6-gliedrige Heteroaryl-Ring oder die Phenyl-Gruppen optional und unabhängig substituiert sind mit bis zu 3 Vorkommen von Halogen, C₁₋₄-Alkyl, C₁₋₄ (Haloalkyl), -OH, -NH₂, -NH(C₁₋₄-Alkyl), -N(C₁₋₄-Alkyl)₂, -CN, -COOH, -CONH₂, -COO(C₁₋₄-Alkyl), -O(C₁₋₄-Alkyl), -O(C₁₋₄-Haloalkyl) oder Oxo;
alternativ zwei Vorkommen von R^{5c}, die an das gleiche oder an verschiedene Atome von R^{f} angelagert sind, zusammen mit dem Atom oder den Atomen an die sie angelagert sind, einen C₃₋₈-Cycloalkyl-Ring, einen 4- bis 6-gliedrigen heterocyclischen Ring; ein Phenyl oder einen 5- oder 6-gliedrigen Heteroaryl-Ring bilden können, was in einem bicylischen System resultiert, wobei die zwei Ringe des bicyclischen Systems in einem Spiro-, kondensierten oder überbrückten Verhältnis in Bezug aufeinander sind; wobei der 4- bis 6-gliedrige Heterocyclus oder der 5- oder 6-gliedriger Heteroaryl-Ring bis zu vier Ring-Heteroatome enthält, die unabhängig ausgewählt sind aus N, O oder S; und wobei der C₃₋₈-Cycloalkyl-Ring, 4- bis 6-gliedrige heterocyclische Ring, Phenyl oder 5- oder 6-gliedrige Heteroaryl-Ring optional und unabhängig substituiert ist mit bis zu 3 Vorkommen von C₁₋₄-Alkyl, C₁₋₄-Haloalkyl, C₁₋₄-Alkoxy, C₁₋₄-Haloalkoxy, Oxo, -C(O)O(C₁₋₄-Alkyl), -C(O)OH, -CONH₂, -NR(CO)O(C₁₋₄-Alkyl), -OH oder Halogen; wobei R Wasserstoff oder ein C₁₋₂-Alkyl ist;
jedes R^{5d} unabhängig ausgewählt ist aus Halogen, -CN, C₁₋₆-Alkyl, -(C₁₋₆-Alkyl)-R⁶, -OR⁶, -SR⁶, -COR⁶, -OC(O)R⁶, -C(O)OR⁶, -C(O)N(R⁶)₂, -N(R⁶)C(O)R⁶, -N(R⁶)C(O)OR⁶, -N(R⁶)C(O)N(R⁶)₂, -N(R⁶)₂, -SO₂OH, -SO₂NHOH, -SO₂N(R⁶)(CO)-R⁶, -SO₂R⁶, -SO₂N(R⁶)₂, -N(R⁶)SO₂R⁶, a C₇₋₁₂-Aralkyl, einem C₃₋₈-Cycloalkyl-Ring, einem 4- bis 7-gliedrigen heterocyclischen Ring, einem 5- oder 6-gliedrigen Heteroaryl-Ring, Phenyl oder einer Oxo-Gruppe; wobei jeder 5- oder 6-gliedrige Heteroaryl-Ring oder 4- bis 7-gliedrige heterocyclische Ring bis zu vier Ring-Heteroatome enthält, unabhängig ausgewählt aus N, O und S, wobei jede von den C₁₋₆-Alkyl-Ketten, das C₇₋₁₂-Aralkyl, der C₃₋₈-Cycloalkyl-Ring, der 4- bis 7-gliedrige heterocyclische Ring, der 5- oder 6-gliedrige Heteroaryl-Ring oder die Phenyl-Gruppen optional und unabhängig substituiert sind mit bis zu 3 Vorkommen von Halogen, C₁₋₄-Alkyl, C₁₋₄ (Haloalkyl), -OH, -NH₂, -NH(C₁₋₄-Alkyl), -N(C₁₋₄-Alkyl)₂, -CN, -COOH, -CONH₂, -COO(C₁₋₄-Alkyl), -O(C₁₋₄-Alkyl), -O(C₁₋₄-Haloalkyl) oder Oxo;
zwei Vorkommen von R⁵ oder zwei Vorkommen von R^{5d}, die an das gleiche oder an verschiedene Atome von J^{D} angelagert sind, zusammen mit dem Atom oder den Atomen an die sie angelagert sind, optional einen C₃₋₈-Cycloalkyl-Ring, einen 4- bis 6-gliedrigen heterocyclischen Ring; ein Phenyl oder einen 5- oder 6-gliedrigen Heteroaryl-Ring bilden können, was in einem bicylischen System resultiert, wobei die zwei Ringe des bicyclischen Systems in einem Spiro-, kondensierten oder überbrückten Verhältnis in Bezug aufeinander sind; wobei der 4- bis 6-gliedrige Heterocyclus oder der 5- oder 6-gliedriger Heteroaryl-Ring bis zu vier Ring-Heteroatome enthält, die unabhängig ausgewählt sind aus N, O oder S; und wobei der C₃₋₈-Cycloalkyl-Ring, 4- bis 6-gliedrige heterocyclische Ring, Phenyl oder 5- oder 6-gliedrige Heteroaryl-Ring optional und unabhängig substituiert ist mit bis zu 3 Vorkommen von C₁₋₄-Alkyl, C₁₋₄-Haloalkyl, C₁₋₄-Alkoxy, C₁₋₄-Haloalkoxy, Oxo, -C(O)O(C₁₋₄-Alkyl), -C(O)OH, -CONH₂, -NR(CO)O(C₁₋₄-Alkyl), -OH oder Halogen; wobei R Wasserstoff oder ein C₁₋₂-Alkyl ist;
jedes R⁶ unabhängig ausgewählt ist aus Wasserstoff, einem C₁₋₆ aliphatischen, Phenyl, Benzyl, einem C₃₋₈-Cycloalkyl-Ring, einem 4- bis 7-gliedrigen heterocyclischen Ring oder einem 5- oder 6-gliedrigen Heteroaryl-Ring, wobei jedes von dem C₁₋₆ aliphatischen, jedes von dem Phenyl, jedes von dem Benzyl, jede von der C₃₋₈-Cycloalkyl-Gruppe, jedes von dem 4- bis 7-gliedriger heterocyclischen Ring und jedes von dem 5- oder 6-gliedrigen Heteroaryl-Ring optional und unabhängig substituiert ist mit bis zu 3 Vorkommen von Halogen, C₁₋₄ -Alkyl, -OH, -NH₂, -NH(C₁₋₄-Alkyl), -N(C₁₋₄-Alkyl)₂, -CN, -COOH, -COO(C₁₋₄-Alkyl), -O(C₁₋₄-Alkyl), -O(C₁₋₄-Haloalkyl) oder Oxo, wobei jedes von dem 5- oder 6-gliedrigen Heteroaryl-Ring oder 4-bis 7-gliedrigen heterocyclischen Ring bis zu 4 Ring-Heteroatome enthält, unabhängig ausgewählt aus N, O und S;
jedes R^{6a} unabhängig ausgewählt ist aus Wasserstoff, einem C₁₋₆ aliphatischen, Phenyl, Benzyl, einem C₃₋₈-Cycloalkyl-Ring, einem 4- bis 7-gliedrigen heterocyclischen Ring oder einem 5- oder 6-gliedrigen Heteroaryl-Ring, wobei jedes von dem C₁₋₆ aliphatischen, jedes von dem Phenyl, jedes von dem Benzyl, jede von der C₃₋₈-Cycloalkyl-Gruppe, jedes von dem 4- bis 7-gliedriger heterocyclischen Ring und jedes von dem 5- oder 6-gliedrigen Heteroaryl-Ring optional und unabhängig substituiert ist mit bis zu 3 Vorkommen von Halogen, C₁₋₄ -Alkyl, -OH, -NH₂, -NH(C₁₋₄-Alkyl), -N(C₁₋₄-Alkyl)₂, -CN, -COOH, -COO(C₁₋₄-Alkyl), -O(C₁₋₄-Alkyl), -O(C₁₋₄-Haloalkyl) oder Oxo, wobei jedes von dem 5- oder 6-gliedrigen Heteroaryl-Ring oder 4-bis 7-gliedrigen heterocyclischen Ring bis zu 4 Ring-Heteroatome enthält, unabhängig ausgewählt aus N, O und S;
jedes R^{6b} unabhängig ausgewählt ist aus Wasserstoff, einem C₁₋₆ aliphatischen, Phenyl, Benzyl, einem C₃₋₈-Cycloalkyl-Ring, einem 4- bis 7-gliedrigen heterocyclischen Ring oder einem 5- oder 6-gliedrigen Heteroaryl-Ring, wobei jedes von dem C₁₋₆ aliphatischen, jedes von dem Phenyl, jedes von dem Benzyl, jede von der C₃₋₈-Cycloalkyl-Gruppe, jedes von dem 4- bis 7-gliedriger heterocyclischen Ring und jedes von dem 5- oder 6-gliedrigen Heteroaryl-Ring optional und unabhängig substituiert ist mit bis zu 3 Vorkommen von Halogen, C₁₋₄ -Alkyl, -OH, -NH₂, -NH(C₁₋₄-Alkyl), -N(C₁₋₄-Alkyl)₂, -CN, -COOH, -COO(C₁₋₄-Alkyl), -O(C₁₋₄-Alkyl), -O(C₁₋₄-Haloalkyl) oder Oxo, wobei jedes von dem 5- oder 6-gliedrigen Heteroaryl-Ring oder 4-bis 7-gliedrigen heterocyclischen Ring bis zu 4 Ring-Heteroatome enthält, unabhängig ausgewählt aus N, O und S; wobei
zwei Vorkommen von R⁶, die mit dem gleichen Stickstoffatom von R⁵- oder R^{5d} verbunden sind, zusammen mit dem Stickstoffatom von R⁵ bzw. R^{5d}, einen 5- bis 8-gliedrigen heterocyclischen Ring oder a 5-gliedriger Heteroaryl-Ring bilden können; wobei jeder von dem 5-bis 8-gliedrigen heterocyclischen Ring und jeder von dem 5-gliedrigen Heteroaryl-Ring optional bis zu 2 zusätzliche Heteroatome enthält, unabhängig ausgewählt aus N, O oder S;
zwei Vorkommen von R^{6a}, die mit einem Stickstoffatom von R^{5a} oder R^{5b} verbunden sind, zusammen mit dem Stickstoff einen 5- bis 8-gliedrigen heterocyclischen Ring oder einen 5-gliedriger Heteroaryl-Ring bilden können; wobei jeder von dem 5- bis 8-gliedrigen heterocyclischen Ring und jeder von dem 5-gliedrigen Heteroaryl-Ring optional bis zu 2 zusätzliche Heteroatome enthält, unabhängig ausgewählt aus N, O oder S;
zwei Vorkommen von R^{6b}, die mit einem Stickstoffatom von R^{5c} verbunden sind, zusammen mit dem Stickstoff einen 5- bis 8-gliedrigen heterocyclischen Ring oder einen 5-gliedrigen Heteroaryl-Ring bilden können; wobei jeder von dem 5- bis 8-gliedrigen heterocyclischen Ring und jeder von dem 5-gliedrigen Heteroaryl-Ring optional bis zu 2 zusätzliche Heteroatome enthält, unabhängig ausgewählt aus N, O oder S;
alternativ zwei J^{D}-Gruppen, angelagert an zwei benachbarte Ring-D-Atome, zusammengenommen mit den zwei benachbarten Ring-D-Atomen, einen 5- bis 7-gliedrigen Heterocyclus oder einen 5-gliedrigen Heteroaryl-Ring, der zu Ring-D kondensiert ist, bilden können; wobei der 5- bis 7-gliedrige Heterocyclus oder das 5-gliedrige Ring-Heteroaryl von 1 bis 3 Heteroatome enthält, unabhängig ausgewählt aus N, O oder S; und wobei der 5- bis 7-gliedrige Heterocyclus oder der 5-gliedrige Heteroaryl-Ring optional und unabhängig substituiert ist mit bis zu 3 Vorkommen von Oxo oder -(Y)-R⁹;
wobei Y entweder fehlt oder eine C₁₋₆-Alkyl-Kette ist, optional substituiert durch bis zu 6 Vorkommen von Fluor; und wobei, wenn Y die C₁₋₆-Alkyl-Kette ist, bis zu 3 Methylen-Einheiten von dieser Alkyl-Kette durch eine Gruppe ersetzt sein kann, ausgewählt aus -O-, -C(O) - oder -N((Y¹)-R⁹⁹)-;
Y¹ entweder fehlt oder eine C₁₋₆-Alkyl-Kette ist, optional substituiert durch bis zu 6 Vorkommen von Fluor;
wenn Y¹ fehlt, jedes R⁹⁹ unabhängig ausgewählt ist aus Wasserstoff, C₁₋₆-Alkyl optional substituiert mit bis zu 9 Fluoratomen, -COR¹⁰, -C(O)OR¹⁰, -C(O)N(R¹⁰)₂, -C(O)N(R¹⁰)SO₂R¹⁰, -SO₂R¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, einem C₃₋₆-Cycloalkyl-Ring, einem 4-8-gliedrigen heterocyclischen Ring, einem Phenyl-Ring oder einem 5-6-gliedrigen Heteroaryl-Ring; wobei jedes von dem 4- bis 8-gliedrigen heterocyclischen Ring oder 5- bis 6-gliedrigen Heteroaryl-Ring bis zu 4 Ring Heteroatome enthält, unabhängig ausgewählt aus N, O oder S; und wobei jedes von den C₃₋₆-Cycloalkyl-Ringen, jeder von den 4- bis 8-gliedrigen heterocyclischen Ringen, jeder von den Phenyl- und jeder von den 5 bis 6-gliedrigen Heteroaryl-Ringen optional und unabhängig substituiert ist mit bis zu 3 Vorkommen von R^{11a};
wenn Y¹ vorhanden ist, jedes R⁹⁹ unabhängig ausgewählt ist aus Wasserstoff, Halogen, -CN, C₁₋₆-Alkyl optional substituiert mit bis zu 9 Fluoratomen, -COR¹⁰, -OR¹⁰, -OC(O)R¹⁰, -C(O)OR¹⁰, -C(O)N(R¹⁰)₂, -C(O)N(R¹⁰)SO₂R¹⁰, -SO₂R¹⁰, -SOR¹⁰, -SR¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, einem C₃₋₆-Cycloalkyl-Ring, einem 4-8-gliedrigen heterocyclischen Ring, einem Phenyl-Ring oder einem 5-6-gliedrigen Heteroaryl-Ring; wobei jeder von dem 4- bis 8-gliedrigen heterocyclischen Ring oder 5- bis 6-gliedrigen Heteroaryl-Ring bis zu 4 Ring Heteroatome enthält, unabhängig ausgewählt aus N, O oder S; und wobei jeder von den C₃₋₆-Cycloalkyl-Ringen, jeder von den 4- bis 8-gliedrigen heterocyclischen Ringen, jeder von den Phenyl- und jeder von den 5- bis 6-gliedrigen Heteroaryl-Ringen optional und unabhängig substituiert ist mit bis zu 3 Vorkommen von R^{11a};
jedes R⁹ unabhängig ausgewählt ist aus Wasserstoff, -CN, -OR¹⁰, -COR¹⁰, -OC(O)R¹⁰, -C(O)OR¹⁰, -C(O)N(R¹⁰)₂, -C(O)N(R¹⁰)SO₂R¹⁰, -N(R¹⁰)C(O)R¹⁰ -N(R¹⁰)C(O)OR¹⁰, -N(R¹⁰)C(O)N(R¹⁰)₂, -N(R¹⁰)₂, -SO₂R¹⁰, -SO₂N(R¹⁰)₂, -SO₂N(R¹⁰)COOR¹⁰, -SO₂N(R¹⁰)C(O)R¹⁰, -N(R¹⁰)SO₂R¹⁰, -SO₂OH, -SO₂NHOH, -SO₂N(R¹⁰)(CO)-R¹⁰, einem C₃₋₆-Cycloalkyl-Ring, einem 4-8-gliedrigen heterocyclischen Ring, einem Phenyl-Ring oder einem 5-6-gliedrigen Heteroaryl-Ring; wobei jeder von dem 4- bis 8-gliedrigen heterocyclischen Ring oder 5- bis 6-gliedrigen Heteroaryl-Ring bis zu 4 Ring Heteroatome enthält, unabhängig ausgewählt aus N, O oder S; und wobei jeder von den C₃₋₆-Cycloalkyl-Ringen, jeder von den 4- bis 8-gliedrigen heterocyclischen Ringen, jeder von den Phenyl- und jeder von den 5 bis 6-gliedrigen Heteroaryl-Ringen optional und unabhängig substituiert ist mit bis zu 3 Vorkommen von R^{11a};
jedes R¹⁰ unabhängig ausgewählt ist aus Wasserstoff, einem C₁₋₆-Alkyl, -(C₁₋₆-Alkyl)-R¹³ Phenyl, Benzyl, einem C₃₋₆-Cycloalkyl-Ring, einem 4- bis 7-gliedrigen heterocyclischen Ring oder einem 5- oder 6-gliedrigen Heteroaryl-Ring, wobei jeder 5- oder 6-gliedrige Heteroaryl-Ring oder 4- bis 7-gliedrige heterocyclische Ring bis zu 4 Ring Heteroatome enthält, unabhängig ausgewählt aus N, O und S; und wobei jedes von dem C₁₋₆-Alkyl, jedes von dem Phenyl, jedes von dem Benzyl, jede von der C₃₋₈-Cycloalkyl-Gruppe, jeder von dem 4- bis 7-gliedrigen heterocyclischen Ring und jeder 5- oder 6-gliedrige Heteroaryl-Ring optional und unabhängig substituiert ist mit bis zu 3 Vorkommen von R^{11b};
jedes R¹³ unabhängig ausgewählt ist aus einem Phenyl, einem Benzyl, einem C₃₋₆-Cycloalkyl-Ring, einem 4- bis 7-gliedrigen heterocyclischen Ring oder einem 5- oder 6-gliedrigen Heteroaryl-Ring, wobei jeder 5- oder 6-gliedrige Heteroaryl-Ring oder 4- bis 7-gliedrige heterocyclische Ring bis zu 4 Ring Heteroatome enthält, unabhängig ausgewählt aus N, O und S; und wobei jedes von dem Phenyl, jedes von dem Benzyl, jede von der C₃₋₈-Cycloalkyl-Gruppe, jeder von dem 4- bis 7-gliedrigen heterocyclischen Ring und jeder 5- oder 6-gliedrige Heteroaryl-Ring optional und unabhängig substituiert ist mit bis zu 3 Vorkommen von R^{11c};
jedes R^{11a} unabhängig ausgewählt ist aus Halogen, C₁₋₆-Alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ oder -N(R¹²)SO₂R¹²; wobei jedes von dem C₁₋₆-Alkyl optional und unabhängig substituiert ist mit bis zu 6 Vorkommen von Fluor und/oder 3 Vorkommen von R¹²¹;
jedes R^{11b} unabhängig ausgewählt ist aus Halogen, C₁₋₆-Alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ oder -N(R¹²)SO₂R¹²; wobei jedes von dem C₁₋₆-Alkyl optional und unabhängig substituiert ist mit bis zu 6 Vorkommen von Fluor und/oder 3 Vorkommen von R¹²¹; und
jedes R^{11c} unabhängig ausgewählt ist aus Halogen, C₁₋₆-Alkyl, -CN, -OR¹², -COR¹², -C(O)OR¹², -C(O)N(R¹²)₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ oder -N(R¹²)SO₂R¹²; wobei jedes von dem C₁₋₆-Alkyl optional und unabhängig substituiert ist mit bis zu 6 Vorkommen von Fluor und/oder 3 Vorkommen von R¹²¹;
jedes R¹² ausgewählt ist aus Wasserstoff, C₁₋₄-Alkyl, C₁₋₄ (Fluoroalkyl), -OH, -NH₂, -NH(C₁₋₄-Alkyl), -N(C₁₋₄-Alkyl)₂, -CN, -COOH, -COO(C₁₋₄-Alkyl), -O(C₁₋₄-Alkyl), -O(C₁₋₄-Fluoralkyl) oder Oxo;
jedes R¹²¹ ausgewählt ist aus C₁₋₄-Alkyl, C₁₋₄ (Fluoroalkyl), -OH, -NH₂, -NH(C₁₋₄-Alkyl), -N(C₁₋₄-Alkyl)₂, -CN, -COOH, -COO(C₁₋₄-Alkyl), -O(C₁₋₄-Alkyl), -O(C₁₋₄-Fluoralkyl) oder Oxo;
R^{C} ausgewählt ist aus Wasserstoff, C₁₋₆ aliphatischem, -(C₁₋₆-Alkyl)-R^{N}, einem 5- oder 6-gliedrigen Heteroaryl, Phenyl, einem 4- bis 7-gliedrigen heterocyclischen, einem C₃₋₈ cycloaliphatischen, -C(O)R⁷, -C(O)OR⁷, -C(O)N(R⁷)₂ und -C(O)N(R⁷)SO₂R⁷; wobei jedes von dem 5- oder 6-gliedrigen Heteroaryl-Ring und 4- bis 7-gliedrigen heterocyclischen Ring bis zu 4 Ring-Heteroatome enthält, unabhängig ausgewählt aus N, O und S; wobei jedes von dem C₁₋₆ aliphatischen und jeder C₁₋₆-Alkyl-Abschnitt von dem -(C₁₋₆-Alkyl)-R^{N} optional und unabhängig substituiert ist mit bis zu 6 Vorkommen von Halogen und bis zu 2 Vorkommen von -CN, -COOR⁸, -OR⁸, Oxo, -N(R⁸)₂, -C(O)N(R⁸)₂, -N(R⁸)C(O)R⁸, -N(R⁸)C(O)OR⁸, -N(R⁸)C(O)N(R⁸)₂, -SO₂R⁸, -SO₂N(R⁸)₂, -NHOR⁸, -SO₂N(R⁸)COOR⁸, -SO₂N(R⁸)C(O)R⁸ und -N(R⁸)SO₂R⁸;
wobei jedes R⁷ unabhängig ausgewählt ist aus Wasserstoff, C₁₋₆-Alkyl, C₁₋₆-Fluoralkyl, einem C₃₋₈-Cycloalkyl-Ring, Phenyl, einem 4- bis 7-gliedrigen heterocyclischen Ring oder einem 5- oder 6-gliedrigen Heteroaryl-Ring; wobei jeder von dem 5- oder 6-gliedrigen Heteroaryl-Ring oder 4- bis 7-gliedrigen heterocyclischen Ring bis zu 4 Ring-Heteroatome enthält, unabhängig ausgewählt aus N, O und S; und wobei jedes von dem C₁₋₆-Alkyl, jedes von dem Phenyl, jede von de C₃₋₈-Cycloalkyl-Gruppe, jeder von dem 4- bis 7-gliedrigen heterocyclischen Ring und jeder von dem 5- oder 6-gliedrigen Heteroaryl-Ring optional und unabhängig substituiert ist mit bis zu 3 Vorkommen von Halogen, C₁₋₄-Alkyl, -OH, -NH₂, -NH(C₁₋₄-Alkyl), -N(C₁₋₄-Alkyl)₂, -CN, -COOH, -COO(C₁₋₄-Alkyl), -O(C₁₋₄-Alkyl), -O(C₁₋₄-Haloalkyl) oder Oxo;
jedes R⁸ unabhängig ausgewählt ist aus Wasserstoff, C₁₋₆-Alkyl, C₁₋₆-Fluoralkyl, einem C₃₋₈-Cycloalkyl-Ring, einem 4- bis 7-gliedrigen heterocyclischen Ring oder einem 5- oder 6-gliedrigen Heteroaryl-Ring; wobei jeder von dem 5- oder 6-gliedrigen Heteroaryl-Ring oder 4-bis 7-gliedrigen heterocyclischen Ring bis zu 4 Ring-Heteroatome enthält, unabhängig ausgewählt aus N, O und S; und wobei jedes von dem C₁₋₆-Alkyl, jedes von dem Phenyl, jede von de C₃₋₈-Cycloalkyl-Gruppe, jeder von dem 4- bis 7-gliedrigen heterocyclischen Ring und jeder von dem 5- oder 6-gliedrigen Heteroaryl-Ring optional und unabhängig substituiert ist mit bis zu 3 Vorkommen von Halogen, C₁₋₄-Alkyl, -OH, -NH₂, -NH(C₁₋₄-Alkyl), -N(C₁₋₄-Alkyl)₂, -CN, -COOH, -COO(C₁₋₄-Alkyl), -O(C₁₋₄-Alkyl), -O(C₁₋₄-Haloalkyl) oder Oxo;
jedes R^{N} unabhängig ausgewählt ist aus einem Phenyl-Ring, einem monocyclischen 5- oder 6-gliedriger Heteroaryl-Ring, einem monocyclischen C₃₋₆ cycloaliphatischen Ring, oder einem monocyclischen 4- bis 6-gliedrigen Heterocyclus; wobei der monocyclische 5- oder 6-gliedrige Heteroaryl-Ring oder der monocyclische 4- bis 6-gliedrige Heterocyclus zwischen 1 und 4 Heteroatome enthält, ausgewählt aus N, O oder S; wobei der monocyclische 5- oder 6-gliedrige Heteroaryl-Ring nicht ein 1,3,5-Triazinyl-Ring ist; und wobei das Phenyl, der monocyclische 5- bis 6-gliedrige Heteroaryl-Ring, der monocyclische C₃₋₆ cycloaliphatische Ring, oder der monocyclische 4- bis 6-gliedrige Heterocyclus optional und unabhängig substituiert ist mit bis zu 6 Vorkommen von Fluor und/oder bis zu 3 Vorkommen von J^{M};
jedes J^{M} unabhängig ausgewählt ist aus -CN, einem C₃₋₆ aliphatischen, -OR^{M}, -SR^{M}, -N(R^{M})₂, einem C₃₋₈ cycloaliphatischen Ring oder einem 4- bis 8-gliedrigen heterocyclischen Ring; wobei der 4- bis 8-gliedrige heterocyclische Ring 1 oder 2 Heteroatome enthält, unabhängig ausgewählt aus N, O oder S; wobei jedes von dem C₁₋₆ aliphatischen, jeder von dem C₃₋₈ cycloaliphatischen Ring und jeder von dem 4- bis 8-gliedrigen heterocyclischen Ring optional und unabhängig substituiert ist mit bis zu 3 Vorkommen von R^{7c}; und
jedes R^{M} unabhängig ausgewählt ist aus Wasserstoff, einem C₁₋₆ aliphatischen, einem C₃₋₈ cycloaliphatischen Ring oder einem 4- bis 8-gliedrigen heterocyclischen Ring; wobei jeder von dem 4- bis 8-gliedrigen heterocylischen Ring zwischen 1 und 3 Heteroatome enthält, unabhängig ausgewählt aus O, N oder S;
jedes R^{7c} unabhängig ausgewählt ist aus Halogen, -CN, -NO₂, C₁₋₄-Alkyl, C₁₋₄-Haloalkyl, C₃₋₈-Cycloalkyl-Ring, -OR^{8b}, -SR^{8b}, -N(R^{8b})₂, -C(O)O(C₁₋₄-Alkyl), -C(O)OH, -NR(CO)CO(C₁₋₄-Alkyl) oder einer Oxo-Gruppe; wobei jede von der Cycloalkyl-Gruppe optional und unabhängig substituiert ist mit bis zu 3 Vorkommen von Halogen;
jedes R^{8b} unabhängig ausgewählt ist aus Wasserstoff, C₁₋₆-Alkyl, C₁₋₆-Fluoralkyl, einem C₃₋₈-Cycloalkyl-Ring, einem 4- bis 7-gliedrigen heterocyclischen Ring oder einem 5- oder 6-gliedrigen Heteroaryl-Ring; wobei jeder von dem 5- oder 6-gliedrigen Heteroaryl-Ring oder 4-bis 7-gliedrigen heterocyclischen Ring bis zu 4 Ring-Heteroatome enthält, unabhängig ausgewählt aus N, O und S; und wobei jedes von dem C₁₋₆-Alkyl, jedes von dem Phenyl, jede von der C₃₋₈-Cycloalkyl-Gruppe, jeder von dem 4- bis 7-gliedrigen heterocyclischen Ring und jeder von dem 5- oder 6-gliedrigen Heteroaryl-Ring optional und unabhängig substituiert ist mit bis zu 3 Vorkommen von Halogen, C₁₋₄-Alkyl, -OH, -NH₂, -NH(C₁₋₄-Alkyl), -N(C₁₋₄-Alkyl)₂, -CN, -COOH, -COO(C₁₋₄-Alkyl), -O(C₁₋₄-Alkyl), -O(C₁₋₄-Haloalkyl) oder Oxo;
wobei der oben beschriebene heterocyclische Ring vollständig gesättigt ist oder eine oder mehrere Unsättigungseinheiten enthält, aber nicht aromatisch ist;
vorausgesetzt, dass die Verbindung keine Verbindung ist, die durch die folgende allgemeine Struktur dargestellt ist: 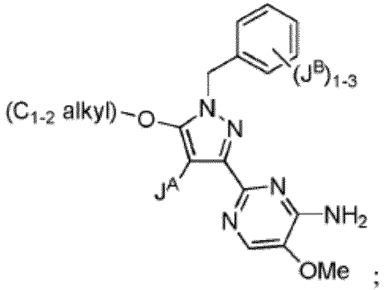
wobei J^{A} entweder Wasserstoff oder C₁₋₄-Alkyl ist; und J^{B} entweder Halogen oder C₁₋₄-(Alkoxy) ist.

2. Verbindung nach Anspruch 1, oder ein pharmazeutisch annehmbares Salz davon, wobei die Verbindung durch **Formel IIa** dargestellt ist: 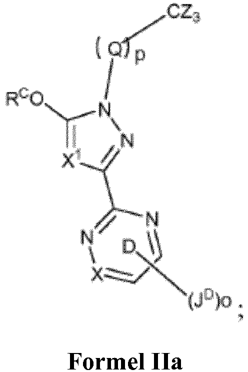 wobei Q eine Gruppe -CZ₂- darstellt, jedes Z unabhängig ausgewählt ist aus Wasserstoff oder Fluor und p eine Ganzzahl ist, ausgewählt aus 1, 2, 3, 4 und 5.

3. Verbindung nach den Ansprüchen 1 oder 2, oder ein pharmazeutisch annehmbares Salz davon, dargestellt durch **Formel IIIa:** 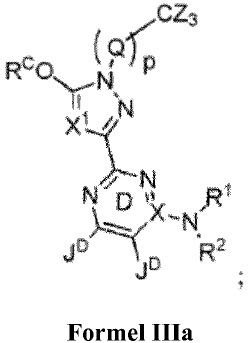 wobei
wenn X N ist, die Gruppierung -N(R¹)(R²) fehlt;
wenn X C ist, die Gruppierung -N(R¹)(R²) vorhanden ist;
R¹ und R², zusammen mit dem Stickstoffatom, an das sie angelagert sind, einen 4- bis 8-gliedrigen heterocyclischen Ring oder 5-gliedrigen Heteroaryl-Ring bilden; wobei der 4- bis 8-gliedrige heterocyclische Ring oder 5-gliedrige Heteroaryl-Ring optional zusätzlich zu dem Stickstoffatom, an das beide R¹ und R² angelagert sind, bis zu 3 Ring Heteroatome enthält, unabhängig ausgewählt aus N, O oder S und optional substituiert ist durch bis zu 5 Vorkommen von R^{5e};
jedes R^{5e} unabhängig ausgewählt ist aus Halogen, -CN, C₁₋₆-Alkyl, -(C₁₋₄-Alkyl)-R⁶, a C₃₋₈-Cycloalkyl-Ring, C₁₋₄ (Cyanoalkyl), -OR⁶, -SR⁶, -OCOR⁶, -COR⁶, -C(O)OR⁶, -C(O)N(R⁶)₂, -N(R⁶)C(O)R⁶ -N(R⁶)₂, -SO₂R⁶, -SO₂N(R⁶)₂, -N(R⁶)SO₂R⁶, -SO₂OH, -SO₂NHOH, -SO₂N(R⁶)(CO)-R⁶, Benzyl, Phenyl oder einer Oxo-Gruppe; wobei jeder von dem Phenyl-Ring und jede von der Benzyl-Gruppe, optional und unabhängig substituiert ist mit bis zu 3 Vorkommen von Halogen, -OH, -NH₂, -NH(C₁₋₄-Alkyl), -N(C₁₋₄-Alkyl)₂, -CN, C₁₋₄-Alkyl, C₁₋₄-Haloalkyl, -CONH₂, -O(C₁₋₄-Alkyl) oder -O(C₁₋₄Haloalkyl); und wobei jede von den C₁₋₆-Alkyl- oder C₁₋₄-Alkyl-Ketten und jeder von dem C₃₋₈-Cycloalkyl-Ring optional und unabhängig substituiert ist mit bis zu 3 Vorkommen von Halogen; wobei
jedes R⁶ unabhängig ausgewählt ist aus Wasserstoff, einem C₁₋₆-Alkyl, einem C₂₋₄-Alkenyl, Phenyl, Benzyl, oder einem C₃₋₈-Cycloalkyl-Ring; wobei jedes von dem C₁₋₆-Alkyl, jedes von dem C₂₋₄-Alkenyl, jedes von dem Phenyl, jedes von dem Benzyl und jede von der C₃₋₈-Cycloalkyl Gruppe optional und unabhängig substituiert ist mit bis zu 3 Vorkommen von Halogen;
alternativ zwei der Vorkommen von R^{5e}, die an das gleiche oder an verschiedene Atome des gebildeten Rings durch R¹, R² angelagert sind, und der Stickstoff, an den R¹ und R² angelagert sind, zusammen mit dem Atom oder den Atomen, optional einen C₃₋₈-Cycloalkyl-Ring, einen 4-bis 6-gliedrigen heterocyclischen Ring; ein Phenyl oder einen 5- oder 6-gliedrigen Heteroaryl-Ring bilden, was in einem bicylischen System resultiert, wobei die zwei Ringe des bicyclischen Systems in einem Spiro-, kondensierten oder überbrückten Verhältnis in Bezug aufeinander sind; wobei der 4- bis 6-gliedrige Heterocyclus oder der 5- oder 6-gliedrige Heteroaryl-Ring bis zu drei Ring-Heteroatome enthält, unabhängig ausgewählt aus N, O oder S; und wobei der C₃₋₈-Cycloalkyl-Ring, 4- bis 6-gliedrige heterocyclische Ring, das Phenyl oder der 5- oder 6-gliedrige Heteroaryl-Ring optional und unabhängig substituiert sind mit bis zu 3 Vorkommen von C₁₋₄-Alkyl, C₁₋₄-Haloalkyl, C₁₋-Alkoxy, C₁₋₄-Haloalkoxy, Oxo, -C(O)O(C₁₋₄-Alkyl), -CONH₂, -C(O)OH, -NR(CO)O(C₁₋₄-Alkyl), -OH oder Halogen; wobei R Wasserstoff oder ein C₁₋₂-Alkyl ist;
alternativ sind R¹ und R² jeweils unabhängig ausgewählt aus Wasserstoff, C₁₋₆-Alkyl, einem C₃₋₈-Cycloalkyl-Ring, einem 4- bis 8-gliedrigen heterocyclischen Ring, einem 5- oder 6-gliedrigen Heteroaryl-Ring, Phenyl oder einem C₁₋₆-Alkyl-R^{Y}; wobei jeder von dem 4- bis 8-gliedrigen heterocyclischen Ring und jeder von dem 5- oder 6-gliedrigen Heteroaryl-Ring bis zu 3 Ring-Heteroatome enthält, unabhängig ausgewählt aus N, O und S; und wobei jedes von dem C₁₋₆-Alkyl, C₃₋₈-Cycloalkyl-Ring, 4- bis 8-gliedrigen heterocyclischen Ring, 5- oder 6-gliedrigen Heteroaryl-Ring, Phenyl und dem C₁₋₆-Alkyl-Abschnitt der C₁₋₆-Alkyl-R^{Y}-Gruppierung, die optional und unabhängig substituiert ist mit bis zu 5 Vorkommen von R^{5f};
R^{y} ausgewählt ist aus einem C₃₋₈-Cycloalkyl-Ring, einem 4- bis 8-gliedrigen heterocyclischen Ring, Phenyl, oder einem 5 bis 6-gliedrigen Heteroaryl-Ring; wobei jeder von dem 4- bis 8-gliedrigen heterocyclischen Ring oder 5- bis 6-gliedrigen heteroaromatischen Ring zwischen 1 und 4 Ring Heteroatome enthält, unabhängig ausgewählt aus N, O oder S; und wobei jeder von dem C₃₋₈-Cycloalkyl-Ring, jeder von dem 4- bis 8-gliedrigen heterocyclischen Ring, jedes von dem Phenyl und jeder von dem 5 bis 6-gliedrigen Heteroaryl-Ring optional substituiert ist mit bis zu 5 Vorkommen von R^{5g};
jedes R^{5f} unabhängig ausgewählt ist aus Halogen, -CN, C₁₋₆-Alkyl, -(C₁₋₄-Alkyl)-R^{6a}, einem C₇₋₁₂-Aralkyl, C₃₋₈-Cycloalkyl-Ring, C₁₋₄(Cyanoalkyl), -OR^{6a}, -SR^{6a}, -OCOR^{6a}, -COR^{6a}, -C(O)OR^{6a}, -C(O)N(R^{6a})₂, -N(R^{6a})C(O)R^{6a}, -N(R^{6a})₂, -SO₂OH, -SO₂NHOH, -SO₂N(R^{6a})(CO)-R^{6a}, -SO₂R^{6a}, -SO₂N(R^{6a})₂, -N(R^{6a})SO₂R^{6a}, Phenyl oder einer Oxo-Gruppe; wobei jede von der Phenyl-Gruppe optional und unabhängig substituiert ist mit bis zu 3 Vorkommen von Halogen, -OH, -NH₂, -NH(C₁₋₄-Alkyl), -N(C₁₋₄-Alkyl)₂, -CN, -CONH₂, C₁₋₄-Alkyl, C₁₋₄-Haloalkyl, -O(C₁₋₄-Alkyl) oder -O(C₁₋₄-Haloalkyl); und wobei jedes von dem C₇₋₁₂-Aralkyl, jedes von dem C₁₋₆-Alkyl, jede von der C₁₋₄-Alkyl-Kette und jeder von dem C₃₋₈-Cycloalkyl-Ring optional und unabhängig substituiert ist mit bis zu drei Vorkommen von Halogen;
jedes R^{6a} unabhängig ausgewählt ist aus Wasserstoff, einem C₁₋₆-Alkyl, einem C₂₋₄-Alkenyl, Phenyl, Benzyl, oder einem C₃₋₈-Cycloalkyl-Ring; wobei jedes von dem C₁₋₆-Alkyl, jedes von dem C₂₋₄ Alkenyl, jedes von dem Phenyl, jedes von dem Benzyl und jeder von dem C₃₋₈-Cycloalkyl-Ring optional und unabhängig substituiert ist mit bis zu 3 Vorkommen von Halogen;
wenn eines von R¹ oder R² der C₃₋₈-Cycloalkyl-Ring, 4- bis 8-gliedrige heterocyclische Ring oder das 5- oder 6-gliedriges Heteroaryl substituiert mit bis zu 5 Vorkommen von R^{5f} ist, zwei der Vorkommen von R^{5f}, die an den gleichen oder verschiedene Ringatome von dem R¹ oder R² angelagert sind, zusammen mit dem Atom oder den Atomen einen C₃₋₈-Cycloalkyl-Ring, einen 4- bis 6-gliedrigen heterocyclischen Ring, ein Phenyl oder einen 5- oder 6-gliedrigen heterocyclischen Ring bilden, was in einem bicylischen System resultiert, wobei die zwei Ringe in einem Spiro-, kondensierten oder überbrückten Verhältnis sind, wobei der 4- bis 6-gliedrige Heterocyclus oder der 5- oder 6-gliedrige heterocyclische Ring bis zu zwei Ring-Heteroatome enthält, unabhängig ausgewählt aus N, O oder S; und wobei der C₃₋₈-Cycloalkyl-Ring, 4- bis 6-gliedrige heterocyclische Ring, das Phenyl oder der 5- oder 6-gliedrige heterocyclische Ring optional substituiert ist durch bis zu 2 Vorkommen von C₁₋₄-Alkyl, C₁₋₄-Haloalkyl, Oxo, -(CO)O(C₁₋₄-Alkyl), -NR'(CO)O(C₁₋₄-Alkyl) oder Halogen; wobei R' Wasserstoff oder ein C₁₋₂-Alkyl ist;
jedes R^{5g} unabhängig ausgewählt ist aus Halogen, -CN, C₁₋₆-Alkyl, -(C₁₋₄-Alkyl)-R^{6b}, einem Benzyl, C₃₋₈-Cycloalkyl-Ring, C₁₋₄(Cyanoalkyl), -OR^{6b}, -SR^{6b}, -OCOR^{6b}, -COR^{6b}, -C(O)OR^{6b}, -C(O)N(R^{6b})₂, -N(R^{6b})C(O)R^{6b}, -N(R^{6b})₂, -SO₂R^{6b}, -SO₂OH, -SO₂NHOH, -SO₂N(R^{6b})(CO)-R^{6b}, -SO₂N(R^{6b})₂, -N(R^{6b})SO₂R^{6b}, Phenyl oder einer Oxo-Gruppe; wobei jedes von dem Phenyl und jede von der Benzyl-Gruppe optional und unabhängig substituiert ist mit bis zu 3 Vorkommen von Halogen, -OH, -NH₂, -NH(C₁₋₄-Alkyl), -N(C₁₋₄-Alkyl)₂, -CN, -CONH₂, C₁₋₄-Alkyl, C₁₋₄-Haloalkyl, -O(C₁₋₄-Alkyl) oder -O(C₁₋₄-Haloalkyl); und wobei jede von der C₁₋₄-Alkyl-Kette und jede von der C₃₋₈-Cycloalkyl-Gruppe optional und unabhängig substituiert ist mit bis zu 3 Vorkommen von Halogen;
jedes R^{6b} unabhängig ausgewählt ist aus Wasserstoff, einem C₁₋₆-Alkyl, einem C₂₋₄-Alkenyl, Phenyl, Benzyl, oder einem C₃₋₈-Cycloalkyl-Ring; wobei jedes von dem C₁₋₆-Alkyl, jedes von dem C₂₋₄-Alkenyl, jedes von dem Phenyl, jedes von dem Benzyl und jede von der C₃₋₈-Cycloalkyl Gruppe optional und unabhängig substituiert ist mit bis zu 3 Vorkommen von Halogen;
alternativ zwei Vorkommen von R^{5g}, die an das gleiche oder an verschiedene Atome von R^{Y} angelagert sind, zusammen mit dem Ringatom oder Ringatomen einen C₃₋₈-Cycloalkyl-Ring, einen 4- bis 6-gliedrigen heterocyclischen Ring; ein Phenyl oder einen 5- oder 6-gliedrigen Heteroaryl-Ring bilden, was in einem bicylischen System resultiert, wobei die zwei Ringe in einem Spiro-, kondensierten oder überbrückten Verhältnis sind, wobei der 4- bis 6-gliedrige Heterocyclus oder der 5- oder 6-gliedrige Heteroaryl-Ring bis zu drei Heteroatome enthält, unabhängig ausgewählt aus N, O oder S; und wobei der C₃₋₈-Cycloalkyl-Ring, 4- bis 6-gliedrige heterocyclische Ring, das Phenyl oder ein 5- oder 6-gliedriger Heteroaryl-Ring optional und unabhängig substituiert ist mit bis zu 3 Vorkommen von C₁₋₄-Alkyl, C₁₋₄-Haloalkyl, C₁₋₄-Alkoxy, C₁₋₄-Haloalkoxy, Oxo, -C(O)O(C₁₋₄-Alkyl), -C(O)OH, -NR"(CO)O(C₁₋₄-Alkyl), -OH oder Halogen; und
R" Wasserstoff oder ein C₁₋₂-Alkyl ist.

4. Verbindung nach Anspruch 1, dargestellt durch **Formel IIb,** oder ein pharmazeutisch annehmbares Salz davon: 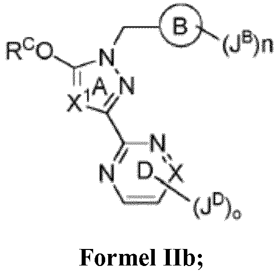 wobei Ring B ein Phenyl oder ein 5- oder 6-gliedriger Heteroaryl-Ring ist, der 1 oder 2 Ring-Heteroatome enthält, ausgewählt aus N, O oder S.

5. Verbindung nach Anspruch 4, oder ein pharmazeutisch annehmbares Salz davon, wobei jedes J^{D} unabhängig ausgewählt ist aus Halogen, einem C₁₋₆ aliphatischen, C₁₋₆ haloaliphatischen, -N(R^{D})₂, -N(R^{d})COR^{D}, -N(R^{d})COOR^{D}, -OR^{D}, -N(R^{d})SO₂R^{D} oder einem optional substituierten C₃₋₈ cycloaliphatischen Ring.

6. Verbindung nach den Ansprüchen 4 oder 5, oder ein pharmazeutisch annehmbares Salz davon, wobei die Verbindung durch **Formel IIIb** dargestellt ist:

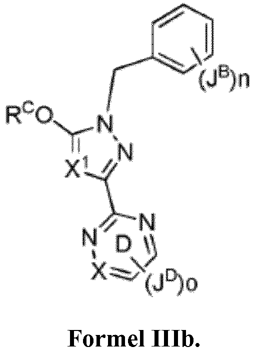

7. Verbindung nach den Ansprüchen 4 oder 6, oder ein pharmazeutisch annehmbares Salz davon, dargestellt durch **Formel IVb:**

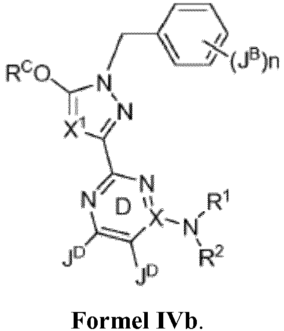

8. Verbindung nach einem der Ansprüche 4 bis 6, oder ein pharmazeutisch annehmbares Salz davon, dargestellt durch **Formel Vb:** 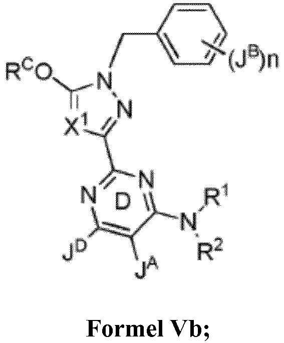 wobei J^{D} fehlt oder ausgewählt ist aus Halogen, Methyl, Hydroxyl, Methoxy, Trifluormethyl, Trifluormethoxy oder -NR^{a}R^{b}; wobei R^{a} und R^{b} jedes unabhängig ausgewählt sind aus Wasserstoff, C₁₋₆-Alkyl oder einem 3-6-Cycloalkyl-Ring; oder wobei R^{a} und R^{b}, zusammen mit dem Stickstoffatom, an das sie beide angelagert sind, einen 4-8-gliedrigen heterocyclischen Ring bilden, oder einen 5-gliedrigenr Heteroaryl-Ring, der optional bis zu zwei zusätzliche Heteroatome enthält, ausgewählt aus N, O und S; wobei jeder von dem 4-8-gliedrigen heterocyclischen Ring und 5-gliedrigen Heteroaryl-Ring optional und unabhängig substituiert ist mit bis zu 5 Vorkommen von Fluor; und J^{A} ausgewählt ist aus Wasserstoff oder Fluor.

9. Verbindung nach einem der Ansprüche 1, 2, 4 und 6, oder ein pharmazeutisch annehmbares Salz davon, wobei jedes J^{D} unabhängig ausgewählt ist aus Halogen, einem C₁₋₆ aliphatischen, einem C₁₋₆ haloaliphatischen -N(R^{D})₂, -N(R^{d})C(O)R^{D}, -N(R^{d})C(O)OR^{D}, -N(R^{d})C(0O)N(R^{D})₂, -SO₂R^{D}, -SO₂N(R^{D})₂, -N(R^{d})SO₂R^{D}, -SR^{D}, -OR^{D}oder einem optional substituierten C₃₋₈ cycloaliphatischen Ring, und wobei o eine Ganzzahl ist, ausgewählt zwischen 1 und 3.

10. Verbindung nach Anspruch 9, oder ein pharmazeutisch annehmbares Salz davon, wobei jedes J^{D} unabhängig ausgewählt ist aus Methyl, Trifluormethyl, Chlor, Fluor, -N(R^{D})₂, N(R^{d})C(O)R^{D}, -N(R^{d})SO₂R^{D} oder- OR^{D}.

11. Verbindung nach Anspruch 1, oder ein pharmazeutisch annehmbares Salz davon, dargestellt durch eine der **Formeln VIIa** oder **VIIIb:**

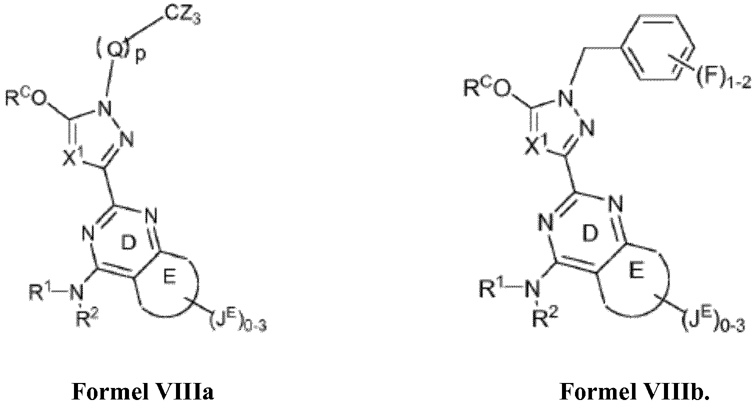

12. Verbindung nach einem der Ansprüche 1, 4, oder 6, oder ein pharmazeutisch annehmbares Salz davon, dargestellt durch eine der **Formeln Xb oder XIb:** 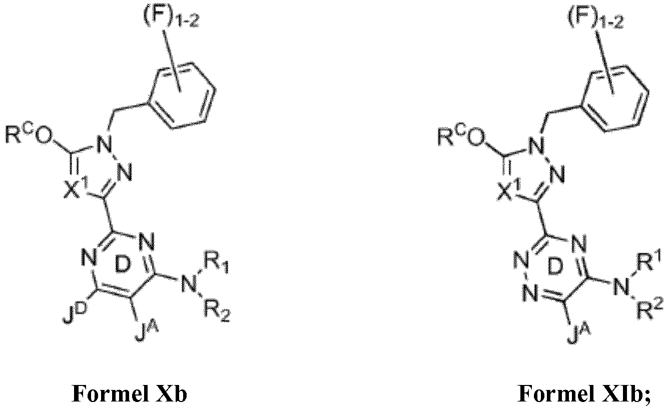
wobei jedes J^{D} unabhängig ausgewählt ist aus -NH₂ oder fehlt; und
jedes J^{A} alternativ wie folgt ist:
i) wenn R¹ und R² nicht gleichzeitig Wasserstoff sind, ist jedes J^{A} unabhängig ausgewählt aus Wasserstoff oder Halogen; oder
ii) wenn R¹ und R² beide gleichzeitig Wasserstoff sind, jedes J^{A} unabhängig ausgewählt ist aus -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂ oder -N(R^{d})SO₂R^{D}.

13. Verbindung nach einem der Ansprüche 1 oder 2, oder ein pharmazeutisch annehmbares Salz davon, dargestellt durch eine der **Formeln XIXa oder Xa:** 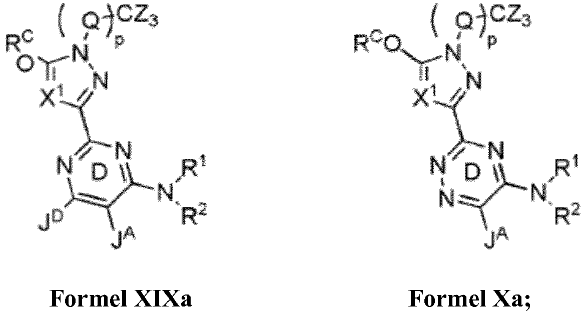 wobei
jedes J^{D} unabhängig ausgewählt ist aus -NH₂ oder fehlt; und jedes J^{A} alternativ wie folgt ist:
i) wenn R¹ und R² nicht gleichzeitig Wasserstoff sind, ist jedes J^{A} unabhängig ausgewählt aus Wasserstoff oder Halogen; oder
ii) wenn R¹ und R² beide gleichzeitig Wasserstoff sind, jedes J^{A} unabhängig ausgewählt ist aus -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂ oder -N(R^{d})SO₂R^{D}.

14. Verbindung nach Anspruch 1, wobei die Verbindung ausgewählt ist aus einer der folgenden Verbindungen: 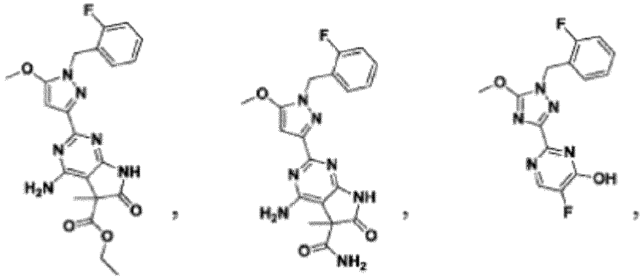 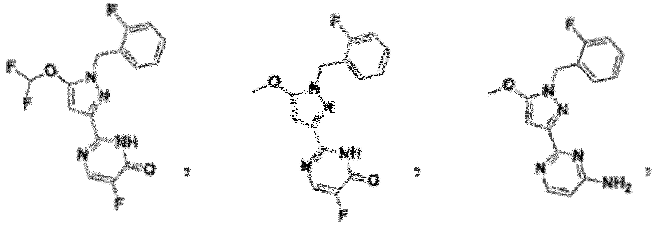 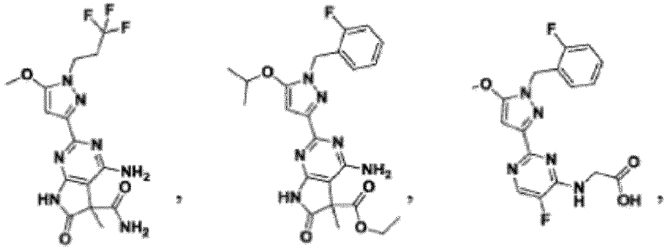 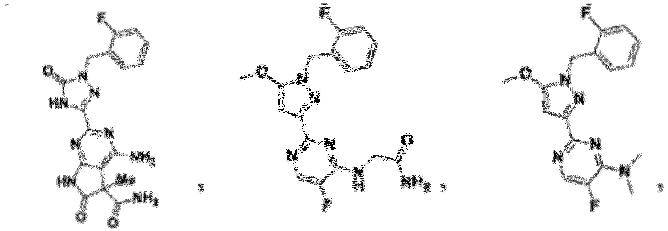 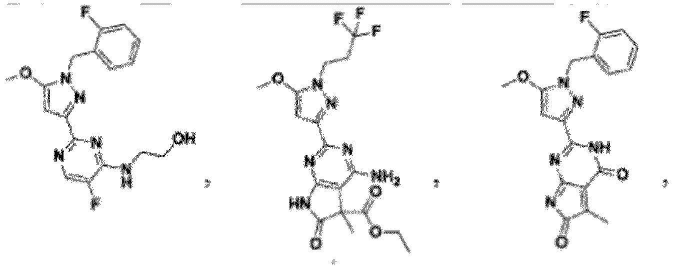 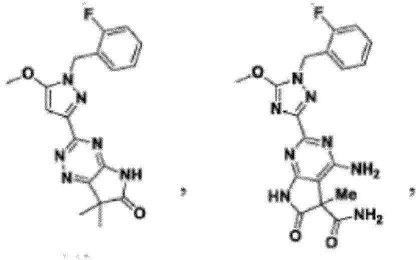 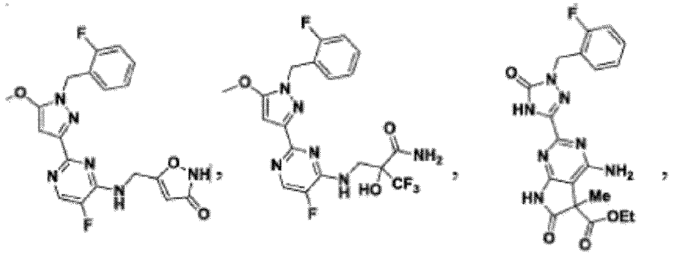 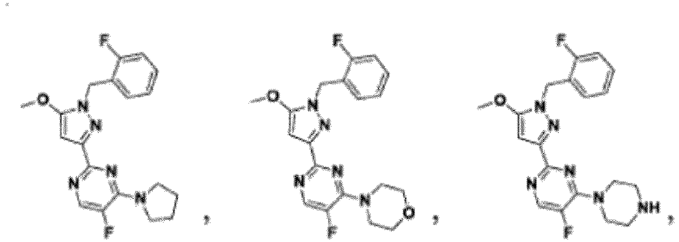 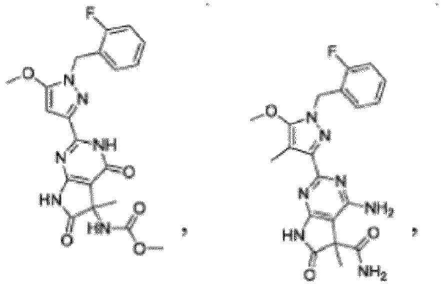 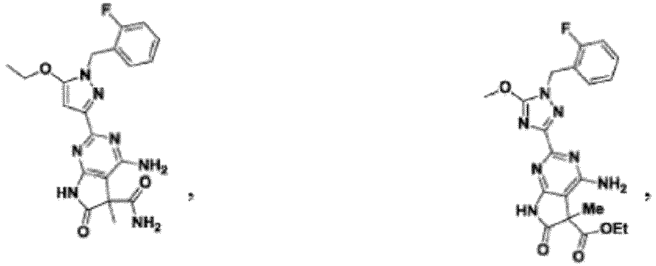 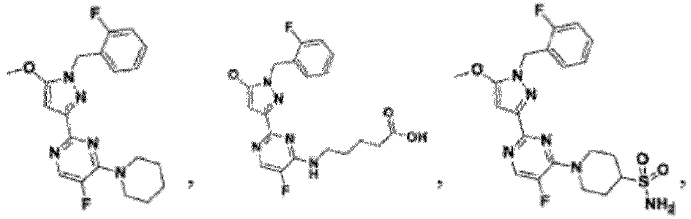 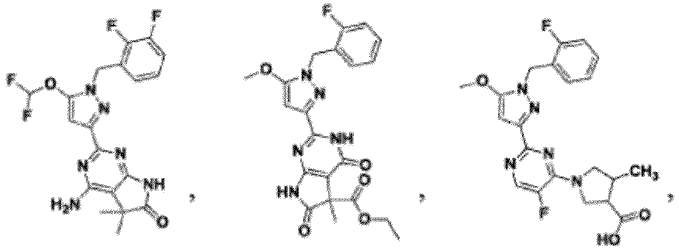 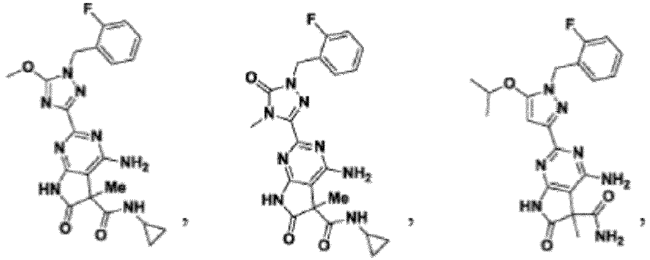 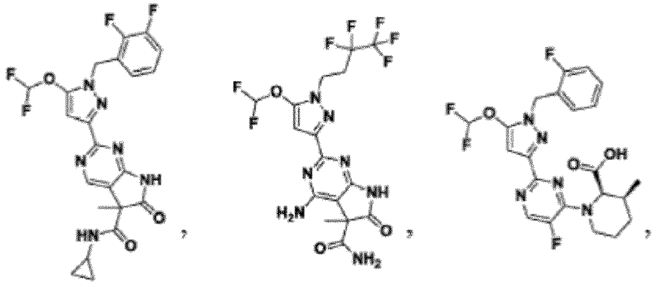 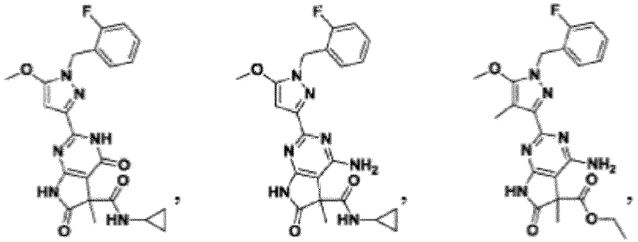 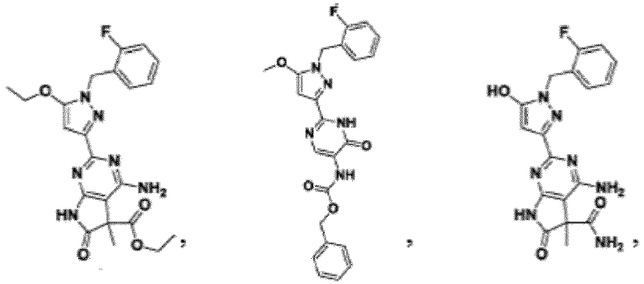 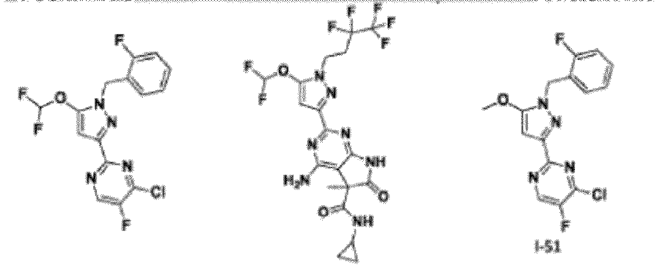 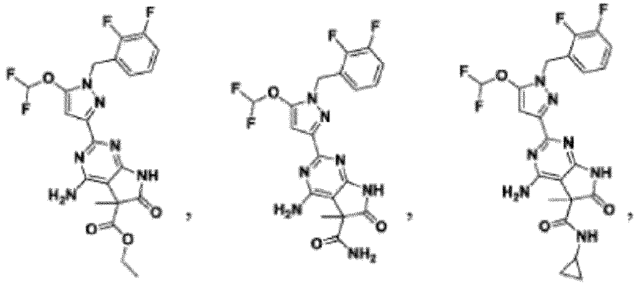 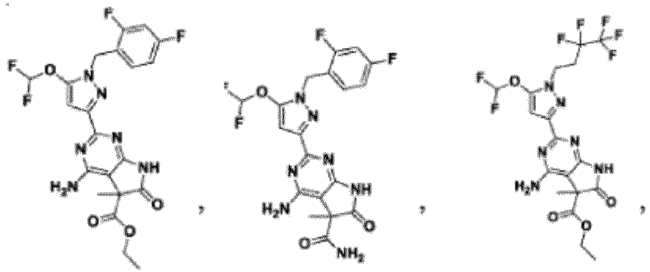 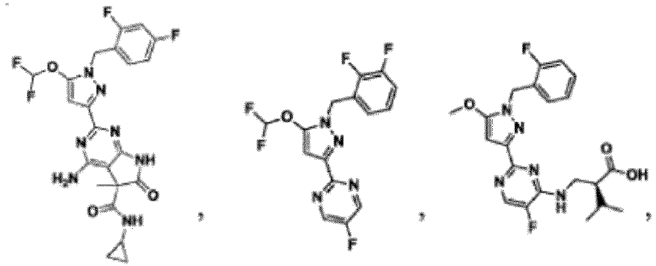 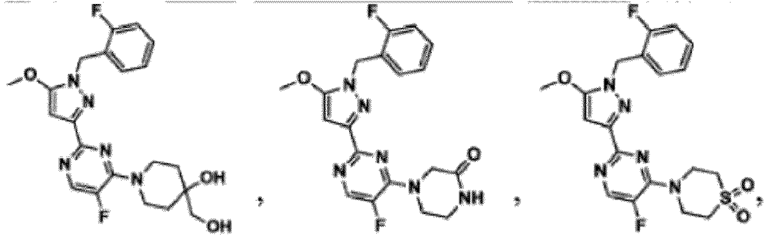 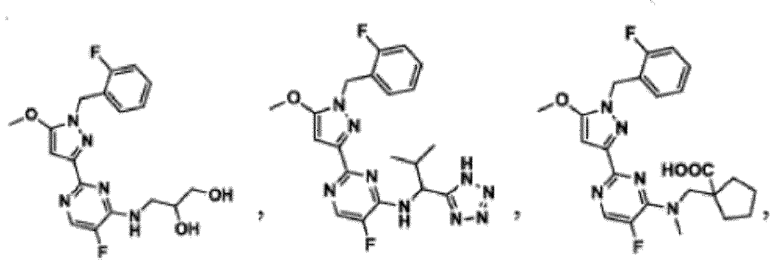 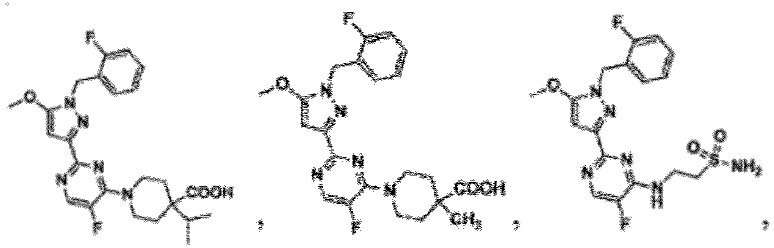 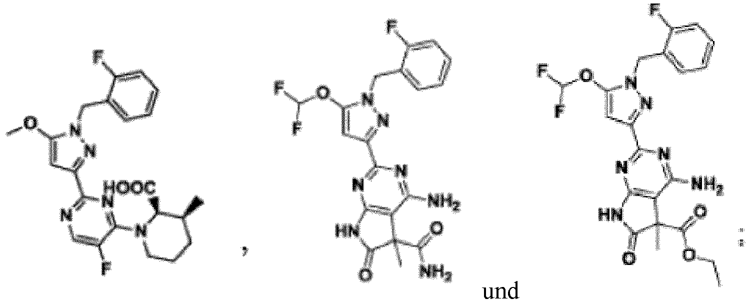 oder einem pharmazeutisch annehmbaren Salz davon.

15. Verbindung nach einem der Ansprüche 1 bis 14 zur Verwendung in der Behandlung von einer Krankheit, eines Gesundheitszustands oder einer Gesundheitsstörung, die aus Folgenden ausgewählt sind:
• Störungen in Bezug auf hohen Blutdruck und verringerten koronaren Blutfluss; erhöhter akuter und chronischer koronarer Blutfluss; arterielle Hypertonie und vaskuläre Störung, die aus kardialen und renalen Komplikationen resultiert; arterielle Hypertonie und vaskuläre Störung, die aus Herzkrankheit, Schlaganfall, zerebraler Ischämie, Nierenversagen oder resistenter Hypertonie resultiert; diabetische Hypertonie; Essentielle Hypotonie; sekundäre Hypertonie;
• Herzversagen; HFPEF; HFREF; akutes und chronisches Herzversagen und spezifischere Formen der Krankheit; akutes dekompensiertes Herzversagen, Rechtsherzversagen, Linksherzversagen, totales Herzversagen, ischämische Kardiomyopathie, dilatierte Kardiomyopathie, kongenitale Herzfehler, Herzversagen mit Herzklappenfehlern, Mitralklappenstenose, Mitralklappeninsuffizienz, Aortenklappenstenose, Aortenklappeninsuffizienz, Trikuspidstenose, trikuspidale Insuffizienz, Pulmonalklappenstenose, Pulmonalklappeninsuffizienz, kombinierte Herzklappenfehler; diabetische Herzfehler; alkoholische Kardiomyopathie, Speicherungs-Kardiomyopathien; diastolisches Herzversagen, systolisches Herzversagen, akute Phasen eines vorliegenden chronischen Herzversagens, degeneratives Herzversagen; diastolische oder systolische Dysfunktion; Koronarinsuffizienz; Arrhythmien; Verringerung von ventrikulärer Vorlast; Herzhypertrophie; Herzfehler/kardiorenales Syndrom; portale Hypertension; endotheliale Dysfunktion oder Verletzung; Störungen des atrialen und ventrikulären Rhythmus und Leitungsstörungen; Atrioventrikularblöcke vom Grad I-III (AVB I-III); supraventrikuläre Tachyarrhythmie, Vorhofflimmern, Vorhofflattern, Herzkammerflimmern, Herzkammerflattern, ventrikuläre Tachyarrhythmie, Torsade-de-Pointes-Tachykardie, atriale und ventrikuläre Extrasystolen, Extrasystolen der AV-Verbindung, Sick-Sinus-Syndrom, Synkopen, Reentry-Tachykardie des AV-Knotens; Wolff-Parkinson-White-Syndrom, akutes Koronarsyndrom; Boxer-Kardiomyopathie; ventrikuläre Extrasystole;
• thromboembolische Störungen und Ischämien, Myokardischämie, Infarzierung, Herzanfall, Herzmuskelschwäche, endotheliale Dysfunktion, Schlaganfall, transitorische ischämische Attacken (transient ischemic attacks, TIAs); obstruktive Thrombangiitis; stabile oder instabile Angina pectoris; Koronarspasmen, Spasmen der Becken-Bein-Arterien; Variantangina, Prinzmetal-Angina; Schlaganfall; Herzhypertrophie; Präeklampsie; thrombogene Störungen; Ischämie-Reperfusions-Schaden; Ischämie-Reperfusion in Verbindung mit Organtransplantation; Ischämie-Reperfusion in Verbindung mit Lungentransplantation, Lungentransplantation oder Herztransplantation; Beibehaltung von Blutsubstituenten bei Traumapatienten;
• periphere Arterienerkrankung; periphere arterielle Verschlusskrankheit; periphere vaskuläre Krankheit; Hypertonie; Raynaud-Syndrom oder Raynaud-Phänomen; primäres und sekundäres Raynaud-Phänomen; Raynaud-Krankheit, Ischämiesyndrom; periphere Embolie; Claudicatio intermitens; gefäßverengende Krise; Duchenne- und Becker-Muskeldystrophie; Mikrozirkulationsanomalien; Kontrolle von Gefäßundichtigkeit oder -permeabilität; lumbale Spinalkanalstenose; okklusive thrombotische Vasculitis; thrombotische Vasculitis; periphere Durchblutungsstörungen; arterielle und venöse Thrombosen; Microalbuminurie; periphere und autonome Neuropathien; diabetische Mikroangiopathien;
• Ödem; Nierenödem wegen Herzversagen;
• Alzheimer-Krankheit; Parkinson-Krankheit; vaskuläre Demenzen; vaskuläre kognitive Beeinträchtigung; zerebraler Vasospasmus; traumatische Hirnverletzung; Verbessern von Wahrnehmung, Konzentrationsfähigkeit, Lernfähigkeit oder Gedächtnisleistung nach kognitiven Störungen, wie sie z. B. jene, die bei einer leichten kognitiven Beeinträchtigung, altersbedingten Lern- und Gedächtnisstörungen, altersbedingtem Gedächtnisverlust, vaskulärer Demenz, Kopfverletzung, Schlaganfall, Demenz nach Schlaganfall, posttraumatischer Kopfverletzung, allgemeinen Konzentrationsstörungen und Konzentrationsstörungen bei Kindern mit Lern- und Gedächtnisproblemen vorkommen; Lewy-Körper-Demenz, Demenz mit Frontallappendegeneration einschließlich Pick-Syndrom; progressive nukleäre Blickparese; Demenz mit kortikobasaler Degeneration; amyotrophe Lateralsklerose (ALS); Huntington-Krankheit; Demyelinisierung, multiple Sklerose, thalamische Degeneration; Creutzfeldt-Jakob-Demenz, HIV-Demenz, Schizophrenie mit Demenz oder Korsakoff-Psychose; multiple Systematrophie und andere Formen von Parkinsonismus Plus; Bewegungsstörungen; Neuroprotektion; Angst, Anspannung und Depression, posttraumatische Belastungsstörung (PTSD); ZNS-bedingte sexuelle Dysfunktion und Schlafstörungen; pathologische Essstörungen und Verwendung von Genussmitteln und Suchtdrogen; Kontrolle von Hirndurchblutung, Kontrolle von Migränen; Prophylaxe und Kontrolle der Folgen von Hirninfarkt (Apoplexia cerebri), wie z. B. Schlaganfall, zerebrale Ischämie und Kopfverletzung;
• Schock; kardiogener Schock; Sepsis oder septischer Schock oder anaphylaktischer Schock; Aneurysma; Kontrolle von Leukozytenaktivierung; Hemmung oder Modulation von Thrombozytenaggregation; Multiorganversagen (MOV);
• pulmonale/respiratorische Bedingungen; pulmonale Hypertonie (PH), pulmonale arterielle Hypertonie (PAH) und assoziierte pulmonale vaskuläre Remodellierung; lokalisierte Thrombose und rechte Ventrikelhypertrophie; pulmonale Hypertonie; primäre pulmonale Hypertonie, sekundäre pulmonale Hypertonie, familiäre pulmonale Hypertonie, sporadische pulmonale Hypertonie, präkapilläre pulmonale Hypertonie, idiopathische pulmonale Hypertonie; thrombotische pulmonale Arteriopathie, plexogene pulmonale Arteriopathie; zystische Fibrose; Bronchialverengung oder pulmonale Bronchialverengung; akutes respiratorisches Distresssyndrom; Lungenfibrose, Lungentransplantation; asthmatische Krankheiten; andere Formen von PH; PH assoziiert mit linksventrikulärer Krankheit, HIV, SCD, Thrombembolie (CTEPH), Sarkoidose, COPD oder pulmonale Fibrose; akutes respiratorisches Distresssyndrom (ARDS), akute Lungenverletzung, Alpha-1-Antitrypsin-Mangel (A1AD), pulmonale Emphysem; durch Rauchen induziertes Emphysem und CF;
• pulmonale Hypertonie assoziiert mit oder in Bezug auf: linksventrikuläre Dysfunktion, Hypoxämie, WHO-Gruppen I, II, III, IV und V Hypertensionen, Mitralklappenkrankheit, konstriktive Perikarditis, Aortastenose, Kardiomyopathie, Mediastinalfibrose, pulmonale Fibrose, anomaler pulmonaler venöser Abfluss, Lungenvenen-Verschlusskrankheitt, pulmonale Vaskulitis, kollagenvaskuläre Krankheit, kongenitale Herzkrankheit, Lungenvenenhypertonie, interstitielle Lungenkrankheit, schlafbezogene Atmungsstörung, Schlafapnoe, alveoläre Hypoventilationsstörungen, chronische Aussetzung an große Höhe, neonatale Lungenkrankheit, alveoläre kapillare Dysplasie, Sichelzellenkrankheit, andere Koagulationsstörungen, chronische Thrombembolie, Lungenembolie, Embolie wegen Tumor, Parasiten oder Fremdmaterial; pulmonale Hypertonie assoziiert mit oder in Bezug auf: Bindegewebekrankheit, Lupus, Schistosomiasis, Sarkoidose, chronische obstruktive pulmonale Krankheit, Asthma, Emphysem, chronische Bronchitis oder pulmonale kapillare Hämangiomatose; Histiozytose X, Lymphangiomatose und komprimierte Lungengefäße; komprimierte Gefäße durch Drüsenerkrankung, Tumor oder fibrosierende Mediastinitis;
• arteriosklerotische Krankheiten oder Zustände; Arteriosklerose, Arteriosklerose assoziiert mit endothelialer Verletzung, Thrombozyt- und Monozytadhäsion und -aggregation, Proliferation und Migration von glattem Muskel; Restenose; Restenose, die sich nach Thrombolyse-Therapien entwickelt hat, perkutane transluminale Angioplastien (PTAs), transluminale Coronar-Angioplastien (PTCAs), Herztransplantations- und Bypass-Operationen; Entzündungsprozesse;
• mikro- und makrovaskulärer Schaden; Vaskulitis; erhöhte Werte von Fibrinogen und DLD niedriger Dichte, erhöhte Konzentration von Plasminogenaktivator-Hemmer 1 (PA-1);
• Krankheiten, die mit metabolischem Syndrom assoziiert sind: Adipositas, Dyslipidämie, Diabetes, hoher Blutdruck; lipid-bedingte Störungen: Dyslipidämie, Hypercholesterinämie, erhöhte Cholesterol-Werte des Lipoproteins hoher Dichte (HDL-Cholesterol) und in einigen Fällen moderat erhöhte Cholesterol-Werte des Lipoproteins niedriger Dichte (LDL-Cholesterol), Hypertriglyceridämien, Hyperglyceridemie, Hypolipoproteinanämien, Sitosterolämie, Fettleberkrankheit und Hepatitis; Präeklampsie; polyzystischer Nierenkrankheitfortschritt; subkutanes Fett; Adipositas; Lebersteatose oder anormale Lipid-Ansammlung in der Leber; Steatose des Herzens, der Nieren oder Muskeln; Abetalipoproteinämie; Sitosterolämie; Xanthomatose; Tangier-Krankheit; Obesitas; kombinierte Hyperlipidämien und metabolisches Syndrom; Hyperammonämie und verwandte Krankheiten und Störungen; hepatische Encephalopathien und andere toxische Encephalopathien und Reye-Syndrom;
• sexuelle, gynäkologische und urologische Störungen oder Zustände; erektile Dysfunktion; Impotenz; vorzeitige Ejakulation; weibliche sexuelle Dysfunktion; weibliche sexuelle Erregungsdysfunktion, hypoaktive sexuelle Erregungsstörung, vaginale Atrophie, Dyspareunie, atrophische Vaginitis, gutartige Prostatahyperplasie (BPH) oder Hypertrophie oder Vergrößerung; Blasenauslassobstruktion; Blasenschmerzsyndrom (BPS); interstitielle Zystitis (IC); überaktive Blase; neurogene Blase und Inkontinenz; diabetische Nephropathie; primäre und sekundäre Dysmenorrhö; Syndrome des unteren Harntrakts (LUTS); Beckenschmerzen; gutartige und bösartige Krankheiten der Organe des männlichen und weiblichen urogenitalen Systems;
• akute und chronische Niereninsuffizienz, akutes und chronisches Nierenversagen, sowie zugrundeliegende oder verwandte Nierenkrankheiten, wie z. B. Hypoperfusion, intradialytische Hypotension, obstruktive Uropathie, Glomerulopathien, Glomerulonephritis, akute Glomerulonephritis, Glomerulosklerose, tubulointerstitielle Krankheiten, nephropathische Krankheiten; primäre und kongenitale Nierenkrankheiten; Nephritis; Krankheiten, die durch abnormal reduzierte Kreatinin- und/oder Wasserausscheidung, abnormal erhöhte Blutkonzentration von Harnstoff, Stickstoff, Kalium und/oder Kreatinin, eine veränderte Aktivität von Nierenenzymen, veränderte Urinosmolarität oder verändertes Urinvolumen, erhöhte Microalbuminurie, Macroalbuminurie, Verletzungen von Glomeruli und Arteriolen, tubuläre Dilatation, Hyperphosphatemie und/oder Notwendigkeit einer Dialyse gekennzeichnet sind; Folgeschäden von Niereninsuffizienz; Lungenödem, Herzversagen, Uremie, Anemie, Elekrolytstörungen; Hyperkaliämie, Hyponatriämie; Störungen des Knochen- und Kohlenhydratmetabolismus;
• Augenkrankheiten oder Augenstörungen; Glaukom, Retinopathie oder diabetische Retinopathie.

## Revendications

1. Composé selon la formule I, ou sel pharmaceutiquement acceptable de celui-ci, 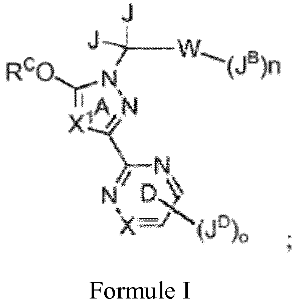
dans laquelle X est choisi parmi un atome N ou C ;
X¹ est choisi parmi un atome N, un groupe CH, C((C₁ à C₄)alkyle), C((C₁ à C₄)fluoroalkyle), C(Cl) et CF ;
W est soit
i) absent, J^{B} étant directement relié à l'atome de carbone portant deux groupes J, chaque groupe J étant indépendamment choisi parmi un atome d'hydrogène, un groupe méthyle ou un atome de fluor, n valant 1 et J^{B} représentant une chaîne (C₁ à C₆)alkyle éventuellement substituée par jusqu'à 6 occurrences d'un atome de fluor ; soit
ii) un noyau B choisi parmi le noyau phényle ou un noyau hétéroaryle à 5 ou 6 chaînons, contenant 1 ou 2 hétéroatomes de cycle choisis parmi un atome N, O ou S ; dans lequel lorsque W représente un noyau B :
chaque groupe J représente un atome d'hydrogène ;
N vaut 0 ou représente un entier choisi parmi 1 à 3 ;
et chaque groupe J^{B} est indépendamment choisi parmi un atome d'halogène, un groupe -CN, un groupe (C₁ à C₆)aliphatique, un groupe -OR^{B} ou un groupe (C₃ à C₈)cycloaliphatique ; chaque dit groupe (C₁ à C₆)aliphatique et chaque dit groupe (C₃ à C₈)cycloaliphatique étant éventuellement et indépendamment substitué par jusqu'à 3 occurrences de R³ ;
chaque groupe R^{B} est indépendamment choisi parmi un atome d'hydrogène, un groupe (C₁ à C₆)aliphatique ou un groupe (C₃ à C₈)cycloaliphatique ; chaque dit groupe R^{B} qui représente un groupe (C₁ à C₆)aliphatique et chaque dit groupe R^{B} qui représente un groupe (C₃ à C₈)cycloaliphatique étant éventuellement et indépendamment substitué par jusqu'à 3 occurrences de R^{3a} ;
chaque groupe R³ étant indépendamment choisi parmi un atome d'halogène, un groupe -CN, un groupe (C₁ à C₄)alkyle, un groupe (C₁ à C₄)halogénoalkyle, un groupe -O((C₁ à C₄)alkyle) ou un groupe -O((C₁ à C₄)halogénoalkyle) ;
chaque groupe R^{3a} est indépendamment choisi parmi un atome d'halogène, un groupe -CN, un groupe (C₁ à C₄)alkyle, un groupe (C₁ à C₄)halogénoalkyle, un groupe -O((C₁ à C₄)alkyle) ou un groupe -O((C₁ à C₄)halogénoalkyle) ;
o vaut 0 ou représente un entier choisi parmi 1 à 3 ;
chaque groupe J^{D} est soit absent soit indépendamment choisi parmi un atome d'hydrogène, un atome d'halogène, un groupe -CN, -NO₂, -OR^{D}, -SR^{D}, -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂, -N(R^{d})SO₂R^{D}, un groupe (C₁ à C₆)aliphatique, un groupe -((C₁ à C₆)aliphatique)-R^{D}, un noyau (C₃ à C₈)cycloaliphatique, un noyau aryle de 6 à 10 chaînons, un noyau hétérocyclique de 4 à 8 chaînons ou un noyau hétéroaryle de 5 à 10 chaînons ; chaque dit noyau hétérocyclique de 4 à 8 chaînons et chaque dit noyau hétéroaryle de 5 à 10 chaînons contenant entre 1 à 3 hétéroatomes indépendamment choisis parmi un atome O, N ou S ; et chacune desdites chaînes (C₁ à C₆)aliphatiques, chaque dit noyau (C₃ à C₈)cycloaliphatique, chaque dit noyau aryle de 6 à 10 chaînons, chaque dit noyau hétérocyclique de 4 à 8 chaînons et chaque dit noyau hétéroaryle de 5 à 10 chaînons étant éventuellement et indépendamment substitué par jusqu'à 5 occurrences de R⁵ ;
chaque groupe R^{D} est indépendamment choisi parmi un atome d'hydrogène, un groupe (C₁ à C₆)aliphatique, un groupe -((C₁ à C₆)aliphatique)-R^{f}, un noyau (C₃ à C₈)cycloaliphatique, un noyau hétérocyclique de 4 à 8 chaînons et un groupe phényle ; chaque dit noyau hétérocyclique de 4 à 8 chaînons contenant entre 1 à 3 hétéroatomes choisis indépendamment parmi un O, N et S ; et chacune desdites chaîne (C₁ à C₆)aliphatique, chaque dit noyau (C₃ à C₈)cycloaliphatique, chaque dit noyau hétérocyclique de 4 à 8 chaînons et chaque dit groupe phényle étant éventuellement et indépendamment substitué par jusqu'à 5 occurrences de R^{5a} ; lorsqu'un quelconque groupe R^{D} est l'un d'un groupe (C₁ à C₆)aliphatique ou d'un groupe -((C₁ à C₆)aliphatique)-R^{f}, une ou deux unités -CH₂- qui forment lesdites chaînes (C₁ à C₆)aliphatiques pouvant, éventuellement, être remplacées par un groupe indépendamment choisi parmi un groupe -C(O)-, -N(R^{d}) - ou -O- ;
chaque groupe R^{d} est indépendamment choisi parmi un atome d'hydrogène, un groupe (C₁ à C₆)aliphatique, un groupe -((C₁ à C₆)aliphatique)-R^{f}, un noyau (C₃ à C₈)cycloaliphatique, un noyau hétérocyclique de 4 à 8 chaînons, un groupe phényle ou un noyau hétéroaryle de 5 ou 6 chaînons ; chaque dit noyau hétérocyclique de 4 à 8 chaînons et chaque dit noyau hétéroaryle de 5 ou 6 chaînons contenant entre 1 à 3 hétéroatomes indépendamment choisis parmi un atome O, N ou S ; et chacune desdites chaînes (C₁ à C₆)aliphatiques, chaque dit noyau (C₃ à C₈)cycloaliphatique, chaque dit noyau hétérocyclique de 4 à 8 chaînons, chaque dit groupe phényle et chaque dit noyau hétéroaryle de 5 à 6 chaînons étant éventuellement et indépendamment substitué par jusqu'à 5 occurrences de R^{5b} ;
chaque groupe R^{f} est indépendamment choisi parmi un groupe (C₁ à C₃)alkyle, un noyau (C₃ à C₈)cycloaliphatique, un noyau hétérocyclique de 4 à 8 chaînons, un groupe phényle ou un noyau hétéroaryle de 5 à 6 chaînons ; chaque dit noyau hétérocyclique de 4 à 8 chaînons et chaque dit noyau hétéroaryle de 5 à 6 chaînons contenant entre 1 et 4 hétéroatomes indépendamment choisi parmi un atome O, N ou S ; et chaque dit noyau (C₃ à C₈)cycloaliphatique, chaque dit noyau hétérocyclique de 4 à 8 chaînons, chaque dit groupe phényle et chaque dit noyau hétéroaryle de 5 à 6 chaînons étant éventuellement et indépendamment substitué par jusqu'à 5 occurrences de R^{5c} ;
lorsque le groupe J^{D} représente un groupe -C(O)N(R^{D})₂, -N(R^{D})₂, -N(R^{d})C(O)N(R^{D})₂, -OC(O)N(R^{D})₂ ou -SO₂N(R^{D})₂, les deux groupes R^{D} conjointement avec l'atome d'azote lié au deux groupes R^{D} peuvent former un noyau hétérocyclique de 4 à 8 chaînons ou un noyau hétéroaryle de 5 chaînons ; chaque dit noyau hétérocyclique de 4 à 8 chaînons et chaque dit noyau hétéroaryle de 5 chaînons contenant éventuellement jusqu'à 3 hétéroatomes supplémentaires indépendamment choisis parmi un atome N, O ou S, en plus de l'atome d'azote auquel les deux groupes R^{D} sont liés ; et chaque dit noyau hétérocyclique de 4 à 8 chaînons et chaque dit noyau hétéroaryle de 5 chaînons étant éventuellement et indépendamment substitué par jusqu'à 5 occurrences de R^{5d};
lorsque le groupe J^{D} représente un groupe -N(R^{d})C(O)R^{D}, le groupe R^{D} conjointement avec l'atome de carbone lié au groupe R^{D}, avec l'atome d'azote lié au groupe R^{d}, et avec le groupe R^{d} peuvent former un noyau hétérocyclique de 4 à 8 chaînons ou un noyau hétéroaryle de 5 chaînons ; chaque noyau hétérocyclique de 4 à 8 chaînons et chaque noyau hétéroaryle de 5 chaînons contenant éventuellement 2 hétéroatomes supplémentaires indépendamment choisis parmi un atome N, O ou S, en plus de l'atome d'azote auquel le groupe R^{d} est lié ; et chaque dit noyau hétérocyclique de 4 à 8 chaînons et chaque dit noyau hétéroaryle de 5 chaînons étant éventuellement et indépendamment substitué par jusqu'à 5 occurrences de R^{5d};
lorsque le groupe J^{D} représente un groupe -N(R^{d})C(O)OR^{D}, le groupe R^{D} conjointement avec l'atome d'oxygène lié au groupe R^{D}, avec l'atome de carbone de la portion -C(O)- du groupe -N(R^{d})C(O)OR^{D}, avec l'atome d'azote lié au groupe R^{d}, et avec ledit groupe R^{d}, peut former un noyau hétérocyclique de 4 à 8 chaînons ; ledit noyau hétérocyclique de 4 à 8 chaînons contenant éventuellement jusqu'à 2 hétéroatomes supplémentaires indépendamment choisis parmi un atome N, O ou S, et étant éventuellement et indépendamment substitué par jusqu'à 5 occurrences de R^{5d};
lorsque le groupe J^{D} représente un groupe -N(R^{d})C(O)N(R^{D})₂, l'un des groupes R^{D} lié à l'atome d'azote, conjointement avec l'atome d'azote, et avec l'atome N lié au groupe R^{d} group et ledit groupe R^{d} peut former un noyau hétérocyclique de 4 à 8 chaînons, ledit noyau hétérocyclique de 4 à 8 chaînons contenant éventuellement jusqu'à 2 hétéroatomes supplémentaires indépendamment choisis parmi un atome N, O or S, et étant éventuellement et indépendamment substitué par jusqu'à 5 occurrences de R^{5d};
lorsque le groupe J^{D} représente un groupe -N(R^{d})SO₂R^{D}, le groupe R^{D} conjointement avec l'atome de soufre lié au groupe R^{D}, avec l'atome d'azote lié au groupe R^{d}, et avec ledit groupe R^{d} peut se combiner pour former un noyau hétérocyclique de 4 à 8 chaînons ; ledit noyau hétérocyclique de 4 à 8 chaînons contenant éventuellement jusqu'à 2 hétéroatomes supplémentaires indépendamment choisis parmi un atome N, O or S, et étant éventuellement et indépendamment substitué par jusqu'à 5 occurrences de R^{5d};
chaque groupe R⁵ étant indépendamment choisi parmi un atome d'halogène, un groupe -CN, un groupe (C₁ à C₆)alkyle, un groupe -((C₁ à C₆)alkyl)-R⁶, un groupe -OR⁶, -SR⁶, -COR⁶, -OC(O)R⁶, -C(O)OR⁶, -C(O)N(R⁶)₂, -N(R⁶)C(O)R⁶, -N(R⁶)C(O)OR⁶, -N(R⁶)C(O)N(R⁶)₂, -N(R⁶)₂, -SO₂R⁶, -SO₂OH, -SO₂NHOH, -SO₂N(R⁶)₂, -SO₂N(R⁶)(CO)-R⁶, -N(R⁶)SO₂R⁶, un groupe (C₇ à C₁₂)aralkyle, un noyau (C₃ à C₈)cycloalkyle, un noyau hétérocyclique de 4 à 7 chaînons, un noyau hétéroaryle de 5 ou 6 chaînons, un groupe phényle ou un groupe oxo; chaque noyau hétéroaryle de 5 ou 6 chaînons ou chaque noyau hétérocyclique de 4 à 7 chaînons contenant jusqu'à quatre hétéroatomes de cycle indépendamment choisis parmi un atome N, O et S, chacun desdites chaînes (C₁ à C₆)alkyle, dudit groupe (C₇ à C₁₂)aralkyle, dudit noyau (C₃ à C₈)cycloalkyle, dudit noyau hétérocyclique de 4 à 7 chaînons, dudit noyau hétéroaryle de 5 ou 6 chaînons ou dudit groupe phényle étant éventuellement et indépendamment substitué par jusqu'à 3 occurrences d'un atome d'halogène, d'un groupe (C₁ à C₄)alkyle, (C₁ à C₄)halogénoalkyle, -OH, -NH₂, -NH((C₁ à C₄)alkyle), -N((C₁ à C₄)alkyle)₂, -CN, -COOH, -CONH₂, -COO((C₁ à C₄)alkyle), -O((C₁ à C₄)alkyle), -O((C₁ à C₄)halogénoalkyle) ou oxo ;
sinon, deux occurrences de R⁵ liées au même atome ou à des atomes différents du groupe J^{D}, conjointement avec ledit atome ou lesdits atomes du groupe J^{D} auxquels elles sont liées, peuvent former un noyau (C₃ à C₈)cycloalkyle, un noyau hétérocyclique de 4 à 6 chaînons, un groupe phényle ou un noyau hétéroaryle de 5 ou 6 chaînons, conduisant à un système bicyclique dans lequel les deux noyaux du système bicyclique se trouvent dans une relation spiro, fusionnée ou pontée l'un par rapport à l'autre, ledit hétérocycle de 4 à 6 chaînons ou ledit noyau hétéroaryle de 5 ou 6 chaînons contenant jusqu'à quatre hétéroatomes de cycle indépendamment choisis parmi un atome N, O ou S ; et ledit noyau (C₃ à C₈)cycloalkyle, noyau hétérocyclique de 4 à 6 chaînons, groupe phényle ou noyau hétéroaryle de 5 ou 6 chaînons étant éventuellement et indépendamment substitué par jusqu'à 3 occurrences d'un groupe (C₁ à C₄)alkyle, (C₁ à C₄)halogénoalkyle, (C₁ à C₄)alcoxy, (C₁ à C₄)halogénoalcoxy, oxo, -C(O)O((C₁ à C₄)alkyle), -C(O)OH, -NR(CO)O((C₁ à C₄)alkyle), -CONH₂, -OH ou d'un atome d'halogène ; où R représente un atome d'hydrogène ou un groupe (C₁ à C₂)alkyle ;
chaque groupe R^{5a} est indépendamment choisi parmi un atome d'halogène, un groupe -CN, un groupe (C₁ à C₆)alkyle, un groupe -((C₁ à C₆)alkyl)-R^{6a}, un groupe -OR^{6a}, -SR^{6a}, -COR^{6a}, -OC(O)R^{6a}, -C(O)OR^{6a}, -C(O)N(R^{6a})₂, -N(R^{6a})C(O)R^{6a}, -N(R^{6a})C(O)OR^{6a}, -N(R^{6a})C(O)N(R^{6a} )₂, -N(R^{6a} )₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, un groupe (C₇ à C₁₂)aralkyle, un noyau (C₃ à C₈)cycloalkyle, un noyau hétérocyclique de 4 à 7 chaînons, un noyau hétéroaryle de 5 ou 6 chaînons, un groupe phényle, ou un groupe oxo ; chaque noyau hétéroaryle de 5 ou 6 chaînons ou chaque noyau hétérocyclique de 4 à 7 chaînons contenant jusqu'à quatre hétéroatomes de cycle indépendamment choisis parmi un atome N, O et S, chaque desdites chaînes (C₁ à C₆)alkyle, chaque dit groupe (C₇ à C₁₂)aralkyle, dit noyau (C₃ à C₈)cycloalkyle, dit noyau hétérocyclique de 4 à 7 chaînons, dit noyau hétéroaryle de 5 ou 6 chaînons ou groupe phényle étant éventuellement et indépendamment substitué par jusqu'à 3 occurrences d'un atome d'halogène, d'un groupe (C₁ à C₄)alkyle, (C₁ à C₄)halogénoalkyle, -OH, -NH₂, -NH((C₁ à C₄)alkyle), -N((C₁ à C₄)alkyle)₂, -CN, -COOH, -COO((C₁ à C₄)alkyle), -CONH₂, -O((C₁ à C₄)alkyle), -O((C₁ à C₄)halogénoalkyle) ou oxo ;
chaque groupe R^{5b} est indépendamment choisi parmi un atome d'halogène, un groupe -CN, un groupe (C₁ à C₆)alkyle, un groupe -((C₁ à C₆)alkyl)-R^{6a}, un groupe -OR^{6a}, -SR^{6a}, -COR^{6a}, -OC(O)R^{6a}, -C(O)OR^{6a}, -C(O)N(R^{6a})₂, -N(R^{6a})C(O)R^{6a}, -N(R^{6a})C(O)OR^{6a}, -N(R^{6a})C(O)N(R^{6a})₂, -N(R^{6a})₂, -SO₂R^{6a}, -SO₂OH, -SO₂NHOH, -SO₂N(R^{6a})₂, -SO₂N(R^{6a})(CO)-R^{6a}, -N(R^{6a})SO₂R^{6a}, un groupe (C₇ à C₁₂)aralkyle, un noyau (C₃ à C₈)cycloalkyle, un noyau hétérocyclique de 4 à 7 chaînons, un noyau hétéroaryle de 5 ou 6 chaînons, un groupe phényle ou un groupe oxo ; chaque noyau hétéroaryle de 5 ou 6 chaînons ou noyau hétérocyclique de 4 à 7 chaînons contenant jusqu'à quatre hétéroatomes de cycle indépendamment choisis parmi un atome N, O et S, chacune desdites chaînes (C₁ à C₆)alkyle, chaque dit groupe (C₇ à C₁₂)aralkyle, dit noyau (C₃ à C₈)cycloalkyle, dit noyau hétérocyclique de 4 à 7 chaînons, dit noyau hétéroaryle de 5 ou 6 chaînons, un groupe phényle étant éventuellement et indépendamment substitué par jusqu'à 3 occurrences d'un atome d'halogène, un groupe (C₁ à C₄)alkyle, (C₁ à C₄)halogénoalkyle, -OH, -NH₂, -NH((C₁ à C₄)alkyle), -N((C₁ à C₄)alkyle)₂, -CN, -COOH, -COO((C₁ à C₄)alkyle), -CONH₂, -O((C₁ à C₄)alkyle), -O((C₁ à C₄)halogénoalkyle) ou oxo ;
sinon, deux occurrences de R^{5a} ou deux occurrences de R^{5b} liées au même atome ou à des atomes différents du groupe R^{D} ou R^{d}, respectivement, conjointement avec ledit atome ou lesdites atomes auxquels elles sont liées, peuvent former un noyau (C₃ à C₈)cycloalkyle, un noyau hétérocycle de 4 à 6 chaînons, un groupe phényle ou un noyau hétéroaryle de 5 ou 6 chaînons, conduisant à un système bicyclique dans lequel les deux noyaux du système bicyclique se trouvent dans une relation spiro, fusionnée ou pontée l'un par rapport à l'autre, ledit hétérocycle de 4 à 6 chaînons ou ledit noyau hétéroaryle de 5 ou 6 chaînons contenant jusqu'à quatre hétéroatomes de cycle indépendamment choisis parmi un atome N, O ou S ; et ledit noyau (C₃ à C₈)cycloalkyle, noyau hétérocyclique de 4 à 6 chaînons, groupe phényle ou noyau hétéroaryle de 5 ou 6 chaînons étant éventuellement et indépendamment substitué par jusqu'à 3 occurrences d'un groupe (C₁ à C₄)alkyle, (C₁ à C₄)halogénoalkyle, (C₁ à C₄)alcoxy, (C₁ à C₄)halogénoalcoxy, oxo, -C(O)O((C₁ à C₄)alkyle), -C(O)OH, -NR(CO)O((C₁ à C₄)alkyle), -CONH₂, -OH ou d'un atome d'halogène ; où R représente un atome d'hydrogène ou un groupe (C₁ à C₂)alkyle ;
chaque groupe R^{5C} est indépendamment choisi parmi un atome d'halogène, un groupe -CN, un groupe (C₁ à C₆)alkyle, un groupe -((C₁ à C₆)alkyl)-R^{6b}, un groupe -OR^{6b}, -SR^{6b}, -COR^{6b}, -OC(O)R^{6b}, -C(O)OR^{6b}, -C(O)N(R^{6b})₂, -N(R^{6b})C(O)R^{6b},-N(R^{6b})C(O)OR^{6b}, -N(R^{6b})C(O)N(R^{6b})₂, -N(R^{6b})₂, -SO₂R^{6b}, -SO₂OH, -SO₂NHOH, -SO₂N(R^{6b})(CO)-R^{6b}, -SO₂N(R^{6b})₂, -N(R^{6b})SO₂R^{6b}, un groupe (C₇ à C₁₂)aralkyle, un noyau (C₃ à C₈)cycloalkyle, un cycle hétérocyclique de 4 à 7 chaînons, un noyau hétéroaryle de 5 ou 6 chaînons, un groupe phényle ou un groupe oxo ; chaque noyau hétéroaryle de 5 ou 6 chaînons ou cycle hétérocyclique de 4 à 7 chaînons contenant jusqu'à quatre hétéroatomes de cycle indépendamment choisis parmi un atomes N, O et S, chacun desdites chaînes (C₁ à C₆)alkyle, dudit groupe (C₇ à C₁₂)aralkyle, dudit noyau (C₃ à C₈)cycloalkyle, dudit cycle hétérocyclique de 4 à 7 chaînons, dudit noyau hétéroaryle de 5 ou 6 chaînons ou desdits groupes phényle étant éventuellement et indépendamment substitué par jusqu'à 3 occurrences d'un atome d'halogène, un groupe (C₁ à C₄)alkyle, (C₁ à C₄)halogénoalkyle, -OH, -NH₂, -NH((C₁ à C₄)alkyle), -N((C₁ à C₄)alkyle)₂, -CN, -COOH, -CONH₂, -COO((C₁ à C₄)alkyle), -O((C₁ à C₄)alkyle), -O((C₂ à C₄)halogénoalkyle) ou oxo ;
sinon, deux occurrences de R^{5c} liées au même atome ou à des atomes différents du groupes R^{f}, conjointement avec ledit atome ou lesdits atomes auxquels elles sont liées, peuvent former un noyau (C₃ à C₈)cycloalkyle, un noyau hétérocyclique de 4 à 6 chaînons, un groupe phényle ou un noyau hétéroaryle de 5 ou 6 chaînons, conduisant à un système bicyclique dans lequel les deux noyaux du système bicyclique se trouvent dans une relation spiro, fusionnée ou pontée l'un par rapport à l'autre, ledit hétérocycle de 4 à 6 chaînons ou ledit noyau hétéroaryle de 5 ou 6 chaînons contenant jusqu'à quatre hétéroatomes de cycle indépendamment choisis parmi un atome N, O ou S ; et ledit noyau (C₃ à C₈)cycloalkyle, noyau hétérocyclique de 4 à 6 chaînons, groupe phényle ou noyau hétéroaryle de 5 ou 6 chaînons étant éventuellement et indépendamment substitué par jusqu'à 3 occurrences d'un groupe (C₁ à C₄)alkyle, (C₁ à C₄)halogénoalkyle, (C₁ à C₄)alcoxy, (C₁ à C₄)halogénoalcoxy, oxo, -C(O)O((C₁ à C₄)alkyle), -C(O)OH, -CONH₂, -NR(CO)O((C₁ à C₄)alkyle), -OH ou d'un atome d'halogène ; où R représente un atome d'hydrogène ou un groupe (C₁ à C₂)alkyle ;
chaque groupe R^{5d} est indépendamment choisi parmi un atome d'halogène, un groupe -CN, un groupe (C₁ à C₆)alkyle, un groupe -((C₁ à C₆)alkyl)-R⁶, un groupe -OR⁶, -SR⁶, -COR⁶, -OC(O)R⁶, -C(O)OR⁶, -C(O)N(R⁶)₂, -N(R⁶)C(O)R⁶, -N(R⁶)C(O)OR⁶, -N(R⁶)C(O)N(R⁶)₂, -N(R⁶)₂, -SO₂OH, -SO₂NHOH, -SO₂N(R⁶)(CO)-R⁶, -SO₂R⁶, -SO₂N(R⁶)₂, -N(R⁶)SO₂R⁶, un groupe (C₇ à C₁₂)aralkyle, un noyau (C₃ à C₈)cycloalkyle, un noyau hétérocyclique de 4 à 7 chaînons, un noyau hétéroaryle de 5 ou 6 chaînons, un groupe phényle ou un groupe oxo ; chaque noyau hétéroaryle de 5 ou 6 chaînons ou noyau hétérocyclique de 4 à 7 chaînons contenant jusqu'à quatre hétéroatomes de cycle indépendamment choisis parmi un atome N, O et S, chacune desdites chaînes (C₁ à C₆)alkyle, dudit groupe (C₇ à C₁₂)aralkyle, dudit noyau (C₃ à C₈)cycloalkyle, dudit noyau hétérocyclique de 4 à 7 chaînons, dudit noyau hétéroaryle de 5 ou 6 chaînons ou dudit groupe phényle étant éventuellement et indépendamment substitué par jusqu'à 3 occurrences d'un atome d'halogène, d'un groupe (C₁ à C₄)alkyle, (C₁ à C₄)halogénoalkyle, -OH, -NH₂, -NH((C₁ à C₄)alkyle), -N((C₁ à C₄)alkyle)₂, -CN, -COOH, -CONH₂, -COO((C₁ à C₄)alkyle), -O((C₁ à C₄)alkyle), -O((C₁ à C₄)halogénoalkyle) ou oxo ;
deux occurrences de R⁵ ou deux occurrences de R^{5d} liées au même atome ou à des atomes différents du groupe J^{D}, conjointement avec ledit atome ou lesdites atomes auxquels elles sont liées, peuvent éventuellement former un noyau (C₃ à C₈)cycloalkyle, un noyau hétérocycle de 4 à 6 chaînons, un groupe phényle ou un noyau hétéroaryle de 5 ou 6 chaînons, conduisant à un système bicyclique dans lequel les deux noyaux du système bicyclique se trouvent dans une relation spiro, fusionnée ou pontée, ledit hétérocycle de 4 à 6 chaînons ou ledit noyau hétéroaryle de 5 ou 6 chaînons contenant jusqu'à quatre hétéroatomes de cycle indépendamment choisis parmi un atome N, O ou S ; et ledit noyau (C₃ à C₈)cycloalkyle, noyau hétérocyclique de 4 à 6 chaînons, groupe phényle ou noyau hétéroaryle de 5 ou 6 chaînons étant éventuellement et indépendamment substitué par jusqu'à 3 occurrences d'un groupe (C₁ à C₄)alkyle, (C₁ à C₄)halogénoalkyle, (C₁ à C₄)alcoxy, (C₁ à C₄)halogénoalcoxy, oxo, -C(O)O((C₁ à C₄)alkyle), -C(O)OH, -CONH₂, -NR(CO)O((C₁ à C₄)alkyle), -OH ou d'un atome d'halogène ; où R représente un atome d'hydrogène ou un groupe (C₁ à C₂)alkyle ;
chaque groupe R⁶ est indépendamment choisi parmi un atome d'hydrogène, un groupe (C₁ à C₆)aliphatique, un groupe phényle, un groupe benzyle, un noyau (C₃ à C₈)cycloalkyle, un noyau hétérocyclique de 4 à 7 chaînons ou un noyau hétéroaryle de 5 ou 6 chaînons, chacun desdits groupes (C₁ à C₆)aliphatiques, chacun desdits groupes phényle, chacun desdits groupes benzyle, chacun desdits groupes (C₃ à C₈)cycloalkyle, chacun desdits noyaux hétérocycliques de 4 à 7chaînons et chacun desdits noyaux hétéroaryle de 5 ou 6 chaînons étant éventuellement et indépendamment substitué par jusqu'à 3 occurrences d'un atome d'halogène, d'un groupe (C₁ à C₄)alkyle, -OH, -NH₂, -NH((C₁ à C₄)alkyle), -N((C₁ à C₄)alkyle)₂, -CN, -COOH, -COO((C₁ à C₄)alkyle), -O((C₁ à C₄)alkyle), -O((C₁ à C₄)halogénoalkyle) ou oxo, chacun dudit noyau hétéroaryle de 5 ou 6 chaînons ou noyau hétérocyclique de 4 à 7 chaînons contenant jusqu'à 4 hétéroatomes de cycle indépendamment choisis parmi N, O et S ;
chaque groupe R^{6a} est indépendamment choisi parmi un atome d'hydrogène, un groupe (C₁ à C₆)aliphatique, un groupe phényle, un groupe benzyle, un noyau (C₃ à C₈)cycloalkyle, un noyau hétérocyclique de 4 à 7 chaînons ou un noyau hétéroaryle de 5 ou 6 chaînons, chacun desdits groupes (C₁ à C₆)aliphatiques, chacun desdits groupes phényle, chacun desdits groupes benzyle, chacun desdits groupes (C₃ à C₈)cycloalkyle, chacun desdits noyaux hétérocycliques de 4 à 7chaînons et chacun desdits noyaux hétéroaryle de 5 ou 6 chaînons étant éventuellement et indépendamment substitué par jusqu'à 3 occurrences d'un atome d'halogène, d'un groupe (C₁ à C₄)alkyle, -OH, -NH₂, -NH((C₁ à C₄)alkyle), -N((C₁ à C₄)alkyle)₂, -CN, -COOH, -COO((C₁ à C₄)alkyle), -O((C₁ à C₄)alkyle), -O((C₁ à C₄)halogénoalkyle) ou oxo, chacun dudit noyau hétéroaryle de 5 ou 6 chaînons ou noyau hétérocyclique de 4 à 7 chaînons contenant jusqu'à 4 hétéroatomes de cycle indépendamment choisis parmi N, O et S ;
chaque groupe R^{a} est indépendamment choisi parmi un atome d'hydrogène, un groupe (C₁ à C₆)aliphatique, un groupe phényle, un groupe benzyle, un noyau (C₃ à C₈)cycloalkyle, un noyau hétérocyclique de 4 à 7 chaînons ou un noyau hétéroaryle de 5 ou 6 chaînons, chacun desdits groupes (C₁ à C₆)aliphatiques, chacun desdits groupes phényle, chacun desdits groupes benzyle, chacun desdits groupes (C₃ à C₈)cycloalkyle, chacun desdits noyaux hétérocycliques de 4 à 7 chaînons et chacun desdits noyaux hétéroaryle de 5 ou 6 chaînons étant éventuellement et indépendamment substitué par jusqu'à 3 occurrences d'un atome d'halogène, d'un groupe (C₁ à C₄)alkyle, -OH, -NH₂, -NH((C₁ à C₄)alkyle), -N((C₁ à C₄)alkyle)₂, -CN, -COOH, -COO((C₁ à C₄)alkyle), -O((C₁ à C₄)alkyle), -O((C₁ à C₄)halogénoalkyle) ou oxo, chacun dudit noyau hétéroaryle de 5 ou 6 chaînons ou noyau hétérocyclique de 4 à 7 chaînons contenant jusqu'à 4 hétéroatomes de cycle indépendamment choisis parmi N, O et S ;
deux occurrences de R⁶ liées au même atome d'azote du groupe R⁵ ou R^{5d} conjointement avec ledit atome d'azote du groupe R⁵ ou R^{5d} respectivement, pouvant former un noyau hétérocyclique de 5 à 8 chaînons ou un noyau hétéroaryle de 5 chaînons ; chaque dit noyau hétérocyclique de 5 à 8 chaînons ou chaque dit noyau hétéroaryle de 5 chaînons contenant éventuellement jusqu'à 2 hétéroatomes supplémentaires indépendamment choisis parmi N, O ou S;
deux occurrences de R^{6a} liées à un atome d'azote du groupe R^{5a} ou R^{5b}, conjointement avec ledit atome d'azote, pouvant former un noyau hétérocyclique de 5 à 8 chaînons ou un noyau hétéroaryle de 5 chaînons ; chaque dit noyau hétérocyclique de 5 à 8 chaînons ou chaque dit noyau hétéroaryle de 5 chaînons contenant éventuellement jusqu'à 2 hétéroatomes supplémentaires indépendamment choisis parmi N, O ou S ;
deux occurrences de R^{6b} liées à un atome d'azote du groupe R^{5c}, conjointement avec ledit atome d'azote, pouvant former un noyau hétérocyclique de 5 à 8 chaînons ou un noyau hétéroaryle de 5 chaînons ; chaque dit noyau hétérocyclique de 5 à 8 chaînons et chaque dit noyau hétéroaryle de 5 chaînons contenant éventuellement jusqu'à 2 hétéroatomes supplémentaires indépendamment choisis parmi N, O ou S ;
sinon, deux groupes J^{D} liés à deux atomes de cycle D vicinaux, pris ensemble avec lesdits deux atomes de cycle D vicinaux, pouvant former un hétérocycle de 5 à 7 chaînons ou un noyau hétéroaryle de 5 chaînons qui est fusionné au cycle D ; ledit hétérocycle de 5 à 7 chaînons ou noyau hétéroaryle de 5 chaînons contenant de 1 à 3 hétéroatomes indépendamment choisis parmi un atome N, O ou S ; et ledit hétérocycle de 5 à 7 chaînons ou ledit noyau hétéroaryle de 5 chaînons étant éventuellement et indépendamment substitué par jusqu'à 3 occurrences d'un groupe oxo ou -(Y)-R⁹ ;
Y étant soit absent soit représentant une chaîne (C₁ à C₆)alkyle, éventuellement substituée par jusqu'à 6 occurrences d'un groupe fluoro ; et lorsque Y représente ladite chaîne (C₁ à C₆)alkyle, jusqu'à 3 unités méthylène de cette chaîne alkyle, peuvent être remplacés par un groupe choisi parmi -O-, -C(O) - or -N((Y¹)-R⁹⁹)- ;
Y¹ étant soit absent soit représentant une chaîne (C₁ à C₆)alkyle, éventuellement substituée par jusqu'à 6 occurrences d'un groupe fluoro ; et lorsque Y¹ est absent, chaque R⁹⁹ étant indépendamment choisi parmi un atome d'hydrogène, un groupe (C₁ à C₆)alkyle éventuellement substitué par jusqu'à 9 atomes de fluor, un groupe -COR¹⁰, -C(O)OR¹⁰,-C(O)N(R¹⁰)₂, -C(O)N(R¹⁰)SO₂R¹⁰, -SO₂R¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, un noyau (C₃ à C₆)cycloalkyle, un noyau hétérocyclique de 4 à 8 chaînons, un noyau phényle ou un noyau hétéroaryle de 5 à 6 chaînons ; chaque dit noyau hétérocyclique de 4 à 8 chaînons ou noyau hétéroaryle de 5 à 6 chaînons contenant jusqu'à 4 hétéroatomes de cycle indépendamment choisis parmi un atome N, O ou S ; et chacun desdits noyaux (C₃ à C₆)cycloalkyle, chacun desdits noyaux hétérocycliques de 4 à 8 chaînons, chacun desdits groupes phényle et chacun desdits noyaux hétéroaryle de 5 à 6 chaînons étant éventuellement et indépendamment substitué par jusqu'à 3 occurrences de R^{11a} ;
lorsque Y¹ est présent, chaque R⁹⁹ étant indépendamment choisi parmi un atome d'hydrogène, un atome d'halogène, un groupe -CN, un groupe (C₁ à C₆)alkyle éventuellement substitué par jusqu'à 9 atomes de fluor, un groupe -COR¹⁰, -OR¹⁰, -OC(O)R¹⁰, -C(O)OR¹⁰,-C(O)N(R¹⁰)₂, -C(O)N(R¹⁰)SO₂R¹⁰, -SO₂R¹⁰, -SOR¹⁰, -SR¹⁰, -SO₂N(R¹⁰)₂, -SO₂N(R¹⁰)COOR¹⁰, -SO₂N(R¹⁰)C(O)R¹⁰, -SO₂OH, -SO₂NHOH, -SO₂N(R¹⁰)(CO)R¹⁰, un noyau (C₃ à C₆)cycloalkyle, un noyau hétérocyclique de 4 à 8 chaînons, un noyau phényle ou un noyau hétéroaryle de 5 à 6 chaînons ; chaque dit noyau hétérocyclique de 4 à 8 chaînons ou noyau hétéroaryle de 5 à 6 chaînons contenant jusqu'à 4 hétéroatomes de cycle indépendamment choisis parmi un atome N, O ou S ; et chacun desdits noyaux (C₃ à C₆)cycloalkyle, chacun desdits noyaux hétérocycliques de 4 à 8 chaînons, chacun desdits groupes phényle et chacun desdits noyaux hétéroaryle de 5 à 6 chaînons étant éventuellement et indépendamment substitué par jusqu'à 3 occurrences de R^{11a} ;
et R⁹ est indépendamment choisi parmi un atome d'hydrogène, un groupe -CN, -OR¹⁰, -COR¹⁰, -OC(O)R¹⁰, -C(O)OR¹⁰, -C(O)N(R¹⁰)₂, -C(O)N(R¹⁰)SO₂R¹⁰ -N(R¹⁰)C(O)R¹⁰ -N(R¹⁰)C(O)OR¹⁰, -N(R¹⁰)C(O)N(R¹⁰)₂, -N(R¹⁰)₂, -SO₂R¹⁰, -SO₂N(R¹⁰)₂, -SO₂N(R¹⁰)COOR¹⁰, -SO₂N(R¹⁰)C(O)R¹⁰, -N(R¹⁰)SO₂R¹⁰, -SO₂OH, -SO₂NHOH, -SO₂N(R¹⁰)(CO)-R¹⁰, un noyau (C₃ à C₆)cycloalkyle, un noyau hétérocyclique de 4 à 8 chaînons, un noyau phényle ou un noyau hétéroaryle de 5 à 6 chaînons ; chaque dit noyau hétérocyclique de 4 à 8 chaînons ou noyau hétéroaryle de 5 à 6 chaînons contenant jusqu'à 4 hétéroatomes de cycle indépendamment choisis parmi un atome N, O ou S ; et chacun desdits noyaux (C₃ à C₆)cycloalkyle, chacun desdits noyaux hétérocycliques de 4 à 8 chaînons, chacun desdits groupes phényle et chacun desdits noyaux hétéroaryle de 5 à 6 chaînons étant éventuellement et indépendamment substitué par jusqu'à 3 occurrences de R^{11a};
chaque groupe R¹⁰ est indépendamment choisi parmi un atome d'hydrogène, un groupe (C₁ à C₆)alkyle, -((C₁ à C₆)alkyl)-R¹³, un groupe phényle, un groupe benzyle, un noyau (C₃ à C₆)cycloalkyle, un noyau hétérocyclique de 4 à 7 chaînons ou un noyau hétéroaryle de 5 à 6 chaînons ; chaque noyau hétéroaryle de 5 à 6 chaînons ou noyau hétérocyclique de 4 à 7 chaînons contenant jusqu'à 4 hétéroatomes de cycle indépendamment choisis parmi un atome N, O et S ; et chacun desdits groupes (C₁ à C₆)alkyle, chaque dit groupe phényle, chaque dit groupe benzyle, chaque dit groupe (C₃ à C₈)cycloalkyle, chaque dit noyau hétérocyclique de 4 à 7 chaînons et chaque noyau hétéroaryle de 5 à 6 chaînons étant éventuellement et indépendamment substitué par jusqu'à 3 occurrences de R^{11b} ;
chaque groupe R¹³ est indépendamment choisi parmi un groupe phényle, un groupe benzyle, un noyau (C₃ à C₆)cycloalkyle, un noyau hétérocyclique de 4 à 7 chaînons ou un noyau hétéroaryle de 5 à 6 chaînons ; chaque noyau hétéroaryle de 5 à 6 chaînons ou noyau hétérocyclique de 4 à 7 chaînons contenant jusqu'à 4 hétéroatomes de cycle indépendamment choisis parmi un atome N, O et S ; et chaque dit groupe phényle, chaque dit groupe benzyle, chaque dit groupe (C₃ à C₈)cycloalkyle, chaque dit noyau hétérocyclique de 4 à 7 chaînons et chaque noyau hétéroaryle de 5 à 6 chaînons étant éventuellement et indépendamment substitué par jusqu'à 3 occurrences de R^{11c} ;
chaque groupe R^{11a} est indépendamment choisi parmi un atome d'halogène, un groupe (C₁ à C₆)alkyle, un groupe -CN, -OR¹², -COR¹², -C(O)OR¹², -C(O)N(R¹²)₂, -N(R¹²)C(O)R¹²,-N(R¹²)C(O)OR¹², -N(R¹²)C(O)N(R¹²)₂, -N(R¹²)₂, -SO₂R¹², -SO₂N(R¹²)₂ ou -N(R¹²)SO2R¹² ; chacun desdits groupes(C₁ à C₆)alkyle étant éventuellement et indépendamment substitué par jusqu'à 6 occurrences d'un groupe fluoro et/ou 3 occurrences de R¹²¹ ;
chaque groupe R^{11b} est indépendamment choisi parmi un atome d'halogène, un groupe (C₁ à C₆)alkyle, un groupe -CN, -OR¹², -COR¹², -C(O)OR¹², -C(O)N(R¹² )₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ ou -N(R¹²)SO₂R¹² ; chacun desdits groupes(C₁ à C₆)alkyle étant éventuellement et indépendamment substitué par jusqu'à 6 occurrences d'un groupe fluoro et/ou 3 occurrences de R¹²¹ ; et
chaque groupe R^{11c} est indépendamment choisi parmi un atome d'halogène, un groupe (C₁ à C₆)alkyle, un groupe -CN, -OR¹², -COR¹², -C(O)OR¹², -C(O)N(R¹² )₂, -N(R¹²)C(O)R¹², -N(R¹²)C(O)OR¹², -N(R¹²)C(O)N(R¹²)₂, -N(R¹²)₂, -SO₂R¹², -SO₂N(R¹²)₂ ou -N(R¹²)SO₂R¹² ; chacun desdits groupes(C₁ à C₆)alkyle étant éventuellement et indépendamment substitué par jusqu'à 6 occurrences d'un groupe fluoro et/ou 3 occurrences de R¹²¹ ;
chaque groupe R¹² est choisi parmi un atome d'hydrogène, un groupe (C₁ à C₄)alkyle, un groupe (C₁ à C₄)fluoroalkyle, -OH, -NH₂, un groupe -NH((C₁ à C₄)alkyle), un groupe -N((C₁ à C₄)alkyle)₂, un groupe -CN, -COOH, un groupe -COO((C₁ à C₄)alkyle), un groupe -O((C₁ à C₄)alkyle), un groupe -O((C₁ à C₄)fluoroalkyle) ou un groupe oxo ;
chaque groupe R¹²¹ est choisi parmi un groupe (C₁ à C₄)alkyle, un groupe (C₁ à C₄)fluoroalkyle, -OH, -NH₂, un groupe -NH((C₁ à C₄)alkyle), -N((C₁ à C₄)alkyle)₂, un groupe -CN, -COOH, un groupe -COO((C₁ à C₄)alkyle), un groupe -O((C₁ à C₄)alkyle), -O((C₁ à C₄)fluoroalkyle) ou un groupe oxo ;
le groupe R^{C} est choisi parmi un atome d'hydrogène, un groupe (C₁ à C₆)aliphatique, un groupe -((C₁ à C₆)alkyl)-R^{N}, un noyau hétéroaryle de 5 ou 6 chaînons, un groupe phényle, un noyau hétérocyclique de 4 à 7 chaînons, un groupe (C₃ à C₈)cycloaliphatique, un groupe -C(O)R⁷, -C(O)OR⁷, -C(O)N(R⁷)₂ et -C(O)N(R⁷)SO₂R⁷ ; chacun dudit noyau hétéroaryle de 5 ou 6 chaînons et noyau hétérocyclique de 4 à 7 chaînons contenant jusqu'à 4 hétéroatomes de cycle indépendamment choisis parmi un atome N, O et S ; chaque dit groupe (C₁ à C₆)aliphatique et chaque portion (C₁ à C₆)alkyle dudit groupe -((C₁ à C₆)alkyl)-R^{N}, étant éventuellement et indépendamment substitué par jusqu'à 6 occurrences d'un atome d'halogène et jusqu'à 2 occurrences d'un groupe -CN, -COOR⁸, -OR⁸, oxo, -N(R⁸)₂, -C(O)N(R⁸)₂, -N(R⁸)C(O)R⁸, -N(R⁸)C(O)OR⁸, -N(R⁸)C(O)N(R⁸)₂, -SO₂R⁸, -SO₂N(R⁸)₂, -NHOR⁸, -SO₂N(R⁸)COOR⁸, -SO₂N(R⁸)C(O)R⁸ et -N(R⁸)SO₂R⁸ ;
chaque groupe R⁷ étant indépendamment choisi parmi un atome d'hydrogène, un groupe (C₁ à C₆)alkyle, (C₁ à C₆)fluoroalkyle, un noyau (C₃ à C₈)cycloalkyle, un groupe phényle, un noyau hétérocyclique de 4 à 7 chaînons ou un noyau hétéroaryle de 5 ou 6 chaînons ; chacun dudit noyau hétéroaryle de 5 ou 6 chaînons ou noyau hétérocyclique de 4 à 7 chaînons contenant jusqu'à 4 hétéroatomes de cycle indépendamment choisis parmi N, O et S ; et chacun desdits groupes (C₁ à C₆)alkyle, chacun desdits groupes phényle, chacun desdits groupes (C₃ à C₈)cycloalkyle, chacun desdits noyaux hétérocycliques de 4 à 7 chaînons et chacun desdits noyaux hétéroaryle de 5 ou 6 chaînons étant éventuellement et indépendamment substitué par jusqu'à 3 occurrences d'un atome d'halogène, d'un groupe (C₁ à C₄)alkyle, -OH, -NH₂, -NH((C₁ à C₄)alkyle), -N((C₁ à C₄)alkyle)₂, -CN, -COOH, -COO((C₁ à C₄)alkyle), -O((C₁ à C₄)alkyle), -O((C₁ à C₄)halogénoalkyle) ou oxo ;
chaque groupe R⁸ étant indépendamment choisi parmi un atome d'hydrogène, un groupe (C₁ à C₆)alkyle, un groupe (C₁ à C₆)fluoroalkyle, un noyau (C₃ à C₈)cycloalkyle, un noyau hétérocyclique de 4 à 7 chaînons ou un noyau hétéroaryle de 5 ou 6 chaînons ; chacun dudit noyau hétéroaryle de 5 ou 6 chaînons ou noyau hétérocyclique de 4 à 7 chaînons contenant jusqu'à 4 hétéroatomes de cycle indépendamment choisis parmi un atome N, O et S ; et chacun desdits groupes (C₁ à C₆)alkyle, chacun desdits groupes phényle, chacun desdits groupes (C₃ à C₈)cycloalkyle, chacun desdits noyaux hétérocycliques de 4 à 7 chaînons et chacun desdits noyaux hétéroaryle de 5 ou 6 chaînons étant éventuellement et indépendamment substitué par jusqu'à 3 occurrences d'un atome d'halogène, d'un groupe (C₁ à C₄)alkyle, -OH, -NH₂, -NH((C₁ à C₄)alkyle), -N((C₁ à C₄)alkyl)₂, -CN, -COOH, -COO((C₁ à C₄)alkyle), -O((C₁ à C₄)alkyle), -O((C₁ à C₄)halogénoalkyle) ou oxo ;
chaque groupe R^{N} étant indépendamment choisi parmi un noyau phényle, un noyau hétéroaryle monocyclique de 5 ou 6 chaînons, un noyau (C₃ à C₆)cycloaliphatique monocyclique, ou un hétérocycle monocyclique de 4 à 6 chaînons ; ledit noyau hétéroaryle monocyclique de 5 ou 6 chaînons ou ledit hétérocycle monocyclique de 4 à 6 chaînons contenant entre 1 et 4 hétéroatomes choisis parmi un atome N, O ou S ; ledit noyau hétéroaryle monocyclique de 5 ou 6 chaînons n'étant pas un noyau 1,3,5-triazinyle ; et ledit groupe phényle, ledit noyau hétéroaryle monocyclique de 5 ou 6 chaînons, ledit noyau (C₃ à C₆)cycloaliphatique monocyclique ou ledit hétérocycle monocyclique de 4 à 6 chaînons étant éventuellement et indépendamment substitué par jusqu'à 6 occurrences d'un groupe fluoro et/ou jusqu'à 3 occurrences de J^{M} ;
chaque groupe J^{M} étant indépendamment choisi parmi un groupe -CN, un groupe (C₁ à C₆)aliphatique, un groupe -OR^{M}, -SR^{M}, -N(R^{M})₂, un noyau (C₃ à C₈)cycloaliphatique ou un noyau hétérocyclique de 4 à 8 chaînons ; ledit noyau hétérocyclique de 4 à 8 chaînons contenant 1 ou 2 hétéroatomes indépendamment choisis parmi N, O ou S ; chaque dit groupe (C₁ à C₆)aliphatique, chaque dit noyau (C₃ à C₈)cycloaliphatique et chaque dit noyau hétérocyclique de 4 à 8 chaînons, étant éventuellement et indépendamment substitué par jusqu'à 3 occurrences de R^{7c} ; et
chaque groupe R^{M} étant indépendamment choisi parmi un atome d'hydrogène, un groupe (C₁ à C₆)aliphatique, un noyau (C₃ à C₈)cycloaliphatique ou un noyau hétérocyclique de 4 à 8 chaînons ; chaque dit noyau hétérocyclique de 4 à 8 chaînons contenant entre 1 et 3 hétéroatomes indépendamment choisis parmi O, N ou S ;
chaque groupe R^{7C} étant indépendamment choisi parmi un atome d'halogène, un groupe -CN, -NO₂, un groupe (C₁ à C₄)alkyle, un groupe (C₁ à C₄)halogénoalkyle, un noyau (C₃ à C₈)cycloalkyle, un groupe -OR^{8b}, -SR^{8b}, -N(R^{8b})₂, -C(O)O((C₁ à C₄)alkyle), -C(O)OH, -NR(CO)CO((C₁ à C₄)alkyle) ou un groupe oxo; chaque dit groupe cycloalkyle étant éventuellement et indépendamment substitué par jusqu'à 3 occurrences d'un atome d'halogène ;
chaque groupe R^{8b} étant indépendamment choisi parmi un atome d'hydrogène, un groupe (C₁ à C₆)alkyle, un groupe (C₁ à C₆)fluoroalkyle, un noyau (C₃ à C₈)cycloalkyle, un noyau hétérocyclique de 4 à 7 chaînons ou un noyau hétéroaryle de 5 ou 6 chaînons ; chacun dudit noyau hétéroaryle de 5 ou 6 chaînons ou noyau hétérocyclique de 4 à 7 chaînons contenant jusqu'à 4 hétéroatomes de cycle indépendamment choisis parmi un atome N, O et S ; et chacun desdits groupes (C₁ à C₆)alkyle, chacun desdits groupe phényle, chacun desdits groupes (C₃ à C₈)cycloalkyle, chacun desdits noyaux hétérocycliques de 4 à 7 chaînons et chacun desdits noyaux hétéroaryle de 5 ou 6 chaînons étant éventuellement et indépendamment par jusqu'à 3 occurrences d'un atome d'halogène, d'un groupe (C₁ à C₄)alkyle, -OH, -NH₂, -NH((C₁ à C₄)alkyle), -N((C₁ à C₄)alkyle)₂, -CN, -COOH, -COO((C₁ à C₄)alkyle), -O((C₁ à C₄)alkyle), -O((C₁ à C₄)halogénoalkyle) ou oxo ;
ledit noyau hétérocyclique décrit ci-dessus étant complètement saturé ou contenant une ou plusieurs unités d'insaturation mais n'étant pas aromatique ;
à condition que le composé ne soit pas représenté par la structure générale : 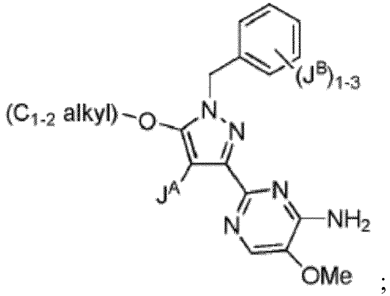
dans laquelle le groupe J^{A} représente soit un atome d'hydrogène soit un groupe (C₁ à C₄)alkyle ; et le groupe J^{B} représente soit un atome d'halogène soit un groupe (C₁ à C₄)alcoxy.

2. Composé selon la revendication 1, ou sel pharmaceutiquement acceptable de celui-ci, ledit composé étant représenté par la formule IIa : 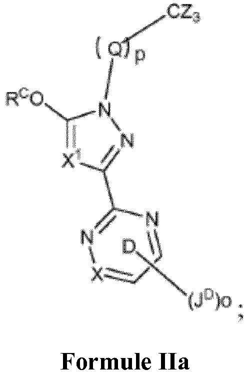 dans laquelle Q représente un groupe -CZ₂-, chaque Z étant indépendamment choisi parmi un atome d'hydrogène ou de fluor et p représente un entier choisi parmi 1, 2, 3, 4 et 5.

3. Composé selon la revendication 1 ou 2, ou sel pharmaceutiquement acceptable de celui-ci, représenté par la **formule IIIa** : 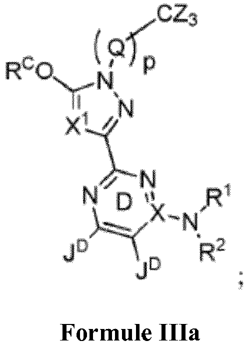 dans laquelle,
lorsque X représente un atome N, le groupement -N(R¹)(R²) est absent ; lorsque X représente un atome C, le groupement -N(R¹)(R²) est présent ;
les groupes R¹ et R², conjointement avec l'atome d'azote auquel ils sont liés, forment un noyau hétérocyclique de 4 à 8 chaînons ou un noyau hétéroaryle de 5 chaînons ; ledit noyau hétérocyclique de 4 à 8 chaînons ou noyau hétéroaryle de 5 chaînons contenant éventuellement, en plus de l'atome d'azote auquel les deux groupes R¹ et R² sont liés, jusqu'à 3 hétéroatomes de cycle indépendamment choisis parmi un atome N, O ou S, et étant éventuellement substitué par jusqu'à 5 occurrences de R^{5e} ;
chaque groupe R^{5e} est indépendamment choisi parmi un atome d'halogène, un groupe -CN, un groupe (C₁ à C₆)alkyle, un groupe -((C₁ à C₄)alkyl)-R⁶, un noyau (C₃ à C₈)cycloalkyle, (C₁ à C₄)cyanoalkyle, un groupe -OR⁶, -SR⁶, -OCOR⁶, -COR⁶, -C(O)OR⁶, -C(O)N(R⁶)₂, -N(R⁶)C(O)R⁶, -N(R⁶)₂, -SO₂R⁶, -SO₂N(R⁶)₂, -N(R⁶)SO₂R⁶, -SO₂OH, -SO₂NHOH, -SO₂N(R⁶)(CO)-R⁶, un groupe benzyle, un groupe phényle ou un groupe oxo ; chaque dit groupe phényle et chaque dit groupe benzyle, étant éventuellement et indépendamment substitué par jusqu'à 3 occurrences d'un atome d'halogène, d'un groupe -OH, -NH₂, -NH((C₁ à C₄)alkyle), -N((C₁ à C₄)alkyle)₂, -CN, (C₁ à C₄)alkyle, (C₁ à C₄)halogénoalkyle, -CONH₂, -O((C₁ à C₄)alkyle) ou -O((C₁ à C₄)halogénoalkyle) ; et chaque dit groupe (C₁ à C₆)alkyle ou chacune desdites chaînes (C₁ à C₄)alkyle et chaque dit noyau (C₃ à C₈)cycloalkyle étant éventuellement et indépendamment substitué par jusqu'à 3 occurrences d'un atome d'halogène ;
chaque groupe R⁶ étant indépendamment choisi parmi un atome d'hydrogène, un groupe (C₁ à C₆)alkyle, un groupe (C₂ à C₄)alcényle, un groupe phényle, un groupe benzyle, ou un noyau (C₃ à C₈)cycloalkyle ; chaque dit groupe (C₁ à C₆)alkyle, chaque dit groupe (C₂ à C₄)alcényle, chaque dit groupe phényle, chaque dit groupe benzyle et chaque dit groupe (C₃ à C₈)cycloalkyle étant éventuellement et indépendamment substitué par jusqu'à 3 occurrences d'un atome d'halogène ;
sinon, deux des occurrences de R^{5e} liées au même atome ou à des atomes différents dudit noyau formé par R¹, R² et l'atome d'azote auquel les groupes R¹ et R² sont liés, conjointement avec ledit atome ou lesdits atomes, forment éventuellement un noyau (C₃ à C₈)cycloalkyle, un noyau hétérocyclique de 4 à 6 chaînons, un groupe phényle ou un noyau hétéroaryle de 5 ou 6 chaînons, conduisant à un système bicyclique dans lequel les deux noyaux du système bicyclique se trouvent dans une relation spiro, fusionnée ou pontée, ledit hétérocycle de 4 à 6 chaînons ou ledit noyau hétéroaryle de 5 ou 6 chaînons contenant jusqu'à trois hétéroatomes de cycle indépendamment choisis parmi un atome N, O ou S ; et ledit noyau (C₃ à C₈)cycloalkyle, noyau hétérocyclique de 4 à 6 chaînons, groupe phényle ou noyau hétéroaryle de 5 ou 6 chaînons étant éventuellement et indépendamment substitué par jusqu'à 3 occurrences d'un groupe (C₁ à C₄)alkyle, (C₁ à C₄)halogénoalkyle, (C₁ à C₄)alcoxy, (C₁ à C₄)halogénoalcoxy, oxo, -C(O)O((C₁ à C₄)alkyle), -CONH₂, -C(O)OH, -NR(CO)O((C₁ à C₄)alkyle), -OH ou d'un atome d'halogène ; où R représente un atome d'hydrogène ou un groupe (C₁ à C₂)alkyle ;
sinon, les groupes R¹ et R² sont chacun indépendamment choisis parmi un atome d'hydrogène, un groupe (C₁ à C₆)alkyle, un noyau (C₃ à C₈)cycloalkyle, un noyau hétérocyclique de 4 à 8 chaînons, un noyau hétéroaryle de 5 ou 6 chaînons, un groupe phényle ou un groupe (C₁ à C₆)alkyl -R^{Y} ; chacun desdits noyaux hétérocycliques de 4 à 8 chaînons et chacun desdits noyaux hétéroaryle de 5 ou 6 chaînons contenant jusqu'à 3 hétéroatomes de cycle indépendamment choisis parmi un atome N, O et S ; et chacun desdits groupe (C₁ à C₆)alkyle, noyau (C₃ à C₈)cycloalkyle, noyau hétérocyclique de 4 à 8 chaînons, noyau hétéroaryle de 5 ou 6 chaînons, groupe phényle et la portion (C₁ à C₆)alkyle du groupement (C₁ à C₆)alkyl-R^{Y}, étant éventuellement et indépendamment substitué par jusqu'à 5 occurrences de R^{5f} ;
R^{Y} est choisi parmi un noyau (C₃ à C₈)cycloalkyle, un noyau hétérocyclique de 4 à 8 chaînons, un groupe phényle ou un noyau hétéroaryle de 5 ou 6 chaînons ; chacun dudit noyau hétérocyclique de 4 à 8 chaînons ou noyau hétéroaromatique de 5 ou 6 chaînons contenant entre 1 et 4 hétéroatomes de cycle choisis parmi un atome N, O ou S ; et chacun desdits noyaux (C₃ à C₈)cycloalkyle, chacun desdits noyaux hétérocyclique de 4 à 8 chaînons, chacun desdits groupes phényle, et chacun desdits noyaux hétéroaryle de 5 à 6 chaînons étant éventuellement substitué par jusqu'à 5 occurrences de R^{5g} ;
chaque groupe R^{5f} est indépendamment choisi parmi un atome d'halogène, un groupe -CN, un groupe (C₁ à C₆)alkyle, un groupe -((C₁ à C₄)alkyl)-R^{6a}, un groupe (C₇ à C₁₂)aralkyle, un noyau (C₃ à C₈)cycloalkyle, un groupe (C₁ à C₄)cyanoalkyle, un groupe -OR^{6a}, -SR^{6a}, -OCOR^{6a}, -COR^{6a}, -C(O)OR^{6a}, -C(O)N(R^{6a})₂, -N(R^{6a})C(O)R^{6a}-N(R^{6a})₂, -SO₂OH, -SO₂NHOH, -SO₂N(R^{6a})(CO)-R^{6a}, -SO₂R^{6a}, -SO₂N(R^{6a})₂, -N(R^{6a})SO₂R^{6a}, un groupe phényle ou un groupe oxo ; chaque dit groupe phényle étant éventuellement et indépendamment substitué par jusqu'à 3 occurrences d'un atome d'halogène, d'un groupe -OH, -NH₂, -NH((C₁ à C₄)alkyle), -N((C₁ à C₄)alkyle)₂, -CN, -CONH₂, (C₁ à C₄)alkyle, (C₁ à C₄)halogénoalkyle, -O((C₁ à C₄)alkyle) ou -O((C₁ à C₄)halogénoalkyle) ; et chaque dit groupe (C₇ à C₁₂)aralkyle, chaque dit groupe (C₁ à C₆)alkyle, chaque dite chaîne (C₁ à C₄)alkyle et chaque dit noyau (C₃ à C₈)cycloalkyle étant éventuellement et indépendamment substitué par jusqu'à trois occurrences d'un atome d'halogène ;
chaque groupe R^{6a} est indépendamment choisis parmi un atome d'hydrogène, un groupe (C₁ à C₆)alkyle, un groupe (C₂ à C₄)alcényle, un groupe phényle, un groupe benzyle, ou un noyau (C₃ à C₈)cycloalkyle ; chaque dit groupe (C₁ à C₆)alkyle, chaque dit groupe (C₂ à C₄)alcényle, chaque dit groupe phényle, chaque dit groupe benzyle et chaque dit noyau (C₃ à C₈)cycloalkyle étant éventuellement et indépendamment substitué par jusqu'à 3 occurrences d'un atome d'halogène ;
lorsque l'une des groupes R¹ ou R² représente le noyau (C₃ à C₈)cycloalkyle, le noyau hétérocyclique de 4 à 8 chaînons ou le noyau hétéroaryle de 5 ou 6 chaînons substitué par jusqu'à 5 occurrences de R^{5f}, deux des occurrences de R^{5f} liées au même atome de cycle ou à des atomes de cycle différents desdits groupes R¹ ou R², conjointement avec ledit atome ou lesdits atomes, forment un noyau (C₃ à C₈)cycloalkyle, un noyau hétérocyclique de 4 à 6 chaînons, un groupe phényle ou un noyau hétérocyclique de 5 ou 6 chaînons, conduisant à un système bicyclique dans lequel les deux noyaux se trouvent dans une relation spiro, fusionnée ou pontée l'un par rapport à l'autre, ledit hétérocycle de 4 à 6 chaînons ou ledit noyau hétérocyclique de 5 ou 6 chaînons contenant jusqu'à deux hétéroatomes de cycle indépendamment choisis parmi un atome N, O ou S ; et ledit noyau (C₃ à C₈)cycloalkyle, noyau hétérocyclique de 4 à 6 chaînons, groupe phényle ou noyau hétérocyclique de 5 ou 6 chaînons étant éventuellement substitué par jusqu'à 2 occurrences d'un groupe (C₁ à C₄)alkyle, (C₁ à C₄)halogénoalkyle, oxo, -C(O)O((C₁ à C₄)alkyle), -NR'(CO)O((C₁ à C₄)alkyle), ou d'un atome d'halogène ; où R' représente un atome d'hydrogène ou un groupe (C₁ à C₂)alkyle ;
chaque groupe R^{5g} est indépendamment choisi parmi un atome d'halogène, un groupe -CN, un groupe (C₁ à C₆)alkyle, un groupe -((C₁ à C₄)alkyl)-R^{6b}, un groupe benzyle, un noyau (C₃ à C₈)cycloalkyle, un groupe (C₁ à C₄)cyanoalkyle, un groupe -OR^{6b}, -SR^{6b}, -OCOR^{6b}, -COR^{6b}, -C(O)OR^{6b}, -C(O)N(R^{6b})₂, -N(R^{6b})C(O)R^{6b}, -N(R^{6b})₂, -SO₂R^{6b}, -SO₂OH, -SO₂NHOH, -SO₂N(R^{6b})(CO)-R^{6b}, -SO₂N(R^{6b})₂, -N(R^{6b})SO₂R^{6b}, un groupe phényle ou un groupe oxo ; chaque dit groupe phényle et chaque dit groupe benzyle étant éventuellement et indépendamment substitué par jusqu'à 3 occurrences d'un atome d'halogène, d'un groupe -OH, -NH₂, -NH((C₁ à C₄)alkyle), -N((C₁ à C₄)alkyle)₂, -CN, -CONH₂, (C₁ à C₄)alkyle, (C₁ à C₄)halogénoalkyle, -O((C₁ à C₄)alkyle) ou -O((C₁ à C₄)halogénoalkyle) ; et chaque dite chaîne (C₁ à C₄)alkyle et chaque dit groupe (C₃ à C₈)cycloalkyle étant éventuellement et indépendamment substitué par jusqu'à 3 occurrences d'un atome d'halogène ;
chaque groupe R^{6b} est indépendamment choisi parmi un atome d'hydrogène, un groupe (C₁ à C₆)alkyle, un groupe (C₂ à C₄)alcényle, un groupe phényle, un groupe benzyle, ou un noyau (C₃ à C₈)cycloalkyle ; chaque dit groupe (C₁ à C₆)alkyle, chaque dit groupe (C₂ à C₄)alcényle, chaque dit groupe phényle, chaque dit groupe benzyle et chaque dit noyau (C₃ à C₈)cycloalkyle étant éventuellement substitué par jusqu'à 3 occurrences d'un atome d'halogène ;
sinon, deux occurrences de R^{5g} liées au même atome de cycle ou à des atomes de cycle différents de R^{Y}, conjointement avec ledit atome ou lesdits atomes de cycle, forment un noyau (C₃ à C₈)cycloalkyle, un noyau hétérocyclique de 4 à 6 chaînons, un groupe phényle ou un noyau hétéroaryle de 5 ou 6 chaînons, conduisant à un système bicyclique dans lequel les deux noyaux se trouvent dans une relation spiro, fusionnée ou pontée, ledit hétérocycle de 4 à 6 chaînons ou ledit noyau hétéroaryle de 5 ou 6 chaînons contenant jusqu'à trois hétéroatomes indépendamment choisis parmi un atome N, O ou S ; et ledit noyau (C₃ à C₈)cycloalkyle, noyau hétérocyclique de 4 à 6 chaînons, groupe phényle ou noyau hétéroaryle de 5 ou 6 chaînons étant éventuellement et indépendamment substitué par jusqu'à 3 occurrences d'un groupe (C₁ à C₄)alkyle, (C₁ à C₄)halogénoalkyle, (C₁ à C₄)alcoxy, (C₁ à C₄)halogénoalcoxy, oxo, -C(O)O((C₁ à C₄)alkyle), -C(O)OH, -NR"(CO)O((C₁ à C₄)alkyle), -OH ou d'un atome d'halogène ; et R" représente un atome d'hydrogène ou un groupe (C₁ à C₂)alkyle.

4. Composé selon la revendication 1, représenté par la **formula IIb**, ou sel pharmaceutiquement acceptable de celui-ci : 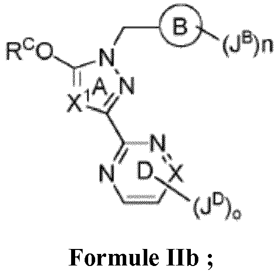 dans laquelle, le noyau B représente un noyau phényle ou un noyau hétéroaryle de 5 ou 6 chaînons, contenant 1 ou 2 hétéroatomes de cycle choisis parmi un atome N, O ou S.

5. Composé selon la revendication 4, ou sel pharmaceutiquement acceptable de celui-ci, chaque groupe J^{D} étant indépendamment choisi parmi un atome d'halogène, un groupe (C₁ à C₆)aliphatique, un groupe (C₁ à C₆)halogénoaliphatique, -N(R^{D})₂, -N(R^{d})COR^{D}, -N(R^{d})COOR^{D}, -OR^{D}, -N(R^{d})SO₂R^{D}, ou un noyau (C₃ à C₈)cycloaliphatique éventuellement substitué.

6. Composé selon la revendication 4 ou 5, ou sel pharmaceutiquement acceptable de celui-ci, ledit composé étant représenté par la **Formule IIIb** :

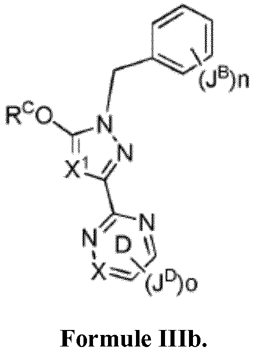

7. Composé selon la revendication 4 ou 6, ou sel pharmaceutiquement acceptable de celui-ci, représenté par la **formula IVb** :

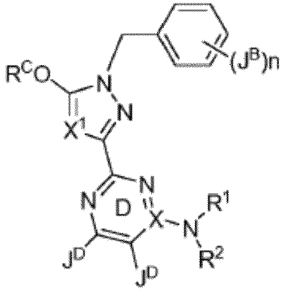

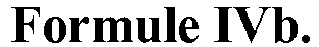

8. Composé selon l'une quelconque des revendications 4 à 6, ou sel pharmaceutiquement acceptable de celui-ci, représenté par la **formule Vb :** 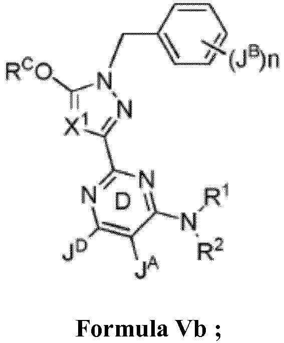 dans laquelle, le groupe J^{D} est absent ou choisi parmi un atome d'halogène, un groupe méthyle, hydroxy, méthoxy, trifluorométhyle, trifluorométhoxy ou -NR^{a}R^{b} ; dans lequel les groupes R^{a} et R^{b} sont chacun indépendamment choisis parmi un atome d'hydrogène, un groupe (C₁ à C₆)alkyle ou un noyau (C₃ à C₆)cycloalkyle ; ou dans lequel les groupes R^{a} et R^{b}, conjointement avec l'atome d'azote auquel ils sont tous les deux liés, forment un noyau hétérocyclique de 4 à 8 chaînons ou un noyau hétéroaryle de 5 chaînons contenant éventuellement jusqu'à deux hétéroatomes supplémentaires choisis parmi un atome N, O et S ; chacun desdits noyau hétérocyclique de 4 à 8 chaînons et noyau hétéroaryle de 5 chaînons étant éventuellement et indépendamment substitué par jusqu'à 5 occurrences d'un atome de fluor ; et le groupe J^{A} est choisi parmi un atome d'hydrogène ou de fluor.

9. Composé selon l'une quelconque des revendications 1, 2, 4 et 6, ou sel pharmaceutiquement acceptable de celui-ci, chaque groupe J^{D} étant indépendamment choisi parmi un atome d'halogène, un groupe (C₁ à C₆)aliphatique, un groupe (C₁ à C₆)halogénoaliphatique, un groupe -N(R^{D})₂, -N(R^{d})C(O)R^{D}, -N(R^{d})C(O)OR^{D}, -N(R^{d})C(O)N(R^{D})₂, -SO₂R^{D}, -SO₂N(R^{D})₂, -N(R^{d})SO₂R^{D}, -SR^{D}', -OR^{D} ou un noyau (C₃ à C₈)cycloaliphatique éventuellement substitué, et o représentant un entier choisi entre 1 et 3.

10. Composé selon la revendication 9, ou sel pharmaceutiquement acceptable de celui-ci, chaque groupe J^{D} étant indépendamment choisi parmi un groupe méthyle, trifluorométhyle, chloro, fluoro, -N(R^{D})₂, N(R^{d})C(O)R^{D}, -N(R^{d})SO₂RD ou -OR^{D}.

11. Composé selon la revendication 1, ou sel pharmaceutiquement acceptable de celui-ci, représenté par l'une des **formules VIIa** ou **VIIIb** :

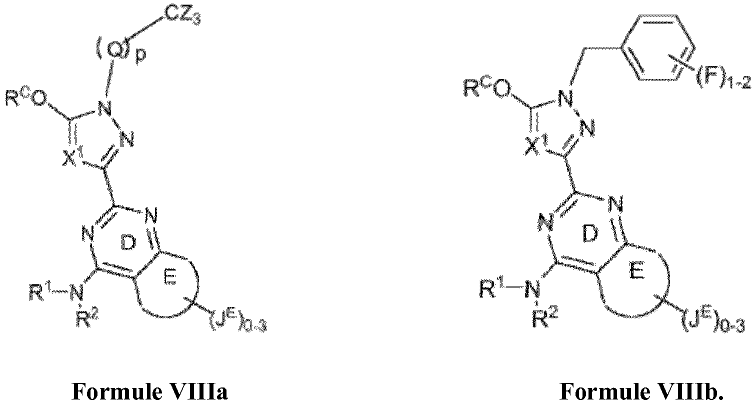

12. Composé selon l'une quelconque des revendications 1, 4, or 6, ou sel pharmaceutiquement acceptable de celui-ci, représenté par l'une des **Formules Xb ou XIb** : 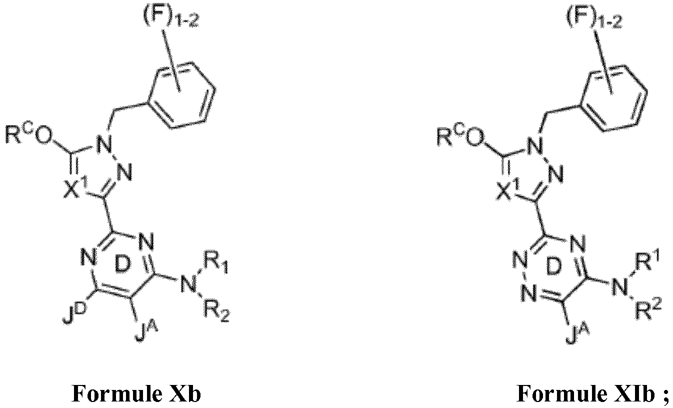 dans lesquelles chaque groupe J^{D} est indépendamment choisi parmi un groupe -NH₂ ou est absent ; et chaque groupe J^{A} représente alternativement :
i) lorsque les groupes R¹ et R² ne représentent pas simultanément un atome d'hydrogène, chaque groupe J^{A} est indépendamment choisi parmi un atome d'hydrogène ou d'halogène ; ou
ii) lorsque les groupes R¹ et R² représentent tous les deux simultanément un atome d'hydrogène, chaque groupe J^{A} est indépendamment choisi parmi un groupe -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂ ou -N(R^{d})SO₂R^{D}.

13. Composés selon l'une quelconque des revendications 1 ou 2, ou sel pharmaceutiquement acceptable de celui-ci, représenté par l'une des **formules XIXa ou Xa** : 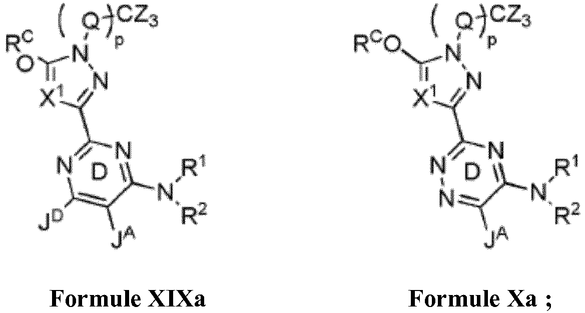 dans lesquelles
chaque groupe J^{D} est indépendamment choisi parmi un groupe -NH₂ ou est absent ; et chaque groupe J^{A} représente alternativement :
i) lorsque les groupes R¹ et R² ne représentent pas simultanément un atome d'hydrogène, chaque groupe J^{A} est indépendamment choisi parmi un atome d'hydrogène ou d'halogène ; ou
ii) lorsque les groupes R¹ et R² représentent tous les deux simultanément un atome d'hydrogène, chaque groupe J^{A} est indépendamment choisi parmi un groupe -C(O)R^{D}, -C(O)OR^{D}, -OC(O)R^{D}, -C(O)N(R^{D})₂, -N(R^{D})₂, -N(R^{d})C(O)R^{D}, -N(R^{d})C(O)OR^{D}, -N(R^{d})C(O)N(R^{D})₂, -OC(O)N(R^{D})₂, -SO₂R^{D}, -SO₂N(R^{D})₂ ou -N(R^{d})SO₂R^{D}

14. Composé selon la revendication 1, ledit composé étant choisi parmi l'un des composés suivants : 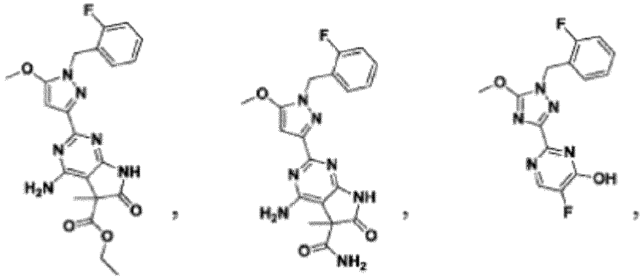 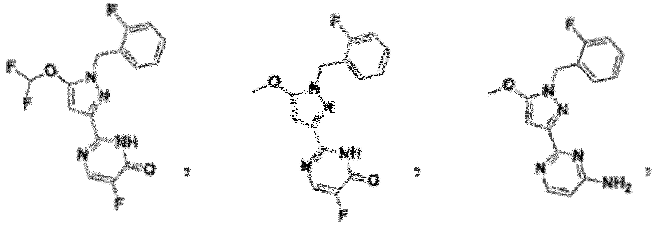 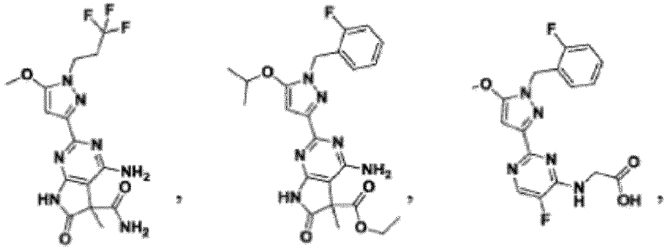 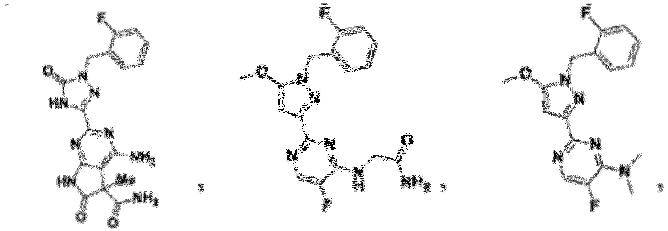 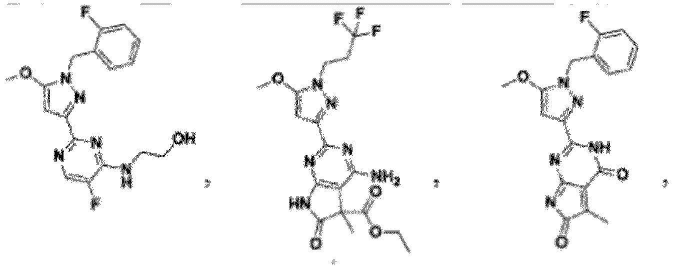 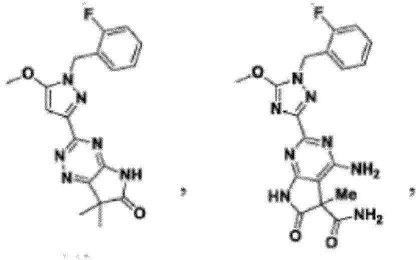 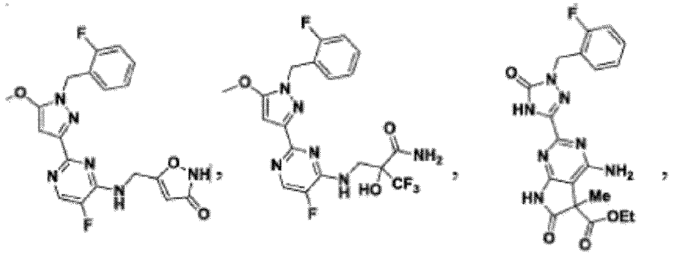 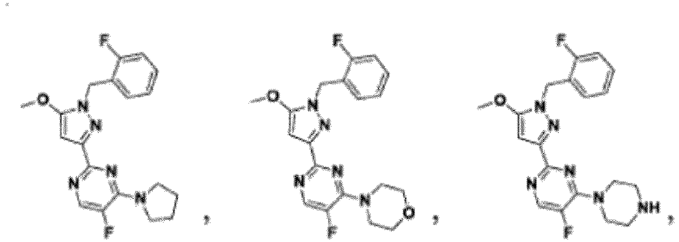 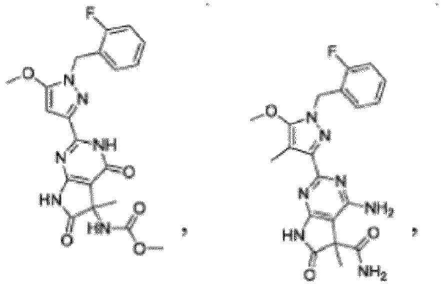 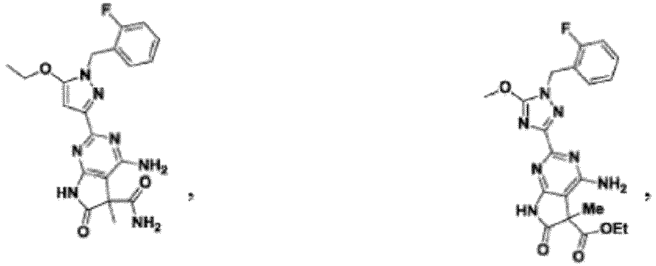 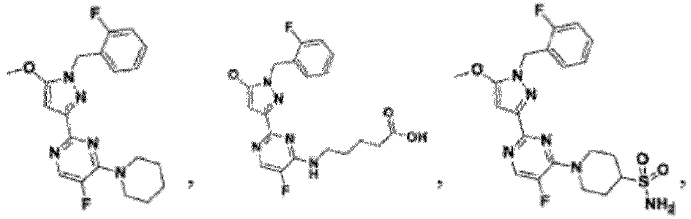 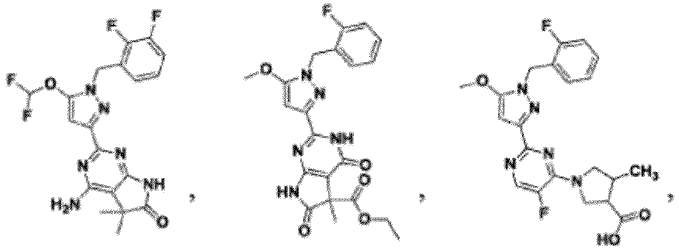 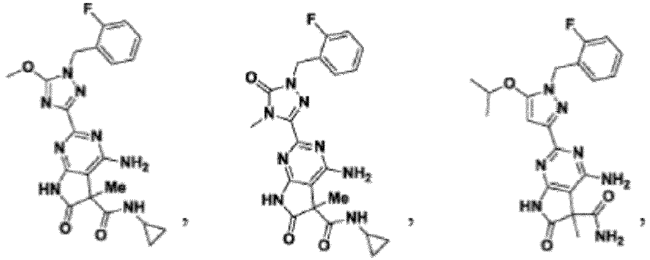 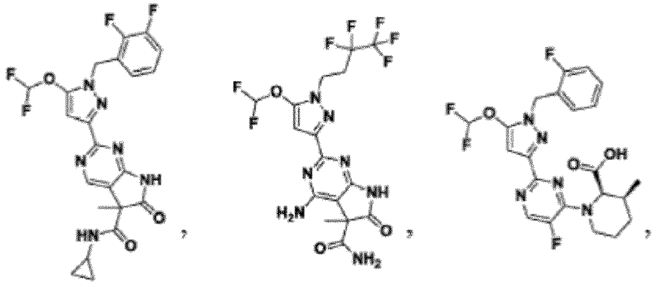 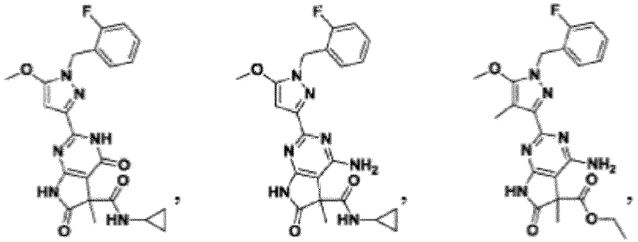 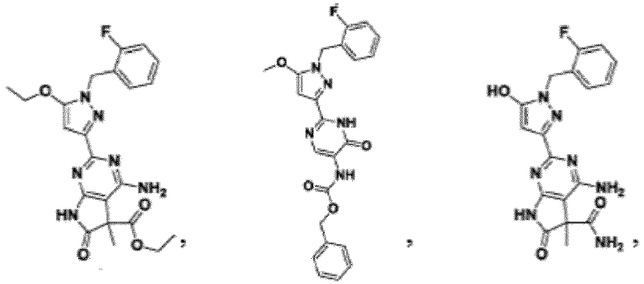 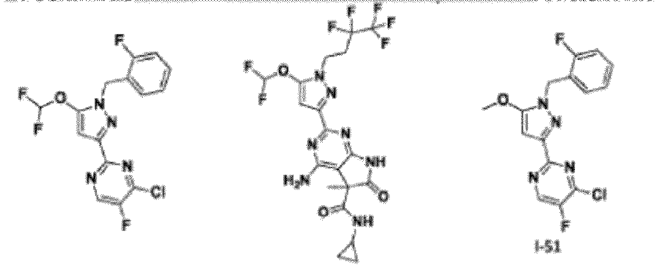 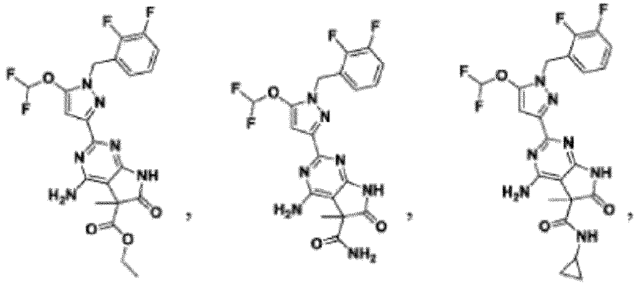 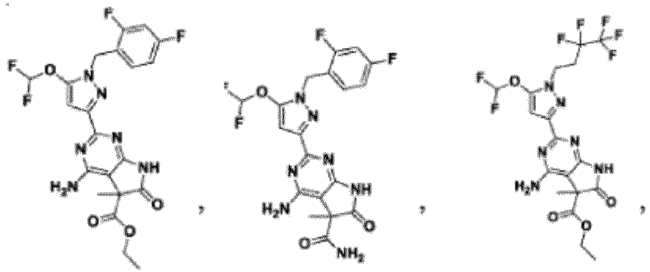 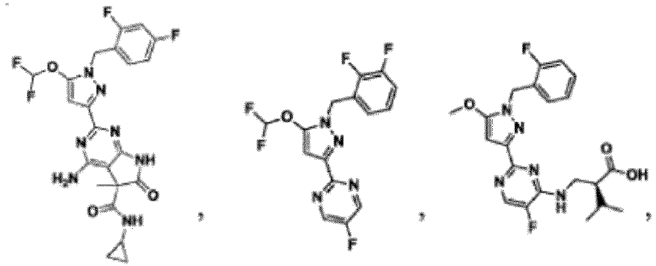 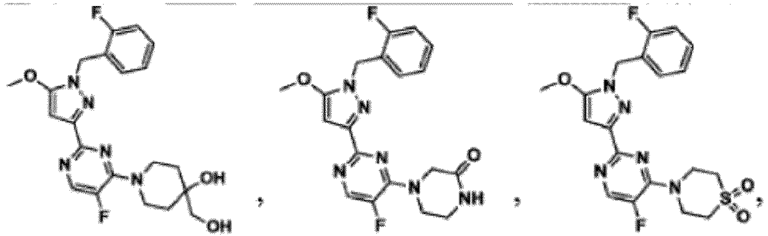 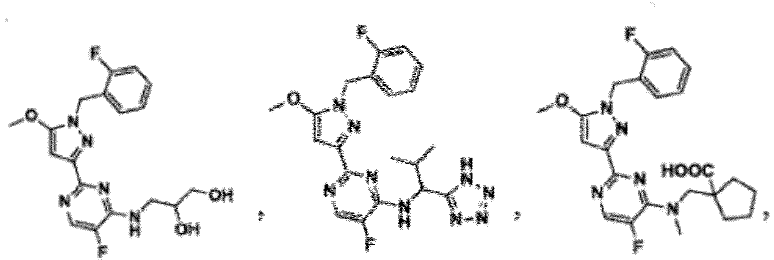 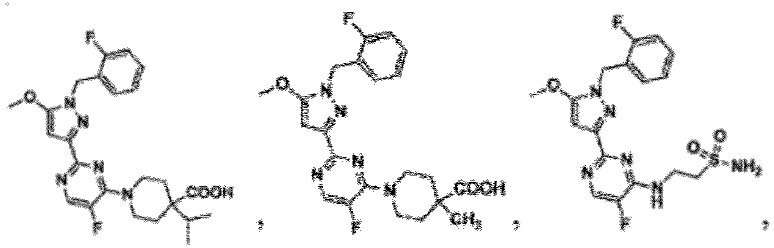 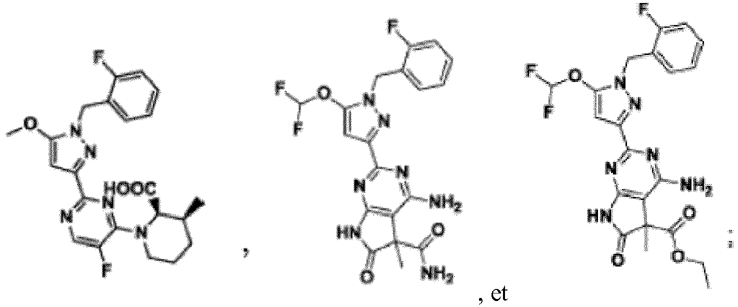 ou sel pharmaceutiquement acceptable de celui-ci.

15. Composé selon l'une quelconque des revendications 1 à 14 pour utilisation dans le traitement d'une maladie, d'un état de santé ou d'un trouble choisi parmi :
• les troubles associés à une pression artérielle élevée et un flux sanguin coronaire réduit ; une pression artérielle coronaire aiguë et chronique ; une hypertension artérielle et un trouble vasculaire résultant de complication cardiaques et rénales ; une hypertension artérielle et un trouble vasculaire résultant d'une attaque cardiaque, d'un accident vasculaire cérébral, d'une ischémie cérébrale, d'un insuffisance rénale ou d'une hypertension résistante ; d'une hypertension diabétique ; d'une hypertension essentielle ; d'une hypertension secondaire ;
• une insuffisance cardiaque ; une HFPEF (insuffisance cardiaque à fraction d'éjection préservée) ; une HFREF (insuffisance cardiaque à fraction d'éjection réduite) ; une insuffisance cardiaque chronique et aiguë, et des fromes plus spécifiques de la maladie ; une insuffisance cardiaque aiguë décompensée, une insuffisance du ventricule droit, une insuffisance du ventricule gauche, un insuffisance cardiaque globale, une cardiomyopathie ischémique, une cardiomyopathie dilatée, des malformations cardiaques congénitales, une insuffisance cardiaque avec vices valvulaires, une sténose de la valve mitrale, une insuffisance de la valve mitrale, une sténose de la valve aortique, une insuffisance de la valve aortique, une sténose tricuspidienne, une insuffisance tricuspidienne, une sténose de valve pulmonaire, une insuffisance de valve pulmonaire, des anomalies valvulaires combinées ; une insuffisance cardiaque diabétique ; une cardiomyopathie alcoolique, des cardiomyopathies de stockage ; une insuffisance cardiaque diastolique, une insuffisance cardiaque systolique, des phases aiguës d'insuffisance cardiaque chronique existante, l'aggravation d'une insuffisance cardiaque ; un dysfonctionnement diastolique ou systolique ; une insuffisance coronaire ; les arythmies ; une réduction de précharge ventriculaire ; une hypertrophie cardiaque ; une insuffisance cardiaque/le syndrome cardio-rénal ; l'hypertension portale ; un dysfonctionnement ou une lésion endothéliale ; des perturbations du rythme ventriculaire ou atrial et des perturbations de conduction ; des blocs auriculo-ventriculaires de degré I-III (AVB I-III) ; une tachyarythmie supraventriculaire, une fibrillation auriculaire, un flutter auriculaire, une fibrillation ventriculaire, un flutter ventriculaire, une tachyarythmie ventriculaire, une tachycardie à torsades de pointes, des extrasystoles auriculaire et ventriculaire, des extrasystoles de la jonction auriculo-ventriculaire, le syndrome du sinus malade, des syncopes, une tachycardie par réentrée dans le noeud auriculo-ventriculaire, le syndrome de Wolff-Parkinson-White, le syndrome coronaire aigu ; la cardiomyopathie de Boxer ; une contraction ventriculaire prématurée ;
• les troubles thromboemboliques et les ischémies, une ischémie du myocarde, un infarctus, une attaque cardiaque, une insuffisance du myocarde, un dysfonctionnement endothélial, un accident vasculaire cérébral, des attaques ischémiques transitoires (AIT) ; une thrombo-angéite obstructive ; une angine de poitrine stable ou instable ; des spasmes coronaires, des spasmes des artères périphériques ; une angine variable, une angine de Prinzmetal ; un accident vasculaire cérébral ; une hypertrophie cardiaque ; une prééclampsie ; des troubles thrombogènes ; des lésions d'ischémie-reperfusion ; une ischémie-reperfusion associée à une greffe d'organe ; une ischémie-reperfusion associée à une greffe de poumon, une greffe pulmonaire ou un greffe cardiaque ; la conservation de substituts sanguins chez des patients atteints de traumatisme ;
• une maladie artérielle périphérique ; une maladie artérielle occlusive périphérique ; une maladie vasculaire périphérique ; l'hypertonie ; le syndrome de Raynaud ou le phénomène de Raynaud ; le phénomène de Raynaud primaire ou secondaire ; la maladie de Raynaud, une ischémie critique des membres ; une embolie périphérique ; une claudication intermittente ; une crise vaso-occlusive ; les dystrophies musculaires de Duchenne et Becker ; des anomalies de la microcirculation ; le contrôle des fuites et de la perméabilité vasculaires ; une sténose du canal rachidien lombaire ; une vascularité thrombotique occlusive ; une vascularité thrombotique ; des perturbations de perfusion périphérique ; des thromboses artérielles et veineuses ; une microalbuminurie ; des neuropathies périphériques et autonomes ; les microangiopathies diabétiques ;
• un oedème ; un oedème rénal dû à une insuffisance cardiaque ;
• la maladie d'Alzheimer, la maladie de Parkinson ; des démences vasculaires ; un trouble cognitif vasculaire ; un vasospasme cérébral ; une lésion cérébrale traumatique ; une amélioration de la perception, de la capacité de concentration, de la capacité d'apprendre ou des performances de la mémoire après des perturbations cognitives telles que celles qui se produisent dans un trouble cognitif léger, des perturbations de la mémoire et d'apprentissage associées à l'âge, une perte de mémoire associée à l'âge, une démence vasculaire, une blessure à la tête, un accident vasculaire cérébral, une démence après accident vasculaire cérébral, une blessure à la tête post-traumatique, des perturbations générales de la concentration et des perturbations de la concentration chez les enfants avec des problèmes de mémoire et d'apprentissage ; la démence à corps de Lewy, la démence avec dégénérescence du lobe frontal dont le syndrome de Pick ; une paralysie nucléaire progressive ; une démence avec dégénérescence corticobasale ; la sclérose latérale amyotrophique (SLA) ; la maladie de Huntington ; la démyélinisation, la sclérose en plaques, une dégénérescence thalamique ; la maladie de Creutzfeldt-Jakob, la démence associée au VIH, la schizophrénie avec démence ou psychose de Korsakoff ; une atrophie multisystémisée et d'autres formes de parkinsonisme plus ; des troubles moteurs ; une neuroprotection ; l'anxiété, la tension et la dépression, un trouble de stress post-traumatique (TPST) ; un dysfonctionnement sexuel et des troubles du sommeil associés au SNC ; des troubles de l'alimentation pathologiques et l'utilisation d'aliments de luxe et de médicaments addictifs ; le contrôle de la perfusion cérébrale, le contrôle des migraines ; la prophylaxie et le contrôle des conséquences d'un infarctus cérébral (apoplexie cérébrale) tel qu'un accident vasculaire cérébral, des ischémies cérébrales et une blessure à la tête ;
• un choc ; un choc cardiogénique ; une septicémie ou un choc septique ou un choc anaphylactique ; un anévrisme ; le contrôle de l'activation des leucocytes ; l'inhibition ou la modulation de l'agrégation des plaquettes ; le syndrome de défaillance multiviscérale (SDMV ou DMV) ;
• des états pulmonaires/respiratoires ; l'hypertension pulmonaire (HP), l'hypertension artérielle pulmonaire (HAP), et une remodélisation vasculaire des poumons associée ; une thrombose localisée et une hypertrophie cardiaque à gauche ; une hypertonie pulmonaire ; une hypertension pulmonaire primaire, un hypertension pulmonaire secondaire, une hypertension pulmonaire familiale, une hypertension pulmonaire sporadique, une hypertension pulmonaire pré-capillaire, une hypertension pulmonaire idiopathique ; une artériopathie pulmonaire thrombotique, une artériopathie pulmonaire plexogénique ; une fibrose kystique ; une bronchoconstriction ou une bronchoconstriction pulmonaire ; le syndrome de détresse respiratoire aiguë ; la fibrose pulmonaire, une greffe de poumon ; des maladies asthmatiques ; d'autres formes de HP ; une HP associée à une maladie du ventricule gauche, au VIH, la MSC, une thrombo-embolie (hypertension pulmonaire post-embolique ou CTEPH), la sarcoïdose, la BPCO ou la fibrose pulmonaire ; le syndrome de détresse respiratoire aiguë (SDRA), une lésion pulmonaire aiguë, le déficit d'alpha-1-antitrypsine (DAAT), l'emphysème pulmonaire ; l'emphysème induit par la fumée de cigarette et la mucoviscidose (fibrose kystique ou FK) ;
• l'hypertension pulmonaire associée à ou liée à : un dysfonctionnement du ventricule gauche, l'hypoxémie, les hypertensions des groupes I, II, III, IV et V de l'OMS, une maladie de la valve mitrale, une péricardite constrictive, une sténose aortique, une cardiomyopathie, une fibrose médiastinale, une fibrose pulmonaire, une drainage veineux pulmonaire anormal, une maladie pulmonaire veino-occlusive, une vascularité pulmonaire, une collagénose vasculaire, une maladie cardiaque congénitale, une hypertension veineuse pulmonaire, une pneumopathie interstitielle, un trouble respiratoire du sommeil, l'apnée du sommeil, les troubles d'hypoventilation alvéolaire, une exposition chronique à la haute altitude, une maladie pulmonaire néonatale, une dysplasie alvéolo-capillaire, une anémie à cellules falciformes, d'autres troubles de la coagulation, une thrombo-embolie chronique, une embolie pulmonaire, une embolie due à une tumeur, des parasites ou une matière étrangère ; l'hypertension pulmonaire associée ou liée à : une maladie du tissu conjonctif, le lupus, la schistosomiase, la sarcoïdose, la bronchopneumopathie chronique obstructive, l'asthme, l'emphysème, la bronchite chronique ou l'hémangiomatose capillaire pulmonaire ; l'histiocytose X, la lymphangiomatose et la compression de vaisseaux pulmonaires; la compression de vaisseaux due à une adénopathie, une tumeur ou une médiastinite fibreuse ;
• les maladies ou états artérosclérotiques ; l'athérosclérose, l'athérosclérose associée à une lésion endothéliale, à l'adhérence et l'agrégation des plaquettes et des monocytes, à la prolifération et la migration des muscles lisses ; la resténose ; la resténose développée après des thérapies par thrombolyse, les angioplasties transluminales percutanées (ATP), les angioplasties coronaires transluminales (ACTP), une greffe de coeur ou des opérations de pontage ; des processus inflammatoires ;
• des dommages micro- et macro-vasculaire ; une vascularité ; des taux accrus de fibrinogène et une faible densité de DLD, concentration accrue de l'inhibiteur 1 de l'activateur du plasminogène (PA-1) ;
• des maladies associées à un syndrome métabolique : l'obésité, la dyslipidémie, le diabète, une pression artérielle élevée ; des troubles associés aux lipides : la dyslipidémie, les hypercholestérolémies, une baisse du taux de cholestérol lié aux lipoprotéines haute densité (HDL-cholestérol) et dans certains cas une hausse modérée du taux de cholestérol lié aux lipoprotéines faible densité (LDL-cholestérol), les hypertriglycéridémies, l'hyperglycéridémie, les lipoprotéinanémies, la sitostérolémie, la stéatose hépatique et l'hépatite ; le prééclampsie ; la progression de la polykystose rénale ; les graisses sous-cutanées ; l'obésité ; la stéatose hépatique ou une accumulation anormale des lipides dans le foie ; la stéatose du coeur, des reins ou des muscles ; l'abêtalipoprotéinémie ; la sitostérolémie ; la xanthomatose ; la maladie de Tangier ; l'adiposité ; les hyperlipidémies combinées et un syndrome métabolique ; l'hyperammonémie et les maladies et troubles associés ; les encéphalopathies hépatiques et d'autres encéphalopathies toxiques et le syndrome de Reye ;
• les troubles ou états sexuels, gynécologiques et urologiques ; un dysfonctionnement érectile ; l'impotence ; l'éjaculation précoce ; un dysfonctionnement sexuel chez la femme ; un dysfonctionnement de l'excitation sexuelle chez la femme, un trouble d'excitation sexuelle hypoactive, une atrophie vaginale, la dyspareunie, une vaginite atrophique ; un élargissement ou une hypertrophie ou une hyperplasie bénigne de la prostate (HBP) ; une obstruction de la sortie de la vessie ; le syndrome de la vessie douloureuse (SVD) ; la cystite interstitielle (CI) ; la vessie hyperactive ; la vessie neurogène et l'incontinence ; la néphropathie diabétique ; la dysménorrhée primaire et secondaire ; les affections du bas appareil urinaire (ABAU) ; les douleurs pelviennes ; les maladies bénignes et malignes des organes de l'appareil urogénital masculin et féminin ;
• l'insuffisance rénale aiguë et chronique, la défaillance rénale aiguë et chronique, ainsi que les maladies rénales sous-jacentes ou associées comme l'hypoperfusion, l'hypotension intradialyse, l'uropathie obstructive, les glomérulopathies, la glomérulonéphrite, la glomérulonéphrite aiguë, la glomérulosclérose, les maladies tubulo-interstitielles, les maladies néphropathiques ; les maladies rénales primaires et congénitales ; la néphrite ; les maladies **caractérisées par** un taux de créatinine et/ou une excrétion d'eau anormalement réduits, des concentrations sanguines anormalement accrues d'urée, d'azote, de potassium et/ou de créatinine, une activité modifiée des enzymes rénales, une osmolarité urinaire ou un volume d'urine modifié, une microalbuminurie ou une macroalbuminuria accrues, des lésions de glomérules et d'artérioles, une dilatation tubulaire, une hyperphosphatémie et/ou un besoin de dialyse ; les séquelles d'une insuffisance rénale ; un lavement pulmonaire, une insuffisance cardiaque, une urémie, une anémie, des perturbations d'électrolyte ; l'hyperkaliémie, l'hyponatrémie ; les perturbations du métabolisme osseux et glucidique ;
• les troubles ou maladies oculaires ; un glaucome, une rétinopathie ou une rétinopathie diabétique.
